(19) Europäisches Patentamt
European Patent Office
Office européen des brevets

(11) EP 0 672 666 B1

(12) **EUROPÄISCHE PATENTSCHRIFT**

(45) Veröffentlichungstag und Bekanntmachung des Hinweises auf die Patenterteilung:
**25.07.2001 Patentblatt 2001/30**

(21) Anmeldenummer: **95103028.7**

(22) Anmeldetag: **03.03.1995**

(51) Int Cl.[7]: **C07D 487/04**, C07D 495/14, A61K 31/55
// (C07D487/04, 243:00, 235:00), (C07D495/14, 333:00, 243:00, 235:00)

# (54) Imidazodiazepine

Imidazodiazepins

Imidazodiazépines

(84) Benannte Vertragsstaaten:
**AT BE CH DE DK ES FR GB GR IE IT LI LU MC NL PT SE**
Benannte Erstreckungsstaaten:
**LT SI**

(30) Priorität: **16.03.1994 CH 78394**
**03.01.1995 CH 1095**

(43) Veröffentlichungstag der Anmeldung:
**20.09.1995 Patentblatt 1995/38**

(73) Patentinhaber: **F. Hoffmann-La Roche AG**
**4002 Basel (CH)**

(72) Erfinder:
- **Godel, Thierry**
  **CH-4056 Basel (CH)**
- **Hunkeler, Walter**
  **CH-4312 Magden (CH)**
- **Stadler, Heinz**
  **CH-4310 Rheinfelden (CH)**
- **Widmer, Ulrich**
  **CH-4310 Rheinfelden (CH)**

(74) Vertreter: **Vergani, Diego, Dr. et al**
**F. Hoffmann-La Roche AG**
**Patent Department (PLP)**
**Grenzacherstrasse 124**
**4070 Basel (CH)**

(56) Entgegenhaltungen:
**EP-A- 0 109 921**
**EP-A- 0 150 040**
**EP-A- 0 197 282**
**US-A- 4 904 654**
**US-A- 4 939 139**

- **JOURNAL OF MEDICINAL CHEMISTRY, Bd. 32, Nr. 10, Oktober 1989 Seiten 2282-2291, F. WATJEN ET AL. 'Novel Benzodiazepine Receptor Partial Agonists: Oxadiazolylimidazobenzodiazepines'**
- **RESEARCH COMMUNICATIONS IN CHEMICAL PATHOLOGY AND PHARMACOLOGY, Bd. 80, Nr. 3, Juni 1993 Seiten 357-362, R.C. SHARMA ET AL. 'Oxadiazolylimidazobenzodiazepines as novel partial agonists with the benzodiazepine receptor: A QSAR analysis'**
- **JOURNAL OF MEDICINAL CHEMISTRY, Bd. 36, 1993 Seiten 479-490, S. ANANTHAN ET AL. 'Synthesis and Structure-Activity Relationships of 3,5-Disubstituted 4,5-Dihydro-6H...'**
- **ADVANCES IN BIOCHEMICAL PSYCHOPHARMACOLOGY, Bd. 45, 1988 Seiten 209-217, L.H. JENSEN ET AL. 'Oxadiazolylimidazobenzodiazepines, a new class of benzodiazepine receptor ligands'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 672 666 B1

Printed by Jouve, 75001 PARIS (FR)

## Beschreibung

Die vorliegende Erfindung betrifft Imidazodiazepine der allgemeinen Formel

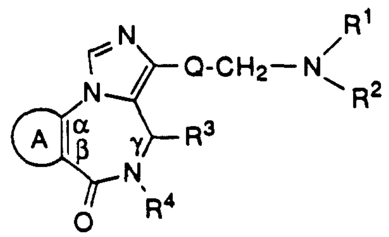

I

worin A zusammen mit den beiden mit α und β bezeichneten Kohlenstoffatomen einen der Reste

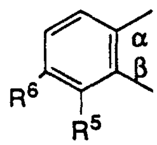

,

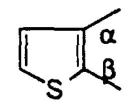

und

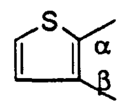

(A[1]) (A[2]) (A[3])

Q einen der Reste

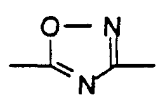

,

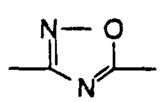

und

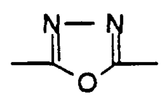

(Q[1]) (Q[2]) (Q[3])

$R^1$ und $R^2$ je Wasserstoff, niederes Alkyl, niederes Alkenyl, niederes Alkinyl, niederes Hydroxyalkyl, niederes Alkoxy-niederes Alkyl, ($C_3$-$C_6$)-Cycloalkyl, ($C_3$-$C_6$)-Cycloalkyl-niederes Alkyl, Amino-niederes Alkyl, niederes Alkylamino-niederes Alkyl, Di-niederes Alkylamino-niederes Alkyl oder Aryl-niederes Alkyl oder zusammen mit dem Stickstoffatom einen 5- bis 8-gliedrigen, gegebenenfalls ein weiteres Heteroatom oder einen ankondensierten Benzolring enthaltenden Heterocyclus,

$R^3$ Wasserstoff und $R^4$ niederes Alkyl oder $R^3$ und $R^4$ zusammen eine Di-oder Trimethylengruppe und

$R^5$ und $R^6$ je Wasserstoff, Halogen, Trifluormethyl, niederes Alkoxy oder Nitro bedeuten,

wobei das mit γ bezeichnete Kohlenstoffatom die S-Konfiguration aufweist, wenn $R^3$ von Wasserstoff verschieden ist,
und pharmazeutisch annehmbare Säureadditionssalze davon.

Diese Verbindungen und Salze sind neu und besitzen wertvolle pharmakodynamische Eigenschaften. Sie eignen sich daher für therapeutische Zwecke, insbesondere für anxiolytische und/oder antikonvulsive und/oder muskelrelaxierende und/oder sedativ-hypnotische Zwecke.

Die Europäische Patentanmeldung EP-A-0197 282 beschreibt Oxadiazolylimidazpbenzodiazepin-Derivate, die als antikonvulsive, anxiolytische, hypnotische und nootropische Wirkstoffe angewendet werden können. Diese Verbindungen weisen aber eine schlechte Löslichkeit im Wasser auf, und sind daher besonders ungünstig für deren parenterale Verabreichung.

Gegenstand der vorliegenden Erfindung sind die erwähnten Verbindungen der Formel I und Salze davon als solche und als therapeutische Wirkstoffe, deren Herstellung und deren Verwendung für therapeutische Zwecke bzw. zur Herstellung entsprechender Arzneimittel sowie Arzneimittel, enthaltend eine Verbindung der Formel I oder ein Salz

davon, und die Herstellung solcher Arzneimittel.

Der Ausdruck "nieder" bezeichnet Reste oder Verbindungen mit höchstens 7, vorzugsweise höchstens 4 Kohlenstoffatomen. Der Ausdruck "Alkyl" bezeichnet geradekettige oder verzweigte gesättigte Kohlenwasserstoffreste, wie Methyl, Aethyl, n-Propyl, iso-Propyl, n-Butyl, sek.-Butyl, iso-Butyl und tert.-Butyl. Der Ausdruck "Alkoxy" bezeichnet über ein Sauerstoffatom gebundene Alkylgruppen, wie Methoxy und Aethoxy. Der Ausdruck "Cycloalkyl" bezeichnet gesättigte zyklische Kohlenwasser-stoffreste, wie z.B. Cyclopropyl. Die Ausdrücke "Alkenyl" und "Alkinyl" bezeichnen geradekettige oder verzweigte Kohlenwasserstoffreste, welche eine C-C-Doppel- bzw. Dreifachbindung enthalten, wie Allyl, But-2-enyl, 3-Methyl-but-2-enyl, Propargyl u. dgl. Der Ausdruck "Aryl" bezeichnet einen gegebenenfalls durch Halogen, Trifluormethyl, niederes Alkyl oder niederes Alkoxy substituierten Phenylrest. Der Ausdruck "Halogen" umfasst Fluor, Chlor, Brom und Jod. Wenn $R^1$ und $R^2$ zusammen mit dem Stickstoffatom einen Heterocyclus bedeuten, so handelt es sich um Reste, wie 1-Pyrrolidinyl, 1-Pyrrolinyl, Piperidino, 2,6-Dimethylpiperidino, 3,3-Dimethylpiperidino, Hexamethylenimin-1-yl, Heptamethylenimin-1-yl, Morpholino, 4-Methyl-1-piperazinyl, Isoindolin-2-yl u. dgl.

Vorzugsweise bedeutet Q in Formel I einen Rest der Formel $Q^2$ oder der Formel $Q^3$. $R^1$ und $R^2$ bedeuten vorzugsweise je niederes Alkyl, niederes Hydroxyalkyl oder ($C_3$-$C_6$)-Cycloalkyl-niederes Alkyl oder zusammen mit dem Stickstoffatom Piperidino oder Isoindolin-2-yl. A bedeutet vorzugsweise einen Rest der Formel $A^1$, worin $R^5$ Wasserstoff, Chlor, Fluor, Trifluormethyl oder niederes Alkoxy und $R^6$ Wasserstoff oder Fluor bedeuten, oder einen Rest der Formel $A^2$. Schliesslich bedeuten vorzugsweise $R^3$ Wasserstoff und $R^4$ Methyl oder $R^3$ und $R^4$ zusammen Dimethylen.

Besonders bevorzugte Verbindungen der Formel I sind:

3-(5-Dipropylaminomethyl-1,2,4-oxadiazol-3-yl)-8-fluoro-5-methyl-5,6-dihydro-4H-imidazo[1,5-a][1,4]benzodiazepin-6-on;

(S)-8-Chlor-1-(5-dipropylaminomethyl-1,2,4-oxadiazol-3-yl)-12,12a-dihydro-9H,11H-azeto[2,1-c]imidazo[1,5-a][1,4]benzodiazepin-9-on;

(S)-1-(5-Dipropylaminomethyl-1,2,4-oxadiazol-3-yl)-11,11a-dihydro-8H,10H-azeto[1,2-a]imidazo[5,1-c]thieno[3,2-e][1,4]diazepin-8-on;

8-Fluor-5-methyl-3-[5-(piperidin-1-ylmethyl)-1,2,4-oxadiazol-3-yl]-5,6-dihydro-4H-imidazo[1,5-a][1,4]benzodiazepin-6-on;

(S)-1-(5-Diallylaminomethyl-1,2,4-oxadiazol-3-yl)-8-chlor-7-fluor-12,12a-dihydro-9H,11H-azeto[2,1-c]imidazo[1,5-a][1,4]benzodiazepin-9-on;

3-(5-Dipropylaminomethyl-1,2,4-oxadiazol-3-yl)-5-methyl-5,6-dihydro-4H-imidazo[1,5-a][1,4]benzodiazepin-6-on;

3-(5-Dipropylaminomethyl-1,3,4-oxadiazol-2-yl)-8-fluoro-5-methyl-5,6-dihydro-4H-imidazo[1,5-c][1,4]benzodiazepin-6-on;

3-(5-Diethylamiomethyl-1,2,4-oxadiazol-3-yl)-8-fluoro-5-methyl-5,6-dihydro-4H-imidazo[1,5-a][1,4]benzodiazepin-6-on;

(S)-8-Chlor-1-[5-(piperidin-1-yl)methyl-1,2,4-oxadiazol-3-yl]-12,12a-dihydro-9H,11H-azeto[2,1-c]imidazo[1,5-a][1,4]benzodiazepin-9-on;

7-Fluor-5-methyl-3-(5-dipropylaminomethyl-1,2,4-oxadiazol-3-yl)-5,6-dihydro-4H-imidazo[1,5-a][1,4]benzodiazepin-6-on;

7-Chlor-3-(5-diäthylaminomethyl-1,2,4-oxadiazol-3-yl)-5-methyl-5,6-dihydro-4H-imidazo[1,5-a][1,4]benzodiazepin-6-on;

7-Chlor-3-(5-dipropylaminomethyl-1,3,4-oxadiazo-2-yl)-5-methyl-5,6-dihydro-4H-imidazo[1,5-a][1,4]benzodiazepin-6-on;

(S)-1-(5-Diäthylaminomethyl-1,2,4-oxadiazol-3-yl)-11,11a-dihydro-8H,10H-azeto[1,2-a]imidazo[5,1-c]thieno[3,2-e][1,4]diazepin-8-on;

(S)-1-(5-Dibutylaminomethyl-1,3,4-oxadiazol-2-yl)-11,11a-dihydro-8H,10H-azeto[1,2-a]imidazo[5,1-c]thieno [3,2-e][1,4]diazepin-8-on;

3-(5-Diisopropylaminomethyl-1,2,4-oxadiazol-3-yl)-5-methyl-5,6-dihydro-4H-imidazo[1,5-a][1,4]benzodiazepin-6-on; und

3-(5-Diisopropylaminomethyl-1,2,4-oxadiazol-3-yl)-8-fluor-5-methyl-5,6-dihydro-4H-imidazo[1,5-a][1,4]benzodiazepin-6-on.

Weitere Beispiele für im Rahmen der vorliegenden Erfindung bevorzugte Verbindungen sind

(S)-1-(5-Diallylaminomethyl-1,2,4-oxadiazol-3-yl)-8-chlor-12,12a-dihydro-9H,11H-azeto[2,1-c]imidazo[1,5-a][1,4] benzodiazepin-9-on;

(S)-1-(5-Diallylaminomethyl-1,2,4-oxadiazol-3-yl)-8-trifluormethyl-12,12a-dihydro-9H,11H-azeto[2,1-c]imidazo [1,5-a][1,4]benzodiazepin-9-on;

3-(5-Diallylaminomethyl-1,2,4-oxadiazol-3-yl)-8-fluor-5-methyl-5,6-dihydro-4H-imidazo[1,5-a][1,4]benzodiazepin-6-on;

8-Fluor-3-(5-isoindolin-2-ylmethyl-1,2,4-oxadiazol-3-yl)-5-methyl-5,6-dihydro-4H-imidazo[1,5-a][1,4]benzodiazepin-6-on;

(S)-1-(5-Diallylaminomethyl-1,2,4-oxadiazol-3-yl)-11,11a-dihydro-8H,10H-azeto[1,2-a]imidazo[5,1-c]thieno [3,2-e][1,4]diazepin-8-on;

3-(5-Dipropylaminomethyl-1,2,4-oxadiazol-3-yl)-5-methyl-5,6-dihydro-4H-imidazo[1,5-a]trieno[2,3-f][1,4]diazepin-6-on;

3-(5-Dipropylaminomethyl-1,2,4-oxadiazol-3-yl)-5-methyl-7-trifluormethyl-5,6-dihydro-4H-imidazo[1,5-a][1,4]benzodiazepin-6-on; und

(S)-8-Chlor-1-(5-dipropylaminomethyl-1,3,4-oxadiazol-2-yl)-12,12a-dihydro-9H,11H-azeto[2,1-c]imidazo[1,5-a] [1,4]benzodiazepin-9-on.

Die eingangs erwähnte Verbindungen der Formel I und deren pharmazeutisch annehmbaren Säureadditionssalze können erfindungsgemäss dadurch hergestellt werden, dass man

a) ein reaktionsfähiges funktionelles Derivat einer Carbonsäure der allgemeinen Formel

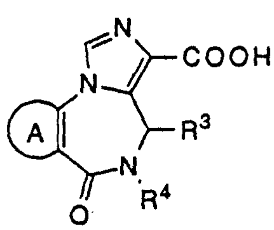

II oder

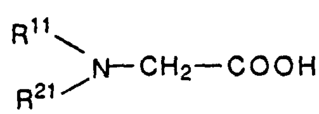

III

mit einem Amidoxim der allgemeinen Formel

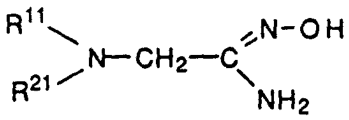 IV bzw. 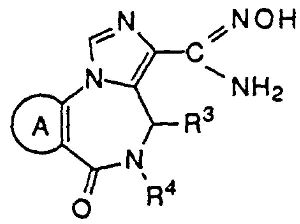 V

oder mit einem Hydrazid der allgemeinen Formel

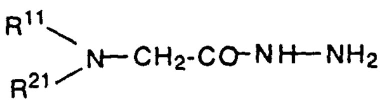 VI bzw. 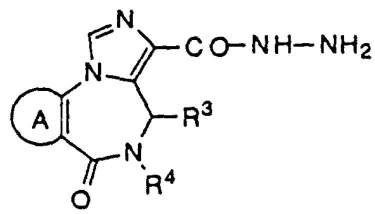 VII

wobei A, $R^3$ und $R^4$ obige Bedeutung besitzen und $R^{11}$ und $R^{21}$ je niederes Alkyl, niederes Alkenyl, niederes Alkinyl, niederes Alkoxy-niederes Alkyl, ($C_3$-$C_6$)-Cycloalkyl, ($C_3$-$C_6$)-Cycloalkylniederes Alkyl, Di-niederes Alkylamino-niederes Alkyl oder Aryl-niederes Alkyl oder zusammen mit dem Stickstoffatom einen 5- bis 8-gliedrigen, gegebenenfalls ein weiteres Heteroatom oder einen ankondensierten Benzolring enthaltenden Heterocyclus bedeuten,
umsetzt, oder

b) eine Verbindung der allgemeinen Formel

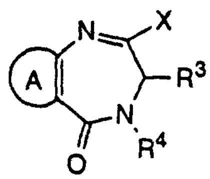 VIII

worin A, $R^3$ und $R^4$ obige Bedeutung besitzen und X eine Abgangsgruppe bedeutet,
in Gegenwart einer Base mit einem Isonitril der allgemeinen Formel

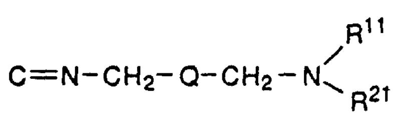 IX

worin Q, $R^{11}$ und $R^{21}$ obige Bedeutung besitzen, umsetzt, oder

c) eine Verbindung der allgemeinen Formel

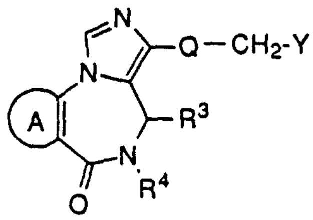

X

worin A, Q, $R^3$ und $R^4$ obige Bedeutung besitzen und Y eine Abgangsgruppe bedeutet, mit einem Amin der allgemeinen Formel

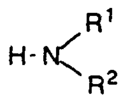

XI

worin $R^1$ und $R^2$ obige Bedeutung besitzen, umsetzt, oder

d) aus einer Verbindung der allgemeinen Formel

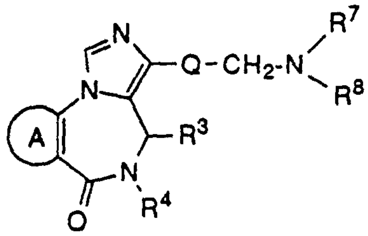

XII

worin A, Q, $R^3$ und $R^4$ obige Bedeutung, besitzen und $R^7$ eine Schutzgruppe, geschütztes niederes Hydroxyalkyl, geschütztes Amino-niederes Alkyl oder geschütztes niederes Alkylamino-niederes Alkyl und $R^8$ Wasserstoff, niederes Alkyl, niederes Alkenyl, niederes Alkinyl, geschütztes niederes Hydroxyalkyl, niederes Alkoxy-niederes Alkyl, ($C_3$-$C_6$)-Cycloalkyl, ($C_3$-$C_6$)-Cycloalkyl-niederes Alkyl, geschütztes Amino-niederes Alkyl, geschütztes niederes Alkylamino-niederes Alkyl, Di-niederes Alkylamino-niederes Alkyl oder Aryl-niederes Alkyl oder $R^7$ und $R^8$ zusammen eine Schutzgruppe bedeuten,
die Schutzgruppe (n) abspaltet, oder

e) eine Verbindung der allgemeinen Formel

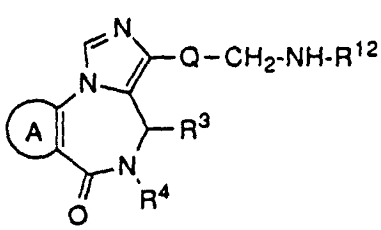

Ia

worin A, Q, $R^3$ und $R^4$ obige Bedeutung besitzen und $R^{12}$ Wasserstoff, niederes Alkyl, niederes Alkenyl, niederes Alkinyl, niederes Hydroxyalkyl, niederes Alkoxy-niederes Alkyl, ($C_3$-$C_6$)-Cycloalkyl, ($C_3$-$C_6$)-Cycloalkyl-niederes Alkyl, Amino-niederes Alkyl, niederes Alkylamino-niederes Alkyl, Di-niederes Alkylamino-niederes Alkyl oder Aryl-niederes Alkyl bedeutet,
entsprechend N-alkyliert, oder

f) eine Verbindung der allgemeinen Formel

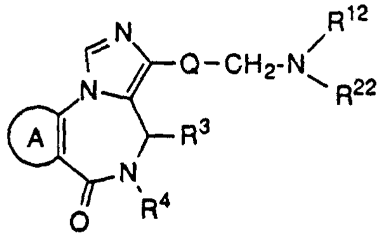

Ib

worin A, Q, $R^3$ und $R^4$ und $R^{12}$ obige Bedeutung besitzen und $R^{22}$ niederes Alkenyl oder niederes Alkinyl bedeutet, reduziert und erwünschtenfalls

g) eine Verbindung der allgemeinen Formel I in ein pharmazeutisch anwendbares Säureadditionssalz überführt.

Gemäss Verfahrensvariante a) werden Verbindungen der Formel I, worin $R^1$ und $R^2$ je niederes Alkyl, niederes Alkenyl, niederes Alkinyl, niederes Alkoxy-niederes Alkyl, ($C_3$-$C_6$)-Cycloalkyl, ($C_3$-$C_6$)-Cycloalkylniederes Alkyl, Di-niederes Alkylamino-niederes Alkyl oder Aryl-niederes Alkyl oder zusammen mit dem Stickstoffatom einen 5- bis 8-gliedrigen, gegebenenfalls ein weiteres Heteroatom oder einen ankondensierten Benzolring enthaltenden Heterocyclus bedeuten, durch Aufbau des Oxadiazolrests Q hergestellt. Dabei entsteht ausgehend von Verbindungen der Formeln II und IV der Rest $Q^1$, von Verbindungen der Formeln III und V der Rest $Q^2$ und von Verbindungen der Formel II und VI oder III und VII der Rest $Q^3$.

Als reaktionsfähige funktionelle Derivate der Carbonsäuren der Formeln II und III verwendet man zweckmässigerweise die entsprechenden Imidazolide, welche nach an sich bekannten Methoden aus den fraglichen freien Carbonsäuren hergestellt werden können, z.B. durch Umsetzung mit 1,1'-Carbonyldiimidazol in einem inerten organischen Lösungsmittel, wie N,N-Dimethylformamid.

Als reaktionsfähige funktionelle Derivate können aber beispielsweise auch Carbonsäurechloride verwendet werden, welche sich aus den entsprechenden freien Carbonsäuren mittels Thionylchlorid herstellen lassen.

Die Umsetzung eines reaktionsfähigen funktionellen Derivats einer Carbonsäure der Formel II oder III mit einem Amidoxim der Formel IV bzw. V erfolgt zweckmässigerweise durch mehrstündiges Erhitzen auf etwa 70 bis 130°C in einem inerten Lösungsmittel, wie N,N-Dimethylformamid. Das intermediär entstehende nicht-cyclisierte Kondensationsprodukt wird zweckmässigerweise nicht gefasst, sondern cyclisiert unter den vorliegenden Reaktionsbedingungen spontan.

Die Umsetzung eines reaktionsfähigen funktionellen Derivats einer Carbonsäure der Formel II oder III mit einem Hydrazid der Formel VI bzw. VII erfolgt zweckmässigerweise bei Raumtemperatur in einem inerten organischen Lösungsmittel, wie N,N-Dimethylformamid. Das dabei entstehende nicht-cyclisierte Kondensationsprodukt kann gefasst und hierauf cyclisiert werden, zweckmässigerweise durch 1- bis mehrstündiges Erhitzen mit Polyphosphorsäure auf ca. 100°C.

Auch gemäss Verfahrensvariante b) erhält man Verbindungen der Formel I, worin $R^1$ und $R^2$ je niederes Alkyl, niederes Alkenyl, niederes Alkinyl, niederes Alkoxy-niederes Alkyl, ($C_3$-$C_6$)-Cycloalkyl, ($C_3$-$C_6$)-Cycloalkyl-niederes Alkyl, Di-niederes Alkylamino-niederes Alkyl oder Aryl-niederes Alkyl oder zusammen mit dem Stickstoffatom einen 5- bis 8-gliedrigen, gegebenenfalls ein weiteres Heteroatom oder einen ankondensierten Benzolring enthaltenden Heterocyclus bedeuten. Die in Formel VIII mit X bezeichnete Abgangsgruppe ist beispielsweise ein leicht abspaltbarer Rest eines Phosphorsäure-Derivats , z.B. eine Gruppe der Formel

$$-O-PO(OR^a)_2 \text{ oder } -O-PO(NR^bR^c)_2$$

worin $R^a$ niederes Alkyl oder Aryl und $R^b$ und $R^c$ je niederes Alkyl, niederes Alkenyl (wie Allyl) oder Aryl oder zusammen mit dem Stickstoffatom einen 5- bis 8-gliedrigen, gegebenenfalls ein weiteres Heteroatom enthaltenden Heterocyclus (wie Morpholin) bedeuten,
ein Halogenatom, eine Alkylthiogruppe, eine Aralkylthiogruppe, eine N-Nitrosoalkylaminogruppe, eine Alkoxygruppe, eine Mercaptogruppe und dergleichen. Die Reaktion einer Verbindung der allgemeinen Formel VIII mit einem Isonitril der Formel IX erfolgt in einem inerten Lösungsmittel, wie Dimethylformamid, Hexamethylphosphorsäuretriamid, Dimethylsulfoxid, Tetrahydrofuran oder in irgend einem anderen geeigneten organischen Lösungsmittel und in Gegenwart einer Base, die genügend stark basisch ist, um das Anion des Isonitrils zu bilden. Als Basen eignen sich Alkalimetallalkoxide, wie Natriummethoxid oder Kalium-t-butoxid, Alkalimetallhydride, wie Natriumhydrid, Alkalimetallamide,

wie Lithiumamid oder Lithiumdiisopropylamid, Butyllithium, tertiäre Amine, wie 1,8-Diazabicyclo[5.4.0]undec-7-en (1,5-5), und dergleichen. Die Reaktionstemperatur liegt zweckmässigerweise zwischen etwa -70°C und etwa Raumtemperatur.

Gemäss Verfahrensvariante c) erhält man Verbindungen der Formel I, worin $R^1$ und $R^2$ die eingangs erwähnte Bedeutung besitzen. Die in Formel X mit Y bezeichnete Abgangsgruppe ist zweckmässigerweise ein Halogenatom, vorzugsweise ein Chlor- oder Bromatom, oder eine leicht abspaltbare Sulfonyloxygruppe, wie Methansulfonyloxy, Benzolsulfonyloxy, p-Toluolsulfonyloxy o. dgl. Die Umsetzung einer Verbindung der Formel X mit einem Amin der Formel XI erfolgt in Gegenwart eines inerten Lösungsmittels, wie N,N-Dimethylformamid, und in Gegenwart einer Base, zweckmässigerweise einer organischen Base, z.B. eines tertiären Amins, wie N-Aethyldiisopropylamin o. dgl., wobei als organische Base auch ein Ueberschuss des Amins der Formel XI dienen kann.

Gemäss Verfahrensvariante d) erhält man Verbindungen der Formel I, worin $R^1$ Wasserstoff niederes Hydroxyalkyl, Amino-niederes Alkyl oder niederes Alkylamino-niederes Alkyl und $R^2$ Wasserstoff, niederes Alkyl, niederes Alkenyl, niederes Alkinyl, niederes Hydroxyalkyl, niederes Alkoxy-niederes Alkyl, ($C_3$-$C_6$)-Cycloalkyl, ($C_3$-$C_6$)-Cycloalkyl-niederes Alkyl, Amino-niederes Alkyl, niederes Alkylamino-niederes Alkyl, Di-niederes Alkylamino-niederes Alkyl oder Aryl-niederes Alkyl bedeuten. Geeignete Schutzgruppen und Methoden zu deren Abspaltung sind jedem Fachmann geläufig, wobei natürlich nur solche Schutzgruppen verwendet werden können, die sich durch Methoden abspalten lassen, unter deren Bedingungen andere Strukturelemente in den Verbindungen der Formel XII nicht in Mitleidenschaft gezogen werden.

Als N-Schutzgruppe eignet sich beispielsweise die tert.-Butoxycarbonylgruppe (BOC), welche mittels Trifluoressigsäure abgespalten werden kann.

Als O-Schutzgruppe eignet sich beispielsweise die tert.-Butylgruppe (tBu), welche ebenfalls mittels Trifluoressigsäure abgespalten werden kann.

Wenn $R^7$ und $R^8$ zusammen eine Schutzgruppe bedeuten, dann bedeutet der Rest $-NR^7R^8$ beispielsweise eine Phthalimidogruppe, welche mittels Methylamin zur $NH_2$-Gruppe gespalten werden kann.

Gemäss Verfahrensvariante e) erhält man Verbindungen der Formel I, worin mindestens eines von $R^1$ und $R^2$ von Wasserstoff verschieden ist. Geeignete Alkylierungsmittel und Alkylierungsmethoden sind jedem Fachmann geläufig. Als Alkylierungsmittel eignen sich vorzugsweise entsprechende Halogenide, wie Propylbromid. Propyljodid, Butyljodid, Allylbromid, Crotylbromid, 4-Brom-1-buten, 3,3-Dimethylallylbromid, Propargylbromid, Cyclopropylmethylbromid, Benzylbromid oder $\alpha,\alpha'$-Dibrom-o-xylol (wobei mittels letzterem eine $NH_2$-Gruppe in eine Isoindolin-2-ylgruppe übergeführt werden kann). Die Alkylierung erfolgt in Gegenwart einer Base, zweckmässigerweise einer organischen Base, wie N-Aethyl-diisopropylamin, 1,8-Diazabicyclo[5.4.0]undec-7-en (1,5-5) o. dgl. Weiterhin erfolgt die Alkylierung zweckmässigerweise in einem inerten Lösungsmittel, wie N,N-Dimethylformamid.

Gemäss Verfahrensvariante f) erhält man Verbindungen der Formel I, worin mindestens eines von $R^1$ und $R^2$ niederes Alkyl bedeutet, aus entsprechenden Verbindungen der Formel I, worin mindestens eines von $R^1$ und $R^2$ niederes Alkenyl oder niederes Alkinyl bedeutet, d.h. aus Verbindungen der Formel Ib, und zwar durch Reduktion der C-C-Doppel- bzw. Dreifachbindung. Diese Reduktion erfolgt zweckmässigerweise durch katalytische Hydrierung, beispielsweise in Gegenwart eines Palladiumkatalysators, wie Pd/C. Weiterhin erfolgt diese Reduktion in einem inerten Lösungsmittel, wie Essigester.

Gemäss Verfahrensvariante g) können die Verbindungen der Formel I in pharmazeutisch annehmbare Säureadditionssalze übergeführt werden. Es kommen dabei sowohl Salze mit anorganischen als auch Salze mit organischen Säuren in Betracht. Beispiele derartiger Salze sind die Hydrochloride, Hydrobromide, Sulfate, Nitrate, Citrate, Acetate, Maleate, Succinate, Methansulfonate, p-Toluolsulfonate und dergleichen. Diese Salze können nach an sich bekannten und jedem Fachmann geläufigen Methoden hergestellt werden.

Die Ausgangsprodukte der obigen Formeln III, IV, VI und XI gehören allgemein bekannten Verbindungsklassen an und sind daher für jeden Fachmann ohne weiteres zugänglich. Auch die Ausgangsprodukte der Formeln II, V, VII und VIII gehören bekannten Verbindungsklassen an (vgl. z.B. EP 0 150 040 A2 und EP 0 027 214 A1). Die Ausgangsprodukte der Formel XII könne beispielsweise in Analogie zu den vorstehend beschriebenen Verfahrensvarianten a) und b) hergestellt werden. Die Herstellung von Ausgangsprodukten der Formel IX und X wird nachstehend anhand der Reaktionsschemata 1 bis 3 bzw. 4 erläutert. Ausserdem enthalten manche der weiter unten beschriebenen Beispiele ausführliche Angaben bezüglich der Herstellung spezifischer Ausgangsprodukte.

Reaktionsschema 1

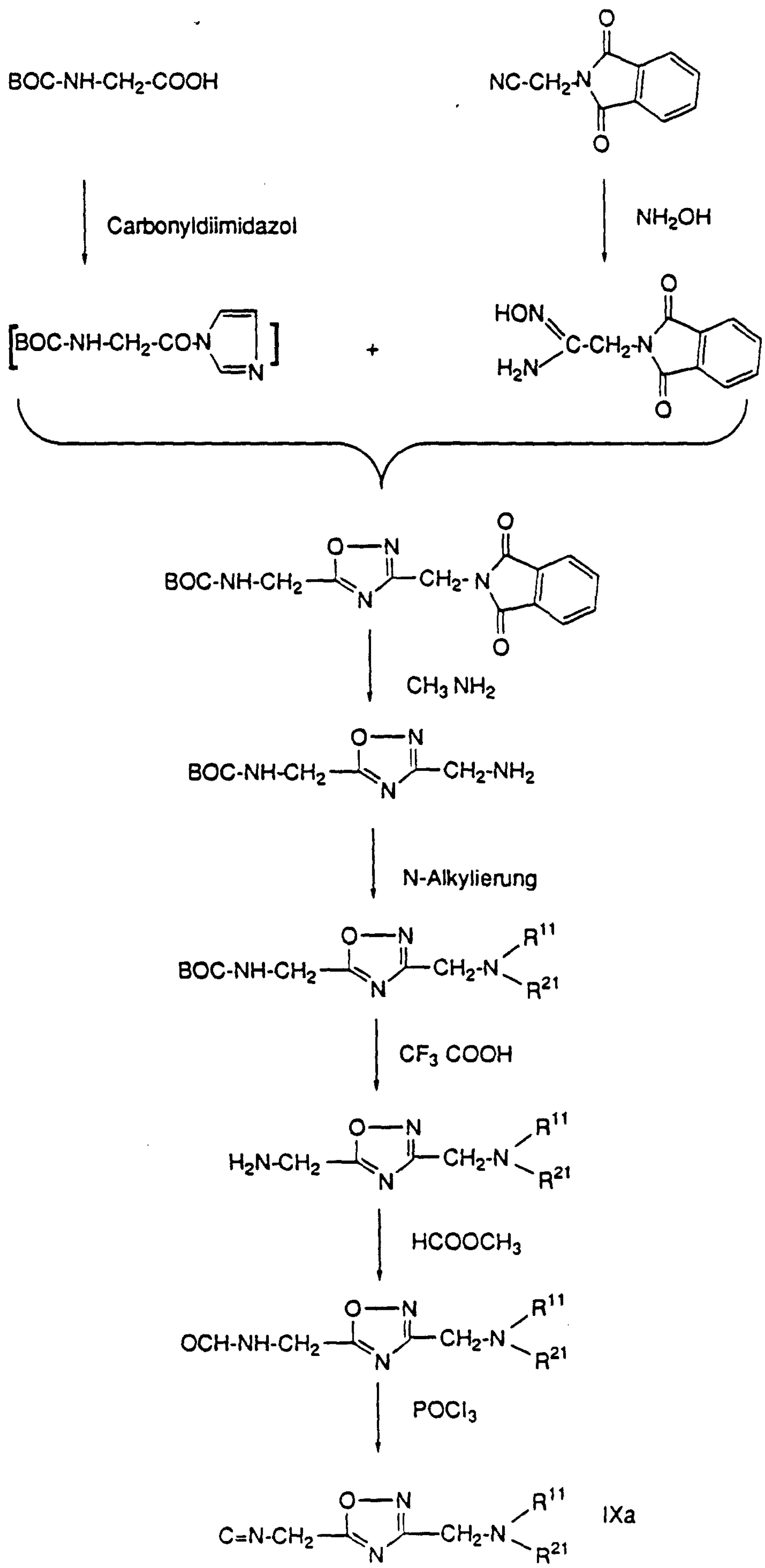


IXa

(IXa: Formel IX, worin Q den Rest $Q^1$ bedeutet)

Reaktionsschema 2

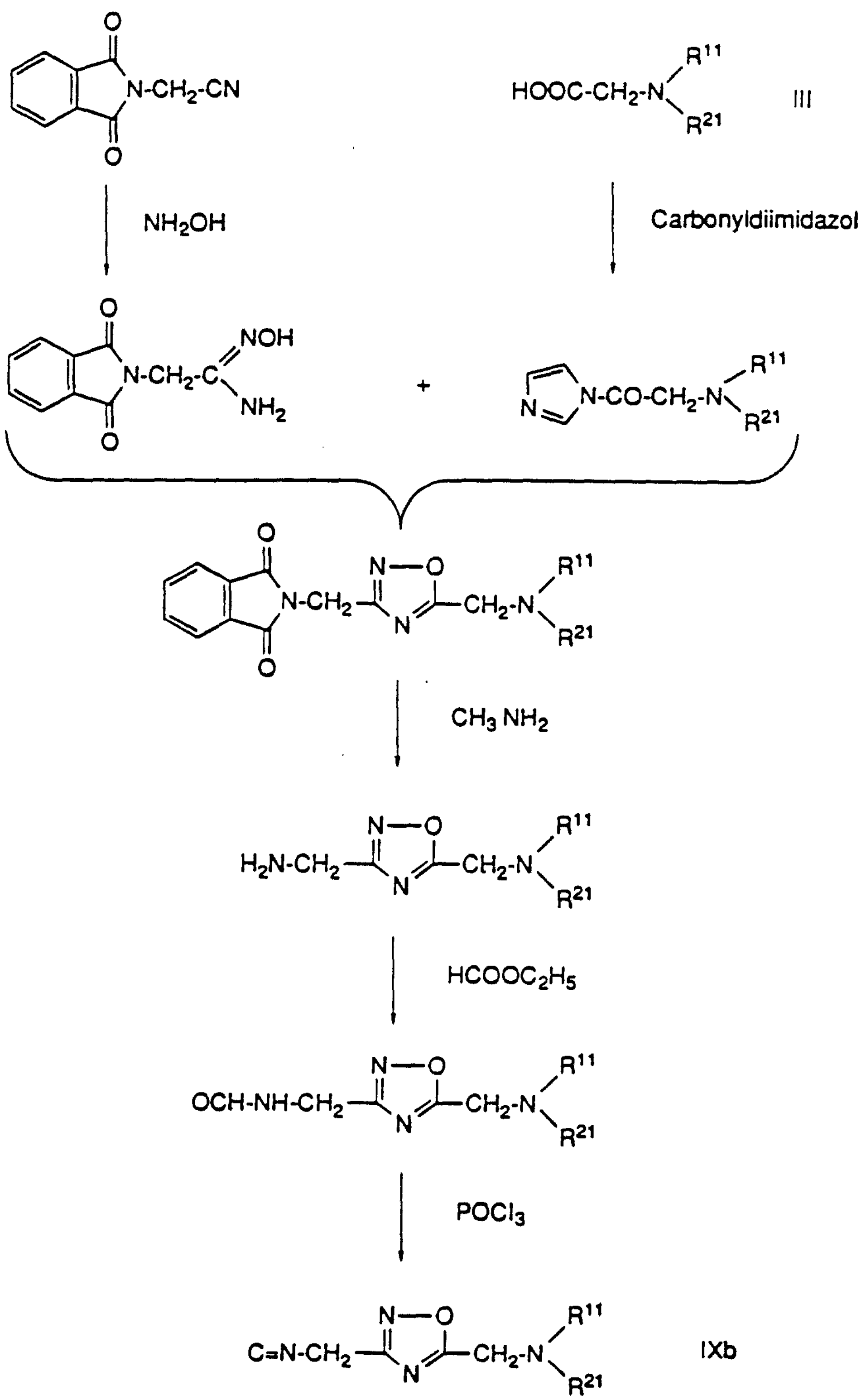


III

IXb

(IXb: Formel IX, worin Q den Rest $Q^2$ bedeutet)

Reaktionsschema 3

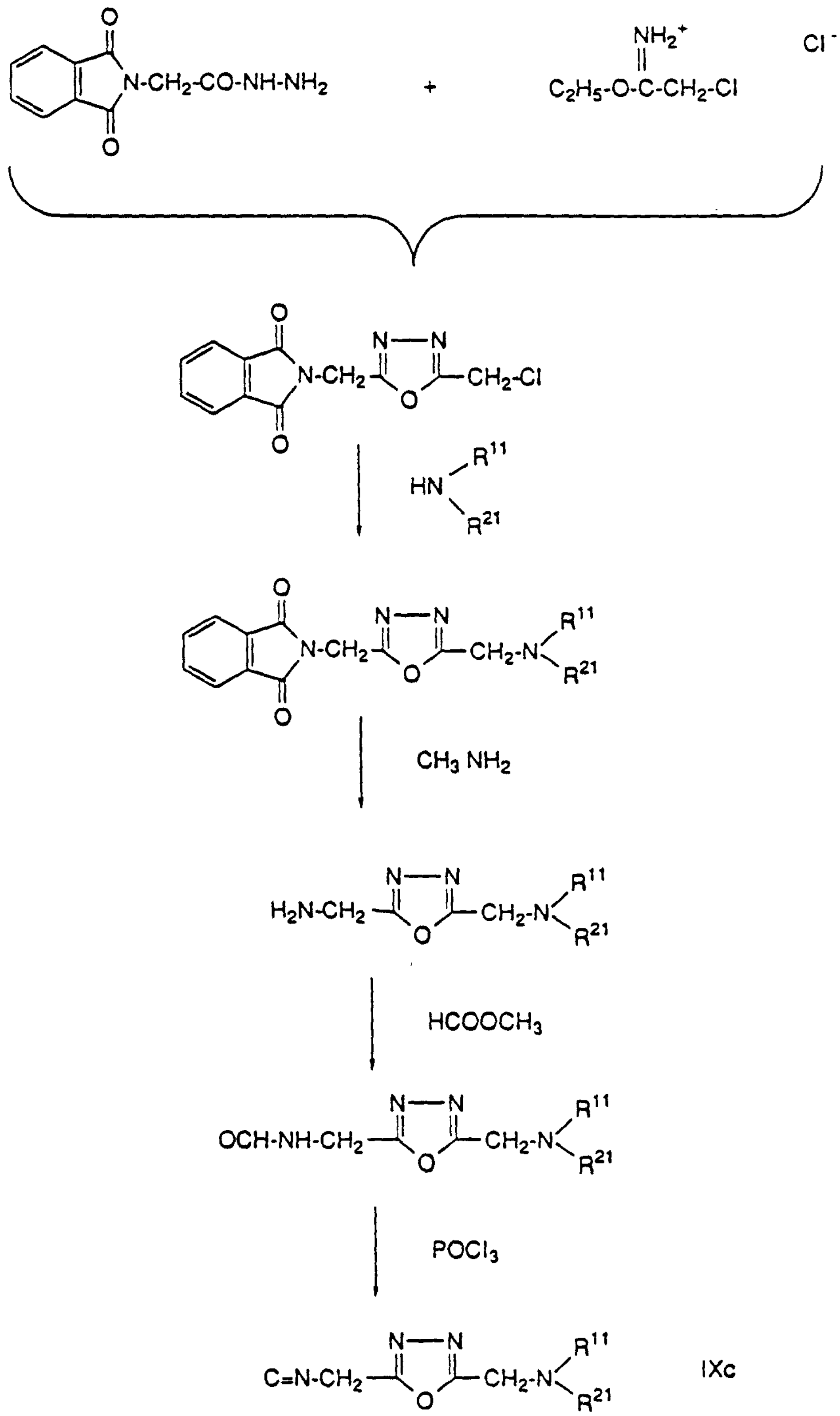


IXc

(IXc: Formel IX, worin Q den Rest $Q^3$ bedeutet)

Reaktionsschema 4

a)

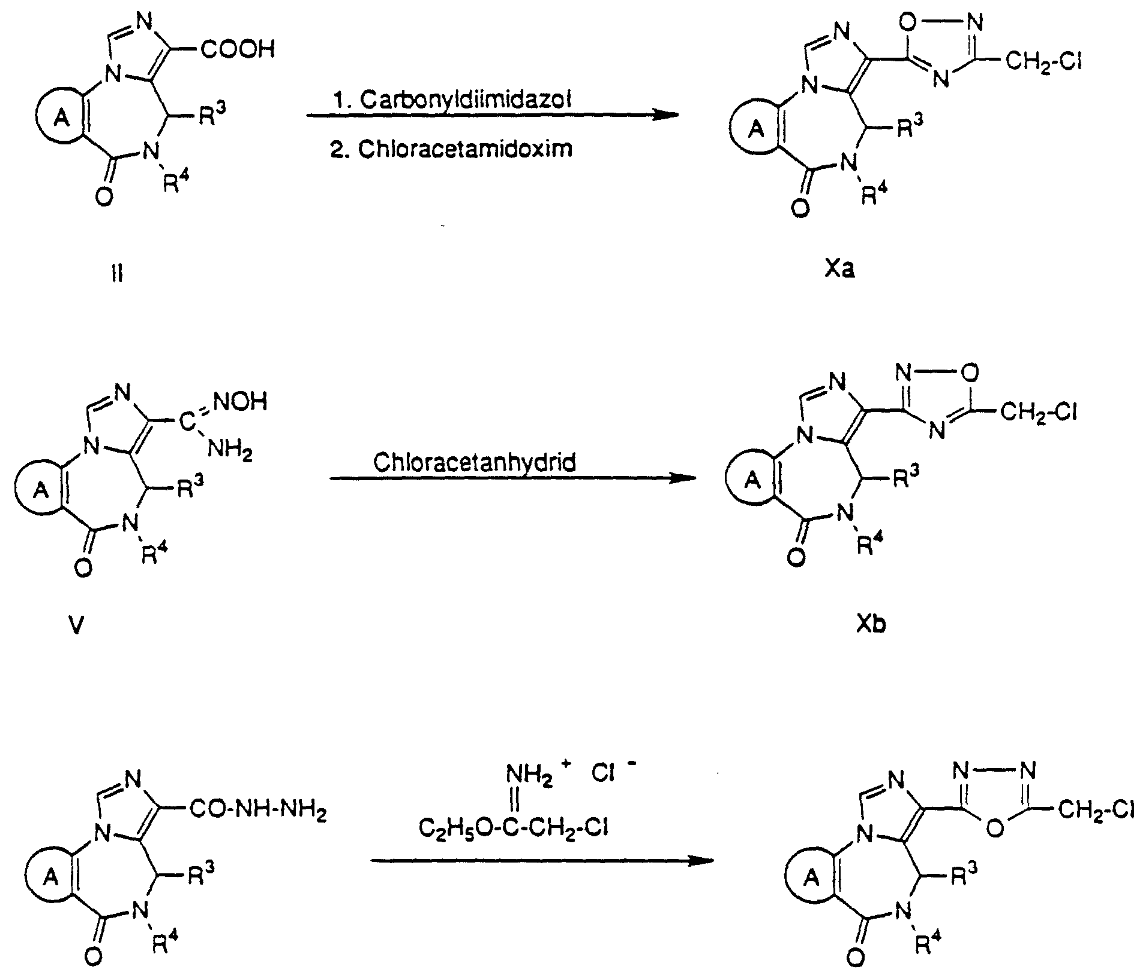


II → Xa

b) V → Xb

c) VII → Xc

(Xa, Xb und Xc: Formel X, worin Y Chlor und Q den Rest $Q^1$ bzw. $Q^2$ bzw. $Q^3$ bedeuten)

Wie eingangs erwähnt sind die Verbindungen der Formel I neu. Sie besitzen wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben aufgrund ihrer agonistischen Wirkung an diesen Rezeptoren ausgeprägte anxiolytische, antikonvulsive, muskelrelaxierende und sedativ-hypnotische Eigenschaften. Sie bilden sehr gut wasserlösliche Säureadditionssalze und eignen sich daher vorzüglich zur Herstellung von wässerigen Injektionslösungen.

Die Affinität von Verbindungen der allgemeinen Formel I zu den zentralen Benzodiazepin-Rezeptoren wurde in vitro nach der in Nature 294, 763-765 (1981) und J. Neurochemistry 37, 714-722 (1981) beschriebenen Methode festgestellt. Nach dieser Methode ermittelt man die Hemmung der Bindung von tritiiertem Flumazenil an die spezifischen Benzodiazepin-Rezeptoren im Ratten-Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung des tritiierten Flumazenils and die spezifischen Benzodiazepin-Rezeptoren im Ratten-Cortex

bewirkt.

Die sedativ/muskelrelaxierenden Eigenschaften der erfindungsgemässen Verbindungen der Formel I können beispielsweise im Rotierstab-Test erfasst werden. Für diesen Test werden Mäuse von 19-21 g Gewicht verwendet. Bis 1 h vor Beginn der Versuches haben sie freien Zugang zu Futter und Trinkwasser. Mindestens 30 min vor dem Versuch werden sie in das Versuchslabor gebracht. Beim Rotierstab-Test werden die Tiere auf einen waagrecht angeordneten, glatten Metallstab von 3 cm Durchmesser, der sich mit 2 Umdrehungen pro min. dreht, aufgesetzt. Zunächst lässt man den Tieren 30 sec Gelegenheit, sich mit der Test-Situation vertraut zu machen. Anschliessend werden diejenigen Tiere ausgewählt, denen es gelingt, mindestens 1 min lang auf dem Stab zu verbleiben. Diesen Tieren werden dann die Versuchpräparate in verschiedenen Dosen intravenös verabreicht. Zu verschieden Zeitpunkten wird dann bestimmt, ob sich die Tiere während einer Mindestzeit auf dem Stab halten können (Mindestzeit: 10 sec; ab 5 min nach Verabreichung: 1 min). Es wird diejenige Dosis ermittelt, bei welcher sich 50% der Tiere auf dem Stab zu halten vermögen (ED 50).

In der nachfolgenden Tabelle werden die Resultate dargestellt, welche mit repräsentativen Vertretern der durch die allgemeine Formel I definierten Verbindungsklasse in den vorstehend geschilderten Versuchen erhalten worden sind.

Tabelle

| Verbindung | Affinität zu BenzodiazepinRezeptoren, IC50, nMol/1 | Rotierstab-Test, ED50 in mg/kg, i.v., ermittelt zu folgenden Zeitpunkten nach Verabreichung: | | | | | | | |
|---|---|---|---|---|---|---|---|---|---|
| | | 15 sec | 30 sec | 60 sec | 2 min | 5 min | 15 min | 30 min | 60 min |
| A | 13 | 0.3 | 0.3 | 0.7 | 0.8 | 1.4 | 2.1 | 7.3 | >10 |
| B | 4.3 | 0.1 | 0.1 | 0.2 | 0.4 | 0.7 | 3.5 | >10 | >10 |
| C | 50 | 0.8 | 0.8 | 1.2 | 1.6 | 3.2 | ≥10 | >10 | >10 |
| D | 3.3 | 0.2 | 0.2 | 0.3 | 0.3 | 0.8 | 2.6 | >10 | >10 |
| E | 10 | 0.2 | 0.3 | 0.3 | 0.4 | 0.8 | 3.9 | ≥10 | >10 |
| F | 6.9 | 0.2 | 0.4 | 0.5 | 0.9 | ≥10 | >10 | >10 | >10 |
| G | 18.3 | 0.3 | 0.3 | 0.7 | 1.1 | 3.6 | ≥10 | >10 | >10 |
| H | 25 | 0.1 | 0.2 | 0.4 | 0.7 | 1.2 | 4.3 | 5.1 | >10 |
| I | 4.4 | 0.1 | 0.7 | 0.7 | 1.0 | 1.1 | 3.1 | ≥10 | >10 |
| J | 2.3 | 0.1 | 0.2 | 0.3 | 0.4 | 1.1 | >10 | >10 | >10 |
| K | 1.4 | 0.1 | 0.3 | 0.3 | 0.6 | 2.0 | ≥10 | ≥10 | ≥10 |
| L | 4.5 | 0.3 | 0.4 | 0.6 | 1.5 | 5.0 | >10 | >10 | >10 |
| M | 6.5 | 0.2 | 0.3 | 0.3 | 1.1 | 2.1 | >10 | >10 | >10 |
| N | 4.8 | 0.3 | 0.3 | 0.3 | 0.6 | 2.4 | >10 | >10 | >10 |
| O | 6.4 | 0.3 | 0.5 | 0.7 | 1.0 | 3.0 | >10 | >10 | >10 |
| P | 4.1 | 0.1 | 0.2 | 0.3 | 0.7 | 1.0 | 4.3 | ≥10 | >10 |

A: (S)-8-Chlor-1-(5-dipropylaminomethyl-1,2,4-oxadiazol-3-yl)-12,12a-dihydro-9H,11H-azeto[2,1-c]imidazo [1,5-a][1,4]benzodiazepin-9-on.

B: (S)-1-(5-Dipropylaminomethyl-1,2,4-oxadiazol-3-yl)-11,11a-dihydro-8H,10H-azeto[1,2-a]imidazo[5,1-c] thieno[3,2-e][1,4]diazepin-8-on.

C: 8-Fluor-5-methyl-3-[5-(piperidin-1-ylmethyl)-1,2,4-oxadiazol-3-yl)-5,6-dihydro-4H-imidazo[1,5-a][1,4]benzodiazepin-6-on.

D: 3-(5-Dipropylaminomethyl-1,2,4-oxadiazol-3-yl)-8-fluoro-5-methyl-5,6-dihydro-4H-imidazo[1,5-a][1,4]-benzodiazepin-6-on.

E: (S)-1-(5-Diallylaminomethyl-1,2,4-oxadiazol-3-yl)-8-chlor-7-fluor-12,12a-dihydro-9H,11H-azeto[2,1-c]imidazo[1,5-a][1,4]benzodiazepin-9-on.

F: 3-(5-Dipropylaminomethyl-1,2,4-oxadiazol-3-yl)-5-methyl-5,6-dihydro-4H-imidazo[1,5-a][1,4]benzodiazepin-6-on.

G: 3-(5-Dipropylaminomethyl-1,3,4-oxadiazol-2-yl)-8-fluoro-5-methyl-5,6-dihydro-4H-imidazo[1,5-c][1,4] benzodiazepin-6-on.

H: 3-(5-Diethylamiomethyl-1,2,4-oxadiazol-3-yl)-8-fluoro-5-methyl-5,6-dihydro-4H-imidazo[1,5-a][1,4]benzodiazepin-6-on.

I: (S)-8-Chlor-1-[5-(piperidin-1-yl)methyl-1,2,4-oxadiazol-3-yl]-12,12a-dihydro-9H,11H-azeto[2,1-c]imidazo[1,5-a][1,4]benzodiazepin-9-on.

J: 7-Fluor-5-methyl-3-(5-dipropylaminomethyl-1,2,4-oxadiazol-3-yl)-5,6-dihydro-4H-imidazo[1,5-a][1,4]benzodiazepin-6-on.

K: 7-Chlor-3-(5-diäthylaminomethyl-1,2,4-oxadiazol-3-yl)-5-methyl-5,6-dihydro-4H-imidazo[1,5-a][1,4]benzodiazepin-6-on.

L: 7-Chlor-3-(5-dipropylaminomethyl-1,3,4-oxadiazo-2-yl)-5-methyl-5,6-dihydro-4H-imidazo[1,5-a][1,4]benzodiazepin-6-on.

M: (S)-1-(5-Diäthylaminomethyl-1,2,4-oxadiazol-3-yl)-11,11a-dihydro-8H,10H-azeto[1,2-a]imidazo[5,1-c]thieno[3,2-e][1,4]diazepin-8-on.

N: (S)-1-(5-Dibutylaminomethyl-1,3,4-oxadiazol-2-yl)-11,11a-dihydro-8H,10H-azeto[1,2-a]imidazo[5,1-c]thieno[3,2-e][1,4]diazepin-8-on.

O: 3-(5-Diisopropylaminomethyl-1,2,4-oxadiazol-3-yl)-5-methyl-5,6-dihydro-4H-imidazo[1,5-a][1,4]benzodiazepin-6-on.

P: 3-(5-Diisopropylaminomethyl-1,2,4-oxadiazol-3-yl)-8-fluor-5-methyl-5,6-dihydro-4H-imidazo[1,5-a][1,4]benzodiazepin-6-on.

Aus der obigen Tabelle ist ersichtlich, dass Verbindungen A bis P eine sehr rasch eintretende und nur relativ kurze Zeit anhaltende sedative Wirkung entfalten.

Die Verbindungen der Formel I können aufgrund ihrer agonistischen Wirkung auf die Benzodiazepin-Rezeptoren als Sedativa/ Hypnotica, Anticonvulsiva, Muskelrelaxantien und Anxiolytica verwendet werden. Sie eignen sich beispielsweise als rasch aber kurz wirksame Hypnotica für perorale Verabreichung, insbesondere aber - in Form wässeriger Lösungen ihrer Säureadditionssalze - als injizierbare Kurzhypnotica für Prämedikation, Sedation sowie Narkoseeinleitung und Aufrechterhaltung der Narkose; bevorzugte Anwendungsmöglichkeiten sind dabei Prämedikation vor der Narkoseeinleitung, Basalsedation vor diagnostischen oder chirurgischen Eingriffen mit oder ohne Lokalanästhesie, Langzeitsedation auf der Intensivpflegestation, Verwendung als Induktionsmittel der Inhalationsnarkose oder als schlafinduzierende Komponente der Kombinationsnarkose (einschliesslich der totalen intravenösen Anästhesie) u.s.w.

Eine Reihe von Verbindungen der Formel I, einschliesslich der obigen Verbindungen A, B, D, und E wurden Mäusen und Ratten in Dosen von 32 und 100 mg/kg i.v. verabreicht, ohne dass dabei Todesfälle eingetreten wären.

Die Verbindungen der Formel I und pharmazeutisch annehmbare Säureadditionssalze davon können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I und pharmazeutisch annehmbare Säureadditionssalze davon mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für wässerige Injektionslösungen wasserlöslicher Säureadditionssalze von Verbindungen der Formel I sind Hilfsstoffe, wie Alkohole, Polyole, Glyzerin, pflanzliche Oele und dergleichen verwendbar aber in der Regel nicht notwendig. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungs-mittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon und ein therapeutisch inertes Excipiens, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe zusammen mit einem oder mehreren therapeutisch inerten Trägern in eine galenische Darreichungsform bringt.

Wie eingangs erwähnt, können die Verbindungen der Formel I und pharmazeutisch annehmbare Säureadditionssalze davon erfindungsgemäss für therapeutische Zwecke verwendet werden und zwar insbesondere für anxiolytische und/oder antikonvulsive und/oder muskelrelaxierende und/oder sedativ-hypnotische Zwecke. Die Dosierung kann innerhalb weiter Grenzen variieren und ist natürlich in jedem einzelnen Fall den individuellen Gegebenheiten

anzupassen. Im allgemeinen dürfte bei intravenöser Verabreichung eine Tagesdosis von etwa 1 mg bis 1000 mg angemessen sein.

Ebenfalls Gegenstand der Erfindung ist schliesslich, wie eingangs erwähnt, die Verwendung von Verbindungen der Formel I und von pharmazeutisch anwendbaren Säureadditionssalzen davon für die Herstellung von Arzneimitteln, insbesondere von anxiolytischen und/oder antikonvulsiven und/oder muskelrelaxierenden und/oder sedativhypnotischen Arzneimitteln.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

a) 1.89 g (10 mMol) BOC-Sarcosin wurden in 10 ml N,N-Dimethylformamid gelöst, portionenweise mit 1.63 g (10 mMol) 1,1'-Carbonyldiimidazol versetzt und während 20' bei 50°C gerührt. Nach Zugabe von 3.05 g (10mMol) 7-Chlor-5,6-dihydro-5-methyl-6-oxo-4H-imidazo[1,5-a][1,4]-benzodiazepin-3-carboxamidoxim wurde während 7 Stunden bei 90° weitergerührt. Man kühlte das Reaktionsgemisch ab und goss es auf 300 ml Wasser. Die erhaltene Suspension wurde filtriert, und die Kristalle wurden mit Wasser nachgewaschen und getrocknet. Man erhielt 3.16 g (69%) 7-Chlor-5,6-dihydro-5-methyl-3-(5-N-BOC-N-methylaminomethyl-1,2,4-oxadiazol-3-yl)-4H-imidazo [1,5-a][1,4]benzodiazepin-6-on vom Smp. 114-117°.

b) 3.09 g (6.73 mMol) 7-Chlor-5,6-dihydro-5-methyl-3-(5-N-BOC-N-methylamino-methyl-1,2,4-oxadiazol-3-yl)-4H-imidazo[1,5-a][1,4]benzodiazepin-6-on wurden während 2.5 Stunden in 20 ml Trifluoressigsäure bei Raumtemperatur gerührt. Die Lösung wurde eingeengt, worauf der Rückstand in Wasser aufgenommen und zweimal mit Methylenchlorid gewaschen wurde. Man stellte die wässrige Phase mit 25%igem Ammoniak alkalisch und extrahierte sie viermal mit Methylenchlorid. Nach Trocknung und Eindampfen der vereinigten organischen Phasen und Umkristallisieren des Rückstandes aus Methanol erhielt man 1.3 g (54%) 7-Chlor-5,6-dihydro-5-methyl-3-(5-methylaminomethyl-1,2,4-oxadiazol-3-yl)4H-imidazo[1,5-a][1,4]benzodiazepin-6-on vom Smp. 192-193°.

Beispiel 2

a) 4.60 g (16.95 mMol) 5,6-Dihydro-5-methyl-6-oxo-4H-imidazo[1,5-a][1,4]benzodiazepin-3-carboxamidoxim wurden mit 3.19 g (18.65 mMol) Chloressigsäureanhydrid in 25 ml N,N-Dimethylformamid während 30 Minuten bei Raumtemperatur und 2 Stunden bei 105° gerührt. Das Reaktionsgemisch wurde eingedampft, der Rückstand wurde in Methylenchlorid gelöst, und die Lösung wurde mit gesättigter Natriumbicarbonat-Lösung gewaschen. Nach Trocknung über Magnesiumsulfat wurde die Lösung eingeengt und der kristalline Rückstand wurde an Kieselgel unter Eluieren mit Essigester chromatographiert. Man erhielt 3.94 g (70%) 3-(5-Chlormethyl-1,2,4-oxadiazol-3-yl)-5,6-dihydro-5-methyl-4H-imidazo[1,5-a]-[1,4]benzodiazepin-6-on vom Smp. 208-209°.

b) 3.3 g (10 mMol) 3-(5-Chlormethyl-1,2,4-oxadiazol-3-yl)-5,6-dihydro-5-methyl-4H-imidazo[1,5-a][1,4]benzodiazepin-6-on wurden in 20 ml N,N-Dimethylformamid mit 5.06 g (50 mMol) Dipropylamin während 4 Stunden bei Raumtemperatur gerührt. Das Reaktionsgemisch wurde eingedampft, und der Rückstand wurde an Kieselgel unter Eluieren mit Essigester chromatographiert. Man erhielt 3.65 g ( 92 %) 3-(5-Dipropylaminomethyl-1,2,4-oxadiazol-3-yl)-5-methyl-5,6-dihydro-4H-imidazo[1,5-a][1,4]benzodiazepin-6-on vom Schmelzpunkt 130-132°, welches in das Hydrochlorid vom Smp. 213-215° überführt wurde.

Beispiel 3

1.4 g (13.5mMol) N,N-Dimethylglycin wurden in 20 ml N,N-Dimethylformamid suspendiert, mit 2.6 g (15.9mMol) 1,1'-Carbonyldiimidazol versetzt und während 1 Stunde bei Raumtemperatur und 1 Stunde bei 75° gerührt. Man gab 3.9 g (12.3 mMol) (S)-8-Chlor-12,12a-dihydro-9-oxo-9H,11H-azeto[2,1-c]imidazo[1,5-a][1,4]-benzodiazepin-1-carboxamidoxim zu und rührte über Nacht bei 90°. Durch Einengen des Reaktionsgemisches und Chromatographieren des Rückstandes an Kieselgel unter Eluieren mit Methylenchlorid und Methanol 19/1 erhielt man nach Umkristallisieren aus Essigester 2.53 g (54%) (S)-8-Chlor-12,12a-dihydro-1-(5-dimethylaminomethyl-1,2,4-oxadiazol-3-yl)-9H, 11H-azeto[2,1-c]imidazo[1,5-a][1,4]benzodiazepin-9-on vom Schmelzpunkt 127-129°, das in das Hydrochlorid vom Smp. 178° überführt wurde.

Beispiel 4

2.55 g (13.5 mMol) 1-Pyrrolidin-essigsäure wurden in 20 ml N,N-Dimethylformamid gelöst und portionenweise mit 2.6 g (15.9 mMol) 1,1'-Carbonyldiimidazol versetzt. Nach 45minütigem Rühren gab man 3.9g (12.3 mMol) (S)-8-Chlor-12,12a-dihydro-9-oxo-9H,11H-azeto[2,1-c]imidazo[1,5-a]-[1,4]benzo-diazepin-1-carboxamidoxim zu und rührte über Nacht bei 90°. Man gab 0.2 g p-Toluolsulfonsäure zu und rührte nochmals während 4 Stunden bei 90°. Das Reaktionsgemisch wurde eingeengt. Durch Chromatographieren des Rückstandes an Kieselgel unter Eluieren mit Methylenchlorid/Methanol 19/1 und Umkristallisieren aus Essigester und Hexan erhielt man 1.6 g (32%) (S)-8-Chlor-12,12a-dihydro-1-[5-(pyrrolidin-1-ylmethyl)-1,2,4-oxadiazol-3-yl]-9H,11H-azeto[2,1-c]-imidazo[1,5-a][1,4] benzodiazepin-9-on vom Smp. 117-120°, das in das Hydrochlorid überführt wurde.

Beispiel 5

1.4 g (13.5 mMol) N,N-Dimethylglycin wurden in 20 ml N,N-Dimethylformamid gelöst und portionenweise mit 2.6 g (15 mMol) 1,1'-Carbonyldiimidazol versetzt. Nach Beendigung der $CO_2$-Abspaltung wurde die Lösung während 30' bei 70° gerührt. Man gab dann 4.02 g (15 mMol) (S)-8-Chlor-11,12,13,13a-tetrahydro-9-oxo-9H-imidazo[1,5-a] pyrrolo[2,1-c][1,4]-benzodiazepin-1-carboxamidoxim zu und rührte über Nacht bei 90°. Durch Eindampfen der Lösung und Chromatographieren des Rückstandes an 340 g Kieselgel unter Eluieren mit Methylenchlorid/Methanol 19/1 erhielt man 1.99 g (40%) (S)-8-Chlor-1-(5-dimethylaminomethyl-1,2.4-oxadiazol-3-yl)-11,12,13,13a-tetrahydro-9H-imidazo [1,5-a]pyrrolo[2,1-c][1,4]-benzodiazepin-9-on, welches in das Hydrochlorid vom Smp. 261° überführt wurde.

Beispiel 6

a) 3.5 g (20 mMol) BOC-Glycin wurden in 20 ml N,N-Dimethylformamid gelöst und portionenweise mit 3,35 g (20 mMol) 1,1'-Carbonyldiimidazol versetzt. Nach 10minütigem Rühren bei 45° gab man 6.35g (20 mMol) (S)-8-Chlor-12,12a-dihydro-9-oxo-9H,11H-azeto[2,1-c]imidazo[1,5-a][1,4]benzodiazepin-1-carboxamidoxim zu und rührte über Nacht bei 90°. Das Reaktionsgemisch wurde eingeengt; der Rückstand wurde in Methylenchlorid gelöst, und die Lösung wurde dreimal mit Wasser gewaschen, über Magnesiumsulfat getrocknet und eingedampft. Durch Chromatographieren des Rückstandes an Kieselgel unter Eluieren mit Essigester erhielt man 7.5 g (82%) (S)-1-(5-BOC-Aminamethyl-1,2,4-oxadiazol-3-yl)-8-Chlor-12,12a-dihydro-9H,11H-azeto[2,1-c]imidazo[1,5-a][1,4]benzodiazepin-9-on, das ohne weitere Reinigung in die nächste Stufe eingesetzt wurde.

b) 7.29 g (16 mMol) rohes (S)-1-(5-BOC-Aminomethyl-1,2,4-osadiazol-3-yl)-8-Chlor-12,12a-dihydro-9H,11H-azeto[2,1-c]imidazo[1,5-a][1,4]benzodiazepin-9-on wurden während 2 Stunden in 25 ml Trifluoressigsäure gerührt. Die Lösung wurde eingeengt, der Rückstand wurde in Wasser aufgenommen, und die wässerige Lösung wurde zweimal mit Methylenchlorid gewaschen. Man stellte die wässrige Phase mit 25%igem Ammoniak alkalisch und extrahierte sie siebenmal mit Methylenchlorid. Durch Trocknung und Eindampfen der vereinigten organischen Phasen erhielt man 5.12 g (90%) (S)-1-(5-Aminomethyl-1,2,4-oxadiazol-3-yl)-8-chlor-12,12a-dihydra-9H,11H-azeta[2,1-c]imidaza[1,5-a][1,4]benzodiazepin-9-on vom Smp. 212-214°, das in das Hydrochlorid vom Smp. 252-255° überführt wurde.

Beispiel 7

a) 3.8 g (20 mMol) BOC-Sarcosin wurden in 20 ml N,N-Dimethylformamid gelöst und portionenweise mit 3,5 g (21.6 mMol) 1,1'-Carbonyldiimidazol versetzt. Nach 10minütigem Rühren bei 45° gab man 6.35g (20 mMol) (S)-8-Chlar-12,12a-dihydro-9-oxa-9H,11H-azeta[2,1-c]imidazo[1,5-a]-[1,4]benzodiazepin-1-carboxamidoxim zu und rührte über Nacht bei 90°. Durch Einengen der Reaktionslösung und Chromatographieren des Rückstandes an Kieselgel unter Eluieren mit Essigester erhielt man 7.58 g (80%) (S)-8-Chlor-12,12a-dihydro-1-[5-(N-BOC-N-methyl)-aminomethyl-1,2,4-oxadiazol-3-yl]-9H,11H-azeto[2,1-c]imidazo[1,5-a][1,4]benzodiazepin-9-on, das ohne weitere Reinigung in die nächste Stufe eingesetzt wurde.

b) 8 g (17 mMol) rohes (S)-8-Chlor-12,12a-dihydro-1-[5-(N-BOC-N-methyl)-aminomethyl-1,2,4-oxadiazol-3-yl]-9H,11H-azeto[2,1-c]imidazo[1,5-a][1,4]benzo-diazepin-9-on wurden während 2 Stunden in 25 ml Trifluoressigsäure bei Raumtemperatur gerührt. Die Lösung wurde eingeengt, der Rückstand wurde in Wasser aufgenommen,

und die wässerige Lösung wurde zweimal mit Methylenchlorid gewaschen. Man stellte die wässrige Phase mit 25%igem Ammoniak alkalisch und extrahierte sie siebenmal mit Methylenchlorid. Durch Trocknung und Eindampfen der vereinigten organischen Phasen erhielt man 4.35 g (69%) (S)-8-Chlor-12,12a-dihydro-1-(5-methylaminomethyl-1,2,4-oxadiazol-3-yl)-9H,11H-azeto[2,1-c]imidazo[1,5-a][1,4]benzodiazepin-9-on, das in das Hydrochlorid vom Smp. 250° überführt wurde.

Beispiel 8

1.96 g (13.5 mMol) 4-Morpholino-essigsäure wurden in 20 ml N,N-Dimethylformamid gelöst und portionenweise mit 2.6 g (15.9 mMol) 1,1'-Carbonyldiimidazol versetzt. Nach 15minütigem Rühren bei 80° gab man 3.9 g (12.3 mMol) (S)-8-Chlor-12,12a-dihydro-9-oxo-9H,11H-azeto[2,1-c]-imidazo[1,5-a][1,4]-benzodiazepin-1-carboxamidoxim zu und rührte über Nacht bei 90°. Das Reaktionsgemisch wurde eingeengt. Durch Chromatographieren des Rückstandes an Kieselgel unter Eluieren mit Methylenchlorid/Methanol 19/1 und Umkristallisieren aus Essigester erhielt man 1.85 g (35%) (S)-8-Chlor-12,12a-dihydro-1-[5-(pyrrolidin-1-ylmethyl)-1,2,4-oxadiazol-3-yl]-9H,11H-azeto[2,1-c]-imidazo[1,5-a][1,4]benzodiazepin-9-on, das in das Hydrochlorid überführt wurde.

Beispiel 9

1.75 g (17 mMol) N,N-Dimethylglycin wurden in 20 ml N,N-Dimethylformamid gelöst und portionenweise mit 3.08 g (15 mMol) 1,1'-Carbonyldiimidazol versetzt. Nach Beendigung der $CO_2$-Abspaltung wurde die Lösung während 30' bei 70° gerührt. Man gab dann 4.02 g (15 mMol) (S)-7-Fluor-12,12a-dihydro-9-oxo-9H,11H-azeto[2,1-c]imidazo [1,5-a][1,4]benzodiazepin-1-carboxamidoxim zu und rührte über Nacht bei 90°C. Durch Eindampfen der Lösung, Chromatographieren des Rückstandes an 240 g Kieselgel unter Eluieren mit Methylenchlorid/Methanol 19/1 und Kristallisieren aus Essigester erhielt man 1.35 g (24%) (S)-7-Fluor-12,12a-dihydro-1-[5-(dimethylaminomethyl)-1,2,4-oxadiazol-3-yl]-9H,11H-azeto[2,1-c]imidazo-[1,5-a][1,4]benzodiazepin-9-on vom Smp. 187-190°, das in das Hydrochlorid vom Smp. 150-155° überführt wurde.

Beispiel 10

a) 6.81 g (36 mMol) BOC-Sarcosin wurden in 30 ml N,N-Dimethylformamid gelöst und portionenweise mit 6.5 g (40 mMol) 1,1'-Carbonyldiimidazol versetzt. Nach Beendigung der $CO_2$-Abspaltung wurde die Lösung während 30' bei 40° gerührt. Man gab dann 9.04 g (15 mMol) (S)-7-Fluor-12,12a-dihydro-9-oxo-9H,11H-azeto[2,1-c]imidazo [1,5-a][1,4]benzodiazepin-1-carboxamidoxim zu und rührte über Nacht bei 90°. Durch Eindampfen der Lösung und Chromatographieren des Rückstandes an 450 g Kieselgel unter Eluieren mit Methylenchlorid/Methanol 19/1 erhielt man 8.81 g (65%) (S)-7-Fluor-12,12a-dihydro-1-[5-(N-BOC-N-methylaminomethyl)-1,2,4-oxadiazol-3-yl]-9H,11H-azeto[2,1-c]imidazo[1,5-a][1,4]benzodiazepin-9-on, das ohne weitere Reinigung in die nächste Stufe eingesetzt wurde.

b) 7.74 g (17mMol) rohes (S)-7-Fluor-12,12a-dihydro-1-[5-(N-BOC-N-methylaminomethyl)-1,2,4-oxadiazol-3-yl]-9H,11H-azeto[2,1-c]imidazo[1,5-a][1,4]benzodiazepin-9-on wurden während 2 Stunden in 25 ml Trifluoressigsäure gerührt. Die Lösung wurde eingeengt, der Rückstand wurde in Wasser aufgenommen, und die Lösung wurde zweimal mit Methylenchlorid gewaschen. Man stellte die wässrige Phase mit 25%igem Ammoniak alkalisch und extrahierte sie siebenmal mit Methylenchlorid. Man erhielt 5.12 g (85%) (S)-7-Fluor-12,12a-dihydro-1-(5-methylaminomethyl-1,2,4-oxadiazol-3-yl)-9H,11H-azeto[2,1-c]imidazo[1,5-a][1,4]benzodiazepin-9-on vom Smp. 187-190°, das in das Hydrochlorid vom Smp. 155-160° überführt wurde.

Beispiel 11

4.13 g (11.13 mMol) (S)-8-Chlor-12,12a-dihydro-1-(5-methylaminomethyl-1,2,4-oxadiazol-3-yl]-9H,11H-azeto[2,1-c]imidazo[1,5-a][1,4]benzodiazepin-9-on, 20 ml N,N-Dimethylformamid, 2 g (13 mMol) 1,8-Diazabicyclo-[5.4.0]-undec-7-en(1,5-5) und 1.58 g (13 mMol) Allylbromid wurden während 60 Stunden bei Raumtemperatur gerührt. Das Reaktionsgemisch wurde durch Chromatographieren an Kieselgel unter Eluieren mit Methylenchlorid/Methanol 19/1 gereinigt. Man erhielt 2.86 g (62%) (S)-1-[5-(N-Allyl-N-methyl)-aminomethyl-1,2,4-oxadiazol-3-yl]-8-chlor-12,12a-dihydro-9H,11H-azeto[2,1-c]imidazo[1,5-a][1,4]-benzodiazepin-9-on, das in das Hydrochlorid vom Smp. 187-192° überführt wurde.

Beispiel 12

a) 8.41 g (48 mMol) BOC-Glycin wurden in 30 ml N,N-Dimethylformamid gelöst und portionenweise mit 7.8 g (48 mMol) 1,1'-Carbonyldiimidazol versetzt. Nach Beendigung der $CO_2$-Abspaltung wurde die Lösung während 10' bei 45° gerührt. Man gab dann 12.05 g (40 mMol) (S)-7-Fluor-12,12a-dihydro-9-oxo-9H,11H-azeto-[2,1-c]imidazo [1,5-a][1,4]benzodiazepin-1-carboxamidoxim zu und rührte über Nacht bei 90°. Durch Eindampfen der Lösung, Chromatographieren des Rückstandes an 550 g Kieselgel unter Eluieren mit Methylenchlorid/Methanol 19/1 und Umkristallisieren aus Essigester erhielt man 10.78 g (61%) (S)-1-[5-(BOC-Aminomethyl)-1,2,4-oxadiazol-3-yl]-7-fluor-12,12a-dihydro-9H,11H-azeto-[2,1-c]imidaza[1,5-a][1,4]benzodiazepin-9-on vom Smp. 147-150°.

b) 4.40 g (10 mMol) (S)-1-[5-(BOC-Aminomethyl)-1,2,4-oxadiazol-3-yl]-7-fluor-12,12a-dihydro-9H,11H-azeto [2,1-c]imidazo[1,5-a][1,4]benzodiazepin-9-on wurden während 2 Stunden in 20 ml Trifluoressigsäure gerührt. Die Lösung wurde eingeengt, der Rückstand wurde in Wasser aufgenommen, und die wässerige Lösung wurde zweimal mit Methylenchlorid gewaschen. Man stellte die wässrige Phase mit 25%igem Ammoniak alkalisch und extrahierte sie siebenmal mit Methylenchlorid. Nach Trocknung und Eindampfen der vereinigten organischen Phasen erhielt man 2.77 g (81%) (S)-1-(5-Aminomethyl-1,2,4-oxadiazol-3-yl)-7-fluor-12,12a-dihydro-9H,11H-azeto-[2,1-c]-imidazo[1,5-a][1,4]benzodiazepin-9-on vom Smp. 195-198°, das in das Hydrochlorid vom Smp. 275° überführt wurde.

Beispiel 13

1.24 g (12 mMol) N,N-Dimethylglycin wurden in 20 ml N,N-Dimethylformamid gelöst und portionenweise mit 2,43 g (15 mMol) 1,1'-Carbonyldiimidazol versetzt. Nach Beendigung der $CO_2$-Abspaltung wurde die Lösung während 30' bei 70° gerührt. Man gab dann 3.35 g (10 mMol) (S)-8-Chlor-7-fluor-12,12a-dihydro-9-oxo-9H,11H-azeto[2,1-c] imidazo[1,5-a][1,4]-benzodiazepin-1-carboxamidoxim zu und rührte während 3.5 Stunden bei 90°. Durch Eindampfen der Lösung, Chromatographieren des Rückstandes an 300 g Kieselgel unter Eluieren mit Essigester/Methanol 9/1 und Kristallisieren aus Essigester und Hexan erhielt man 0.98 g (24%) (S)-8-Chlor-7-fluor-12,12a-dihydro-1-[5-(dimethylaminomethyl)-1,2,4-oxadiazol-3-yl]-9H,11H-azeto[2,1-c]imidazo[1,5-a][1,4]benzodiazepin-9-on vom Smp. 166-169°, das in das Hydrochlorid vom Smp. 223-227° überführt wurde.

Beispiel 14

470 mg (1.32mMol) (S)-7-Fluor-12,12a-dihydro-1-(5-methylaminomethyl-1,2,4-oxadiazol-3-yl)-9H,11H-azeto [2,1-c]imidazo[1,5-a][1,4]benzodiazepin-9-on und 750 mg (1.92 mMol) (S)-7-Fluor-12,-12a-dihydro-1-(5-methylaminomethyl-1,2,4-oxadiazol-3-yl)-9H,11H-azeto[2,1-c]imidazo[1,5-a][1,4]benzo-diazepin-9-on hydrochlorid, 837 mg (5.5 mMol) 1,8-Diazabicyclo[5.4.0]undec-7-en(1,5-5) und 450 mg (3.5 mMol) Allylbromid wurden in 15ml N,N-Dimethylformamid über Nacht bei Raumtemperatur und während 2.5 Stunden bei 55° gerührt. Das Reaktionsgemisch wurde durch Chromatographieren an Kieselgel unter Eluieren mit Methylenchlorid/Methanol 19/1 gereinigt. Man erhielt 1.15g (88%) (S)-1-[5-(N-Allyl-N-methyl)-aminomethyl-1,2,4-oxadiazol-3-yl]-7-fluor-12,12a-dihydro-9H,11H-azeto[2,1-c]imidazo-[1,5-a][1,4]benzodiazepin-9-on, das in das Hydrochlorid vom Smp. 224-226° überführt wurde.

Beispiel 15

a) 3.02 g (16 mMol) BOC-Sarcosin wurden in 15 ml N,N-Dimethylformamid gelöst und portionenweise mit 2,75 g (17 mMol) 1,1'-Carbonyldiimidazol versetzt. Nach 15-minütigem Rühren bei 50° gab man 5.04 g (15 mMol) (S)-8-Chlor-7-fluor-12,12a-dihydro-9-oxo-9H,11H-azeto[2,1-c]imidazo-[1,5-a][1,4]benzodiazepin-1-carboxamidoxim zu und rührte über Nacht bei 90°. Durch Einengen der Reaktionslösung und Chromatographieren des Rückstandes an Kieselgel unter Eluieren mit Essigester erhielt man 5.0 g (68%) (S)-8-Chlor-7-fluor-12,12a-dihydro-1-[5-(N-BOC-N-methyl)-aminomethyl-1,2,4-oxadiazol-3-yl]-9H,11H-azeto[2,1-c]imidazo-[1,5-a][1,4]-benzodiazepin-9-on, das ohne weitere Reinigung in die nächste Stufe eingesetzt wurde.

b) 4.42 g (9 mMol) (S)-8-Chlor-7-fluor-12,12a-dihydro-1-[5-(N-BOC-N-methyl)-aminomethyl-1,2,4-oxadiazol-3-yl]-9H,11H-azeto[2,1-c]imidazo-[1,5-a][1,4]benzodiazepin-9-on wurden über Nacht in 20 ml Trifluoressigsäure stehen gelassen. Die Lösung wurde eingedampft, der Rückstand wurde in Wasser gelöst, und die Lösung wurde dreimal

mit Methylenchlorid gewaschen. Die wässrige Phase wurde mit konz. Ammoniak alkalisch gestellt und achtmal mit Methylenchlorid (insgesamt ca 11) extrahiert. Durch Eindampfen der vereinigten und über Magnesiumsulfat getrockneten organischen Phasen erhielt man 2.97 g (84%) (S)-8-Chlor-7-fluor-12,12a-dihydro-1-(5-methylaminomethyl-1,2,4-oxadiazol-3-yl)-9H,11H-azeto[2,1-c]imidazo[1,5-a][1,4]benzodiazepin-9-on, das ohne weitere Reinigung als Ausgangsprodukt für das nachstehend beschriebende Beispiel verwendet wurde.

Beispiel 16

2.95 g (7.6 mMol) rohes (S)-8-Chlor-7-fluor-12,12a-dihydro-1-(5-methylaminomethyl-1,2,4-oxadiazol-3-yl)-9H,11H-azeto[2,1-c]imidazo[1,5-a][1,4]-benzodiazepin-9-on, 15 ml N,N-Dimethylformamid, 1.4 g (9.4 mMol) 1,8-Diazabicyclo[5.4.0]undec-7-en(1,5-5) und 1.1 g (9 mMol) Allylbromid wurden über Nacht bei Raumtemperatur und während 6 Stunden bei 55° gerührt. Das Reaktionsgemisch wurde durch Chromatographieren an Kieselgel unter Eluieren mit Methylenchlorid/Methanol 19/1 gereinigt. Man erhielt 1.39 g (42%) (S)-1-[5-(N-Allyl-N-methyl)-aminomethyl-1,2,4-oxadiazol-3-yl]-8-chlor-7-fluor-12,12a-dihydro-9H,11H-azeto[2,1-c]imidazo[1,5a][1,4]benzodiazepin-9-on, das in das Hydrochlorid vom Smp. 199-203° überführt wurde.

Beispiel 17

a) 3.02 g (16 mMol) BOC-Sarcosin wurden in 25 ml N,N-Dimethylformamid gelöst und portionenweise mit 2.75 g (17 mMol) 1,1'-Carbonyldiimidazol versetzt. Nach Beendigung der $CO_2$-Abspaltung wurde die Lösung während 30' bei 60° gerührt. Man gab dann 4.02 g (15 mMol) (S)-8-Chlor-11,12,13,13a-tetrahydro-9-oxo-9H-imidazo[1,5-a]pyrrolo[2,1-c][1,4]-benzodiazepin-1-carboxamidoxim zu und rührte über Nacht bei 95°. Durch Eindampfen der Lösung und Chromatographieren des Rückstandes an 300 g Kieselgel unter Eluieren mit Essigester erhielt man 3.6 g (49%) (S)-8-Chlor-11,12,13,13a-tetrahydro-1-(5-N-BOC-N-methylaminomethyl-1,2,4-oxadiazol-3-yl)-9H-imidazo[1,5-a]pyrrolo[2,1-c][1,4]-benzodiazepin-9-on, das ohne weitere Reinigung in die nächste Stufe eingesetzt wurde.

b) 3.6 g (7.4 mMol) rohes (S)-8-Chlor-11,12,13,13a-tetrahydro-1-(5-N-BOC-N-methylaminomethyl-1,2,4-oxadiazol-3-yl)-9H-imidazo[1,5-a]pyrrolo-[2,1-c][1,4]benzodiazepin-9-on wurden während 1 Stunde in 25 ml Trifluoressigsäure bei Raumtemperatur gerührt. Die Lösung wurde eingeengt, der Rückstand wurde in Wasser aufgenommen, und die wässerige Lösung wurde zweimal mit Methylenchlorid gewaschen. Man stellte die wässrige Phase mit 25%igem Ammoniak alkalisch und extrahierte sie fünfmal mit Methylenchlorid. Durch Trocknung und Eindampfen der vereinigten organischen Phasen erhielt man 2.47 g (87%) (S)-8-Chlor-11,12,13,13a-tetrahydro-1-(5-methylaminomethyl-1,2,4-oxadiazol-3-yl)-9H-imidazo[1,5-a]pyrrolo[2,1-c][1,4]-benzodiazepin-9-on vom Smp. 161-163°.

Beispiel 18

2.19 g (5.7 mMol) (S)-8-Chlor-11,12,13,13a-tetrahydro-1-(5-methylaminomethyl-1,2,4-oxadiazol-3-yl)-9H-imidazo[1,5-a]pyrrolo[2,1-c][1,4]benzodiazepin-9-on, 20 ml N,N-Dimethylformamid, 1.07 g (7 mMol) 1,8-Diazabicyclo[5.4.0]undec-7-en-(1,5-5) und 850 mg (7 mMol) Allylbromid wurden während 4.5 Stunden bei 60° gerührt. Das Reaktionsgemisch wurde eingeengt, und der Rückstand wurde durch Chromatographieren an Kieselgel unter Eluieren mit Methylenchlorid/Methanol 19/1 gereinigt. Man erhielt 1.32 g (54%) (S)-1-(5-N-Allyl-N-methylaminomethyl-1,2,4-oxadiazol-3-yl)-8-chlor-11,12,13,13a-tetrahydro-9H-imidazo[1,5-a]pyrrolo[2,1-c][1,4]-benzodiazepin-9-on, das in das Hydrochlorid übergeführt wurde.

Beispiel 19

a) 7.47 g (16.35 mMol) (S)-1-(5-BOC-Aminomethyl-1,2,4-oxadiazol-3-yl)-8-chlor-12,12a-dihydro-9H,11H-azeto[2,1-c]imidazo[1,5-a][1,4]benzodiazepin-9-on, 20 ml N,N-Dimethylformamid, 800 mg Natriumhydrid und 4,34 g (36 mMol) Allylbromid wurden über Nacht bei 65° gerührt. Nach Eindampfen des Lösungsmittels wurde der Rückstand in Methylenchlorid aufgenommen, worauf die Lösung zweimal mit Wasser gewaschen, getrocknet und eingedampft wurde. Durch Chromatographieren des Rückstandes an Kieselgel unter Eluieren mit Methylenchlorid/Methanol 19/1 erhielt man 2.08 g (25%) (S)-1-(5-N-Allyl-N-BOC-aminomethyl-1,2,4-oxadiazol-3-yl)-8-Chlor-12,12a-dihydro-9H,11H-azeto[2,1-c]imidazo[1,5-a]-[1,4]benzodiazepin-9-on, das ohne weitere Reinigung in die nächste Stufe eingesetzt wurde.

b) 2.08 g (4.2 mMol) rohes (S)-1-(5-N-Allyl-N-BOC-aminomethyl-1,2,4-oxadiazol-3-yl)-8-chlor-12,12a-dihydro-9H, 11H-azeto[2,1-c]imidazo[1,5-a][1,4]-benzodiazepin-9-on wurden während 3/4 Stunden in 10 ml Trifluoressigsäure gerührt. Nach Eindampfen des Reaktionsgemisches wurde der Rückstand in Methylenchlorid gelöst, worauf die Lösung mit gesättigter Natriumbicarbonat-lösung und mit Wasser gewaschen, über Magnesium-sulfat getrocknet und eingeengt wurde. Der Rückstand wurde durch Chromatographieren an Kieselgel unter Eluieren mit Methylenchlorid/ Methanol 19/1 gereinigt. Man erhielt 0.75 g (48%) (S)-1-(5-Allylaminomethyl-1,2,4-oxadiazol-3-yl)-8-Chlor-12,12a-dihydro-9H,11H-azeto[2,1-c]imidazo-[1,5-a][1,4]benzodiazepin-9-on, das in das Hydrochlorid vom Smp. 227-228° überführt wurde.

Beispiel 20

36.5 g (235 mMol) Diallylglycin wurden in 165 ml N,N-Dimethylformamid gelöst und portionenweise mit 40.5 g (250 mMol) 1,1'-Carbonyldiimidazol versetzt. Nach 10minütigem Rühren gab man 40 g (126mMol) (S)-8-Chlor-12,12a-dihydro-9-oxo-9H,11H-azeto[2,1-c]imidazo[1,5-a][1,4]benzodiazepin-1-carboxamidoxim zu und rührte während 1 Stunde bei Ramtemperatur und 3.5 Stunden bei 110°. Das Reaktionsgemisch wurde eingeengt, und der Rückstand wurde durch Chromatographieren an Kieselgel unter Eluieren mit Methylenchlorid/Essigester 1/1 gereinigt. Man erhielt 27.1 g (45%) (S)-1-(5-Diallylaminomethyl-1,2,4-oxadiazol-3-yl)-8-chlor-12,12a-dihydro-9H,11H-azeto[2,1-c]imidazo[1,5-a][1,4]benzodiazepin-9-on, das in das Hydrochlorid vom Smp. 181-182.5° überführt wurde.

Beispiel 21

2.70 g (7.2 mMol) (S)-1-(5-Aminomethyl-1,2,4-oxadiazol-3-yl)-8-chlor-7-fluor-12,12a-dihydro-9H,11H-azeto [2,1-c]imidazo[1,5-a][1,4]benzodiazepin-9-on, 35 ml N,N-Dimethylformamid, 3.39 g (22.2 mMol) 1,8-Diazabicyclo-[5.4.0]undec-7-en(1,5-5) und 2.61 g (21.6 mMol) Allylbromid wurden während 20 Stunden bei Raumtemperatur gerührt. Nach Eindampfen des Reaktionsgemisches nahm man den Rückstand in Methylenchlorid auf, wusch die Lösung dreimal mit Wasser, trocknete sie über Magnesiumsulfat und dampfte sie ein. Der Rückstand wurde an 300 g Kieselgel unter Eluieren mit Methylenchlorid/Methanol 19/1 chromatographiert . Die einheitlichen Fraktionen mit dem $R_f$ = 0.19 wurden eingeengt. Man erhielt 1.21 g (37%) (S)-1-(5-Allylaminomethyl-1,2,4-oxadiazol-3-yl)-8-chlor-7-fluor-12,12a-dihydro-9H,11H-azeto[2,1-c]imidazo[1,5-a][1,4]benzo-diazepin-9-on, das in das Hydrochlorid vom Zersetzungspunkt 190° überführt wurde.

Beispiel 22

a) 25.4 g (100 mMol) (S)-5-Chlor-6-fluor-1,10a-dihydro-2H-azeto[2,1-c]-[1,4]benzodiazepin-4,10(9H)-dion wurden in 125 ml N,N-Dimethylformamid gelöst, bei -30° mit 4.8 g (110 mMol) Natriumhydrid-Dispersion (55-65% in Oel, mit n-Hexan gewaschen) versetzt und während 40 min bei -30° bis -18° deprotoniert. Bei -60° gab man eine Lösung von 26.86 g (100 mMol) Phosphorsäurediphenylesterchlorid in 5 ml N,N-Dimethylformamid zu und rührte während 35 min bei max. -45°. Inzwischen wurden separat 12.3 g (110 mMol) Kalium-tert.-butylat in 30 ml N,N-Dimethylformamid gelöst und bei -60° mit 12.2 g (107 mMol) Isocyanessigsäure-aethylester versetzt. Man kühlte den deprotonierten Isocyanessigsäure-aethylester auf -70° ab und tropfte mit einem mit Trockeneis gekühlten Tropftrichter das Reaktionsgemisch bei max. -65° innerhalb von 5/4 Stunden zu. Man rührte während 1 Stunde im Aceton/Trockeneisbad weiter, neutralisierte mit 12 ml Essigsäure und goss auf 500 ml Eiswasser. Man extrahierte fünfmal mit Methylenchlorid (insgesamt 1.2 l), trocknete über Magnesiumsulfat und dampfte zur Trockene ein. Durch Chromatographieren des Rückstandes an 1.5 kg Kieselgel unter Eluieren mit Essigester erhielt man 14.4 g (41%) Aethyl (S)-8-chlor-7-fluor-12,12a-dihydro-9-oxo-9H,11H-azeto[2,1-c]imidazo-[1,5-a][1,4]-benzodiazepin-1-carboxylat vom Smp. 161-163°.

b) 57.3 g (164 mMol) Aethyl (S)-8-chlor-7-fluor-12,12a-dihydro-9-oxo-9H,11H-azeto[2,1-c]imidazo[1,5-a][1,4]benzodiazepin-1-carboxylat, 40 ml Ethanol, 60 ml Wasser und 51.5 ml (206 mMol) 4N-Natronlauge wurden während 30 min auf dem Dampfbad zum Rückfluss erhitzt. Man dampfte den Alkohol am Rotationsverdampfer ab. Die verbleibende wässrige Phase wurde zweimal mit Methylenchlorid gewaschen und mit 51.5 ml (206 mMol) 4N-Salzsäure auf pH 3-4 angesäuert. Die erhaltene Suspension wurde gekühlt und filtriert, und der Filterrückstand wurde mit wenig Eiswasser gewaschen und getrocknet. Man erhielt 47.92 g (91%) (S)-8-Chlor-7-fluor-12,12a-dihydro-9-oxo-9H,11H-azeto[2,1-c]imidazo[1,5-a][1,4]benzodiazepin-1-carbonsäure vom Smp. 225-226°.

c) 40 g (124 mMol) (S)-8-Chlor-7-fluor-12,12a-dihydro-9-oxo-9H,11H-azeto[2,1-c]-imidazo[1,5-a][1,4]benzodiazepin-1-carbonsäure wurden in 190 ml N,N-Dimethylformamid suspendiert und bei Raumtemperatur portionenweise mit 21 g (129.5 mMol) 1,1'-Carbonyldiimidazol versetzt. Nach Beendigung der $CO_2$-Abspaltung wurde die klare braune Lösung während 30 min bei 50° gerührt, gekühlt und bei einer Temperatur unter 25° innerhalb von ca 10 min tropfenweise mit 30 ml konz. Ammoniak versetzt. Nach 30-minütigem Rühren wurde die erhaltene Suspension auf 700 ml Eiswasser gegossen, 30 min bei Raumtemperatur gerührt und filtriert, worauf die Kristalle mit wenig Wasser nachgewaschen wurden. Nach Trocknung erhielt man 31.36 g (78%) (S)-8-Chlor-7-fluor-12,12a-dihydro-9-oxo-9H,11H-azeto[2,1-c]imidazo[1,5-a][1,4]benzodiazepin-1-carboxamid vom Smp. 296-298°.

d) 33.67 g (105 mMol) (S)-8-Chlor-7-fluor-12,12a-dihydro-9-oxo-9H,11H-azeto[2,1-c]imidazo[1,5-a][1,4]benzodiazepin-1-carboxamid wurden in 140 ml Dioxan und 18 ml Pyridin suspendiert und bei einer Temperatur von < 8° innerhalb von 30 min tropfenweise mit 22.6 g (107.6 mMol) Trifluoressigsäure-anhydrid versetzt. Man rührte während 2,5 Stunden bei 50° und goss auf 700 ml Wasser. Man filtrierte die Suspension und erhielt nach Trocknung des Rückstandes 28.62 g (90%) (S)-8-Chlor-7-fluor-12,12a-dihydro-9-oxo-9H,11H-azeto[2,1-c]imidazo[1,5-a][1,4] benzodiazepin-1-carbonitril vom Smp. 225-228°.

e) 3.1 g (134.8 mMol) Natrium wurden in 140 ml Methanol gelöst. Bei Raumtemperatur gab man aufeinanderfolgend 10 g (145 mMol) Hydroxylaminhydrochlorid und 28.6 g (94.5 mMol) (S)-8-Chlor-7-fluor-12,12a-dihydro-9-oxo-9H,11H-azeto[2,1-c]imidazo[1,5-a][1,4]benzodiazepin-1-carbonitril zu. Man rührte die Suspension über Nacht bei Raumtemperatur, kühlte sie während 30 min auf 0° ab, filtrierte die Kristalle ab, schlämmte sie in 50 ml Wasser auf und filtrierte sie ab. Die Methanol-Lösung wurde eingeengt, worauf man den Rückstand in 30 ml Wasser aufschlämmte und die Kristalle abfiltrierte. Durch Trocknung der vereinigten Kristallisate erhielt man 30.94 g (97%) (S)-8-Chlor-7-fluor-12,12a-dihydro-9-oxo-9H,11H-azeto[2,1-c]-imidazo[1,5-a][1,4]benzodiazepin-1-carboxamidoxim vom Smp. 236-238°.

f) 17.08 g ( 97mMol) BOC-Glycin wurden in 165 ml N,N-Dimethylformamid gelöst und portionenweise mit 16.9 g (104 mMol) 1,1'-Carbonyldiimidazol versetzt. Nach Beendigung der $CO_2$-Abspaltung wurde die Lösung während 30 min bei 50° gerührt. Man gab dann 30.8 g (91 mMol) (S)-8-Chlor-7-fluor-12,12a-dihydro-9-oxo-9H,11H-azeto [2,1-c]imidazo[1,5-a][1,4]-benzodiazepin-1-carboxamidoxim zu und rührte über Nacht bei 90°. Durch Eindampfen der Lösung und Chromatographieren des Rückstandes an 1.5 kg Kieselgel unter Eluieren mit Methylenchlorid/ Methanol 19/1 erhielt man 36.8 g (84%) (S)-1-[5-(BOC-Aminomethyl)-1,2,4-oxadiazol-3-yl]-8-chlor-7-fluor-12,12a-dihydro-9H,11H-azeto[2,1-c]-imidazo[1,5-a][1,4]benzodiazepin-9-on, das ohne weitere Reinigung in die nächste Stufe eingesetzt wurde.

g) 36.8 g (77.5 mMol) rohes (S)-1-[5-(BOC-Aminomethyl)-1,2,4-oxadiazol-3-yl]-8-chlor-7-fluor-12,12a-dihydro-9H, 11H-azeto[2,1-c]imidazo[1,5-a][1,4]-benzodiazepin-9-on und 90 ml Trifluoressigsäure wurden während 2 Stunden bei Raumtemperatur gerührt. Die Lösung wurde eingedampft, der Rückstand wurde in Wasser gelöst, und die wässerige Lösung wurde dreimal mit Methylenchlorid gewaschen. Die wässrige Phase wurde mit konz. Ammoniak alkalisch gestellt und achtmal mit Methylenchlorid (insgesamt ca 11) extrahiert. Durch Eindampfen der vereinigten und über Magnesiumsulfat getrockneten organischen Phasen erhielt man 23.8 g (82%) (S)-1-(5-Aminomethyl-1,2,4-oxadiazol-3-yl)-8-chlor-7-fluor-12,12a-dihydro-9H,11H-azeto[2,1-c]imidazo[1,5-a][1,4]benzodiazepin-9-on, das ohne weitere Reinigung als Ausgangsprodukt für das nachstehend beschriebene Beispiel verwendet wurde.

Beispiel 23

26.6 g (71 mMol) rohes (S)-1-(5-Aminomethyl-1,2,4-oxadiazol-3-yl)-8-chlor-7-fluor-12,12a-dihydro-9H,11H-azeto[2,1-c]imidazo[1,5-a][1,4]benzodiazepin-9-on, 400 ml Methylenchlorid, 85 ml (496 mMol) N-Aethyldiisopropylamin und 34.5 g (285 mMol) Allylbromid wurden während 20 Stunden bei Raumtemperatur gerührt. Man wusch die Reaktionslösung dreimal mit Wasser, trocknete sie über Magnesiumsulfat und dampfte sie ein. Der Rückstand wurde an 2 kg Kieselgel unter Eluieren mit Essigester chromatographiert. Die einheitlichen Fraktionen wurden eingedampft und aus Toluol und n-Hexan umkristallisiert. Man erhielt 22.34 g (69%) (S)-1-(5-Diallylaminomethyl-1,2,4-oxadiazol-3-yl)-8-chlor-7-fluor-12,12a-dihydro-9H,11H-azeto[2,1-c]imidazo[1,5-a][1,4]benzodiazepin-9-on vom Smp. 103-104°.

Beispiel 24

a) 4.16 g (23.6 mMol) BOC-Glycin wurden in 30 ml N,N-Dimethylformamid gelöst und portionenweise mit 4.08 g (25.2 mMol) 1,1'-Carbonyldiimidazol versetzt. Nach Beendigung der $CO_2$-Abspaltung wurde die Lösung während 20 min bei 50° gerührt. Man gab dann 7.36 g (22.2 mMol) (S)-8-Chlor-11,12,13,13a-tetrahydro-9-oxo-9H-imidazo [1,5-a]pyrrolo[2,1-c]-[1,4]benzodiazepin-1-carboxamidoxim zu und rührte über Nacht bei 90°. Man dampfte das Reaktionsgemisch ein, löste den Rückstand in Methylenchlorid und wusch die Lösung einmal mit Wasser und einmal mit gesättigter Natriumbicarbonatlösung. Nach Trocknung und Einengen erhielt man 7.75 g (74%) (S)-1-(5-BOC-Aminomethyl-1,2,4-oxadiazol-3-yl)-8-chlor-11,12,13,13a-tetrahydro-9H-imidazo[1,5-a]pyrrolo[2,1-c][1,4] benzodiazepin-9-on, das ohne weitere Reinigung in die nächste Stufe eingesetzt wurde.

b) 7.75 g (16.5 mMol) rohes (S)-1-(5-BOC-Aminomethyl-1,2,4-oxadiazol-3-yl)-8-chlor-11,12,13,13a-tetrahydro-9H-imidazo[1,5-a]pyrrolo[2,1-c][1,4)-benzodiazepin-9-on wurden während 1.5 Stunden in 25 ml Trifluoressigsäure bei Raumtemperatur gerührt. Die Lösung wurde eingeengt, der Rückstand wurde in gesättigter Natriumbicarbonatlösung aufgenommen, und die Lösung wurde zehnmal mit Methylenchlorid extrahiert. Durch Trocknung und Eindampfen der vereinigten organischen Phasen erhielt man 5.53 g (90%) (S)-1-(5-Aminomethyl-1,2,4-oxadiazol-3-yl)-8-chlor-11,12,13,13a-tetrahydro-9H-imidazo[1,5-a]pyrrolo[2,1-c][1,4]-benzodiazepin-9-on, das ohne weitere Reinigung als Ausgangsprodukt für das nachstehend beschriebene Beispiel verwendet wurde.

Beispiel 25

2.47 g (6.7 mMol) rohes (S)-1-(5-Aminomethyl-1,2,4-oxadiazol-3-yl)-8-chlor-11,12,13,13a-tetrahydro-9H-imidazo[1,5-a]pyrrolo[2,1-c][1,4]benzodiazepin-9-on, 20 ml N,N-Dimethylformamid, 9 ml (60 mMol) 1,8-Diazabicyclo [5.4.0]undec-7-en(1,5-5) und 10.26 g (54 mMol) Benzylbromid wurden über Nacht bei Raumtemperatur und während 6 Stunden bei 50° gerührt. Das Reaktionsgemisch wurde eingeengt, und der Rückstand wurde durch Chromatographieren an Kieselgel unter Eluieren mit Methylenchlorid/Methanol 19/1 gereinigt. Nach Umkristallisieren aus Essigester und Hexan erhielt man 2.16 g (58%) (S)-1-(5-Dibenzylaminomethyl-1,2,4-oxadiazol-3-yl)-8-chlor-11,12,13,13a-tetrahydro-9H-imidazo-[1,5-a]pyrrolo[2,1-c][1,4]-benzodiazepin-9-on vom Smp. 107-109°.

Beispiel 26

3.0 g (8.1 mMol) (S)-1-(5-Aminomethyl-1,2,4-oxadiazol-3-yl)-8-chlor-11,12,13,13a-tetrahydro-9H-imidazo [1,5-a]pyrrolo[2,1-c][1,4]benzodiazepin-9-on, 20 ml N,N-Dimethylformamid, 3 ml (20 mMol) 1,8-Diazabicyclo [5.4.0] undec-7-en(1,5-5) und 6.54 g (54 mMol) Allylbromid wurden über Nacht bei 40° gerührt. Das Reaktionsgemisch wurde eingeengt, der Rückstand wurde in Methylenchlorid gelöst, und die Lösung wurde mit Wasser gewaschen. Nach Trocknung wurde das Produkt durch Chromatographie an Kieselgel unter Eluieren mit Methylenchlorid/ Methanol 19/1 gereinigt. Man erhielt 1.8 g (49%) (S)-1-(5-Diallylaminomethyl-1,2,4-oxadiazol-3-yl)-8-chlor-11,12,13,13a-tetrahydro-9H-imidazo[1,5-a]-pyrrolo[2,1-c][1,4]-benzodiazepin-9-on, das in das Hydrochlorid überführt wurde.

Beispiel 27

a) 6.68 g (22 mMol) (S)-8-Chlor-12,12a-dihydro-9-oxo-9H,11H-azeto[2,1-c]-imidazo[1,5-a][1,4]benzodiazepin-1-carbonsäure wurden in 70 ml N,N-Dimethylformamid gelöst, bei 40° portionenweise mit 3.84 g (24 mMol) 1,1'-Carbonyldiimidazol versetzt und während 30 min bei dieser Temperatur gerührt. Nach Zugabe von 5.78 g (26 mMol) Phthaloylglycinamidoxim liess man während 2.5 Stunden bei 60° und während 18 Stunden bei 110° rühren und engte das Reaktionsgemisch ein. Durch Chromatographieren des Rückstandes an Kieselgel unter Eluieren mit Essigester erhielt man 6.01 g (56%) (S)-8-Chlor-12,12a-dihydro-1-(3-phthalimidomethyl-1,2,4-oxadiazol-5-yl)-9H,11H-azeto[2,1-c]imidazo[1,5-a][1,4]benzodiazepin-9-on vom Smp. 189-192°.

b) 6.01 g (12.3 mMol) (S)-8-Chlor-12,12a-dihydro-1-(3-phthalimidomethyl-1,2,4-oxadiazol-5-yl)-9H,11H-azeto [2,1-c]imidazo[1,5-a][1,4]benzodiazepin-9-on wurden in 60 ml Aethanol vorgelegt und bei 60° innerhalb von 30 min tropfenweise mit 120 ml Methylamin (33%ig in Aethanol) versetzt. Die Lösung wurde während 2 Stunden bei 70° gerührt und anschliessend eingeengt. Durch Chromatographieren des Rückstandes an Kieselgel unter Eluieren mit Essigester/Methanol 8/2 erhielt man 4.0 g (91%) (S)-1-(3-Aminomethyl-1,2,4-oxadiazol-5-yl)-8-chlor-12,12a-dihydro-9H,11H-azeto[2,1-c]-imidazo[1,5-a][1,4]benzodiazepin-9-on vom Smp. 206-208°.

Beispiel 28

5.02 g (14 mMol) (S)-1-(3-Aminomethyl-1,2,4-oxadiazol-5-yl)-8-chlor-12,12a-dihydro-9H,11H-azeto[2,1-c] imidazo[1,5-a][1,4]benzodiazepin-9-on, 75 ml N,N-Dimethylformamid, 21 g (173.8 mMol) Allylbromid und 29.4 g (193 mMol) 1,8-Diazabicyclo[5.4.0]undec-7-en(1,5-5) wurden während 60 Stunden bei 75° gerührt. Die Lösung wurde eingeengt, und der Rückstand wurde durch Chromatographieren an 350 g Kieselgel unter Eluieren mit Essigester gereinigt. Man erhielt 1.97 g (35%) (S)-1-(3-Diallylaminomethyl-1,2,4-oxadiazol-5-yl)-8-chlor-12,12a-dihydro-9H,11H-azeto [2,1-c]imidazo[1,5-a]-[1,4]benzodiazepin-9-on, das in das Hydrochlorid vom Smp. 190-192° überführt wurde.

Beispiel 29

a) 0.60 g (26 mMol) Natrium wurden in 32 ml Methanol gelöst. Bei Raumtemperatur gab man 1.95 g (28.1 mMol) Hydroxylaminhydrochlorid und nach 1 Stunde 5.77g (346 mMol) (S)-7-Fluor-12,12a-dihydro-9-oxo-9H,11H-azeto [2,1-c]-imidazo[1,5-a][1,4]benzodiazepin-1-carbonitril zu. Man rührte die Suspension während 3.5 Stunden bei 70°, kühlte sie während 30 min auf 0° ab und filtrierte die Kristalle ab. Durch Trocknung des Kristallisats erhielt man 6.5 g (100%) (S)-7-Fluor-12,12a-dihydro-9-oxo-9H,11H-azeto[2,1-c]imidazo[1,5-a][1,4]benzodiazepin-1-carboxamidoxim vom Smp.248-250°.

b) 2.32 g (15 mMol) Diallylglycin wurden in 15 ml N,N-Dimethylformamid gelöst und mit 2.75 g (17 mMol) 1.1'-Carbonyldiimidazol versetzt. Nach 20-minütigem Rühren bei 50°C gab man 3.01 (10 mMol) (S)-7-Fluor-12,12a-dihydro-9-oxo-9H,11H-azeto[2,1-c]imidazo[1,5-a][1,4]benzodiazepin-1-carboxamidoxim zu und rührte während 16 Stunden bei 90° und während 2 Stunden bei 120°. Die Lösung wurde eingeengt, und der Rückstand wurde durch Chromatographieren an 320 g Kieselgel unter Eluieren mit Essigester gereinigt. Man erhielt 1.54 g (37%) (S)-1-(5-Diallylaminomethyl-1,2,4-oxadiazol-3-yl)-7-fluor-12,12a-dihydro-9H,11H-azeto[2,1-c]-imidazo[1,5-a]-[1,4]benzodiazepin-9-on, das in das Hydrochlorid vom Smp.100-105° überführt wurde.

Beispiel 30

a) 8 g (23.7 mMol) (S)-8-Trifluormethyl-12,12a-dihydro-9-oxo-9H,11H-azeto[2,1-c]imidazo[1,5-a][1,4]benzodiazepin-1-carbonsäure wurden in 50 ml N,N-Dimethylformamid gelöst, portionenweise mit 4.06 g (25 mMol) 1,1'-Carbonyldiimidazol versetzt und während 30 min bei 55° gerührt. Nach Zugabe von 5.26 g (24 mMol) Phthaloylglycinamidoxim liess man während 20 Stunden bei 105° rühren. Man dampfte das Reaktionsgemisch ein und chromatographierte den Rückstand an Kieselgel unter Eluieren mit Essigester. Man erhielt 5.34 g (43%) (S)-8-Trifluormethyl-12,12a-dihydro-1-(3-phthalimidomethyl-1,2,4-oxadiazol-5-yl)-9H,11H-azeto[2,1-c]imidazo[1,5-a]-[1,4] benzodiazepin-9-on vom Smp.243-245°.

b) 5.3 g (10.2 mMol) (S)-8-Trifluormethyl-12,12a-dihydro-1-(3-phthalimidomethyl-1,2,4-oxadiazol-5-yl)-9H,11H-azeto[2,1-c]imidazo[1,5-a]-[1,4]benzodiazepin-9-on in 70 ml Aethanol wurden bei 60° innerhalb 45 min tropfenweise mit 150 ml Methylamin (33%ig in Aethanol) versetzt. Die Lösung wurde während 2 Stunden bei 70° gerührt und anschliessend eingeengt. Der Rückstand wurde in Methylenchlorid und 30 ml 4 N Salzsäure aufgenommen, und die Lösung wurde dreimal mit Methylenchlorid gewaschen. Man stellte die wässrige Phase mit 30 ml 4 N Natronlauge alkalisch und extrahierte fünfmal mit Methylenchlorid. Nach Trocknung der vereinigten organischen Lösungen und Abdampfen des Lösungsmittels erhielt man 3.98 g (100%) (S)-1-(3-Aminomethyl-1,2,4-oxadiazol-5-yl)-8-trifluormethyl-12,12a-dihydro-9H,11H-azeto[2,1-c]-imidazo[1,5-a][1,4]benzo-diazepin-9-on das ohne weitere Reinigung als Ausgangsprodukt für das nachstehend beschriebene Beispiel verwendet wurde.

Beispiel 31

2 g (5.1 mMol) rohes (S)-1-(3-Aminomethyl-1,2,4-oxadiazol-5-yl)-8-trifluormethyl-12,12a-dihydro-9H,11H-azeto[2,1-c]imidazo[1,5-a][1,4]benzodiazepin-9-on, 30 ml Methylenchlorid, 6.2 ml (36 mMol) N-Aethyldiisopropylamin und 2.57 g (1.8 mMol) Allylbromid wurden während 18 Stunden bei Raumtemperatur gerührt. Man wusch die Reaktionslösung dreimal mit Wasser, trocknete sie über Magnesiumsulfat und dampfte sie ein. Der Rückstand wurde an 300

g Kieselgel unter Eluieren mit Essigester chromatographiert. Man erhielt 1.62 g (67%) (S)-1-(3-Diallylaminomethyl-1,2,4-oxadiazol-5-yl)-8-trifluormethyl-12,12a-dihydro-9H,11H-azeto[2,1-c]-imidazo[1,5-a][1,4]benzodiazepin-9-on, das in das Hydrochlorid vom Smp.147-150° überführt wurde.

Beispiel 32

3.56 g (10mMol) (S)-1-(5-Aminomethyl-1,2,4-oxadiazol-3-yl)-8-chlor-12,12a-dihydro-9H,11H-azeto[2,1-c]imidazo[1,5-a][1,4]benzodiazepin-9-on, 15 ml Methylenchlorid, 8.6 ml (50 mMol) N-Aethyldiisopropylamin und 2.64 g (10 mMol) α,α'-Dibrom-o-xylol wurden während 20 Stunden bei Raumtemperatur gerührt. Man wusch die Reaktionslösung dreimal mit Wasser, trocknete sie über Magnesiumsulfat und dampfte sie ein. Der Rückstand wurde an 500 g Kieselgel unter Eluieren mit Methylenchlorid/ Methanol 19/1 chromatographiert . Man erhielt 1.55 g (33%) (S)-8-Chlor-12,12a-dihydro-1-(5-isoindolin-2-ylmethyl-1,2,4-oxadiazol-3-yl)-9H,11H-azeto[2,1-c]imidazo[1,5-a][1,4]benzodiazepin-9-on, das in das Hydrochlorid vom Smp.218-222° überführt wurde.

Beispiel 33

473 mg (1.1 mMol) (S)-1-(5-Diallylaminomethyl-1,2,4-oxadiazol-3-yl)-8-chlor-12,12a-dihydro-9H,11H-azeto[2,1-c]imidazo[1,5-a][1,4]benzodiazepin-9-on wurden in 10 ml Essigester in Gegenwart von 20 mg 5%iger Palladiumkohle bei Raumtemperatur und Normaldruck hydriert. Nach Abtrennen des Katalysators wurde die Lösung eingeengt. Man erhielt 0.42 g (80%) (S)-8-Chlor-1-(5-di-propylaminomethyl-1,2,4-oxadiazol-3-yl)-12,12a-dihydro-9H,11H-azeto[2,1-c]imidazo[1,5-a][1,4]benzodiazepin-9-on, das in das Hydrochlorid vom Smp.147-153° überführt wurde.

Beispiel 34

2,27 g (5mMol) (S)-1-(5-Diallylaminomethyl-1,2,4-oxadiazol-3-yl)-8-chlor-7-fluor-12,12a-dihydro-9H,11H-azeto[2,1-c]imidazo[1,5-a)[1,4]benzodiazepin-9-on wurden in 80 ml Essigester in Gegenwart von 35 mg 5%iger Palladiumkohle bei Raumtemperatur und Normaldruck hydriert. Nach Abtrennen des Katalysators wurde das Reaktionsgemisch durch Chromatographieren an Kieselgel unter Eluieren mit Essigester gereinigt. Man erhielt 1.83 g (80%) (S)-8-Chlor-7-fluor-12,12a-dihydro-1-(5-di-propylaminomethyl-1,2,4-oxadiazol-3-yl)-9H,11H-azeto[2,1-c]imidazo[1,5-a]-[1,4]benzodiazepin-9-on, das in das Hydrochlorid überführt wurde.

Beispiel 35

a) Zu einer auf -70° gekühlten Lösung von 57.2 g (250 mmol) N-(tert.-Butoxycarbonyl)-3,4-difluoranilin in 500 ml Tetrahydrofuran wurden innerhalb einer 1 Stunde 400 ml (600 mmol) tert. Butyllithium (1,5 M in Pentan) zugetropft. Anschliessend gab man zu der gelben Suspension 160 g Trockeneis in kleinen Portionen, liess auf 0° erwärmen und tropfte 400 ml Wasser zu. Das Tetrahydrofuran und das Pentan wurden abdestilliert und die Wasserphase wurde zweimal mit Aether gewaschen und anschliessend mit konz. Salzsäure auf pH=1 gestellt. Die saure Wasserphase wurde dreimal mit Methylenchlorid extrahiert; die organische Phase wurde über Natriumsulfat getrocknet, filtriert und eingedampft. Der erhaltene beige Feststoff wurde aus Aethylenchlorid umkristallisiert, und man erhielt 52 g (76%) 2-(tert.-Butoxycarbonyl)amino-5,6-difluorobenzoesäure als farblose Nadeln vom Smp.159.5 - 160.5°.

b) Zu einer Lösung von 100 g (366 mmol) 2-(tert.-Butoxycarbonyl)-amino-5,6-difluorobenzoesäure in 1.5 l l trockenem Tetrahydrofuran wurde unter Eiskühlung eine Lösung von 108 ml Thionylchlorid in 300 ml Tetrahydrofuran zugetropft, worauf 16 Stunden bei Raumtemperatur gerührt wurde. Die braune Lösung wurde eingedampft, und der erhaltene braune Feststoff wurde mit Methylenchlorid verrührt. Das erhaltene beige Pulver wurde abfiltriert und am Hochvakuum getrocknet. Man erhielt 56.5 g (77 %) 5,6-Difluoro-2,4-dihydro-1H-3,1-benzoxazin-2,4-dion als beiges Pulver vom Smp. > 240°.

c) Eine Lösung von 19.6 g (98.4 mmol) 5,6-Difluoro-2,4-dihydro-1H-3,1-benzoxazin-2,4-dion und 9.95 g ( 98.4 mmol) L-Azetidin-2-carbonsäure in 125 ml Dimethylformamid und 25 ml Essigsäure wurde bei 120° während 16 Stunden gerührt. Die braune Lösung wurde eingedampft, und der erhaltene braune Rückstand wurde aus Aethanol kristallisiert. Man erhielt 16 g (68%) (S)-5,6-Difluoro-1,2,4,9,10,10a-hexahydro-azeto[2,1-c] [1,4]benzodiazepin-4,10-dion als farblose Nadeln vom Smp. > 250°.

d) Zu einer Suspension von 2.7 g (62.3 mmol) NaH (55%, gewaschen mit Hexan) in 5 ml Dimethylformamid wurde

bei -30° eine Lösung von 13.5 g (56.7 mmol) (S)-5,6-Difluoro-1,2,4,9,10,10a-hexahydro-azeto[2,1-c][1,4]-benzodiazepin-4,10-dion in 65 ml Dimethylformamid zugetropft und während 40 Minuten bei -30° gerührt. Nach Abkühlen auf -60° wurde eine Lösung von 12.1 ml (56.7 mmol) Phosphorsäure-diphenylesterchlorid in 3 ml Dimethylformamid so zugetropft, dass die Temperatur nicht über -45° stieg. Anschliessend rührte man noch 30 Minuten nach.

Unterdessen wurden 7.0 g (62.3 mmol) Kalium-tert.butylat in 20 ml Dimethylformamid gelöst und bei -60° mit 7 ml (60.6 mmol) Isocyanessigsäureäthylester (95%) versetzt. Zu der so erhaltenen Lösung wurde bei -70° das oben erhaltene Reaktionsgemisch via einen auf -40° gekühlten Tropftrichter zugetropft. Die so erhaltene dunkelbraune zähe Lösung wurde 1 Stunde bei -60° nachgerührt und nach Neutralisation mit 7 ml Essigsäure bei -40° auf 300 ml Eiswasser gegossen, worauf fünf Mal mit Methylenchlorid extrahiert wurde. Die vereinigten organischen Phasen wurden über Natriumsulfat getrocknet, filtriert und eingedampft. Der erhaltene hellbraune Rückstand wurde aus Aethanol umkristallisiert. Man erhielt 8.9 g (47%) (S)-7,8-Difluoro-9-oxo-12,12a-dihydro-9H,11H-azeto [2,1-c]imidazo-[1,5-a][1,4]benzodiazepin-1-carbonsäureäthylester als farblose Nadeln vom Smp. 233.5-235.5°.

e) Zu einer Suspension von 8.8 g (26.4 mmol) (S)-7,8-Difluoro-9-oxo-12,12a-dihydro-9H,11H-azeto[2,1-c]imidazo [1,5-a][1,4]benzo-diazepin-1-carbonsäureäthylester in 20 ml Aethanol und 30 ml Wasser wurden 8.6 ml (34.3 mmol) 4N Natronlauge zugetropft, und das Gemisch wurde 30 Minuten am Rückfluss erhitzt. Anschliessend wurde das Aethanol abdestilliert. Die Wasserphase wurde zwei Mal mit Methylenchlorid gewaschen und mit 4N Salzsäure auf pH=3 gestellt. Extraktion mit Methylenchlorid (fünf Mal), Trocknen über Natriumsulfat, Filtrieren und Eindampfen lieferten 7.2 g (89%) (S)-7,8-Difluoro-9-oxo-12,12a-dihydro-9H,11H-azeto[2,1-c]imidazo[1,5-a][1,4]benzodiazepin-1-carbonsäure als farblose Nadeln vom Smp. 218.0-219.5° (Zers.).

f) Zu einer Suspension von 7.2 g (23.6 mmol) (S)-7,8-Difluoro-9-oxo-12,12a-dihydro-9H,11H-azeto[2,1-c]imidazo [1,5-a][1,4]benzo-diazepin-1-carbonsäure in 50 ml Dimethylformamid wurden 4.2 g (26 mmol) 1,1'-Carbonyldiimidazol portionenweise zugegeben. Die entstandene hellbraune Lösung erwärmte man während 45 Minuten auf 50°. Anschliessend kühlte man die Lösung auf Raumtemperatur ab und tropfte 6 ml wässerige Ammoniaklösung zu. Nach weiterem 30 minütigem Rühren wurde das Reaktionsgemisch auf 100 ml Eiswasser gegossen und mit Methylenchlorid sieben Mal extrahiert. Trocknen der organischen Phasen über Natriumsulfat, Filtrieren, Eindampfen und anschliessende Chromatographie (Kieselgel, Methylenchlorid/Methanol 19:1) lieferten 7.0 g (97%) (S)-7,8-Difluoro-9-oxo-12,12a-dihydro-9H,11H-azeto[2,1-c]imidazo[1,5-a][1,4]benzodiazepin-1-carbonsäureamid als farbloses Pulver vom Smp. 200.5-204.0°.

g) Zu einer Suspension von 6.3 g (20.7 mmol) (S)-7,8-Difluoro-9-oxo-12,12a-dihydro-9H,11H-azeto[2,1-c]imidazo [1,5-a][1,4]benzo-diazepin-1-carbonsäureamid in 30 ml Dioxan und 5 ml Pyridin wurden bei 5-8° 3 ml (21.7 mmol) Trifluoressigsäureanhydrid zugetropft. Die erhaltene beige Lösung wurde während 2.5 Stunden bei 50° gerührt und anschliessend auf 50 ml Eiswasser gegossen. Extraktion mit Methylenchlorid (vier Mal), Trocknen über Natriumsulfat, Filtrieren und Eindampfen lieferten 4.8 g (81%) (S)-7,8-Difluoro-9-oxo-12,12a-dihydro-9H,11H-azeto [2,1-c]imidazo[1,5-a]-benzodiazepin-1-carbonitril als farbloses Pulver vom Smp.>250°.

h) Zu einer frisch hergestellten Lösung von Natriummethylat in Methanol (aus 750 mg (32.8 mmol) Natrium in 25 ml Methanol) wurden 4.7 g (16.4 mmol) (S)-7,8-Difluoro-9-oxo-12,12a-dihydro-9H,11H-azeto[2,1-c]-imidazo[1,5-a] [1,4]benzodiazepin-1-carbonitril und 2.7 g (36.1 mmol) Hydroxylamin-hydrochlorid zugegeben und 16 Stunden bei Raumtempera-tur gerührt. Anschliessend wurde die Suspension eingedampft, und der Rückstand wurde zwischen Methylenchlorid und Wasser verteilt. Der unlösliche Anteil wurde abfiltriert und am Hochvakuum getrocknet. Die organische Phase wurde über Natriumsulfat getrocknet, filtriert und eingedampft. Der erhaltene Schaum wurde zusammen mit dem unlöslichen Anteil chromatographiert ( Kieselgel, Methylenchlorid/ Methanol 9:1), und man erhielt 4.4 g (84%) (E)- und/oder (Z)-(S)-7-Fluoro-8-methoxy-12,12a-dihydro-9-oxo-9H,11H-azeto[2,1-c]imidazo [1,5-a][1,4]-benzodiazepin-1-carboxamidoxim als farblosen Schaum, Rf=0.33 (Kieselgel, Methylenchlorid/Methanol 9:1).

i) Zu einer Lösung von 2.5 g (14.4 mmol) BOC-Glycin in 25 ml Dimethylformamid wurden 2.5 g (15.4 mmol) 1,1'-Carbonyldiimidazol zugegeben und während 30 Minuten bei 50° gerührt. Anschliessend gab man 4.3 g (13.5 mmol) (E)- und/oder (Z)-(S)-7-Fluoro-8-methoxy-12,12a-dihydro-9-oxo-9H,11H-azeto[2,1-c]imidazo-[1,5-a][1,4] benzodiazepin-1-carboxamidoxim zu und rührte 16 Stunden bei 90°. Die so erhaltene braune Lösung wurde am Hochvakuum eingedampft, und der erhaltene braune Rückstand wurde chromatographiert (Kieselgel, Methylenchlorid /Methanol 19:1). Man erhielt 4.1 g (65%) (S)-1-(5-BOC-Aminomethyl-1,2,4-oxadiazol-3-yl)-7-fluoro-8-methoxy-12,12a-dihydro-9H,11H-azeto[2,1-c]imidazo[1,5-a][1,4]-benzodiazepin-9-on als farblosen Schaum, Rf=0.18 (Kieselgel, Methylenchlorid/Methanol 19:1).

k) Eine Lösung von 4.0 g (8.5 mmol) (S)-1-(5-BOC-Aminomethyl-1,2,4-oxadiazol-3-yl)-7-fluoro-8-methoxy-12,12a-dihydro-9H,11H-azeto[2,1-c]-imidazo[1,5-a][1,4]benzodiazepin-9-on in 10 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 5 ml wässerige Ammoniaklösung basisch gestellt und sechs Mal mit Methylenchlorid extrahiert. Die organischen Phasen wurden über Natriumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 2.3 g (73%) (S)-1-(5-Aminomethyl-1,2,4-oxadiazol-3-yl)-7-fluoro-8-methoxy-12,12a-dihydro-9H,11H-azeto[2,1-c]imidazo[1,5-a][1,4]benzodiazepin-9-on als farblose Kristalle vom Smp.196-198°.

Beispiel 36

Zu einer Lösung von 800 mg (2.16 mmol) (S)-1-(5-Aminomethyl-1,2,4-oxadiazol-3-yl)-7-fluoro-8-methoxy-12,12a-dihydro-9H,11H-azeto[2,1-c]-imidazo[1,5-a][1,4]benzodiazepin-9-on in 12 ml Methylenchlorid wurden 5.1 ml (29.8 mmol) N-Aethyldiisopropylamin und 1.46 ml (17.34 mmol) Allylbromid zugegeben. Die Reaktionslösung wurde 20 Stunden bei Raumtemperatur gerührt, anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/Methanol 19:1). Man erhielt 770 mg (79 %) (S)-1-(5-Diallyl-aminomethyl-1,2,4-oxadiazol-3-yl)-7-fluoro-8-methoxy-12,12a-dihydro-9H,11H-azeto[2,1-c]imidazo[1,5-a]-[1,4]benzodi'azepin-9-on als farblosen Schaum, Rf=0.37 (Kieselgel, Methylenchlorid/Methanol 19:1).

Beispiel 37

Zu einer Lösung von 800 mg (2.16 mmol) (S)-2-(5-Aminomethyl-1,2,4-oxadiazol-3-yl)-7-fluoro-8-methoxy-12,12a-dihydro-9H,11H-azeto[2,1-c]-imidazo[1,5-a][1,4]benzodiazepin-9-on in 12 ml Methylenchlorid wurden 2.55 ml (14.9 mmol) N-Aethyldiisopropylamin und 0.73 ml (8.67 mmol) Allylbromid zugegeben. Die Reaktionslösung wurde 20 Stunden bei Raumtemperatur gerührt, anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/Methanol 19:1). Man erhielt 340 mg (35%) (S)-1-(5-Diallylaminomethyl-1,2,4-oxadiazol-3-yl)-7-fluoro-8-methoxy-12,12a-dihydro-9H,11H-azeto[2,1-c]imidazo[1,5-a]-[1,4]benzodiazepin-9-on als farblosen Schaum, Rf=0.37 (Kieselgel, Methylenchlorid/Methanol 19:1), 30 mg Mischfraktion und 120 mg (13%) (S)-1-(5-Allylaminomethyl-1,2,4-oxadiazol-3-yl)-7-fluoro-8-methoxy-12,12a-dihydro-9H,11H-azeto[2,1-c]imidazo[1,5-a][1,4]benzodiazepin-9-on als farblosen Schaum, Rf=0.21 (Kieselgel, Methylenchlorid/Methanol 19:1).

Beispiel 38

a) Zu einer Natriummethylatlösung, welche auf übliche Weise aus 2.0 g (86.9 mmol) Natrium und 85 ml Methanol hergestellt wurde, wurden unter Argon bei Raumtemperatur nacheinander 6.2 g (89.2 mmol) Hydroxylamin hydrochlorid und 16.3 g (63.6 mmol) 8-Fluor-5,6-dihydro-5-methyl-6-oxo-4H-imidazo[1,5-a][1,4]benzodiazepin-3-carbonitril zugesetzt. Die Reaktionsmischung wurde 24 Std. bei Raumtemperatur gerührt und dann im Eisbad abgekühlt. Die ausgefallenen Kristalle wurden abfiltriert und in 35 ml Wasser verrührt. Die weissen Kristalle wurden abfiltriert und im Vakuum bei 60° getrocknet. Man erhielt 12.4 g (67 %) 8-Fluor-5,6-dihydro-5-methyl-6-oxo-4H-imidazo [1,5-a][1,4]benzodiazepin-3-carboxamidoxim als weisse Kristalle vom Smp. 249-250° (Zers.).

Durch Eindampfen des Filtrates und Wiederholung des oben beschriebenen Vorgehens wurde zusätzliches, noch mit Ausgangsprodukt verunreinigtes Produkt (2.8 g) gewonnen. Dieses wurde an 100 g Kieselgel zuerst mit Methylenchlorid/Aceton 9:1, 2:1 und schliesslich mit Methylenchlorid/Methanol 9:1 chromatographiert, wobei weitere 1.72 g 8-Fluor-5,6-dihydro-5-methyl-6-oxo-4H-imidazo[1,5-a][1,4]benzodiazepin-3-carboxamidoxim gewonnen wurden. Totalausbeute: 77 %.

b) Zu einer Suspension von 0.4 g (2.2 mmol) Morpholin-4-yl-essigsäurehydrochlorid in 4 ml DMF wurden 0.38 ml (2.2 mmol) N-Aethyldiisopropylamin zugesetzt. Bei Raumtemperatur wurden portionenweise 390 mg (2.4 mmol) 1,1'-Carbonyldiimidazol zugegeben, worauf die Lösung 30 Min. bei 50° gerührt und dann bei Raumtemperatur mit 0.58 g (2.0 mmol) 8-Fluor-5,6-dihydro-5-methyl-6-oxo-4H-imidazo[1,5-a][1,4]benzodiazepin-3-carboxamidoxim versetzt wurde. Das Reaktionsgemisch wurde während 20 Std. auf 90° erwärmt. Das Lösungsmittel wurde im Vakuum entfernt, der Rückstand wurde in 15 ml Wasser aufgenommen, und die Lösung wurde mehrmals mit

Essigester extrahiert. Die vereinigten Extrakte wurden über Natriumsulfat getrocknet, filtriert und eingedampft. Das Rohmaterial wurde durch Chromatographie an Kieselgel gereinigt (Methylenchlorid/ Methanol 19:1). Die Lösungsmittel wurden im Vakuum entfernt, der Rückstand wurde in 5 ml Acetonitril gelöst, und die Lösung wurde durch Zugabe von ätherischer HCl-Lösung sauer gestellt. Die weissen Kristalle wurden abgesaugt und aus Acetonitril umkristallisiert. Man erhielt 0.5 g (54 %) 8-Fluor-5-methyl-3-(5-morpholin-4-ylmethyl-1,2,4-oxadiazol-3-yl)-5,6-dihydro-4H-imidazo[1,5-a][1,4]benzodiazepin-6-on- hydrochlorid (3:5) vom Smp. 198-205° (Zers.).

Beispiel 39

a) 11.8 g (39.2 mMol) (S)-7-Fluor-11,12,13,13a-tetrahydro-9-oxo-9H-imidazo[1,5-a]-pyrrolo[2,1-c][1,4]benzodiazepin-1-carbonsäure wurden in 80 ml N,N-Dimethylformamid gelöst, portionenweise mit 6.71 g (41.4 mMol) 1,1'-Carbonyldiimidazol versetzt und während 20 min bei 50° gerührt. Nach Zugabe von 8.77 g (40 mMol) Phthaloylglycinamidoxim liess man über Nacht bei 100° und während 5 Stunden bei 120° rühren. Nach Eindampfen des Lösungsmittels wurde der Rückstand in Methylenchlorid gelöst, worauf die Lösung dreimal mit Wasser gewaschen, über Magnesiumsulfat getrocknet und eingeengt wurde. Durch Chromatographieren an Kieselgel unter Eluieren mit Essigester erhielt man 9.6 g (50%) (S)-7-Fluor-11,12,13,13a-tetrahydro-9H-1-(3-phthalimidomethyl-1,2,4-oxadiazol-5-yl)-imidazo[1,5-a]-pyrrolo[2,1-c][1,4]benzodiazepin-9-on vom Smp. 258-260°, das ohne weitere Reinigung in die nächste Stufe eingesetzt wurde.

b) 9.6 g (19.8 mMol) (S)-7-Fluor-11,12,13,13a-tetrahydro-9H-1-(3-phthalimidomethyl-1,2,4-oxadiazol-5-yl)-imidazo[1,5-a]pyrrolo[2,1-c]-[1,4]benzodiazepin-9-on wurden in 130 ml Aethanol gelöst und bei 65° innerhalb von 30 min tropfenweise mit 130 ml Methylamin (33%ig in Aethanol) versetzt. Die Lösung wurde während 2 Stunden bei 70° gerührt und anschliessend eingeengt. Der Rückstand wurde in Methylenchlorid und 20 ml 4 N Salzsäure aufgenommen, und die Lösung wurde dreimal mit Methylenchlorid gewaschen. Man stellte die wässrige Phase mit 20 ml 4 N Natronlauge alkalisch und extrahierte sie dreimal mit Methylenchlorid und fünfmal mit Essigester. Nach Trocknung der vereinigten organischen Lösungen und Eindampfen des Lösungsmittels erhielt man 6.73 g (96%) (S)-1-(3-Aminomethyl-1,2,4-oxadiazol-5-yl)-7-fluor-11,12,13,13a-tetrahydro-9H-imidazo[1,5-a]pyrrolo [2,1-c]-[1,4]benzodiazepin-9-on, das ohne weitere Reinigung als Ausgangsprodukt für das nachstehend beschriebene Beispiel verwendet wurde.

Beispiel 40

4 g (11.3 mMol) rohes (S)-1-(3-Aminomethyl-1,2,4-oxadiazol-5-yl)-7-fluor-11,12,13,13a-tetrahydro-9H-imidazo[1,5-a]pyrrolo[2,1-c][1,4]-benzodiazepin-9-on, 60 ml Methylenchlorid, 13.6 ml (79 mMol) N-Aethyldiisopropylamin und 5.63 g (46 mMol) Allylbromid wurden während 60 Stunden bei Raumtemperatur gerührt. Man wusch die Reaktionslösung dreimal mit Wasser, trocknete sie über Magnesiumsulfat und dampfte sie ein. Der Rückstand wurde an 360 g Kieselgel unter Eluieren mit Essigester chromatographiert . Die einheitlichen Fraktionen wurden eingedampft. Man erhielt 2.19 g (45%) (S)-1-(5-Diallylaminomethyl-1,2,4-oxadiazol-3-yl)-7-fluor-11,12,13,13a-tetrahydro-9H-imidazo [1,5-a]pyrrolo[2,1-c][1,4]benzodiazepin-9-on (Oel: $R_f$: 0.43; Kieselgel 60 $F_{254}$; Laufmittel: Essigester), das in das Hydrochlorid überführt wurde.

Beispiel 41

a) 6.31 g (21 mMol) (S)-7-Fluor-9-oxo-11,12,13,13a-tetrahydro-9H-imidazo[1,5-a]pyrrolo[2,1-c][1,4]benzodiazepin-1-carboxamid wurden in 20 ml Dioxan und 3.6 ml Pyridin suspendiert und bei 5° bis 10° tropfenweise mit 3.3 ml Trifluoressigsäureanhydrid versetzt. Das Reaktionsgemisch wurde über Nacht bei Raumtemperatur gerührt und auf 150 ml Wasser gegossen. Die erhaltene Suspension wurde filtriert, und die Kristalle wurden getrocknet. Man erhielt 5.36 g (90%) (S)-7-Fluor-9-oxo-11,12,13,13a-tetrahydro-9H-imidazo[1,5-a]pyrrolo[2,1-c][1,4]benzodiazepin-1-carbonitril vom Smp. 254-256°.

b) 615 mg (26.8 mMol) Natrium wurden in 35 ml Methanol gelöst. Bei Raumtemperatur gab man aufeinanderfolgend 2 g (28.8 mMol) Hydroxylaminhydrochlorid und 5.3 g (18.8 mMol) (S)-7-Fluor-11,12,13,13a-tetrahydro-9-oxo-9H-imidazo[1,5-a]pyrrolo[2,1-c][1,4]benzodiazepin-1-carbonitril zu. Man rührte die Suspension über Nacht bei Raumtemperatur, kühlte sie während 30 min auf 0° ab und filtrierte die Kristalle ab. Nach Trocknung erhielt man

4.97 (84%) (S)-7-Fluor-11,12,13,13a-tetrahydro-9-oxo-9H-imidazo[1,5-a]pyrrolo[2,1-c][1,4]benzodiazepin-1-carboxamidoxim vom Smp. 267-268°.

c) 2.93 g (15.7 mMol) BOC-Glycin wurden in 30 ml N,N-Dimethylformamid gelöst und portionenweise mit 2.76 g (17 mMol) 1,1'-Carbonyldiimidazol versetzt. Nach Beendigung der $CO_2$-Abspaltung wurde die Lösung während 20 min bei 55° gerührt. Man gab dann 4.95 g (22.2 mMol) (S)-7-Fluor-11,12,13,13a-tetrahydro-9-oxo-9H-imidazo [1,5-a]pyrrolo[2,1-c][1,4]benzodiazepin-1-carboxamidoxim zu und rührte über Nacht bei 90°. Man dampfte das Reaktionsgemisch ein und chromatographierte den Rückstand an Kieselgel unter Eluieren mit Methylenchlorid/ Methanol 19/1. Man erhielt 4.45 g (61%) (S)-1-(5-BOC-Aminomethyl-1,2,4-oxadiazol-3-yl)-7-fluor-11,12,13,13a-tetrahydro-9H-imidazo[1,5-a]pyrrolo[2,1-c][1,4]-benzodiazepin-9-on das ohne weitere Reinigung in die nächste Stufe eingesetzt wurde.

d) 4.4 g (9.7 mMol) (S)-1-(5-BOC-Aminomethyl-1,2,4-oxadiazol-3-yl)-7-fluor-11,12,13,13a-tetrahydro-9H-imidazo [1,5-a]pyrrolo[2,1-c][1,4]benzodiazepin-9-on wurden während 1.5 Stunden in 15 ml Trifluoressigsäure bei Raumtemperatur gerührt. Die Lösung wurde eingeengt. Der Rückstand wurde in gesättigter Natriumbicarbonatlösung aufgenommen und zehnmal mit Methylenchlorid extrahiert. Durch Trocknung und Eindampfen der vereinigten organischen Phasen erhielt man 2.76 g (80%) (S)-1-(5-Aminomethyl-1,2,4-oxadiazol-3-yl)-7-fluor-11,12,13,13a-tetrahydro-9H-imidazo[1,5-a]pyrrolo[2,1-c][1,4]benzodiazepin-9-on, das ohne weitere Reinigung als Ausgangsprodukt für das nachstehend beschriebene Beispiel verwendet wurde.

Beispiel 42

2.75 g (7.6 mMol) rohes (S)-1-(5-Aminomethyl-1,2,4-oxadiazol-3-yl)-7-fluor-11,12,13,13a-tetrahydro-9H-imidazo[1,5-a]pyrrolo[2,1-c][1,4]benzodiazepin-9-on, 50 ml Methylenchlorid, 9 ml (53.3 mMol) N-Aethyldiisopropylamin und 3.75 g (31 mMol) Allylbromid wurden während 96 Stunden bei Raumtemperatur gerührt. Man wusch die Reaktionslösung dreimal mit Wasser, trocknete sie über Magnesiumsulfat und dampfte sie ein. Der Rückstand wurde an 360 g Kieselgel unter Eluieren mit Essigester chromatographiert . Die einheitlichen Fraktionen wurden eingedampft. Man erhielt 2.46 g (74%) (S)-1-(5-Diallylaminomethyl-1,2,4-oxadiazol-3-yl)-7-fluor-11,12,13,13a-tetrahydro-9H-imidazo [1,5-a]pyrrolo[2,1-c][1,4]-benzodiazepin-9-on, das in das Hydrochlorid überführt wurde.

Beispiel 43

a) 10 g (29.7 mMol) (S)-8-Trifluormethyl-12,12a-dihydro-9-oxo-9H,11H-azeto[2,1-c]imidazo[1,5-a][1,4]benzodiazepin-1-carbonsäure wurden in 50 ml N,N-Dimethylformamid suspendiert und bei Raumtemperatur portionenweise mit 5.1 g (31.2 mMol) 1,1'-Carbonyldiimidazol versetzt. Nach Beendigung der $CO_2$-Abspaltung wurde die klare braune Lösung während 20 min bei 50° gerührt, gekühlt und bei einer Temperatur unter 15° innerhalb von ca 15' tropfenweise mit 8 ml konz. Ammoniak versetzt. Nach 40minütigem Rühren wurde die erhaltene Suspension auf 300 ml Eiswasser gegossen, worauf 20' bei Raumtemperatur gerührt, filtriert und mit wenig Wasser nachgewaschen wurde. Nach Trocknung erhielt man 7.51 g (75%) (S)-8-Trifluormethyl-12,12a-dihydro-9-oxo-9H,11H-azeto [2,1-c]imidazo[1,5-a]-[1,4]benzodiazepin-1-carboxamid vom Smp. >300°.

b) 7.5 g (22.3 mMol) (S)-8-Trifluormethyl-12,12a-dihydro-9-oxo-9H,11H-azeto[2,1-c]-imidazo[1,5-a][1,4]benzodiazepin-1-carboxamid wurden in 40 ml Dioxan und 4 ml Pyridin suspendiert und bei 7 bis 10° innerhalb von 10 min tropfenweise mit 3.6 ml Trifluoressigsäureanhydrid versetzt. Man rührte während 3/4 Stunden bei Raumtemperatur und goss auf 300 ml Wasser. Man filtrierte die erhaltene Suspension und erhielt nach Trocknung des Rückstandes 3.75 g (52%) (S)-8-Trifluormethyl-12,12a-dihydro-9-oxo-9H,11H-azeto[2,1-c]imidazo[1,5-a][1,4]benzodiazepin-1-carbonitril vom Smp. 115-119°.

c) 730 mg (31.8 mMol) Natrium wurden in 40 ml Methanol gelöst. Bei Raumtemperatur gab man aufeinanderfolgend 2.37 g (34.1 mMol) Hydroxylaminhydrochlorid und 7.1 g (22.3 mMol) (S)-8-Trifluormethyl-12,12a-dihydro-9-oxo-9H,11H-azeto[2,1-c]imidazo[1,5-a][1,4]benzodiazepin-1-carbonitril zu und liess über Nacht bei Raumtemperatur rühren. Durch Eindampfen des Lösungsmittels erhielt man 7.8 g (100%) (S)-8-Trifluormethyl-12,12a-dihydro-9-oxo-9H,11H-azeto[2,1-c]-imidazo[1,5-a][1,4]benzodiazepin-1-carboxamidoxim, das ohne weitere Reinigung in die nächste Stufe eingesetzt wurde.

d) 4.2 g (24 mMol) BOC-Glycin wurden in 40 ml N,N-Dimethylformamid gelöst und portionenweise mit 4.05 g (25

mMol) 1,1'-Carbonyldiimidazol versetzt. Nach Beendigung der $CO_2$-Abspaltung wurde die Lösung während 20 min bei 50° gerührt. Man gab dann 7.7 g (22 mMol) (S)-8-Trifluormethyl-12,12a-dihydro-9-oxo-9H,11H-azeto[2,1-c]-imidazo[1,5-a][1,4]benzodiazepin-1-carboxamidoxim zu und rührte über Nacht bei 90°. Das Reaktionsgemisch wurde eingeengt, und der Rückstand wurde in Methylenchlorid gelöst. Durch Waschen, Trocknen und Eindampfen der organische Phase erhielt man 7.82 g (72%) (S)-1-[5-(N-BOC-Aminomethyl)-1,2,4-oxadiazol-3-yl]-8-trifluormethyl-12,12a-dihydro-9H,11H-azeto[2,1-c]imidazo[1,5-a][1,4]benzodiazepin-9-on, das ohne weitere Reinigung in die nächste Stufe eingesetzt wurde.

e) 7.8 g (15.9 mMol) rohes (S)-1-[5-(N-BOC-Aminomethyl)-1,2,4-oxadiazol-3-yl]-8-trifluormethyl-12,12a-dihydro-9H,11H-azeto[2,1-c]imidazo-[1,5-a][1,4]benzodiazepin-9-on und 30 ml Trifluoressigsäure wurden während 1 Stunde bei Raumtemperatur gerührt. Die Lösung wurde eingedampft. Der Rückstand wurde in Wasser gelöst und dreimal mit Methylenchlorid gewaschen. Die wässrige Phase wurde mit konz. Ammoniak alkalisch gestellt und neunmal mit Essigester (insgesamt ca 11) extrahiert. Durch Eindampfen der vereinigten und über Magnesiumsulfat getrockneten organischen Phasen erhielt man 4.57 g (73%) (S)-1-(5-Aminomethyl-1,2,4-oxadiazol-3-yl)-8-trifluormethyl-12,12a-dihydro-9H,11H-azeto[2,1-c]imidazo[1,5-a][1,4]benzodiazepin-9-on, das ohne weitere Reinigung als Ausgangsprodukt für das nachstehend beschriebene Beispiel verwendet wurde.

Beispiel 44

3 g (7.7 mMol) rohes (S)-1-(5-Aminomethyl-1,2,4-oxadiazol-3-yl)-8-trifluormethyl-12,12a-dihydro-9H,llH-azeto[2,1-c]imidazo[1,5-a][1,4]-benzodiazepin-9-on, 50 ml Methylenchlorid, 14.2 ml (72 mMol) N-Aethyldiisopropylamin und 5.14 g (3.6 mMol) Allylbromid wurden über Nacht bei Raumtemperatur und während 4 Stunden bei 40° gerührt. Man wusch die Reaktionslösung dreimal mit Wasser, trocknete sie über Magnesiumsulfat und dampfte sie ein. Der Rückstand wurde an 390 g Kieselgel unter Eluieren mit Essigester chromatographiert. Man erhielt 1.50 g (41%) (S)-1-(5-Diallylaminomethyl-1,2,4-oxadiazol-3-yl)-8-trifluormethyl-12,12a-dihydro-9H,11H-azeto[2,1-c]imidazo[1,5-a][1,4]benzodiazepin-9-on, welches mit methanolischer Salzsäure in das Dihydrochlorid überführt wurde.

Beispiel 45

1.51 g (7.7 mMol) (S)-1-(5-Aminomethyl-1,2,4-oxadiazol-3-yl)-8-trifluormethyl-12,12a-dihydro-9H,11H-azeto[2,1-c]imidazo[1,5-a][1,4]benzodiazepin-9-on, 15 ml Methylenchlorid, 4 ml (23.4 mMol) N-Aethyldiisopropylamin und 1.06 g (4 mMol) α,α'-Dibrom-o-xylol wurden über Nacht bei Raumtemperatur gerührt. Man wusch die Reaktionslösung zweimal mit Wasser, trocknete sie über Magnesiumsulfat und dampfte sie ein. Der Rückstand wurde an 240 g Kieselgel unter Eluieren mit Essigester/Hexan/ Triäthylamin l7/2/lchromatographiert. Man erhielt 0.5 g (26%) (S)-8-Trifluormethyl-12,12a-dihydro-1-(5-isoindolin-2-ylmethyl-1,2,4-oxadiazol-3-yl)-9H,11H-azeto[2,1-c]imidazo[1,5-a][1,4]benzodiazepin-9-on, das in das Hydrochlorid vom Smp. 185-188° überführt wurde.

Beispiel 46

a) 40.58 g (128.7 mMol) Aethyl (S)-7-fluor-12,12a-dihydro-9-oxo-9H,11H-azeto-[2,1-c]imidazo[1,5-a][1,4]benzodiazepin-1-carboxylat, 300 ml Ethanol und 32.5 ml (130 mMol) 4N-Natronlauge wurden während 1.5 Stunden auf dem Dampfbad zum Rückfluss erhitzt. Man dampfte den Alkohol am Rotationsverdampfer ein. Die wässrige Phase wurde zweimal mit Methylenchlorid gewaschen und mit 32.5 ml (130 mMol) 4N-Salzsäure auf pH 3-4 angesäuert. Die erhaltene Suspension wurde gekühlt und filtriert Der Filterrückstand wurde mit wenig Eiswasser gewaschen und getrocknet. Man erhielt 36.67 g (99%) (S)-7-Fluor-12,12a-dihydro-9-oxo-9H,11H-azeto-[2,1-c]imidazo[1,5-a][1,4]-benzodiazepin-1-carbonsäure vom Smp. 159-160°.

b) 20 g (69.6 mMol) (S)-7-Fluor-12,12a-dihydro-9-oxo-9H,11H-azeto[2,1-c]-imidazo[1,5-a][1,4]benzodiazepin-1-carbonsäure wurden in 40 ml N,N-Dimethylformamid gelöst, portionenweise mit 13 g (80.2 mMol) 1,1'-Carbonyldiimidazol versetzt und während 10 min bei 60° weitergerührt. Nach Zugabe von 16.78 g (76.6 mMol) Phthaloylglycinamidoxim liess man während 1 Stunde bei 90° rühren, gab 14 ml Trifluoressigsäure zu und rührte während 18 Stunden bei 85° weiter. Man kühlte die erhaltene Suspension ab, filtrierte die Kristalle ab und wusch sie mit Methanol nach. Man erhielt 16.74 g (51%) (S)-7-Fluor-12,12a-dihydro-1-(3-phthalimidomethyl-1,2,4-oxadiazol-5-yl)-9H,11H-azetc[2,1-c]imidazo[1,5-a][1,4]benzodiazepin-9-on vom Smp. 287-288°, das ohne weitere Kristallisation in die nächste Stufe eingesetzt wurde.

c) 10.8 g (23 mMol) rohes (S)-7-Fluor-12,12a-dihydro-1-(3-phthalimidomethyl-1,2,4-oxadiazol-5-yl)-9H,11H-azeto [2,1-c]imidazo[1,5-a][1,4]benzodiazepin-9-on in 150 ml Aethanol wurden bei 60°C innerhalb von 30 min tropfenweise mit 150 ml Methylamin (33%ig in Aethanol) versetzt. Die Lösung wurde während 2 Stunden bei 70° gerührt und anschliessend eingeengt. Der Rückstand wurde in Methylenchlorid und 30 ml 4 N Salzsäure aufgenommen, und die Lösung wurde dreimal mit Methylenchlorid gewaschen. Man stellte die wässrige Phase mit 30 ml 4 N Natronlauge alkalisch und extrahierte fünfmal mit Methylenchlorid. Nach Trocknung der vereinigten organischen Lösungen und Eindampfen des Lösungsmittels erhielt man 7.5 g (96%) (S)-1-(3-Aminomethyl-1,2,4-oxadiazol-5-yl)-7-fluor-12,12a-dihydro-9H,11H-azeto[2,1-c]imidazo[1,5-a]-[1,4]benzodiazepin-9-on, das ohne weitere Kristallisation als Ausgangsprodukt für das nachstehend beschriebene Beispiel verwendet wurde.

Beispiel 47

10.35 g (30.4 mMol) rohes (S)-1-(3-Aminomethyl-1,2,4-oxadiazol-5-yl)-7-fluor-12,12a-dihydro-9H,11H-azeto [2,1-c]imidazo[1,5-a][1,4]benzodiazepin-9-on, 100 ml N,N-Dimethylformamid, 13 ml (76mMol) Aethyldiisopropylamin und 7.72 g (63.8 mMol) Allylbromid wurden während 3 Stunden bei Raumtemperatur gerührt. Nach Eindampfen des Reaktionsgemisches wurde der Rückstand durch Chromatographie an Kieselgel unter Eluieren mit Essigester gereinigt. Durch Umkristallisieren aus Essigester und Hexan erhielt man 8.11 g (63%) (S)-1-(3-Diallylaminomethyl-1,2,4-oxadiazol-5-yl)-7-fluor-12,12a-dihydro-9H,11H-azeto[2,1-c]-imidazo[1,5-a][1,4]-benzodiazepin-9-on, das in das Hydrochlorid vom Smp. 188-190° überführt wurde.

Beispiel 48

a) Unter Argon wurden 0.92 g (5.3 mmol) BOC-Glycin in 7 ml N,N-Dimethylformamid gelöst und bei Raumtemperatur portionenweise mit 0.9 g (5.6 mmol) 1,1'-Carbonyldiimidazol versetzt. Nach beendeter $CO_2$- Entwicklung wurde das Gemisch noch 30 Min. bei Raumtemperatur gerührt und darauf mit 1.44 g (5.0 mmol) 8-Fluor-5,6-dihydro-5-methyl-6-oxo-4H-imidazo[1,5-a][1,4]benzodiazepin-3-carboxamidoxim versetzt. Man erhitzte während 18 Std. auf 90°, liess auf Raumtemperatur abkühlen und verdünnte mit 50 ml Wasser, wobei das Produkt auskristallisierte. Das Produkt wurde abgesaugt und in Methylenchlorid gelöst, und die Lösung wurde mit Natriumsulfat getrocknet. Man erhielt duch Eindampfen im Vakuum 1.25 g weissen Schaum.

Das wässerige Filtrat wurde mehrmals mit Methylenchlorid extrahiert, und die Extrakte wurden mit Natriumsulfat getrocknet, filtriert und eingedampft, wobei weitere 0.95 g eines Feststoffes erhalten wurden. Dieser wurde in 10 ml Wasser aufgenommen und verrührt, wobei sich weiteres Produkt abtrennte. Das ausgefallene Material wurde abfiltriert, in Methylenchlorid gelöst, mit Natriumsulfat getrocknet, filtriert und eingedampft. Es wurden nochmals 0.47 g Rohprodukt erhalten.

Die vereinigten Rohprodukte (1.72 g) wurden aus 7 ml Essigester kristallisiert. Man erhielt 1.23 g (58 %) 3-(5-BOC-Aminomethyl-1,2,4-oxadiazol-3-yl)-8-fluor-5-methyl-5,6-dihydro-4H-imidazo[1,5-a][1,4]benzodiazepin-6-on vom Smp. 170-172°.

b) Unter Argon wurden 0.64 g (1.5 mmol) 3-(5-BOC-Aminomethyl-1,2,4-oxadiazol-3-yl)-8-fluor-5-methyl-5,6-dihydro-4H-imidazo[1,5-a][1,4]benzodiazepin-6-on in 1.5 ml Trifluoressigsäure aufgenommen und während 1 Std. bei Raumtemperatur gerührt. Das Lösungsmittel wurde im Vakuum abgedampft, und der Rückstand wurde in 10 ml Wasser gelöst. Die Lösung wurde mit Methylenchlorid extrahiert, und die Wasserphase wurde mit konz. Ammoniaklösung basisch gestellt und mehrmals mit Methylenchlorid extrahiert. Die vereinigten organischen Extrakte wurden mit Natriumsulfat getrocknet, filtriert und eingedampft. Die erhaltenen schwach rosa gefärbten Kristalle wurden in 10 ml Methanol erhitzt, worauf über Celite filtriert und das Filtrat eingedampft wurde. Der Rückstand wurde aus 9 ml Acetonitril/ methanolische Salzsäure 2:1 umkristallisiert. Man erhielt 0.30 g g (50 %) 3-(5-Aminomethyl-1,2,4-oxadiazol-3-yl)-8-fluor-5-methyl-5,6-dihydro-4H-imidazo[1,5-a][1,4]benzodiazepin-6-on hydrochlorid (1:1.9) vom Smp. 264-266° (Zers.).

Beispiel 49

Unter Argon wurde eine Suspension von 0.49 g (1.5 mmol) 3-(5-Aminomethyl-1,2,4-oxadiazol-3-yl)-8-fluor-5-methyl-5,6-dihydro-4H-imidazo[1,5-a][1,4]benzodiazepin-6-on hydrochlorid (1:1.9) in 8 ml Methylenchlorid mit 1.8 ml (10.5 mmol) N-Aethyl-diisopropylamin und 0.51 ml (6.0 mmol) Allylbromid versetzt und 42 Std. unter Argon bei Raumtemperatur gerührt. Die Lösung wurde dreimal mit 10 ml Wasser gewaschen, mit Natriumsulfat getrocknet, filtriert und eingedampft. Das Rohprodukt wurde durch Chromatographie an 50 g Kieselgel (Essigester) gereinigt, worauf das

Eluat eingedampft wurde und der Rückstand in 5 ml Methanol aufgenommen wurde. Die Lösung wurde mit ätherischer Salzsäure angesäuert, und die Lösungsmittel wurden im Vakuum entfernt. Der Rückstand wurde in 4 ml Methanol gelöst, worauf durch Celite filtriert, auf ca. 0° gekühlt und mit 8 ml Aether verdünnt wurde. Dabei fielen langsam weisse Kristalle aus, welche abfiltriert wurden. Man erhielt 345 mg (50%) 3-[5-(Diallyl-aminomethyl)-1,2,4-oxadiazol-3-yl]-8-fluor-5-methyl-5,6-dihydro-4H-imidazo[1,5-a][1,4]benzodiazepin-6-on hydrochlorid (2:3) vom Smp. 133-140° (Zers.).

Beispiel 50

Unter Argon wurde eine Suspension von 0.49 g (1.5 mmol) 3-(5-Aminomethyl-1,2,4-oxadiazol-3-yl)-8-fluor-5-methyl-5,6-dihydro-4H-imidazo[1,5-a][1,4]benzodiazepin-6-on in 8 ml Methylenchlorid mit 0.77 ml (4.5 mmol) N-Aethyldiisopropylamin und 0.475 g (1.8 mmol) $\alpha,\alpha'$-Dibrom-oxylol versetzt und 7 Std. unter Argon bei Raumtemperatur gerührt. Die Lösung wurde einmal mit 10 ml Wasser gewaschen, mit Natriumsulfat getrocknet, filtriert und eingedampft. Das Rohprodukt wurde durch Chromatographie an 40 g Kieselgel (Methylenchlorid/Aceton 4:1, dann 2:1) gereinigt, worauf das Eluat eingedampft und der Rückstand in 3 ml Methanol aufgenommen wurde. Die Lösung wurde mit ätherischer Salzsäure angesäuert, und die Lösungsmittel wurden im Vakuum entfernt. Der Rückstand wurde in 3 ml Methanol gelöst, worauf die Lösung auf ca. 0° gekühlt und tropfenweise mit 3 ml Aether verdünnt wurde. Dabei fielen langsam weisse Kristalle aus, welche abfiltriert wurden. Man erhielt 190 mg (26 %) 8-Fluor-3-(5-isoindolin-2-ylmethyl-1,2,4-oxadiazol-3-yl)-5-methyl-5,6-dihydro-4H-imidazo[1,5-a][1,4]benzodiazepin-6-on hydrochlorid (2:3) vom Smp. 177-184° (Zers.).

Beispiel 51

1.85 g (5 mMol) (S)-1-(3-Aminomethyl-1,2,4-oxadiazol-5-yl)-8-chlor-11,12,13,13a-tetrahydro-9H-imidazo [1,5-a]pyrrolo[2,1-c][1,4]benzodiazepin-9-on, 20 ml N,N-Dimethylformamid, 3.4 ml (20 mMol) N-Aethyldiisopropylamin und 1.45 g (5.5 mMol) $\alpha,\alpha'$-Dibrom-o-xylol wurden über Nacht bei Raumtemperatur gerührt. Nach Eindampfen der Reaktionslösung wurde der Rückstand an 430 g Kieselgel unter Eluieren mit Essigester/Methanol 9/1 chromatographiert . Man erhielt 0.74 g (31%) (S) 8-Chlor-11,12,13,13a-tetrahydro-1-(3-isoindolin-2-ylmethyl-1,2,4-oxadiazol-5-yl)-9H-imidazo[1,5-a]-pyrrolo[2,1-c][1,4]benzodiazepin-9-on, das in das Hydrochlorid vom Smp. 165-170° überführt wurde.

Beispiel 52

5 g (14 mMol) (S)-1-(3-Aminomethyl-1,2,4-oxadiazol-5-yl)-8-chlor-12,12a-dihydro-9H,11H-azeto[2,1-c]imidazo[1,5-a][1,4]benzodiazepin-9-on, 50 ml Methylenchlorid, 12 ml (70 mMol) N-Aethyldiisopropylamin und 3.7 g (14 mMol) $\alpha,\alpha'$-Dibrom-o-xylol wurden während 72 Stunden bei Raum-temperatur gerührt. Man wusch die Reaktionslösung dreimal mit Wasser, trocknete sie über Magnesium-sulfat und dampfte sie ein. Der Rückstand wurde an 70 g Kieselgel unter Eluieren mit Methylenchlorid/ Essigester 7/3 chromatographiert . Man erhielt 4.0 g (62%) (S)-8-Chlor-12,12a-dihydro-1-(3-isoindolin-2-ylmethyl-1,2,4-oxadiazol-5-yl)-9H,11H-azeto[2,1-c]imidazo[1,5-a]-[1,4]benzodiazepin-9-on, das in das Hydrochlorid vom Smp. 170-175° überführt wurde.

Beispiel 53

a) Unter Argon wurden 2.75 g (10.0 mmol) 8-Fluor-5,6-dihydro-5-methyl-6-oxo-4H-imidazo[1,5-a][1,4]benzodiazepin-3-carbonsäure in 20 ml N,N-Dimethylformamid suspendiert, und bei Raumtemperatur wurden portionenweise 1.95 g (12.0 mmol) 1,1'-Carbonyldiimidazol zugesetzt. Nach Beendigung der Gasentwicklung wurde das Reaktionsgemisch noch 30 Min. bei 50° gerührt, dann auf Raumtemperatur abgekühlt und mit 2.41 g (11.0 mmol) Phthaloylglycinamidoxim versetzt. Das Gemisch wurde während 18 Std. auf 90° erhitzt, im Eisbad abgekühlt und mit 40 ml Aether verdünnt, worauf die ausgefallenen Kristalle abfiltriert und im Vakuum getrocknet wurden. Man erhielt 2.88 g (63 %) 8-Fluor-5,6-dihydro-5-methyl-3-(3-phthalimidomethyl-1,2,4-oxadiazol-5-yl)-4H-imidazo [1,5-a][1,4]benzodiazepin-6-on vom Smp. 273-276°.

b) 10.1 g (22 mmol) 8-Fluor-5,6-dihydro-5-methyl-3-(3-phthalimidomethyl-1,2,4-oxadiazol-5-yl)-4H-imidazo[1,5-a] [1,4]benzodiazepin-6-on wurden in 220 ml Aethanol auf 65° erwärmt, worauf innerhalb von 100 Min. 150 ml 33%ige äthanolische Methylamin-Lösung zugetropft wurden. Nach beendeter Zugabe wurde die Lösung noch 17 Std. weiter erhitzt und dann im Eisbad abgekühlt, wobei weisse Kristalle ausfielen. Diese wurden abfiltriert und im Vakuum getrocknet. Man erhielt nach Umkristallisation aus Aethanol 3.05 g 3-(3-Aminomethyl)-1,2,4-oxadiazol-5-yl)-8-fluor-5,6-dihydro-5-methyl-4H-imidazo[1,5-a][1,4]benzodiazepin-6-on vom Smp. 208-217°. Weitere 2.5 g des Produktes wurden durch Chromatographie an Kieselgel mit Methylenchlorid/Methanol 19:1, dann 9:1 gewonnen. Gesamtausbeute: 5.55 g (77 %).

Beispiel 54

Unter Argon wurden 492 mg (1.5 mmol) 3-(3-Aminomethyl)-1,2,4-oxadiazol-5-yl)-8-fluor-5,6-dihydro-5-methyl-4H-imidazo[1,5-a][1,4]benzodiazepin-6-on in 8 ml Methylenchlorid mit 1.8 ml (10.5 mmol) N-Aethyldiisopropylamin und 0.51 ml (6.0 mmol) Allylbromid versetzt und 24 Std. unter Argon bei Raumtemperatur gerührt. Die Lösung wurde einmal mit 10 ml Wasser gewaschen, mit Natriumsulfat getrocknet, filtriert und eingedampft. Das Rohprodukt wurde durch Chromatographie an 40 g Kieselgel (Essigester) gereinigt, das Eluat wurde eingedampft, und der Rückstand wurde in 5 ml Methanol aufgenommen. Die Lösung wurde mit ätherischer Salzsäure angesäuert, und die Lösungsmittel wurden im Vakuum entfernt. Der Rückstand wurde in 5 ml Methanol gelöst, und die Lösung wurde auf ca. 0° gekühlt und mit 18 ml Aether verdünnt. Es fielen langsam weisse Kristalle aus, welche abfiltriert wurden. Man erhielt 270 mg (40 %) 3-(3-(Diallylaminomethyl)-1,2,4-oxadiazol-5-yl)-8-fluor-5-methyl-5,6-dihydro-4H-imidazo[1,5-a][1,4]benzodiazepin-6-on hydrochlorid (1:1) vom Smp. 181-185° (Zers.).

Beispiel 55

Unter Argon wurden 492 mg (1.5 mmol) 3-(3-Aminomethyl)-1,2,4-oxadiazol-5-yl)-8-fluor-5,6-dihydro-5-methyl-4H-imidazo[1,5-a][1,4]benzodiazepin-6-on in 8 ml Methylenchlorid mit 1.3 ml (7.5 mmol) N-Aethyldiisopropylamin und 0.42 ml (3.6 mmol) 3,3-Dimethylallylbromid versetzt und 2 Std. unter Argon bei Raumtemperatur gerührt. Die Lösung wurde eingedampft, und das Rohprodukt wurde durch Chromatographie an 40 g Kieselgel (Essigester) gereinigt. Das Eluat wurde eingedampft, und der Rückstand wurde in 3 ml Methanol aufgenommen. Die Lösung wurde mit ätherischer Salzsäure angesäuert und eingedampft, worauf der Rückstand in 10 ml Essigester aufgenommen wurde. Die Lösung wurde auf ca. 0° gekühlt, und die weissen Kristalle wurden abfiltriert. Man erhielt 220 mg (29 %) 3-[3-[Bis-(3-methyl-but-2-enyl)-aminomethyl)-1,2,4-oxadiazol-5-yl]-8-fluor-5-methyl-5,6-dihydro-4H-imidazo[1,5-a][1,4]benzodiazepin-6-on hydrochlorid (1:1) vom Smp. 181-184° (Zers.).

Beispiel 56

Unter Argon wurden 492 mg (1.5 mmol) 3-(5-Aminomethyl)-1,2,4-oxadiazol-3-yl)-8-fluor-5,6-dihydro-5-methyl-4H-imidazo[1,5-a][1,4]benzodiazepin-6-on in 8 ml Methylenchlorid mit 1.3 ml (7.5 mmol) N-Aethyldiisopropylamin und 0.42 ml (3.6 mmol) 3,3-Dimethylallylbromid versetzt und 2 Std. unter Argon bei Raumtemperatur gerührt. Die Lösung wurde eingedampft, und das Rohprodukt wurde durch Chromatographie an 40 g Kieselgel (Essigester) gereinigt. Das Eluat wurde eingedampft, und der Rückstand wurde in 3 ml Methanol aufgenommen. Die Lösung wurde mit ätherischer Salzsäure angesäuert und eingedampft, worauf der Rückstand in 10 ml Essigester aufgenommen wurde. Die Lösung wurde 30 Min. zum Rückfluss erhitzt und auf ca. 0° gekühlt, worauf die weissen Kristalle abfiltriert wurden. Man erhielt 315 mg (40 %) 3-[5-[Bis-(3-methyl-but-2-enyl)aminomethyl]-1,2,4-oxadiazol-3-yl]-8-fluor-5-methyl-5,6-dihydro-4H-imidazo[1,5-a][1,4]benzodiazepin-6-on hydrochlorid (2:3) vom Smp. 144-148° (Zers.).

Beispiel 57

a) Eine Lösung von 26.9 g (171 mmol) 2H-Thieno-[3,2-d][1,3]oxazin-2,4(1H)-dion (EP 27214) und 16.1 g (171 mmol) L-Azetidin-2-carbonsäure in 200 ml Dimethylformamid und 40 ml Essigsäure wurde bei 120° während 3 Stunden gerührt. Die braune Lösung wurde eingedampft, und der erhaltene braune Rückstand wurde aus Aethanol kristallisiert. Man erhielt 15.2 g (43%) (S)-6,7-Dihydroazeto(1,2-a]thieno[3,2-e][1,4]diazepin-5,9(4H,5aH)-dion als farblose Kristalle vom Smp. 274°.

b) Zu einer Suspension von 1.92 g (44 mmol) NaH (55%, gewaschen mit Hexan) in 5 ml Dimethylformamid wurde bei -30° eine Lösung von 8.32 g (40 mmol) (S)-6,7-Dihydroaceto[1,2-a]thieno[3,2-e][1,4]diazepin-5,9(4H,5aH)-dion in 45 ml Dimethylformamid zugetropft, und die Mischung wurde während 40 Minuten bei -30° gerührt. Nach Abkühlen auf -60° wurde eine Lösung von 8.26 ml (40 mmol) Phosphorsäurediphenylesterchlorid in 3 ml Dimethylformamid so dazugetropft, dass die Temperatur nicht über -45° stieg. Anschliessend rührte man noch 30 Minuten nach.

Unterdessen wurden 4.92 g (44 mmol) Kalium-tert.butylat in 20 ml Dimethylformamid gelöst und bei -60° mit

4.7 ml (42.8 mmol) Isocyanessigsäure-äthylester (95%) versetzt. Zu der so erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und nach Neutralisation mit 4.8 ml Essigsäure bei -40° auf 300 ml Eiswasser gegossen, worauf fünf Mal mit Methylenchlorid extrahiert wurde. Die vereinigten organischen Phasen wurden über Natriumsulfat getrocknet, filtriert und eingedampft. Der erhaltene hellbraune Rückstand wurde aus Aethanol umkristallisiert. Man erhielt 8.12 g (67%) (S)-8-Oxo-11,11a-dihydro-8H,10H-azeto[1,2-a]imidazo [5,1-c]-thieno[3,2-e][1,4]diazepin-1-carbonsäureäthylester als farblose Kristalle vom Smp.188-191°.

c) Zu einer Suspension von 13.5 g (44.5 mmol) (S)-8-Oxo-11,11a-dihydro-8H,10H-azeto[1,2-a]imidazo[5,1-c]thieno[3,2-e][1,4]diazepin-1-carbonsäureäthylester in 10 ml Aethanol und 16 ml Wasser wurden 13.9 ml (55.6 mmol) 4N Natronlauge zugetropft . Die Mischung wurde 30 Minuten am Rückfluss erhitzt, und anschliessend wurde das Aethanol abdestilliert. Die Wasserphase wurde zwei Mal mit Methylenchlorid gewaschen und mit 4N Salzsäure auf pH=3 gestellt. Der entstandene Niederschlag wurde abfiltriert und mit Wasser, Aethanol und anschliessend mit Diäthyläther gewaschen. Man erhielt 10.8 g (88%) (S)-8-Oxo-11,11a-dihydro-8H,10H-azeto[1,2-a]-imidazo [5,1-c]thieno[3,2-e][1,4]diazepin-1-carbonsäure als farbloses Pulver vom Smp. 260° (Zers.).

d) Zu einer Suspension von 12.43 g (45 mmol) (S)-8-Oxo-11,11a-dihydro-8H,10H-azeto-[1,2-a]imidazo[5,1-c]thieno[3,2-e][1,4]diazepin-1-carbonsäure in 70 ml Dimethylformamid wurden portionenweise 7.65 g (47 mmol) 1,1'-Carbonyldiimidazol zugegeben. Die entstandene hellbraune Lösung erwärmte man während 45 Minuten auf 50°. Anschliessend kühlte man die Lösung auf Raumtemperatur ab und tropfte 10.9 ml wässerige Ammoniaklösung dazu. Nach weiterem 30- minütigem Rühren wurde das Reaktionsgemisch auf 100 ml Eiswasser gegossen, und der entstandene Niederschlag wurde abfiltriert und mit Wasser, Aethanol und anschliessend mit Aether nachgewaschen. Man erhielt nach Trocknen bei 70°/10 Torr 11.0 g (89%) (S)-8-Oxo-11,11a-dihydro-8H,10H-azeto-[1,2-a]-imidaza[5,1-c]thieno[3,2-e][1,4]diazepin-1-carbonsäureamid als farblose Kristalle vom Smp.>250°.

e) Zu einer Suspension von 11.05 g (40.2 mmol) (S)-8-Oxo-11,11a-dihydro-8H,10H-azeto(1,2-a]imidazo[5,1-c] thieno[3,2-e][1,4]diazepin-1-carbonsäureamid in 55 ml Dioxan und 7 ml Pyridin wurden bei 5-8° 5.75 ml (41.3 mmol) Trifluoressigsäureanhydrid zugetropft. Die erhaltene beige Lösung wurde während 2.5 Stunden bei 50° gerührt und anschliessend auf 50 ml Eiswasser gegossen. Extraktion mit Methylenchlorid (vier Mal), Trocknen über Natriumsulfat, Filtrieren und Eindampfen lieferten einen hellbraunen Rückstand, welcher chromatographiert wurde (Kieselgel, Methylenchlorid/Methanol 10:1). Man erhielt 8.5 g (82%) (S)-8-Oxo-11,11a-dihydro-8H,10H-azeto[1,2-a]imidazo[5,1-c]thieno[3,2-e][1,4]diazepin-1-carbonitril als weisses Pulver vom Smp. 211-213°.

f) Zu einer frisch hergestellten Lösung von Natriummethylat in Methanol (aus 1.08 g (47 mmol) Natrium in 50 ml Methanol) wurden 8.44 g (32.9 mmol) (S)-8-Oxo-11,11a-dihydro-8H,10H-azeto[1,2-a]imidazo[5,1-c]-thieno[3,2-e] [1,4]diazepin-1-carbonitril und 3.48 g (50.5 mmol) Hydroxylamin-hydrochlorid zugegeben, worauf die Mischung 48 Stunden bei Raumtemperatur gerührt wurde. Anschliessend wurde die Suspension eingedampft und mit 100 ml Wasser versetzt. Der erhaltene Niederschlag wurde abfiltriert und am Hochvakuum getrocknet. Man erhielt 7.8 g (82%) (E)-und/oder (Z)- (S)-1-(Amino-hydroxyimino-methyl)-11,11a-dihydro-8H,10H-azeto[1,2-a]imidazo[5,1-c] thieno[3,2-e][1,4]diazepin-8-on als farbloses Pulver vom Smp. 195-198°.

g) Zu einer Lösung von 5.17 g (29.4 mmol) BOC-Glycin in 55 ml Dimethylformamid wurden 5.12 g (31.5 mmol) 1,1'-Carbonyldiimidazol zugegeben, und die Mischung wurde während 30 Minuten bei 50° gerührt. Anschliessend gab man 7.95 g (27.5 mmol) (E)-und/oder (Z)- (S)-1-(Aminohydroxyimino-methyl)-11,11a-dihydro-8H,10H-azeto [1,2-a]imidazo[5,1-c]-thieno[3,2-e][1,4]diazepin-8-on zu und rührte 15 Stunden bei 90°. Die erhaltene braune Lösung wurde am Hochvakuum eingedampft, und der erhaltene braune Rückstand wurde chromatographiert (Kieselgel, Methylenchlorid /Methanol 10:1). Man erhielt 11.6 g (98%) (S)-1-(5-BOC-Aminomethyl-1,2,4-oxadiazol-3-yl)-11,11a-dihydro-8H,10H-azeto[1,2-a]-imidazo[5,1-c]thieno[3,2-e][1,4]diazepin-8-on als farblosen Schaum, Rf=0.48 (Kieselgel, Methylenchlorid/Methanol 10:1).

h) Eine Lösung von 11.6 g (27 mmol) (S)-1-(5-BOC-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 20 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 10 ml wässeriger Ammoniaklösung basisch gestellt und sechs Mal mit Methylenchlorid extrahiert. Die organischen Phasen wurden über Natriumsulfat getrocknet, filtriert und eingedampft. Der erhaltene Rückstand wurde chromatographiert (Kieselgel, Methylenchlorid/Methanol 9:1). Man erhielt 5.0 g (56%) (S)-1-(5-Aminomethyl-1,2,4-oxa,diazol-3-yl)-11,11a-dihydro-8H,10H-azeto[1,2-a]imidazo[5,1-c]thieno[3,2-e][1,4]diazepin-8-on als farblose Kristalle vom Smp.

235-238°.

Beispiel 58

Zu einer Lösung von 1.2 g (3.65 mmol) (S)-1-(5-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 20 ml Methylenchlorid wurden 4.4 ml (25.2 mmol) N-Aethyldiisopropylamin und 1.77 ml (14.65 mmol) Allylbromid zugegeben, und die Mischung wurde 48 Stunden bei Raumtemperatur gerührt. Die Reaktionslösung wurde anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/Methanol 19:1). Man erhielt so 980 mg (65 %) (S)-1-(5-Diallylaminomethyl-1,2,4-oxadiazol-3-yl)-11,11a-dihydro-8H,10H-azeto[1,2-a]imidazo[5,1-c]thieno[3,2-e]-[1,4]diazepin-8-on als farblosen Schaum, Rf=0.52 (Kieselgel, Methylenchlorid/Methanol 10:1).

Beispiel 59

1.6 g (2.3 mMol) (S)-1-(3-Diallylaminomethyl-1,2,4-oxadiazol-5-yl)-8-chlor-12,12a-dihydro-9H,11H-azeto [2,1-c]imidazo[1,5-a][1,4]benzodiazepin-9-on wurden in 80 ml Essigester in Gegenwart von 50 mg 5%iger Palladiumkohle bei Raumtemperatur und Normaldruck hydriert. Nach Abtrennen des Katalysators wurde der Rückstand durch Chromatographie an Kieselgel unter Eluieren mit Methylenchlorid/Essigester 1/1 und Kristallisieren aus Essigester und Hexan gereinigt. Man erhielt 0.71 g (44%) (S)-8-Chlor-12,12a-dihydro-1-(3-di-n-propyl-aminomethyl-1,2,4-oxadiazol-5-yl)-9H,11H-azeto-[2,1-c]imidazo[1,5-a][1,4]benzodiazepin-9-on, das in das Hydrochlorid vom Smp. 179-181° überführt wurde.

Beispiel 60

a) 13.46 g (50 mMol) (S)-12,12a-Dihydro-9-oxo-9H,11H-azeto(2,1-c]-imidazo[1,5-a][1,4]-benzodiazepin-1-carbonsäure wurden in 40 ml N,N-Dimethylformamid gelöst, portionenweise mit 10 g (61.7 mMol) 1,1'-Carbonyldiimidazol versetzt und während 30 min bei 55° gerührt. Nach Zugabe von 12.06 g (55 mMol) Phthaloylglycinamidoxim liess man während 2 Stunde bei 85° rühren. Man gab 10 ml Trifluoressigsäure zu und rührte über Nacht bei 85° weiter. Das Reaktionsgemisch wurde auf 10° gekühlt, und die Kristalle wurden abfiltriert. Nach Umkristallisieren aus Methanol erhielt man 9.16 g (40%) (S)-12,12a-Dihydro-1-(3-phthalimidomethyl-1,2,4-oxadiazol-5-yl)-9H,11H-azeto[2,1-c]imidazo[1,5-a][1,4]-benzodiazepin-9-on vom Smp. 290-292°.

b) 9 g (19.9 mMol) (S)-12,12a-Dihydro-1-(3-phthalimidomethyl-1,2,4-oxadiazol-5-yl)-9H,11H-azeto[2,1-c]imidazo [1,5-a][1,4]benzodiazepin-9-on, wurden in 20 ml Aethanol und 65 ml Methylamin (33%ig) in Aethanol während 2 Stunden bei 70° gerührt. Die Lösung wurde eingeengt, und der Rückstand wurde mit 70 ml Methylenchlorid verrührt.Die erhaltene Suspension wurde filtriert. Nach Trocknung des Filterrückstands erhielt man (S)-1-(3-Aminomethyl-1,2,4-oxadiazol-5-yl)-12,12a-dihydro-9H,11H-azeto[2,1-c]imidazo[1,5-a][1,4]benzodiazepin-9-on, das ohne weitere Kristallisation als Ausgangsprodukt für das nachstehend beschriebene Beispiel verwendet wurde.

Beispiel 61

4.72 g (14.6 mMol) rohes (S)-1-(3-Aminomethyl-1,2,4-oxadiazol-5-yl)-12,12a-dihydro-9H,11H-azeto[2,1-c] imidazo[1,5-a][1,4]benzodiazepin-9-on, 70 ml Methylenchlorid, 6 ml (35mMol) N-Aethyldiisopropylamin und 3.53 g (29.2 mMol) Allylbromid wurden während 60 Stunden bei Raumtemperatur gerührt. Das Reaktionsgemisch wurde eingedampft, und der Rückstand wurde an 180 g Kieselgel unter Eluieren mit Essigester chromatographiert. Durch Einengen der einheitlichen Fraktionen erhielt man (S)-1-(3-Diallylaminomethyl-1,2,4-oxadiazol-5-yl)-12,12a-dihydro-9H,11H-azeto[2,1-c]-imidazo[1,5-a][1,4]benzodiazepin-9-on, das in das Hydrochlorid vom Smp. 147-150° überführt wurde.

Beispiel 62

1.78 g (5 mMol) (S)-1-(5-Aminomethyl-1,2,4-oxadiazol-3-yl)-8-chlor-12,12a-dihydro-9H,11H-azeto[2,1-c]imidazo[1,5-a][1,4]benzo-diazepin-9-on, 40 ml Aethylenchlorid, 8.6 ml (50 mMol) N-Aethyldiisopropylamin und 9.10 g (60 mMol) Brommethylcyclopropan wurden über Nacht bei 75° gerührt. Man dampfte die Reaktionslösung ein. Der Rückstand wurde an 200 g Kieselgel unter Eluieren mit Essigester chromatographiert. Man erhielt 0.7 g (30%) (S)-1-[5-(Bis-cyclopropylmethyl)-aminomethyl-1,2,4-oxadiazol-3-yl]-8-chlor-12,12a-dihydro-9H,11H-azeto[2,1-c]imidaza [1,5-a][1,4]benzodiazepin-9-on vom Smp. 96-99°, das in das Hydrochlorid überführt wurde

Beispiel 63

a) Zu einer Suspension von 4.9 g (35.2 mmol) Kaliumcarbonat in 40 ml Dimethylformamid wurden bei Raumtemperatur 2.6 g (37.7 mmol) Hydroxylaminhydrochlorid zugegeben. Anschliessend tropfte man eine Lösung von 7.2 g (25.2 mmol) (S)-7,8-Difluoro-9-oxo-12,12a-dihydro-9H,11H-azeto[2,1-c]imidazo[1,5-a][1,4]benzodiazepin-1-carbonitril in 100 ml Dimethylformamid zu und rührte während 60 Stunden bei Raumtemperatur. Die erhaltene gelbe Suspension wurde eingedampft, der Rückstand wurde zwischen Methylenchlorid und Wasser verteilt, und die Wasserphase wurde vier Mal mit Methylenchlorid extrahiert. Die organischen Phasen wurden über Natriumsulfat getrocknet, filtriert und eingedampft. Anschliessende Chromatographie (Kieselgel, Methylenchlorid/Methanol 9:1) lieferte 3 g (37%) (E)- und/oder (Z)-(S)-1-(Amino-hydroxylimino-methyl)-7,8-difluoro-12,12a-dihydro-9H, 11H-azeto[2,1-c]imidazo[1,5-a][1,4]benzodiazepin-9-on als fabloses Pulver vom Smp. > 250°.

b) Zu einer Lösung von 4.5 g (14.1 mmol) BOC-Glycin in 30 ml Dimethylformamid wurden 2.7 g (16.9 mmol) 1,1'-Carbonyldiimidazol i zugegeben, und das Gemisch wurde während 30 Minuten bei 50° gerührt. Anschliessend gab man 4.5 g (14.1 mmol) (E)- und/oder (Z)-(S)-1-(Aminohydroxylimino-methyl)-7,8-difluoro-12,12a-dihydro-9H, 11H-azeto[2,1-c]-imidazo[1,5-a][1,4]benzodiazepin-9-on zu und rührte 16 Stunden bei 90°. Die erhaltene braune Lösung wurde am Hochvakuum eingedampft, und der erhaltene braune Rückstand wurde chromatographiert (Kieselgel, Methylenchlorid /Methanol 19:1). Man erhielt 4.9 g (76%) (S)-1-(5-BOC-Aminomethyl-1,2,4-oxadiazol-3-yl)-7,8-difluoro-12,12a-dihydro-9H,11H-azeto[2,1-c]imidazo[1,5-a][1,4]benzodiazepin-9-on als farblosen Schaum, Rf=0.21 (Kieselgel, Methylenchlorid/Methanol 19:1).

c) Eine Lösung von 330 mg (0.72 mmol) (S)-1-(5-BOC-Aminomethyl-1,2,4-oxadiazol-3-yl)-7,8-difluoro-12,12a-dihydro-9H,11H-azeto[2,1-c]-imidazo[1,5-a][1,4]benzodiazepin-9-on in 2 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 2 ml wässeriger Ammoniaklösung basisch gestellt und sechs Mal mit Methylenchlorid extrahiert. Die organischen Phasen wurden über Natriumsulfat getrocknet, filtriert und eingedampft. Der erhaltene Rückstand wurde aus Methylenchlorid/Aether kristallisiert. Man erhielt 190 mg (73%) (S)-1-(5-Aminomethyl-1,2,4-oxadiazol-3-yl)-7,8-difluoro-12,12a-dihydro-9H,11H-azeto[2,1-c]imidazo[1,5-a][1,4]-benzodiazepin-9-on als beiges Pulver vom Smp. 240° (Zers.).

Beispiel 64

Zu einer Lösung von 800 mg (2.23 mmol) (S)-2-(5-Aminomethyl-1,2,4-oxadiazol-3-yl)-7,8-difluoro-12,12a-dihydro-9H,11H-azeto[2,1-c]imidazo[1,5-a]-[1,4]benzodiazepin-9-on in 60 ml Methylenchlorid wurden 1.9 ml (11.2 mmol) N-Aethyldiisopropylamin und 710 mg (2.7 mmol) $\alpha,\alpha'$-Dibrom-ortho-xylol zugegeben, und das Gemisch wurde 20 Stunden bei Raumtemperatur gerührt. Die Reaktionslösung wurde anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/Methanol 19:1). Man erhielt 130 mg (12%) (S)-1-(5-(Isoindolin-2-ylmethyl-1,2,4-oxadiazol-3-yl)-7,8-difluoro-12,12a-dihydro-9H,11H-azeto-[2,1-c] imidazo[1,5-a][1,4]benzodiazepin-9-on als farblosen Schaum, Rf=0.15 (Kieselgel, Methylenchlorid/Methanol 19:1).

Beispiel 65

Zu einer Lösung von 800 mg (2.23 mmol) (S)-1-(5-Aminomethl-1,2,4-oxadiazol-3-yl)-7,8-difluoro-12,12a-dihydro-9H,11H-azeto[2,1-c]imidazo[1,5-a]-[1,4]benzodiazepin-9-on in 60 ml Methylenchlorid wurden 2.6 ml (15.4 mmol) N-Aethyldiisopropylamin und 1 ml (8.93 mmol) 3,3-Dimethyl-allylbromid zugegeben, und die Mischung wurde 20 Stunden bei Raumtemperatur gerührt. Die Reaktionslösung wurde mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/ Methanol 19:1). Man erhielt 300 mg (27%) (S)-1-[5-[Bis-(3-methyl-but-2-enyl)-aminomethyl]-1,2,4-oxadiazol-3-yl]-7,8-difluoro-12,12a-dihydro-9H,11H-azeto[2,1-c]imidazo[1,5-a][1,4]benzodiazepin-9-on als farblosen Schaum, Rf=0.19 (Kieselgel, Methylenchlorid/Methanol 19:1).

Beispiel 66

Zu einer Lösung von 700 mg (1.95 mmol) (S)-1-(5-Aminomethyl-1,2,4-oxadiazol-3-yl)-7,8-difluoro-12,12a-dihydro-9H,11H-azeto[2,1-c]imidazo[1,5-a]-[1,4]benzodiazepin-9-on in 40 ml Methylenchlorid wurden 2.3 ml (13.5 mmol) N-Aethyldiisopropylamin und 0.66 ml (7.8 mmol) Allylbromid zugegeben, und die Mischung wurde 20 Stunden bei Raumtemperatur gerührt. Die Reaktionslösung wurde mit Methylenchlorid verdünnt und drei Mal mit Wasser gewaschen. Die organischen Phasen wurden über Magnesium-sulfat getrocknet, filtriert und eingedampft. Der erhaltene Rückstand wurde chromatographiert (Kieselgel, Methylenchlorid/ Methanol 19:1). Man erhielt 400 mg (46%) (S)-1-[5-(Diallylamino-methyl)-1,2,4-oxadiazol-3-yl]-7,8-difluoro-12,12a-dihydro-9H,11H-azeto[2,1-c]imidazo[1,5-a][1,4]benzadiazepin-9-an als farblosen Schaum, Rf=0.17 (Kieselgel, Methylenchlorid/Methanol 19:1).

Beispiel 67

4 g (11.2 mMol) (S)-1-(3-Aminomethyl-1,2,4-oxadiazol-5-yl)-8-chlor-12,12a-dihydro-9H,11H-azeto[2,1-c]imidazo[1,5-a][1,4]benzodiazepin-9-on, 50 ml Methylenchlorid, 7.3 ml (42.6 mMol) N-Aethyldiisopropylamin und 3 ml (24.6 mMol) Crotylbromid wurden während 7 Stunden bei Raumtemperatur gerührt. Man wusch die Reaktionslösung dreimal mit Wasser, trocknete sie über Magnesiumsulfat und dampfte sie ein. Der Rückstand wurde an 200 g Kieselgel chromatographiert unter Eluieren mit Methylenchlorid/Essigester 1/1. Man erhielt 2.4 g (46%) (S)-1-[3-Bis-(but-2-enyl)-aminomethyl-1,2,4-oxadiazol-5-yl]-8-chlor-12,12a-dihydro-9H,11H-azeto[2,1-c]imidazo[1,5-a][1,4]-benzodiazepin-9-on als 4/1 E/Z Gemisch, das in das Hydrochlorid vom Smp. 109-113° überführt wurde.

Beispiel 68

5 g (14 mMol) (S)-1-(3-Aminomethyl-1,2,4-oxadiazol-5-yl)-8-chlor-12,12a-dihydro-9H,11H-azeto[2,1-c]imidazo[1,5-a][1,4]-benzodiazepin-9-on, 50 ml Methylenchlorid, 9.1 ml (53.2 mMol) N-Aethyldiisopropylamin und 3.8 ml (30.8 mMol) 3,3-Dimethylallylbromid wurden während 2 Stunden bei Raumtemperatur gerührt. Man wusch die Reaktionslösung dreimal mit Wasser, trocknete sie über Magnesiumsulfat und dampfte sie ein. Der Rückstand wurde an 190 g Kieselgel unter Eluieren mit Methylenchlorid/ Essigester 1/1 chromatographiert. Man erhielt 3.25 g (47%) (S)-1-[3-Bis-(3-methyl-but-2-enyl)amino-methyl-1,2,4-oxadiazol-5-yl]-8-chlor-12,12a-dihydro-9H,11H-azeto[2,1-c]-imidazo[1,5-a][1,4]-benzo-diazepin-9-on, das in das Hydrochlorid vom Smp. 188-189° überführt wurde.

Beispiel 69

4 g (11.2 mMol) (S)-1-(3-Aminomethyl-1,2,4-oxadiazol-5-yl)-8-chlor-12,12a-dihydro-9H,11H-azeto[2,1-c]-imidazo[1,5-a][1,4]-benzodiazepin-9-on, 25 ml N,N-Dimethylformamid, 7.5 ml (43.7 mMol) N-Aethyldiisopropylamin und 2.9 ml (28 mMol) 4-Brom-1-buten wurden während 25 Stunden bei 80° gerührt. Man dampfte die Reaktionslösung ein, löste den Rückstand in Methylenchlorid, wusch diese Lösung dreimal mit Wasser, trocknete sie über Magnesiumsulfat und dampfte sie ein. Der Rückstand wurde an 160 g Kieselgel unter Eluieren mit Methylenchlorid/Essigester 1/1 chromatographiert. Man erhielt 2.8 g (53%) (S)-1-(3-[Bis-(but-3-enyl)aminomethyl]-1,2,4-oxadiazol-5-yl}-8-chlor-12,12a-dihydro-9H,11H-azeto[2,1-c]imidazo-[1,5-a][1,4]benzodiazepin-9-on, das in das Hydrochlorid vom Smp. 115-120° überführt wurde.

Beispiel 70

4.6 g (12.9 mMol) (S)-1-(3-Aminomethyl-1,2,4-oxadiazol-5-yl)-8-chlor-12,12a-dihydro-9H,11H-azeto[2,1-c]-imidazo[1,5-a][1,4]-benzodiazepin-9-on, 25 ml N,N-Dimethylformamid, 8.6 ml (50.3 mMol) N-Aethyldiisopropylamin und 3.4 ml (32.2 mMol) Brommethylcyclopropan wurden während 18 Stunden bei 80° gerührt. Man dampfte die Reaktionslösung ein, löste den Rückstand in Methylenchlorid, wusch diese Lösung dreimal mit Wasser, trocknete sie über Magnesiumsulfat und dampfte sie ein. Der Rückstand wurde an 110 g Kieselgel unter Eluieren mit Methylenchlorid/ Essigester 1/1 chromatographiert. Man erhielt 2.3 g (38%) (S)-1-[3-(Bis-cyclopropylmethylaminomethyl)-1,2,4-oxadiazol-5-yl]-8-chlor-12,12a-dihydro-9H,11H-azeto-[2,1-c]imidazo[1,5-a][1,4]benzodiazepin-9-on, das in das Hydrochlorid vom Smp. 155-160° überführt wurde.

Beispiel 71

3.7 g (10 mMol) (S)-1-(3-Aminomethyl-1,2,4-oxadiazol-5-yl)-8-chlor-11,12,13,13a-tetrahydro-9H-imidazo [1,5-a]pyrrolo[2,1-c][1,4]benzodiazepin-9-on, 30 ml N,N-Dimethylformamid, 4.27 ml (25 mMol) N-Aethyldiisopropylamin und 2.38 g (20 mMol) Propargylbromid wurden während 1 Stunde bei Raumtemperatur gerührt. Nach Eindampfen

der Reaktionslösung wurde der Rückstand an 430 g Kieselgel unter Eluieren mit Essigester/Methanol 9/1 chromatographiert. Die einheitlichen Fraktionen mit dem kleineren $R_f$ wurden eingedampft. Man erhielt 1.19 g (29%) (S)-8-Chlor-11,12,13,13a-tetrahydro-1-(3-propargylamino-methyl-1,2,4-oxadiazol-5-yl)-9H-imidazo-[1,5-a]pyrrolo[2,1-c][1,4]benzodiazepin-9-on vom Smp. 144-145°, das in das Hydrochlorid vom Smp. 189-192° überführt wurde.

Beispiel 72

a) 6.35 g (20 mMol) (S)-8-Chlor-11,12,13,13a-tetrahydro-9-oxo-9H-imidazo[1,5-a]pyrrolo[2,1-c][1,4]benzodiazepin-1-carbonsäure wurden in 30 ml N,N-Dimethylformamid gelöst, portionenweise mit 3.57 g (41.4 mMol) 1,1'-Carbonyldiimidazol versetzt und während 20 min bei 50° gerührt. Nach Zugabe von 9.22 g (42 mMol) Phthaloylglycinamidoxim liess man über Nacht bei 90° rühren, gab 1 ml Trifluoressigsäure zu und rührte während 20 Stunden bei 110° weiter. Die erhaltene Suspension wurde gekühlt, und die Kristalle wurden abfiltriert. Man erhielt 5.74 g (57%) (S)-8-Chlor-11,12,13,13a-tetrahydro-9H-1-(3-phthalimidomethyl-1,2,4-oxadiazol-5-yl)-imidazo[1,5-a]pyrrolo[2,1-c][1,4]benzodiazepin-9-on vom Smp. 275-277°.

b) 54.25 g (108.3 mMol) (S)-8-Chlor-11,12,13,13a-tetrahydro-9H-1-(3-phthalimidomethyl-1,2,4-oxadiazol-5-yl)-imidazo[1,5-a]pyrrolo[2,1-c]-[1,4]benzodiazepin-9-on wurden während 1.5 Stunden mit 280 ml Methylamin (33%ig) in Aethanol bei 75° gerührt. Die Lösung wurde eingeengt, der Rückstand wurde in Methylenchlorid und 115 ml 4 N Salzsäure aufgenommen, und die Lösung wurde dreimal mit Methylenchlorid gewaschen. Man stellte die wässrige Phase mit 115 ml 4 N Natronlauge alkalisch und extrahierte sechsmal mit Methylenchlorid. Nach Trocknung der vereinigten organischen Lösungen und Eindampfen des Lösungsmittels erhielt man 35.4 g (88%) (S)-1-(3-Aminomethyl-1,2,4-oxadiazol-5-yl)-8-chlor-11,12,13,13a-tetrahydro-9H-imidazo-[1,5-a]pyrrolo-[2,1-c][1,4]benzodiazepin-9-on, das ohne weitere Reinigung als Ausgangsprodukt für das nachstehend beschriebene Beispiel verwendet wurde.

Beispiel 73

3.7 g (10 mMol) rohes (S)-1-(3-Aminomethyl-1,2,4-oxadiazol-5-yl)-8-chlor-11,12,13,13a-tetrahydro-9H-imidazo[1,5-a]pyrrolo[2,1-c][1,4]benzodiazepin-9-on, 30 ml Methylenchlorid, 4.3 ml (25 mMol) N-Aethyldiisopropylamin und 2.3 ml (20 mMol) 3,3-Dimethylallylbromid wurden während 3 Stunden bei Raumtemperatur gerührt. Die Reaktionslösung wurde an 250 g Kieselgel unter Eluieren mit Essigester chromatographiert. Man erhielt 2.44 g (48%) (S)-1-[3-Bis-(3-methyl-but-2-enyl)aminomethyl-1,2,4-oxadiazol-5-yl]-8-chlor-11,12,13,13a-tetrahydro-9H-imidazo[1,5-a]pyrrolo[2,1-c][1,4]benzodiazepin-9-on, das in das Hydrochlorid vom Smp. 137-140° überführt wurde.

Beispiel 74

1 g (2.2 mMol) (S)-1-(3-Diallylaminomethyl-1,2,4-oxadiazol-5-yl)-7-fluor-12,12a-dihydro-9H,11H-azeto[2,1-c]imidazo[1,5-a][1,4]benzodiazepin-9-on wurden in 5 ml Methanol in Gegenwart von 22 mg 5%iger Palladiumkohle bei Raumtempera-tur und Normaldruck hydriert. Nach Abtrennen des Katalysators wurde der Rückstand durch Chromatographie an Kieselgel unter Eluieren mit Essigester/Hexan/Triaethylamin 17/2/1 gereinigt. Man erhielt 0.53 g (56%) (S)-7-Fluor-12,12a-dihydro-1-(3-di-n-propylaminomethyl-1,2,4-oxadiazol-5-yl)-9H,11H-azeto[2,1-c]imidazo[1,5-a][1,4]benzodiazepin-9-on (Oel: $R_f$: 0.36; Kieselgel 60 $F_{254}$. Laufmittel: Essigester/Hexan/Triaethylamin 17/2/1), das in das Hydrochlorid überführt wurde.

Beispiel 75

Unter Argon wurden 328 mg (1.0 mmol) 3-(3-Aminomethyl-1,2,4-oxadiazol-5-yl)-8-fluor-5,6-dihydro-5-methyl-4H-imidazo[1,5-a][1,4]benzodiazepin-6-on in 6 ml Methylenchlorid mit 1.2 ml (7.0 mmol) N-Aethyldiisopropylamin und 0.65 ml (6.0 mmol) Propargylbromid (80 % in Toluol) versetzt, und die Mischung wurde 19 Std. unter Argon bei Raumtemperatur gerührt. Die Lösung wurde mit 10 ml Wasser gewaschen und eingedampft, und das Rohprodukt wurde durch Chromatographie an 15 g Kieselgel (Methylenchlorid/Aceton 9:1) gereinigt. Die Eluate wurde eingedampft, und der Rückstand wurde in 5 ml Methanol aufgenommen. Die Lösung wurde mit 5 ml ätherischer Salzsäure angesäuert, und die weissen Kristalle wurden abfiltriert. Man erhielt 200 mg (45 %) 3-[3-[Bis-(prop-2-inyl)-aminomethyl]-1,2,4-oxadiazol-5-yl]-8-fluor-5-methyl-5,6-dihydro-4H-imidazo[1,5-a][1,4]benzodiazepin-6-on hydrochlorid (4:5) vom Smp. 186-189° (Zers.).

Beispiel 76

Zu einer Lösung von 660 mg (2 mmol) (S)-1-(5-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 20 ml Dimethylformamid wurden 2.5 g Kaliumcarbonat und 0.48 ml (4.1 mmol) Dimethylallylbromid zugegeben, und die Mischung wurde 12 Stunden bei Raumtemperatur gerührt. Die Reaktionslösung wurde anschliessend filtriert, und das Filtrat wurde zwischen Methylenchlorid und Wasser verteilt. Die Wasserphase wurde drei Mal mit Methylenchlorid nachextrahiert; die vereinigten organischen Phasen wurden anschliessend über Natriumsulfat getrocknet, filtriert und eingedampft. Der erhaltene Rückstand wurde chromatographiert (Kieselgel, Methylenchlorid/ Methanol/wässeriges Ammoniak 300:10:1), und man erhielt 650 mg (70%) (S)-1-[5-[Bis-(3-methyl-but-2-enyl)-aminomethyl]-1,2,4-oxadiazol-3-yl]-11,11a-dihydro-8H,10H-azeto[1,2-a]imidazo [5,1-c]-thieno[3,2-e][1,4]-diazepin-8-on als farblosen Schaum, Rf=0.16 (Kieselgel, Methylenchlorid/Methanol/wässeriges Ammoniak 300:10:1).

Beispiel 77

Zu einer Lösung von 660 mg (2 mmol) (S)-1-(5-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 20 ml Dimethylformamid wurden 2.5 g Kaliumcarbonat und 0.55 g (2.1 mmol) $\alpha,\alpha'$-Dibrom-o-xylol zugegeben, und die Mischung wurde 12 Stunden bei Raumtemperatur gerührt. Die Reaktionslösung wurde anschliessend filtriert, und das Filtrat wurde zwischen Methylenchlorid und Wasser verteilt. Die Wasserphase wurde drei Mal mit Methylenchlorid nachextrahiert; die vereinigten organischen Phasen wurden anschliessend über Natriumsulfat getrocknet, filtriert und eingedampft. Der erhaltene Rückstand wurde chromatographiert (Kieselgel, Methylenchlorid/Methanol/wässeriges Ammoniak 300:10:1), und man erhielt 535 mg (62%) (S)-1-[5-(Isoindolin-2-ylmethyl)-1,2,4-oxadiazol-3-yl)-11,11a-dihydro-8H,10H-azeto[1,2-a]imidazo[5,1-c]thieno[3,2-a] [1,4]-diazepin-8-on als farblosen Schaum, Rf=0.12 (Kieselgel, Methylenchlorid/ Methanol/wässeriges Ammoniak 300: 10:1).

Beispiel 78

a) Zu einer Lösung von 11.88 g (41.2 mmol) (S)-10,11,12,12a-Tetra-hydro-8-oxo-8H-imidazo[5,1-c]pyrrolo[1,2-a] thieno[3,2-e][1,4]diazepin-1-carbonsäure (EP 59390 A1) in 65 ml Dimethylformamid wurden bei Raumtemperatur 6.98 g (43 mmol) 1,1'-Carbonyldiimidazol auf einmal zugegeben, und die Mischung wurde 30 Minuten bei 50° gerührt. Anschliessend wurden 9.2 g (41.9 mmol) Phthaloylglycinamidoxim auf einmal zugegeben, und die Mischung wurde 15 Stunden bei 110° gerührt. Das Dimethylformamid wurde am Hochvakuum eingedampft, und der erhaltene Rückstand wurde mit 150 ml Wasser versetzt. Extraktion mit Methylenchlorid (zwei Mal), Trocknen über Natriumsulfat, Filtrieren und Eindampfen lieferten einen rötlichen Rückstand, welcher anschliessend chromatographiert wurde (Kieselgel, Methylenchlorid/Methanol 20:1). Man erhielt 7.3 g (38%) (S)-2-[5-(8-Oxo-10,11,12,12a-tetrahydro-8H-imidazo[5,1-c]pyrrolo[1,2-a]thieno[3,2-e]-[1,4]diazepin-1-yl)-1,2,4-oxadiazol-3-yl-methyl]-1,3-dihydro-isoindol-1,3-dion als farblose Kristalle vom Smp. 248-250°.

(b) Zu einer Lösung von 7.28 g (15.4 mmol) (S)-2-[5-(8-Oxo-10,11, 12,12a-tetrahydro-8H-imidazo[5,1-c]pyrrolo [1,2-a]thieno[3,2-e][1,4]diazepin-1-yl)-1,2,4-oxadiazol-3-yl-methyl]-1,3-dihydroisoindol-1,3-dion in 100 ml Aethanol tropfte man 100 ml Methylamin (33% in Aethanol) bei 70° zu und rührte noch zwei Stunden bei 70°. Das Reaktionsgemisch wurde eingedampft, und der Rückstand wurde chromatographiert (Kieselgel, Methylenchlorid/ Methanol 20:1). Man erhielt 4.36 g (83%) (S)-1-(3-Aminomethyl-1,2,4-oxadiazol-5-yl)-10,11,12,12a-tetrahydro-8H-imidazo[5,1-c]pyrrolo[1,2-a]-thieno[3,2-e][1,4]diazepin-8-on als farblose Kristalle vom Smp. 156-158°.

Beispiel 79

Zu einer Lösung von 685 mg (2mmol) (S)-1-(3-Aminomethyl-1,2,4-oxadiazol-5-yl)-10,11,12,12a-tetrahydro-8H-imidazo[5,1-c]pyrrolo[1,2-a]-thieno[3,2-e][1,4]diazepin-8-on in 20 ml Methylenchlorid wurden 2.4 ml (13.8 mmol) N-Aethyldiisopropylamin und 0.67 ml (8 mmol) Allylbromid zugegeben, und die Mischung wurde 20 Stunden bei Raumtemperatur gerührt. Die Reaktionslösung wurde anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/ Methanol/wässeriges Ammoniak 200:10:1). Man erhielt 693 mg (82%) (S)-1-(5-Diallylaminomethyl-1,2,4-oxadiazol-5-yl)-10,11,12,12a-tetrahydro-8H-imidazo[5,1-c]pyrrolo[1,2-a]thieno[3,2-e][1,4]diazepin-8-on als farblosen Schaum, Rf=0.32 (Kieselgel, Methylenchlorid/Methanol /wässeriges Ammoniak 200:10:1).

Beispiel 80

Unter Argon wurden 328 mg (1.0 mmol) 3-(5-Aminomethyl)-1,2,4-oxadiazol-3-yl)-6-fluor-5,6-dihydro-5-methyl-4H-imidazo[1,5-a][1,4]benzodiazepin-6-on in 6 ml Methylenchlorid mit 0.51 ml (3.0 mmol) N-Aethyldiisopropylamin und 0.27 ml (2.5 mmol) Propargylbromid (80 % in Toluol) versetzt, und die Mischung wurde 22 Std. unter Argon bei Raumtemperatur gerührt. Es wurden nochmals 0.09 ml Propargylbromid (80 % in Toluol) und 0.05 ml N-Aethyldiisopropylamin zugesetzt, und es wurde weitere 74 Std. bei Raumtemperatur gerührt. Die Reaktionsmischung wurde mit 10 ml Wasser gewaschen, mit Natriumsulfat getrocknet, filtriert und eingedampft. Das Rohprodukt wurde durch Chromatographie an 50 g Kieselgel (Essigester) gereinigt. Die Eluate wurden eingedampft, und der Rückstand wurde in 5 ml Methanol aufgenommen. Die Lösung wurde mit ätherischer Salzsäure angesäuert und eingedampft, und der Rückstand wurde in 5 ml Methanol/Aether 3:2 aufgenommen. Die weissen Kristalle wurden abfiltriert und getrocknet. Man erhielt 87 mg (20 %) 3-[5-[Bis-(prop-2-inyl)-aminomethyl]-1,2,4-oxadiazol-3-yl]-8-fluor-5-methyl-5 ,6-dihydro-4H-imidazo[1,5-a][1,4]benzodiazepin-6-on hydrochlorid (1:1) vom Smp. 175-177° (Zers.).

Beispiel 81

a) 2.45 g (14 mMol) BOC-Glycin wurden in 20 ml N,N-Dimethylformamid gelöst, worauf die Mischung portionenweise mit 2.43g (15 mMol) 1,1'-Carbonyldiimidazol versetzt und während 20 min bei 50° gerührt wurde. Nach Zugabe von 3.8 g (12.4 mMol) 7-Chlor-5,6-dihydro-5-methyl-6-oxo-4H-imidazo[1,5-a][1,4]benzodiazepin-3-carboxamidoxim wurde über Nacht bei 90° weitergerührt. Das Reaktionsgemisch wurde eingeengt; der Rückstand wurde in Methylenchlorid gelöst und dreimal mit Wasser gewaschen. Nach Trocknung der Lösung, Eindampfen des Lösungsmittels und Kristallisation des Rückstandes aus Essigester und Hexan erhielt man 5.33 g (96%) 3-(5-BOC-Aminomethyl-1,2,4-oxadiazol-3-yl)-7-chlor-5,6-dihydro-5-methyl-4H-imidazo[1,5-a][1,4]benzodiazepin-6-on vom Smp. 144-146°.

b) 7.03 g (16.4 mMol) 3-(5-BOC-Aminomethyl-1,2,4-oxadiazol-3-yl)-7-chlor-5,6-dihydro-5-methyl-4H-imidazo [1,5-a ][1,4]benzodiazepin-6-on wurden während 1 Stunde in 30 ml Trifluoressigsäure bei Raumtemperatur gerührt. Die Lösung. wurde eingeengt, der Rückstand wurde in Wasser aufgenommen, und die Lösung wurde zweimal mit Methylenchlorid gewaschen. Man stellte die wässrige Phase mit 25%igem Ammoniak alkalisch und extrahierte sie siebenmal mit Methylenchlorid. Nach Trocknung und Eindampfen der vereinigten organischen Phasen erhielt man 4.5 g (79%) 3-(5-Aminomethyl-1,2,4-oxadiazol-3-yl)-7-chlor-5,6-dihydro-5-methyl-4H-imidazo[1,5-a][1,4]benzodiazepin-6-on, das ohne weitere Reinigung als Ausgangsprodukt für das nachstehend beschriebene Beispiel verwendet wurde.

Beispiel 82

1.5 g (4.4 mMol) 3-(5-Aminomethyl-1,2,4-oxadiazol-3-yl)-7-chlor-5,6-dihydro-5-methyl-4H-imidazo[1,5-a][1,4]benzodiazepin-6-on, 20 ml N,N-Dimethyl-formamid, 2.10 ml (12 mMol) N-Aethyldiisopropylamin und 1.16 g (9.6 mMol) Allylbromid wurden während 4 Stunden bei Raumtemperatur gerührt. Die Reaktionslösung wurde eingedampft, und der Rückstand wurde an 250 g Kieselgel unter Eluieren mit Essigester chromatographiert . Die einheitlichen Fraktionen wurden eingedampft. Man erhielt 1.54 g (82%) 3-(5-Diallylaminomethyl-1,2,4-oxadiazol-3-yl)-7-chlor-5,6-dihydro-5-methyl-4H-imidazo[1,5-a][1,4]benzodiazepin-6-on, das in das Hydrochlorid vom Smp. 126-130° überführt wurde.

Beispiel 83

a) 38.8 g (133 mMol) 7-Chlor-5,6-dihydro-5-methyl-6-oxo-4H-imidazo-[1,5-a][1,4]-benzodiazepin-3-carbonsäure wurden in 200 ml N,N-Dimethylformamid gelöst, portionenweise mit 23.7 g (146 mMol) 1,1'-Carbonyldiimidazol versetzt und während 20 min bei 70° gerührt. Nach Zugabe von 43.73 g (199.5 mMol) Phthaloylglycinamidoxim liess man während 1 Stunde bei 90° rühren, gab 1.5 ml Trifluoressigsäure zu und rührte über Nacht bei 90° und während 3 Stunden bei 120° weiter. Die erhaltene Suspension wurde auf die Hälfte ihres Voluments eingeengt und gekühlt, und die ausgefallenen Kristalle wurden abfiltriert. Man erhielt 35.3 g (56%) 7-Chlor-5,6-dihydro-5-methyl-3-(3-phthalimidomethyl-1,2,4-oxadiazol-5-yl)-4H-imidazo-[1,5-a][1,4]benzodiazepin-6-on vom Smp.

237-239°.

b) 35.1 g (74 mMol) 7-Chlor-5,6-dihydro-5-methyl-3-(3-phthalimidomethyl-1,2,4-oxadiazol-5-yl)-4H-imidazo[1,5-a][1,4]benzodiazepin-6-on wurden während 3 Stunden mit 190 ml Methylamin (33%ig in Aethanol) und 100 ml Aethanol bei 80° gerührt. Die Lösung wurde eingeengt, der Rückstand wurde in Methylenchlorid und 100 ml 4 N Salzsäure aufgenommen, und die Lösung wurde dreimal mit Methylenchlorid gewaschen. Man stellte die wässrige Phase mit 105 ml 4 N Natronlauge alkalisch und extrahierte sechsmal mit Methylenchlorid. Nach Trocknung der vereinigten organischen Lösungen und Eindampfen des Lösungsmittels erhielt man 18.14 g (71%) 3-(3-Aminomethyl-1,2,4-oxadiazol-5-yl)-7-chlor-5,6-dihydro-5-methyl-4H-imidazo[1,5-a][1,4]benzodiazepin-6-on, das ohne weitere Reinigung als Ausgangsprodukt für das nachstehend beschriebene Beispiel verwendet wurde.

Beispiel 84

5.17 g (15 mMol) rohes 3-(3-Aminomethyl-1,2,4-oxadiazol-5-yl)-7-chlor-5,6-dihydro-5-methyl-4H-imidazo[1,5-a][1,4]benzodiazepin-6-on, 45 ml N,N-Dimethylformamid, 6.5 ml (37.5 mMol) N-Aethyldiisopropylamin und 3.63g (30 mMol) Allylbromid wurden während 1 Stunde bei Raumtemperatur gerührt. Die Reaktionslösung wurde eingedampft, und der Rückstand wurde an 250 g Kieselgel unter Eluieren mit Essigester chromatographiert. Die einheitlichen Fraktionen mit dem grösseren $R_f$-Wert wurden eingedampft. Man erhielt 4.84 g (76%) 3-(3-Diallyl-aminomethyl-1,2,4-oxadiazol-5-yl)-7-chlor-5,6-dihydro-5-methyl-4H-imidazo[1,5-a][1,4]benzodiazepin-6-on, das in das Hydrochlorid vom Smp. 125-130° überführt wurde.

Beispiel 85

3.44 g (10 mMol) 3-(3-Aminomethyl-1,2,4-oxadiazol-5-yl)-7-chlor-5,6-dihydro-5-methyl-4H-imidazo[1,5-a][1,4]benzodiazepin-6-on, 30 ml N,N-Dimethylformamid, 3.7 ml (22 mMol) N-Aethyldiisopropylamin und 2.9 g (11 mMol) α,α'-Dibrom-o-xylol wurden während 6 Stunden bei Raumtempera-tur gerührt. Nach Eindampfen des Lösungsmittels wurde der Rückstand an 230 g Kieselgel unter Eluieren mit Essigester chromatographiert . Die einheitlichen Fraktionen wurden eingedampft. Man erhielt 1.56 g (35%) 7-Chlor-5,6-dihydro-3-(3-isoindolin-2-yl-methyl-1,2,4-oxadiazol-5-yl)-5-methyl-4H-imidazo[1,5-a][1,4]-benzodiazepin-6-on, das in das Hydrochlorid vom Smp. 180-184° überführt wurde.

Beispiel 86

a) 15 g (5.83 mMol) 5,6-Dihydro-5-methyl-6-oxo-4H-imidazo[1,5-a]-[1,4]benzo-diazepin-3-carbonsäure wurden in 100 ml N,N-Dimethylformamid gelöst, portionenweise mit 11.3 g (7 mMol) 1,1'-Carbonyldiimidazol versetzt und während 20 min bei 70° gerührt. Nach Zugabe von 19.2 g (8.75 mMol) Phthaloylglycinamidoxim liess man während 3 Stunde bei 80° rühren, gab 5 ml Trifluoressigsäure zu und rührte über Nacht bei 110°. Das Reaktionsgemisch wurde eingeengt, und der Rückstand wurde an 200 g Kieselgel unter Eluieren mit Essigester chromatographiert. Man erhielt 13 g (50%) 5,6-Dihydro-5-methyl-3-(3-phthalimidomethyl-1,2,4-oxadiazol-5-yl)-4H-imidazo[1,5-a][1,4]benzodiazepin-6-on vom Smp. 239-240°.

b) 13 g (29.5 mMol) 5,6-Dihydro-5-methyl-3-(3-phthalimidomethyl-1,2,4-oxadiazol-5-yl)-4H-imidazo[1,5-a][1,4]benzodiazepin-6-on wurden während 3 Stunden mit 90 ml Methylamin (33%ig in Aethanol) und 40 ml Aethanol bei 80° gerührt. Die Lösung wurde eingeengt, der Rückstand wurde in Methylenchlorid und 53 ml 4 N Salzsäure aufgenommen, und die Lösung wurde dreimal mit Methylenchlorid gewaschen. Man stellte die wässrige Phase mit 55 ml 4 N Natronlauge alkalisch und extrahierte sechsmal mit Methylenchlorid. Nach Trocknung der vereinigten organischen Lösungen und Eindampfen des Lösungsmittels erhielt man 9 g (100%) (3-(3-Aminomethyl-1,2,4-oxadiazol-5-yl)-5,6-dihydro-5-methyl-4H-imidazo[1,5-a]-[1,4]benzodiazepin-6-on vom Smp. 183-185°, das ohne weitere Reinigung als Ausgangsprodukt für das nachstehend beschriebene Beispiel verwendet wurde.

Beispiel 87

3.10 g (10 mMol) rohes 3-(3-Aminomethyl-1,2,4-oxadiazol-5-yl)-5,6-dihydro-5-methyl-4H-imidazo[1,5-a][1,4]benzodiazepin-6-on, 20 ml N,N-Dimethylformamid, 3.7 ml (22 mMol) N-Aethyldiisopropylamin und 1.81 g (15 mMol) Allylbromid wurden während 1.5 Stunden bei Raumtemperatur gerührt. Nach Eindampfen des Lösungsmittels wurde der Rückstand an 220 g Kieselgel unter Eluieren mit Essigester chromatographiert. Man erhielt 0.86 g (22%) 3-(3-Diallylaminomethyl-1,2,4-oxadiazol-5-yl)-5,6-dihydro-5-methyl-4H-imidazo[1,5-a][1,4]benzodiazepin-6-on, das in das

Hydrochlorid vom Smp. 203-205° überführt wurde.

Beispiel 88

1.85 g (5 mMol) (S)-1-(3-Aminomethyl-1,2,4-oxadiazol-5-yl)-8-chlor-11,12,13,13a-tetrahydro-9H-imidazo [1,5-a]pyrrolo[2,1-c][1,4]benzodiazepin-9-on, 20 ml N,N-Dimethylformamid, 2.14 ml (12,5 mMol) N-Aethyldiisopropylamin und 0.97 ml (10 mMol) Propyljodid wurden über Nacht bei 80° gerührt. Nach Eindampfen des Lösungsmittels wurde der Rückstand an 210 g Kieselgel unter Eluieren mit Essigester chromatographiert. Man erhielt 0.81 g (36%) (S)-8-Chlor-11,12,13,13a-tetrahydro-1-(3-dipropylaminomethyl-1,2,4-oxadiazol-5-yl)-9H-imidazo[1,5-a]pyrrolo[2,1-c] [1,4]benzodiazepin-9-on, das in das Hydrochlorid vom Smp. 155-158° überführt wurde.

Beispiel 89

5.17 g (15 mMol) 3-(3-Aminomethyl-1,2,4-oxadiazol-5-yl)-7-chlor-5,6-dihydro-5-methyl-4H-imidazo[1,5-a] [1,4]benzodiazepin-6-on, 45 ml N,N-Dimethylformamid, 6.5 ml (37.5 mMol) N-Aethyldiisopropylamin und 3.63g (30 mMol) Allylbromid wurden während 1 Stunde bei Raumtemperatur gerührt. Die Reaktionslösung wurde eingedampft, und der Rückstand wurde an 250 g Kieselgel unter Eluieren mit Essigester chromatographiert. Die einheitlichen Fraktionen mit dem kleineren $R_f$-Wert wurden eingedampft. Man erhielt 0.344 g (6%) 3-(3-Allylaminomethyl-1,2,4-oxadiazol-5-yl)-7-chlor-5,6-dihydro-5-methyl-4H-imidazo[1,5-a][1,4]benzodiazepin-6-on, das in das Hydrochlorid vom Smp. 171-174° überführt wurde.

Beispiel 90

1.5 g (4.65 mMol) (S)-1-(3-Aminomethyl-1,2,4-oxadiazol-5-yl)-12,12a-dihydro-9H,11H-azeto[2,1-c]imidazo [1,5-a][1,4]benzodiazepin-9-on, 20 ml N,N-Dimethylformamid, 2.8 ml (16.3 mMol) N-Aethyldiisopropylamin und 1.1 ml (10.7 mMol) Brommethylcyclopropan wurden während 18 Stunden bei 80° gerührt. Man dampfte die Reaktionslösung ein, löste den Rückstand in Methylenchlorid, wusch diese Lösung dreimal mit Wasser, trocknete sie über Magnesiumsulfat und dampfte sie ein. Der Rückstand wurde an 110 g Kieselgel unter Eluieren mit Methylenchlorid/Essigester 1/1 chromatographiert. Man erhielt 1 g (49%) (S)-1-[3-(Bis-cyclopropylmethyl)-aminomethyl-1,2,4-oxadiazol-5-yl]-12,12a-dihydro-9H,11H-azeto[2,1-c]imidazo-[1,5-a][1,4]benzodiazepin-9-on, das in das Hydrochlorid vom Smp. 174-176° überführt wurde.

Beispiel 91

1.28 g (4.1 mMol) 3-(3-Aminomethyl-1,2,4-oxadiazol-5-yl)-5,6-dihydro-5-methyl-4H-imidazo[1,5-a][1,4]benzodiazepin-6-on, 25 ml N,N-Dimethylformamid, 2 ml (11.7 mMol) N-Aethyldiisopropylamin und 1.19 g (4.5 mMol) α,α'-Dibrom-o-xylol wurden während 4 Stunden bei Raumtemperatur gerührt. Nach Eindampfen des Lösungsmittels wurde der Rückstand an 180 g Kieselgel unter Eluieren mit Essigester chromatographiert. Die einheitlichen Fraktionen wurden eingedampft. Man erhielt 0.39 g (23%) 5,6-Dihydro-3-(3-isoindolin-2-ylmethyl-1,2,4-oxadiazol-5-yl)-5-methyl-4H-imidazo[1,5-a][1,4]benzodiazepin-6-on, das in das Hydrochlorid vom Smp. 155-160° überführt wurde.

Beispiel 92

3.44 g (10 mMol) 3-(3-Aminomethyl-1,2,4-oxadiazol-5-yl)-7-chlor-5,6-dihydro-5-methyl-4H-imidazo[1,5-a] [1,4]benzodiazepin-6-on, 20 ml N,N-Dimethylformamid, 4.3 ml (25 mMol) N-Aethyldiisopropylamin und 2.98 g (20 mMol) 3,3-Dimethylallylbromid wurden während 1.5 Stunden bei Raumtemperatur gerührt. Die Reaktionslösung wurde eingedampft, und der Rückstand wurde an 215 g Kieselgel unter Eluieren mit Essigester chromatographiert. Man erhielt 1.8 g (37%) 3-{3-[Bis-(3-methyl-but-2-enyl)aminomethyl]-1,2,4-oxadiazol-5-yl)-7-chlor-5,6-dihydro-5-methyl-4H-imidazo[1,5-a][1,4]benzo-diazepin-6-on, das in das Hydrochlorid vom Smp. 139-142° überführt wurde.

Beispiel 93

1.5 g (4.65 mMol) (S)-1-(3-Aminomethyl-1,2,4-oxadiazol-5-yl)-12,12a-dihydro-9H,11H-azeto[2,1-c]imidazo [1,5-a][1,4]benzodiazepin-9-on, 20 ml N,N-Dimethylformamid, 2.4 ml (13.9 mMol) N-Aethyldiisopropylamin und 1.3 g (4.9 mMol) α,α'-Dibrom-o-xylol wurden während 1 Stunde bei Raumtemperatur gerührt. Man dampfte die Reaktionslösung ein, löste den Rückstand in Methylenchlorid und chromatographierte ihn an 50 g Kieselgel unter Eluieren mit Methylenchlorid/Essigester 1/1. Man erhielt 1.2 g (61%) (S)-12,12a-Dihydro-1-(3-isoindolin-2-ylmethyl-1,2,4-oxadiazol-5-yl)-9H,11H-azeto[2,1-c]imidazo[1,5-a][1,4]benzodiazepin-9-on, das in das Hydrochlorid vom Smp. 210-213°

überführt wurde.

Beispiel 94

5 g (15.5 mMol) (S)-1-(3-Aminomethyl-1,2,4-oxadiazol-5-yl)-12,12a-dihydro-9H,11H-azeto[2,1-c]imidazo [1,5-a][1,4]benzodiazepin-9-on, 50 ml N,N-Dimethylformamid, 8 ml (46.5 mMol) N-Aethyldiisopropylamin und 4 ml (32.5 mMol) 3,3-Dimethylallylbromid wurden während 2 Stunden bei Raumtemperatur gerührt. Man dampfte die Reaktionslösung ein, löste den Rückstand in Methylenchlorid, wusch diese Lösung dreimal mit Wasser, trocknete sie über Magnesiumsulfat und dampfte sie ein. Der Rückstand wurde an 130 g Kieselgel unter Eluieren mit Methylenchlorid/Essigester 1/1 chromatographiert . Man erhielt 3.3 g (46%) (S)-1-[3-Bis-(3-methyl-but-2-enyl)aminomethyl-1,2,4-oxadiazol-5-yl]-12,12a-dihydro-9H,11H-azeto[2,1-c]-imidazo[1,5-a][1,4]benzodiazepin-9-on, das in das Hydrochlorid vom Smp. 119-122° überführt wurde.

Beispiel 95

1.5 g (3.7 mMol) (S)-1-(3-Diallylaminomethyl-1,2,4-oxadiazol-5-yl)-12,12a-dihydro-9H,11H-azeto[2,1-c]imidazo[1,5-a][1,4]benzodiazepin-9-on wurden in 50 ml Essigester in Gegenwart von 50 mg 5%iger Palladiumkohle bei Raumtemperatur und Normaldruck hydriert. Nach Abtrennen des Katalysators wurde der Rückstand durch Chromatographie an Kieselgel unter Eluieren mit Essigester/Methylenchlorid 1/1 gereinigt. Man erhielt 0.9 g (59%) (S)-12,12a-Dihydro-1-(3-dipropylaminomethyl-1,2,4-oxadiazol-5-yl)-9H,11H-azeto[2,1-c]imidazo[1,5-a][1,4]-benzodiazepin-9-on, das in das Hydrochlorid vom Smp. 185-187° überführt wurde.

Beispiel 96

a) 10 g (31.09 mMol) (S)-8-Chlor-7-fluor-12,12a-dihydro-9-oxo-9H,11H-azeto[2,1-c]-imidazo[1,5-a][1,4]benzodiazepin-1-carbonsäure wurden in 100 ml N,N-Dimethylformamid gelöst, portionenweise mit 6.05 g (37.3 mMol) 1,1'-Carbonyldiimidazol versetzt und während 1 Stunde bei Raumtempera-tur gerührt. Nach Zugabe von 6.8 g (31.09 mMol) Phthaloylglycinamidoxim liess man über Nacht bei Raumtemperatur rühren, gab 10 ml Trifluoressigsäure zu und rührte über Nacht bei 90°. Nach Eindampfen des Lösungsmittel wurde der Rückstand aus Methylenchlorid und Methanol kristallisiert. Man erhielt 8.55 g (54%) (S)-8-Chlor-7-fluor-12,12a-dihydro-1-(3-phthalimidomethyl-1,2,4-oxadiazol-5-yl)-9H,11H-azeto[2,1-c]-imidazo[1,5-a]-[1,4]benzodiazepin-9-on vom Smp. 292-294°.

b) 16 g (31.85 mMol) (S)-8-Chlor-7-fluor-12,12a-dihydro-1-(3-phthalimidomethyl-1,2,4-oxadiazol-5-yl)-9H,11H-azeto[2,1-c]imidazo[1,5-a][1,4]benzodiazepin-9-on, wurden mit 100 ml Methylamin (33%ig in Aethanol) versetzt. Die Lösung wurde während 1 Stunde bei 70° gerührt und anschliessend gekühlt. Die erhaltene Suspension wurde filtriert, und das Kristallisat wurde in 100 ml Methylenchlorid teilweise gelöst. Durch Filtrieren und Eindampfen des Filtrats erhielt man 11.7 g (99%) (S)-1-(3-Aminomethyl-1,2,4-oxadiazol-5-yl)-8-chlor-7-fluor-12,12a-dihydro-9H,11H-azeto[2,1-c]-imidazo[1,5-a][1,4]benzodiazepin-9-on, das ohne weitere Reinigung als Ausgangsprodukt für das nachstehend beschriebene Beispiel verwendet wurde.

Beispiel 97

11.7 g (31.2 mMol) rohes (S)-1-(3-Aminomethyl-1,2,4-oxadiazol-5-yl)-8-chlor-7-fluor-12,12a-dihydro-9H,11H-azeto[2,1-c]imidazo[1,5-a][1,4]benzodiazepin-9-on, 100 ml Methylenchlorid, 6.04 g (50 mMol) Allylbromid und 7.75 g (60 mMol) N-Aethyldiisopropylamin wurden über Nacht bei Raumtemperatur gerührt. Die Lösung wurde eingeengt, und der Rückstand wurde durch Chromatographieren an 500 g Kieselgel unter Eluieren mit Essigester gereinigt. Nach Umkristallisation aus Methanol erhielt man 5.3 g (47%) (S)-1-(3-Diallylaminomethyl-1,2,4-oxadiazol-5-yl)-8-chlor-7-fluor-12,12a-dihydro-9H,11H-azeto[2,1-c]imidazo[1,5-a][1,4]benzodiazepin-9-on vom Smp. 139-140°, das in das Hydrochlorid vom Smp. 127° überführt wurde.

Beispiel 98

Unter Argon wurde eine Suspension von 328 mg (1.0 mmol) 3-(3-Aminomethyl-1,2,4-oxadiazol-5-yl)-8-fluor-5-methyl-5,6-dihydro-4H-imidazo[1,5-a][1,4]benzodiazepin-6-on in 5 ml Methylenchlorid mit 0.38 ml (2.2 mmol) N-Aethyldiisopropylamin und 317 mg (1.2 mmol) $\alpha,\alpha'$-Dibrom-oxylol versetzt und 5 Std. unter Argon bei Raumtemperatur gerührt. Die Lösung wurde einmal mit 5 ml Wasser gewaschen, mit Natriumsulfat getrocknet, filtriert und eingedampft.

Das Rohprodukt wurde durch Chromatographie an 30 g Kieselgel (Methylenchlorid/Aceton 4:1, dann 2:1) gereinigt. Die Eluate wurden eingedampft, und der Rückstand wurde in 2.5 ml Methanol aufgenommen. Die Lösung wurde mit ätherischer Salzsäure angesäuert, und die Lösungsmittel wurden im Vakuum entfernt. Der Rückstand wurde in 6 ml heissem Methanol aufgenommen; dann wurde auf ca. 0° gekühlt, und die weissen Kristalle wurden durch Absaugen isoliert. Man erhielt 150 mg (32 %) 8-Fluor-3-(3-isoindolin-2-ylmethyl-1,2,4-oxadiazol-5-yl)-5-methyl-5,6-dihydro-4H-imidazo[1,5-a][1,4]benzodiazepin-6-on hydrochlorid (1:1) vom Smp. 229-233° (Zers.).

Beispiel 99

a) Eine Lösung von 28.8 g (144.6 mmol) 5,6-Difluoro-2,4-dihydro-1H-3,1-benzoxazin-2,4-dion und 16.7 g ( 144.6 mmol) L-Prolin in 110 ml Dimethylformamid und 20 ml Essigsäure wurde bei 120° während 16 Stunden gerührt. Die braune Lösung wurde eingedampft, und der erhaltene braune Rückstand wurde aus Aethanol kristallisiert. Man erhielt 30 g (82%) (S)-6,7-Difluoro-2,3,5,10,11,11a-hexahydro-1H-pyrrolo[2,1-c][1,4]benzodiazepin-5,11-dion als farblose Kristalle vom Smp. > 250°.

b) Zu einer Suspension von 5.7 g (130 mmol) NaH (55%, gewaschen mit Hexan) in 10ml Dimethylformamid wurde bei -30° eine Lösung von 29.8 g (118.2 mmol) (S)-6,7-Difluoro-2,3,5,10,11,11a-hexahydro-1H-pyrrolo[2,1-c]-[1,4] benzodiazepin-5,11-dion in 140 ml Dimethylformamid zugetropft und während 40 Minuten bei -30° gerührt. Nach Abkühlen auf -60° wurde eine Lösung von 25.2 ml (118.2 mmol) Phosphorsäurediphenylesterchlorid in 50 ml Dimethylformamid so zugetropft, dass die Temperatur nicht über -45° stieg. Anschliessend rührte man noch 30 Minuten nach.

Unterdessen wurden 14.6 g (130 mmol) Kalium-tert.butylat in 50 ml Dimethylformamid gelöst und bei -60° mit 14.5 ml (126.4 mmol) Isocyanessigsäureäthylester (95%) versetzt. Zu der erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und, nach Neutralisation mit 20 ml Essigsäure bei -40°, auf 400 ml Eiswasser gegossen, worauf fünf Mal mit Methylenchlorid extrahiert wurde. Die vereinigten organischen Phasen wurden über Natriumsulfat getrocknet, filtriert und eingedampft. Der erhaltene hellbraune Rückstand wurde chromatographiert (Kieselgel, Essigester). Man erhielt 22 g (54%) (S)-7,8-Difluoro-9-oxo-11,12,13,13a-tetrahydro-9H-imidazo[1,5-a]pyrrolo[2,1-c][1,4]benzodiazepin-1-carbon-säureäthlester als farblose Kristalle vom Smp. 199-200°.

c) Zu einer Suspension von 22.1 g (63.6 mmol) (S)-7,8-Difluoro-9-oxo-11,12,13,13a-tetrahydro-9H-imidazo[1,5-a] pyrrolo[2,1-c][1,4]benzodiazepin-1-carbonsäureäthlester in 55 ml Aethanol und 90 ml Wasser wurden 20.7 ml (82.7 mmol) 4N Natronlauge zugetropft und 45 Minuten am Rückfluss erhitzt. Anschliessend wurde das Aethanol abdestilliert. Die Wasserphase wurde zwei Mal mit Methylenchlorid gewaschen und mit 4N Salzsäure auf pH=3 gestellt. Extraktion mit Methylenchlorid (fünf Mal), Trocknen mit Natriumsulfat, Filtrieren und Eindampfen lieferten 20 g (98%) (S)-7,8-Difluoro-9-oxo-11,12,13,13a-tetrahydro-9H-imidazo[1,5-a]pyrrolo[2,1-c][1,4]-benzodiazepin-1-carbonsäure als farblose Kristalle vom Smp. 214.5-215.5°(Zers.).

d) Zu einer Suspension von 11 g (34.5 mmol) (S)-7,8-Difluoro-9-oxo-11,12,13,13a-tetrahydro-9H-imidazo[1,5-a] pyrrolo[2,1-c][1,4]benzodiazepin-1-carbonsäure in 80 ml Dimethylformamid wurden 6.1 g (37.9 mmol) 1,1'-Carbonyldiimidazol portionenweise zugegeben. Die entstandene hellbraune Lösung erwärmte man während 45 Minuten auf 50°. Anschliessend kühlte man die Lösung auf Raumtemperatur ab und tropfte 12 ml wässerige Ammoniaklösung dazu. Nach weiterem 30 minütigem Rühren wurde das Reaktionsgemisch auf 150 ml Eiswasser gegossen und mit Methylenchlorid sieben Mal extrahiert. Trocknen der organischen Phasen mit Natriumsulfat, Filtrieren und Eindampfen lieferten einen farblosen Rückstand, welcher mit Methylenchlorid verrührt wurde. Nach Trocknen am Hochvakuum erhielt man 9.8 g (S)-7,8-Difluoro-9-oxo-11,12,13,13a-tetrahydro-9H-imidazo[1,5-a]pyrrolo[2,1-c] [1,4]benzodiazepin-1-carbonsäureamid vom Smp. 221-224°.

e) Zu einer Suspension von 10.9 g (34.2 mmol) (S)-7,8-Difluoro-9-oxo-11,12,13,13a-tetrahydro-9H-imidazo[1,5-a] pyrrolo[2,1-c][1,4]benzodiazepin-1-carbonsäureamid in 50 ml Dioxan und 10 ml Pyridin wurden bei 5-8° 5.2 ml (37.7 mmol) Trifluoressigsäure-anhydrid dazugetropft. Die erhaltene beige Lösung wurde während 2.5 Stunden bei 50° gerührt und anschliessend auf 50 ml Eiswasser gegossen. Extraktion mit Methylenchlorid (sieben Mal), Trocknen mit Natriumsulfat, Filtrieren und Eindampfen lieferten nach Umkristallisation aus Aethanol 8.5 g (83%) (S)-7,8-Difluoro-9-oxo-11,12,13,13a-tetrahydro-9H-imidazo[1,5-a]pyrrolo[2,1-c][1,4]benzodiazepin-1-carbonitril als farblose Kristalle vom Smp.>250°.

f) Zu einer Suspension von 5.4 g (93.1 mmol) Kaliumcarbonat in 150 ml Dimethylformamid wurden bei Raumtemperatur 2.9 g (41.9 mmol) Hydroxylaminhydrochlorid zugegeben. Anschliessend tropfte man eine Lösung von 8.9 g (28 mmol) (S)-7,8-Difluoro-9-oxo-11,12,13, 13a-tetrahydro-9H-imidazo[1,5-a]pyrrolo[2,1-c][1,4]benzodiazepin-1-carbonitril in 100 ml Dimethylformamid zu und rührte während 60 Stunden bei Raumtempera-tur. Die erhaltene gelbe Suspension wurde eingedampft, der Rückstand wurde zwischen Methylenchlorid und Wasser verteilt, und die Wasserphase wurde vier Mal mit Methylenchlorid extrahiert. Die organischen Phasen wurden mit Natriumsulfat getrocknet, filtriert und eingedampft. Anschliessende Chromatographie (Kieselgel, Methylenchlorid/Methanol 9:1) lieferte 2.4 g (37%) (E)- und/oder (Z)-(S)-7,8,Difluoro-9-oxo-11,12,13,13a-tetrahydro-9H-imidazo[1,5-a]pyrrolo [2,1-c][1,4]benzodiazepin-1-carboxamidoxim als fablosen Schaum, Rf=0.1 (Kieselgel, Methylenchlorid/Methanol 9:1).

g) Zu einer Lösung von 2.2 g (12.5 mmol) BOC-Glycin in 70 ml Dimethylformamid wurden 2.2 g (13.7 mmol) 1,1'-Carbonyldiimidazol zugegeben und während 30 Minuten bei 50° gerührt. Anschliessend gab man 3.8 g (11.4 mmol) (E)- und/oder (Z)-(S)-7,8,Difluoro-9-oxo-11,12,13,13a-tetrahydro-9H-imidazo[1,5-a]pyrrolo[2,1-c][1,4]benzodiazepin-1-carboxamidoxim zu und rührte 16 Stunden bei 90°. Die erhaltene braune Lösung wurde am Hochvakuum eingedampft, und der erhaltene braune Rückstand wurde chromatographiert (Kieselgel, Methylenchlorid /Methanol 19:1). Man erhiell 4.2 g (78%) (S)-1-(5-BOC-Aminomethyl-1,2,4-oxadiazol-3-yl)-7,8-difluoro-11,12,13,13a-tetrahydro-9H-imidazo[1,5-a]pyrrolo[2,1-c][1,4]benzodiazepin-9-on als hellgelben Schaum, Rf=0.22 (Kieselgel, Methylenchlorid/Methanol 19:1).

h) Eine Lösung von 3.3 g (7 mmol) (S)-1-(5-BOC-Aminomethyl-1,2,4-oxadiazol-3-yl)-7,8-difluoro-11,12,13,13a-tetrahydro-9H-imidazo[1,5-a]pyrrolo-[2,1-c][1,4]benzodiazepin-9-on in 20 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 2 ml wässeriger Ammoniaklösung basisch gestellt und sechs Mal mit Methylenchlorid extrahiert. Die organischen Phasen wurden mit Natriumsulfat getrocknet, filtriert und eingedampft. Der erhaltene Rückstand wurde chromatographiert (Kieselgel, Methylenchlorid/ Methanol/wässeriges Ammoniak 110:10:0.1). Man erhielt 2 g (77%) (S)-1-(5-Aminomethyl-1,2,4-oxadiazol-3-yl)-7,8-difluoro-11,12,13,13a-tetrahydro-9H-imidazo[1,5-a]pyrrolo[2,1-c] [1,4]benzodiazepin-9-on als beiges Pulver vom Smp. 218-220° (Zers.).

Beispiel 100

Zu einer Lösung von 650 mg (1.76 mmol) (S)-1-(5-Aminomethyl-1,2,4-oxadiazol-3-yl)-7,8-difluoro-11,12,13,13a-tetrahydro-9H-imidazo[1,5-a]pyrrolo-[2,1-c][1,4]benzodiazepin-9-on in 40 ml Methylenchlorid wurden 2.1 ml (10.5 mmol) N-Aethyldiisopropylamin und 0.6 ml (7 mmol) Allylbromid zugegeben und 20 Stunden bei Raumtemperatur gerührt. Die Reaktionslösung wurde anschliessend mit Methylenchlorid verdünnt und drei Mal mit Wasser gewaschen. Die organischen Phasen wurden mit Magnesium-sulfat getrocknet, filtriert und eingedampft. Der erhaltene Rückstand wurde zwei Mal chromatographiert (Kieselgel, Methylenchlorid/Methanol/ wässeriges Ammoniak 140:10: 0.1). Man erhielt 625 mg (78 %) (S)-1-[5-(Diallylaminomethyl)-1,2,4-oxadiazol-3-yl]-7,8-difluoro-11,12,13,13a-tetrahydro-9H-imidazo[1,5-a]pyrrolo[2,1-c][1,4]benzodiazepin-9-on als farblosen Schaum, Rf=0.45 (Kieselgel, Methylenchlorid/Methanol /wässeriges Ammoniak 140:10:0.1).

Beispiel 101

Zu einer Lösung von 650 mg (1.76 mmol) (S)-1-(5-Aminomethyl-1,2,4-oxadiazol-3-yl)-7,8-difluoro-11,12,13,13a-tetrahydro-9H-imidazo[1,5-a]pyrrolo-[2,1-c][1,4]benzodiazepin-9-on in 40 ml Methylenchlorid wurden 2.1 ml (10.5 mmol) N-Aethyldiisopropylamin und 0.8 ml (7 mmol) Dimethylallylbromid zugegeben und 20 Stunden bei Raumtemperatur gerührt. Die Reaktionslösung wurde anschliessend mit Methylenchlorid verdünnt und drei Mal mit Wasser gewaschen. Die organischen Phasen wurden mit Magnesium-sulfat getrocknet, filtriert und eingedampft. Der erhaltene Rückstand wurde zwei Mal chromatographiert (Kieselgel, Methylenchlorid/Methanol/ wässeriges Ammoniak 140:10:0.1). Man erhielt 589 mg (66%) (S)-1-[5-[Bis-(3-methyl-but-2-enyl)-aminomethyl]-1,2,4-oxadiazol-3-yl]-11,12,13,13a-tetrahydro-9H-imidazo[1,5-a]pyrrolo[2,1-c][1,4]benzodiazepin-9-on als farblosen Schaum, Rf=0.51 (Kieselgel, Methylenchlorid/Methanol /wässeriges Ammoniak 140:10:0.1).

Beispiel 102

2 g (5.95 mMol) (S)-1-(3-Aminomethyl-1,2,4-oxadiazo]-5-yl)-8-chlor-11,12,13,13a-tetrahydro-9H-imidazo [1,5-a]pyrrolo[2,1-c][1,4]benzodiazepin-9-on, 30 ml N,N-Dimethylformamid, 3.1 ml (17.8 mMol) N-Aethyldiisopropylamin und 1.1 ml (13.1 mMol) Allylbromid wurden während 1.5 Stunden bei Raumtemperatur gerührt. Die Reaktionslösung wurde an 250 g Kieselgel unter Eluieren mit Methylenchlorid/Essigester 7/3 chromatographiert . Man erhielt 2 g (81%) (S)-1-(3-Diallylaminomethyl-1,2,4-oxadiazol-5-yl)-8-chlor-11,12,13,13a-tetrahydro-9H-imidazo[1,5-a]pyrrolo [2,1-c][1,4]benzodiazepin-9-on, das in das Hydrochlorid vom Smp. 175-177° überführt wurde.

Beispiel 103

2 g (6.2 mMol) (S)-1-(3-Aminomethyl-1,2,4-oxadiazol-5-yl)-12,12a-dihydro-9H,11H-azeto[2,1-c]imidazo [1,5-a][1,4]benzodiazepin-9-on, 30 ml N,N-Dimethylformamid, 3.7 ml (21.7 mMol) N-Aethyldiisopropylamin und 1.6 ml (14.3 mMol) 1-Jodbutan wurden während 6 Stunden bei Raum-temperatur und 1.5 Stunden bei 80° gerührt.Das Reaktionsgemisch wurde eingedampft, und der Rückstand wurde an 180 g Kieselgel unter Eluieren mit Methylenchlorid/ Essigester 7/3 chromatographiert . Durch Einengen der einheitlichen Fraktionen erhielt man 1.6 g (59%) (S)-1-(3-Dibutylaminomethyl-1,2,4-oxadiazol-5-yl)-12,12a-dihydro-9H,11H-azeto[2,1-c]-imidazo[1,5-a][1,4]benzodiazepin-9-on, das in das Hydrochlorid überführt wurde.

Beispiel 104

2g 6.44 mMol) 3-(3-Aminomethyl-1,2,4-oxadiazol-5-yl) -5,6-dihydro-5-methyl-4H-imidazo[1,5-a][1,4]benzodiazepin-6-on, 20 ml N,N-Dimethylformamid, 3.3 ml (19.3 mMol) N-Aethyldiisopropylamin und 1.65 ml (13.5 mMol) 3,3-Dimethylallylbromid wurden während 2 Stunden bei Raum-temperatur gerührt. Nach Eindampfen des Lösungsmittel wurde der Rückstand in Methylenchlorid gelöst, und die Lösung wurde mit Wasser gewaschen, getrocknet und eingeengt. Durch Chromatographieren an 220 g Kieselgel unter Eluieren mit Methylenchlorid/Essigester 1/1 erhielt man 1.9 g (69%) 3-[3-Bis-(3-methyl-but-2-enyl)aminomethyl-1,2,4-oxadiazol-5-yl]-5,6-dihydro-5-methyl-4H-imidazo [1,5-a][1,4]benzodiazepin-6-on, das in das Hydrochlorid überführt wurde.

Beispiel 105

2 g (6.44 mMol) 3-(3-Aminomethyl-1,2,4-oxadiazol-5-yl)-5,6-dihydro-5-methyl-4H-imidazo[1,5-a][1,4]benzodiazepin-6-on, 20 ml N,N-Dimethylformamid, 3.8 ml (22.5 mMol) N-Aethyldiisopropylamin und 1.6 ml (14.8 mMol) Brommethylcyclopropan wurden während 4 Stunden bei 80° gerührt. Nach Eindampfen des Lösungsmittels wurde der Rückstand in Methylenchlorid gelöst, und die Lösung wurde mit Wasser gewaschen, getrocknet und eingeengt. Durch Chromatographieren an 220 g Kieselgel unter Eluieren mit Methylenchlorid/Essigester 1/1 erhielt man 1.3 g (48%) 3-[3-(Bis-cyclopropylmethylaminomethyl)-1,2,4-oxadiazol-5-yl]-5,6-dihydro-5-methyl-4H-imidazo[1,5-a][1,4]benzodiazepin-6-on, das in das Hydrochlorid vom Smp. 170-174° überführt wurde.

Beispiel 106

a) Eine Suspension von 5.8 g (0.020 Mol) 8-Fluoro-5,6-dihydro-5-methyl-6-oxo-4H-imidazo[1,5-a][1,4]benzodiazepin-3-carboxamidoxim in 60 ml Dimethylformamid wurde mit 3.9 g (0.023 Mol) Chloressigsäureanhydrid versetzt. Die erhaltene gelbe Lösung wurde 1½ Std. bei 100° gerührt und dann vollständig von den Lösungsmitteln befreit. Das ölige Produkt kristallisierte aus Acetonitril und wurde abfiltriert. Die Mutterlauge wurde eingeengt, der Rückstand wurde über Kieselgel mit Dichloromethan/ Methanol 97:3 als Laufmittel chromatographiert, und die erhaltene zusätzliche Portion des Produkts wurde aus Acetonitril umkristallisiert. Man erhielt insgesamt 4.05 g (59%) 3-(5-Chloromethyl-1,2,4-oxadiazol-3-yl)-8-fluoro-5-methyl-5,6-dihydro-4H-imidazo[1,5-a][1,4]benzodiazepin-6-on als weisse Kristalle; Smp. 245-247°.

b) Eine Suspension von 1.5 g (0.0043 Mol) 3-(5-Chloromethyl-1,2,4-oxadiazol-3-yl)-8-fluoro-5-methyl-5,6-dihydro-4H-imidazo[1,5-a][1,4]benzodiazepin-6-on in 15 ml Dimethylformamid wurde mit 1.6 g (0.022 Mol) Diethylamin versetzt. Nach 16 Std. Rühren bei Raumtemperatur wurde die erhaltene Lösung vollständig von den Lösungsmitteln befreit. Der Rückstand wurde über Kieselgel mit Dichloromethan/Methanol 9:1 als Laufmittel chromatographiert. Man erhielt 1.33 g (81%) 3-(5-Diethylaminomethyl-1,2,4-oxadiazol-3-yl)-8-fluoro-5-methyl-5,6-dihydro-4H-imidazo[1,5-a]-[1,4]benzodiazepin-6-on als weisse Kristalle; Smp. 172-174°.

c) 1.30 g (0.0034 Mol) 3-(5-Diethylaminomethyl-1,2,4-oxadiazol-3-yl)-8-fluoro-5-methyl-5,6-dihydro-4H-imidazo [1,5-a][1,4]benzodiazepin-6-on in 20 ml Ethanol wurden mit 0.79 ml (0.0037 Mol) 4.78 N ethanolischer Salzsäure versetzt. Nach Zugabe von 100 ml Ether fielen Kristalle aus. Man erhielt 1.28 g (90%) 3-(5-Diethylaminomethyl-1,2,4-oxadiazol-3-yl)-8-fluoro-5-methyl-5,6-dihydro-4H-imidazo[1,5-a][1,4]benzodiazepin-6-on hydrochlorid (1:1) als weisse Kristalle; Smp. 220-223° (Zers.).

Beispiel 107

a) Eine Suspension von 1.30 g (0.0037 Mol) 3-(5-Chloromethyl-1,2,4-oxadiazol-3-yl)-8-fluoro-5-methyl-5,6-dihydro-4H-imidazo[1,5-a][1,4]benzodiazepin-6-on in 15 ml Dimethylformamid wurde mit 2.4 g (0.019 Mol) Dibutylamin versetzt. Nach 65 Std. Rühren bei Raumtemperatur wurde die erhaltene orange Lösung vollständig vom Lösungsmittel befreit. Der Rückstand wurde über Kieselgel mit Dichloromethan/Methanol 19:1 als Laufmittel chromatographiert. Das Produkt wurde aus Methanol/Ether umkristallisiert, und man erhielt 1.27 g (77%) 3-(5-Dibutylaminomethyl-1,2,4-oxadiazol-3-yl)-8-fluoro-5-methyl-5,6-dihydro-4H-imidazo[1,5-a][1,4]-benzodiazepin-6-on als weisse Kristalle; Smp. 137-140°.

b) 1.17 g (0.0027 Mol) 3-(5-Dibutylaminomethyl-1,2,4-oxadiazol-3-yl)-8-fluoro-5-methyl-5,6-dihydro-4H-imidazo [1,5-a][1,4]benzodiazepin-6-on in 30 ml Ethanol wurden mit 0.65 ml (0.0031 Mol) 3.7 N ethanolischer Salzsäure versetzt. Die Lösung wurde vollständig vom Lösungsmittel befreit, und der Rückstand wurde aus Aceton umkristallisiert. Man erhielt 0.87 g (69%) 3-(5-Dibutylaminomethyl-1,2,4-oxadiazol-3-yl)-8-fluoro-5-methyl-5,6-dihydro-4H-imidazo[1,5-a][1,4]benzodiazepin-6-on hydrochlorid (1:1) als weisse Kristalle; Smp. 183-185°.

Beispiel 108

a) Eine Suspension von 1.50 g (0.0043 Mol) 3-(5-Chloromethyl-1,2,4-oxadiazol-3-yl)-8-fluoro-5-methyl-5,6-dihydro-4H-imidazo[1,5-a][1,4]benzodiazepin-6-on in 15 ml Dimethylformamid wurde mit 1.5 g (0.022 Mol) Pyrrolidin versetzt. Nach 65 Std. Rühren bei Raumtemperatur wurde die erhaltene gelborange Lösung vollständig vom Lösungsmittel befreit. Der Rückstand wurde über Kieselgel mit Dichloromethan/Methanol 19:1 als Laufmittel chromatographiert. Das Produkt wurde aus Methanol/Ether umkristallisiert. Man erhielt 1.35 g (82%) 8-Fluoro-5-methyl-3-(5-pyrrolidin-1-ylmethyl-1,2,4-oxadiazol-3-yl)-5,6-dihydro-4H-imidazo[1,5-a][1,4]benzodiazepin-6-on als weisse Kristalle; Smp. 158-160°.

b) 1.32 g (0.0035 Mol) 8-Fluoro-5-methyl-3-(5-pyrrolidin-1-ylmethyl-1,2,4-oxadiazol-3-yl)-5,6-dihydro-4H-imidazo [1,5-a][1,4]benzodiazepin-6-on wurden in 50 ml heissem Ethanol gelöst. Bei Raumtemperatur fügte man 0.8 ml (0.0038 Mol) 4.78 N ethanolischer Salzsäure zu. Die Lösung wurde auf ein Volumen von ~20 ml eingeengt, wobei Kristallisation begann. Man gab 80 ml Ether zu, filtrierte ab und erhielt 1.38 g (96%) 8-Fluoro-5-methyl-3-(5-pyrrolidin-1-ylmethyl-1,2,4-oxadiazol-3-yl)-5,6-dihydro-4H-imidazo[1,5-a]-[1,4]benzodia-zepin-6-on hydrochlorid (1:1) als weisse Kristalle; Smp. 243-245° (Zers.).

Beispiel 109

a) Zu einer Suspension von 7.0 g (23 mmol) 8-Fluoro-5-methyl-6-oxo-5,6-dihydro-4H-imidazo[1,5-a][1,4]benzodiazepin-3-carbonsäureäthylester in 70 ml Aethanol wurden 13 ml Hydrazinhydrat zugegeben, und das Gemisch wurde 3 Stunden am Rückfluss erhitzt. Nach Abkühlen auf 0° filtrierte man die erhaltenen Kristalle ab und erhielt 6.43 g (96 %) 8-Fluoro-5-methyl-6-oxo-5,6-dihydro-4H-imidazo[1,5-a][1,4]benzodiazepin-3-carbonsäurehydrazid als farblose Nadeln vom Smp. 288 - 290°.

b) Eine Lösung von 4.46 g ( 21.75 mmol) N-Phthaloylglycin in 35 ml Dimethylformamid versetzte man bei Raumtemperatur mit 3.66 g (22.6 mmol) 1,1'-Carbonyldiimidazol und erhitzte anschliessend auf 50°. Nach 30 Minuten kühlte man auf Raumtemperatur ab, gab 6.43 g ( 22.15 mmol) 8-Fluoro-5-methyl-6-oxo-5,6-dihydro-4H-imidazo [1,5-a][1,4]benzodiazepin-3-carbonsäurehydrazid dazu und rührte 12 Stunden bei Raumtemperatur. Die erhaltene Suspension wurde filtriert, und das erhaltene farblose Pulver wurde mit Aethanol und Diäthyläther gewaschen. Man erhielt 10.2 g ( 98 %) 8-Fluoro-5-methyl-6-oxo-5,6-dihydro-4H-imidazo[1,5-a][1,4]benzodiazepin-3-carbonsäure N'-(2,3-dioxo-2,3-dihydro-1H-isoindol-2-ylacetyl)-hydrazid vom Smp. > 280°.

c) Eine Lösung von 6.0 g ( 12.6 mmol) 8-Fluoro-5-methyl-6-oxo-5,6-dihydro-4H-imidazo[1,5-a][1,4]benzodiazepin-3-carbonsäure N'-(2,3-dioxo-2,3-dihydro-1H-isoindol-2-ylacetyl)-hydrazid in 38 g Polyphosphosphorsäure wurde während 1.5 Stunden bei 100° gerührt. Nach Abkühlen auf Raumtemperatur wurde das Gemisch unter gutem Rühren auf 300 ml Eiswasser gegossen, worauf bis zu pH=8 festes Natriumcarbonat zugegeben wurde. Extraktion mit Methylenchlorid und Chromatographie (Kieselgel, Methylenchlorid/Methanol 20 : 1) lieferte 4.9 g (85 %) 2-[5-(8-Fluoro-5-methyl-6-oxo-5,6-dihydro-4H-imidazo[1,5-a][1,4]benzodiazepin-3-yl)-1,3,4-oxadiazol-2-ylmethyl]-2.3-dihydro-1H-isoindol-1.3-dion als farbloses Pulver vom Smp. >250.

d) Zu einer Suspension von 4.9 g (10.7 mmol) 2-[5-(8-Fluoro-5-methyl-6-oxo-5,6-dihydro-4H-imidazo[1,5-a][1,4] benzodiazepin-3-yl)-1.3.4-oxadiazol-2-ylmethyl]-2.3-dihydro-1H-isoindol-1.3-dion in 100 ml Aethanol tropfte man bei 70° 60 ml Methylamin (33 % in Aethanol) zu und rührte während einer Stunde bei 70°. Der erhaltene Niederschlag wurde heiss abfiltriert, und das erhaltene gelbliche Pulver wurde mit Aethanol farblos gewaschen. Man erhielt 2.6 g (80 %) 3-(5-Aminomethyl-1,3,4-oxadiazol-2-yl)-8-fluoro-5-methyl-5,6-dihydro-4H-imidazo[1,5-c][1,4] benzodiazepin-6-on als farbloses Pulver vom Smp. 227-231°.

Beispiel 110

Zu einer Lösung von 0.656 g (2.0 mmol) 3-(5-Aminomethyl-1,3,4-oxadiazol-2-yl)-8-fluoro-5-methyl-5,6-dihydro-4H-imidazo[1,5-c][1,4]benzodiazepin-6-on in 15 ml Dimethylformamid wurden 3.5 ml N-Aethyldiisopropylamin und 1.1 ml (12 mmol) 1-Brompropan zugegeben, worauf 12 Stunden bei 70° gerührt wurde. Das Dimethylformamid wurde abgedampft, und der Rückstand wurde zwischen Methylenchlorid und 2N Natrimcarbonatlösung verteilt. Die wässerige Phase wurde zwei Mal mit Methylenchlorid gewaschen; die organischen Phasen wurden mit Natriumsulfat getrocknet, filtriert und eingedampft. Chromatographie (Kieselgel, Essigsäureäthylester/Methanol 20:1) lieferte 0.450 g (55%) 3-(5-Dipropylaminomethyl-1,3,4-oxadiazol-2-yl)-8-fluoro-5-methyl-5,6-dihydro-4H-imidazo[1,5-c][1,4]benzodiazepin-6-on als farblosen Schaum, Rf=0.48 (Kieselgel, EssigsäureäthylesterMethanol 20:1).

Beispiel 111

Zu einer Lösung von 0.500 g (1.52 mmol) (S)-1-(5-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 15 ml Dimethylformamid wurden 1.74 ml N-Aethyl-diisopropylamin und 0.55 ml (6 mmol) 1-Brompropan dazugegeben und 12 Stunden bei 70° gerührt. Das Dimethylformamid wurde mit eingedampft, und der Rückstand wurde zwischen Methylenchlorid und 2N Natriumcarbonatlösung verteilt. Die wässerige Phase wurde zwei Mal mit Methylenchlorid extrahiert, und die organischen Phasen wurden mit Natriumsulfat getrocknet, filtriert und eingedampft. Chromatographie (Kieselgel, Essigsäureäthylester/Methanol 20:1) lieferte 0.350 g (55%) (S)-1-(5-Dipropylaminomethyl-1,2,4-oxadiazol-3-yl)-11,11a-dihydro-8H,10H-azeto-[1,2-a]imidazo[5,1-c]thieno[3,2-e][1,4]diazepin-8-on als farblosen Schaum, Rf=0.44 (Kieselgel, Essigsäureäthylester/Methanol 20: 1).

Beispiel 112

1.04 g (3.0 mMol) 3-(5-Chlormethyl-1,2,4-oxadiazol-3-yl)-8-fluor-5,6-dihydro-5-methyl-4H-imidazo[1,5-a] [1,4]-benzodiazepin-6-on, 0.64 g (7.5 mMol) Piperidin und 10 ml N,N-Dimethylformamid wurden während 4 Stunden bei Raumtemperatur gerührt. Das Reaktionsgemisch wurde eingeengt, der Rückstand wurde in Methylenchlorid gelöst, und die Lösung wurde mit 4 N Natronlauge alkalisch gestellt. Man wusch die Lösung einmal mit gesättigter Natriumchlorid-Lösung, trocknete sie mit Magnesiumsulfat und dampfte sie ein. Durch Chromatographieren des Rückstandes an Kieselgel unter Eluieren mit Methylenchlorid/Methanol 9/1 erhielt man 0.89 g (75%) 8-Fluor-5-methyl-3-[5-(piperidin-1-yl-methyl)-1,2,4-oxadiazol-3-yl]-5,6-dihydro-4H-imidazo[1,5-a][1,4]-benzodiazepin-6-on vom Smp. 182-4°, welches in das Hydrochlorid vom Smp. 254-6° überführt wurde.

Beispiel 113

Eine Suspension von 4.77 g (13.7 mMol) 3-(5-Chloromethyl-1,2,4-oxadiazol-3-yl)-8-fluoro-5-methyl-5,6-dihydro-4H-imidazo[1,5-a][1,4]benzodiazepin-6-on in 70 ml Dimethylformamid wurde mit 5.6 ml (41.0 mMol) Dipropylamin versetzt. Nach 18 Std. Rühren bei Raumtemperatur wurde die erhaltene Lösung eingeengt, und der Rückstand wurde in 70 ml Wasser aufgenommen. Die Kristalle wurden abfiltriert, mit 10 ml Wasser gewaschen und bei 60° im Vakuum getrocknet. Der Rückstand wurde über 100 g Kieselgel mit Dichloromethan/Aceton 2:1 als Laufmittel chromatographiert. Man erhielt 4.19 g weisse Kristalle. Diese wurden zweimal aus Essigester umkristallisiert, wobei nach Trocknung bei 600/0.03 mbar 2.77 g (49%) 3-(5-Dipropylaminomethyl-1,2,4-oxadiazol-3-yl)-8-fluoro-5-methyl-5,6-dihydro-4H-imidazo[1,5a][1,4]benzodiazepin-6-on als weisse Kristalle mit Smp. 153-154° erhalten wurden.

Beispiel 114

Zu einer Lösung von 0.600 g (1.75 mmol) (S)-1-(5-Aminomethyl-1,3,4-oxadiazol-2-yl)-10,11,12,12a-tetrahydro-8H-imidazo[5,1-c]pyrrolo[1,2-aJ-thieno[3,2-e][1,4]diazepin-8-on in 30 ml Methylenchlorid wurden 2.4 ml (13.8 mmol) N-Aethyldiisopropylamin und 0.97 ml (8 mmol) Allylbromid zugegeben, worauf 12 Stunden bei 70° gerührt wurde. Die Reaktionslösung wurde mit Methylenchlorid verdünnt und mit 2N Natriumcarbonatlösung gewaschen. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.580 g (78 %) (S)-1-(5-Diallylaminomethyl-1,3,4-oxadiazol-2-yl)-10,11,12,12a-tetrahydro-8H-imidazo[5,1-c]pyrrolo[1,2-a] thieno[3,2-e][1,4]diazepin-8-on als farblosen Schaum, Rf=0.56 (Kieselgel, Essigsäureäthylester/Methanol 20:1).

Beispiel 115

Zu einer Lösung von 0.443 g (1.3 mmol) (S)-1-(5-Aminomethyl-1,3,4-oxadiazol-2-yl)-10,11,12,12a-tetrahydro-8H-imidazo[5,1-c]pyrrolo[1,2-a]-thieno[3,2-e][1,4]diazepin-8-on in 30 ml Methylenchlorid wurden 2 ml (11.5 mmol) N-Aethyldiisopropylamin und 0.3 ml (2.61 mmol) Dimethylallylbromid zugegeben, worauf 12 Stunden bei 70° gerührt wurde. Die Reaktionslösung wurde mit Methylenchlorid verdünnt und mit 2N Natrimcarbonatlösung gewaschen. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 10:1) lieferte 0.190 g (30 %) (S)-1-[5-[Bis-(3-methyl-but-2-enyl)-aminomethyl]-1,3,4-oxadiazol-2-yl)-10,11,12,12a-tetrahydro-8H-imidazo[5,1-c]pyrrolo[1,2-a]thieno[3,2-e][1,4]diazepin-8-on als farblosen Schaum, Rf=0.50 (Kieselgel, Essigsäureäthylester/Methanol 10:1).

Beispiel 116

Zu einer Lösung von 0.500 g (1.52 mmol) (S)-1-(5-Aminomethyl-1,3,4-oxadiazol-2-yl)-11,11a-dihydro-8H,10H-azeto[1,2-a]imidazo[5,1-c]thieno[3,2-e]-[1,4]diazepin-8-on in 30 ml Methylenchlorid wurden 2 ml (11.5 mol) N-Aethyldiisopropylamin und 0.97 ml (8 mmol) Allylbromid zugegeben, worauf 12 Stunden bei 70° gerührt wurde. Die Reaktionslösung wurde mit Methylenchlorid verdünnt und mit 2N Natrimcarbonatlösung gewaschen. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.476 g (77 %) (S)-1-[5-Diallylaminomethyl-1,3,4-oxadiazol-2-yl]-11,11a-dihydro-8H,10H-azeto[1,2-a]imidazo[5,1-c]thieno [3,2-e][1,4]diazepin-8-on als farblosen Schaum, Rf=0.36 (Kieselgel, Essigsäureäthylester/ Methanol 20:1).

Beispiel 117

Zu einer Lösung von 0.426 g (1.3 mmol) (S)-1-(5-Aminomethyl-1,3,4-oxadiazol-2-yl)-11,11a-dihydro-8H,10H-azeto[1,2-a]imidazo[5,1-c]thieno[3,2-e]-[1,4]diazepin-8-on in 30 ml Methylenchlorid wurden 2 ml (11.5 mlmol) N-Aethyldiisopropylamin und 0.3 ml (2.61 mmol) Dimethylallylbromid zugegeben, worauf 12 Stunden bei 70° gerührt wurde. Die Reaktionslösung wurde mit Methylenchlorid verdünnt und mit 2N Natrimcarbonatlösung gewaschen. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 40:1) lieferte 0.270 g (44 %) (13)-1-[5-[Bis-(3-methyl-but-2-enyl)-aminomethyl]-1,3,4-oxadiazol-2-yl]-11,11a-dihydro-8H,10H-azeto[1,2-a] imidazo[5,1-c]thieno[3,2-e][1,4]diazepin-8-on als farblosen Schaum, Rf=0.24 (Kieselgel, EssigsäureäthylesterMethanol 40:1).

Beispiel 118

Zu einer Lösung von 0.550 g (1.68 mmol) (S)-1-(5-Aminomethyl-1,3,4-oxadiazol-2-yl)-11,11a-dihydro-8H,10H-azeto[1,2-a]imidazo[5,1-c]thieno[3,2-e]-[1,4]diazepin-8-on in 30 ml Dimethylformamid wurden 3 ml (17.25 mmol) N-Aethyldiisopropylamin und 0.8 ml (8.8 mmol) Propylbromid zugegeben, worauf 12 Stunden bei 70° gerührt wurde. Das Dimethylformamid wurde abgedampft, und der Rückstand wurde zwischen Methylenchlorid und 2N Natrimcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.300 g (43 %) (S)-1-(5-Dipropylaminomethyl-1,3,4-oxadiazol-2-yl)-11,11a-dihydro-8H,10H-azeto [1,2-a]imidazo[5,1-c]thieno[3,2-e][1,4]diazepin-8-on als farblosen Schaum, Rf=0.28 (Kieselgel, Essigsäureäthylester-

Methanol 20:1).

Beispiel 119

Zu einer Lösung von 0.300 g (0.87 mmol) (S)-1-(5-Aminomethyl-1,3,4-oxadiazol-2-yl)-10,11,12,12a-tetrahydro-8H-imidazo[5,1-c]pyrrolo[1,2-a]thieno-[3,2-e][1,4]diazepin-8-on in 20 ml Dimethylformamid wurden 1.5 ml (8.6 mmol) N-Aethyldiisopropylamin und 0.4 ml (4.4 mmol) Propylbromid zugegeben, worauf 12 Stunden bei 70° gerührt wurde. Das Dimethylformamid wurde abgedampft, und der Rückstand wurde zwischen Methylenchlorid und 2N Natrimcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.140 g (38 %) (S)-1-(5-Dipropylaminomethyl-1,3,4-oxadiazol-2-yl)-10,11,12,12a-tetrahydro-8H-imidazo[5,1-c]pyrrolo[1,2-a]thieno[3,2-e][1,4]-diazepin-8-on als farblosen Schaum, Rf=0.28 (Kieselgel, Essigsäureäthylester/Methanol 20:1).

Beispiel 120

Zu einer Lösung von 0.656 g (2.0 mmol) 3-(5-Aminomethyl-1,3,4-oxadiazol-2-yl)-8-fluoro-5-methyl-5,6-dihydro-4H-imidazo[1,5-c][1,4]benzodiazepin-6-on in 15 ml Dimethylformamid wurden 3.5 ml N-Aethyl-diisopropylamin und 1.29 ml (12 mmol) 1-Brombutan zugegeben, worauf 12 Stunden bei 70° gerührt wurde. Das Dimethylformamid wurde abgedampft, und der Rückstand wurde zwischen Methylenchlorid und 2N Natrimcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.470 g (53%) 3-(5-Dibutylaminomethyl-1,3,4-oxadiazol-2-yl)-8-fluoro-5-methyl-5,6-dihydro-4H-imidazo[1,5-a][1,4]benzodiazepin-6-on als farblosen Schaum, Rf=0.66 (Kieselgel, EssigsäureäthylesterMethanol 20:1).

Beispiel 121

a) Zu einer Suspension von 7.0 g (23.54 mmol) (S)-12,12a-Dihydro-9-oxo-9H,11H-azeto[2,1-c]imidazo[1,5-a][1,4] diazepin-1-carbonsäureäthylester in 70 ml Aethanol wurden 13 ml Hydrazinhydrat zugegeben, und das Gemisch wurde 3 Stunden am Rückfluss erhitzt. Nach Abkühlen auf 0° filtrierte man die erhaltenen Kristalle ab und erhielt 3.6 g (96 %) (S)-12,12a-Dihydro-9-oxo-9H,11H-azeto[2,1-c]imidazo[1,5-a][1,4]diazepine-1-carbonsäurehydrazid als farblose Nadeln vom Smp. 246 - 248°.

b) Eine Lösung von 5 g (17.64 mmol) (S)-12,12a-Dihydro-9-oxo-9H,11H-azeto[2,1-c]imidazo[1,5-a][1,4]benzodiazepin-1-carbonsäurehydrazid und 3.06 g (19.41 mmol) Chloracetimidsäureäthylester in 40 ml Dimethylformamid und 10 ml Aethanol wurde 12 Stunden bei 90° gerührt. Das Lösungsmittel wurde eingedampft, und der Rückstand wurde zwischen Methylenchlorid und Wasser verteilt. Die Wasserphase wurde einmal mit Methylenchlorid nachgewaschen. Die organischen Phasen wurden mit Magnesiumsulfat getrocknet, filtriert und eingedampft. Chromatographie des Rückstandes (Kieselgel, Methylenchlorid/Methanol 93 : 3) lieferte 3.26 g (55 %) (S)-1-(5-Chlormethyl-1,3,4-oxadiazol-2-yl)-12,12a-dihydro-9H,11H-azeto[2,1-c]imidazo[1,5-a][1,4]benzodiazepin-9-on als farblosen Schaum, Rf= 0.5 (Methylenchlorid/Methanol 10 : 1).

c) Zu einer Lösung von 1.17 g (3.42 mmol) (S)-1-(5-Chlormethyl-1,3,4-oxadiazol-2-yl)-12,12a-dihydro-9H,11H-azeto[2,1-c]imidazo[1,5-a][1,4]benzodiazepin-9-on in 10 ml Dimethylformamid wurden 0.94 ml (73 mmol) Dipropylamin zugegeben, worauf 12 Stunden bei Raumtemperatur gerührt wurde. Das Dimethylformamid wurde abgedampft, und der Rückstand wurde zwischen Methylenchlorid und 2N Natriumcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Methylenchlorid/Methanol 95 : 5) lieferte 0.900 g (65%) (S)-1-(5-Dipropylaminomethyl-1,3,4-oxadiazol-2-yl)-12,12a-dihydro-9H,11H-azeto[2,1-c]imidazo[1,5-a][1,4]benzodiazepin-9-onals farblosen Schaum, Rf=0.46 (Kieselgel, Methylenchlorid/Methanol 95 : 5).

Beispiel 122

Zu einer Lösung von 4 g (12.18 mmol) (S)-1-(5-Aminomethyl-1,3,4-oxadiazol-2-yl)-11,11a-dihydro-8H,10H-azeto[1,2-a]imidazo[5,1-c]thieno[3,2-e]-[1,4]diazepin-8-on in 100 ml Dimethylformamid wurden 17.4 ml (100 mmol) N-Aethyldiisopropylamin und 7.87 ml (73 mmol) Butylbromid zugegeben, worauf 12 Stunden bei 70° gerührt wurde. Das

Dimethylformamid wurde abgedampft, und der Rückstand wurde zwischen Methylenchlorid und 2N Natriumcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 10:1) lieferte 2.1 g (39 %) (S)-1-(5-Dibutylaminomethyl-1,3,4-oxadiazol-2-yl)-11,11a-dihydro-8H,10H-azeto-[1,2-a]imidazo[5,1-c]thieno[3,2-e][1,4]diazepin-8-on als farblosen Schaum, Rf=0.4 (Kieselgel, Essigsäureäthylester/Methanol 10:1).

Beispiel 123

a) Eine Lösung von 5 g (17.28 mmol) 8-Fluoro-5,6-dihydro-5-methyl-6-oxo-4H-imidazo[1,5-a][1,4]benzodiazepin-3-carbonsäurehydrazid und 2.87 g (18.16 mmol) Chloracetimidsäureäthylester in 40 ml Dimethylformamid und 10 ml Aethanol wurde 12 Stunden bei 90° gerührt . Das Lösungsmittel wurde abgedampft, und der Rückstand wurde zwischen Methylenchlorid und Wasser verteilt. Die Wasserphase wurde einmal mit Methylenchlorid nachgewaschen. Die organischen Phasen wurden mit Magnesiumsulfat getrocknet, filtriert und eingedampft. Chromatographie des Rückstandes (Kieselgel, Methylenchlorid/Methanol 93 : 3) lieferte 3.66 g (55 %) (S)-1-(5-Chlormethyl-1,3,4-oxadiazol-2-yl)- 8-fluoro-5,6-dihydro-5-methyl-4H-imidazo[1,5-a][1,4]benzodiazepin-6 on als farblose Kristalle mit Smp. 260-262° (Zers.).

b) Zu einer Lösung von 2 g (5.8 mmol) (S)-1-(5-Chlormethyl-1,3,4-oxadiazol-2-yl)- 8-fluoro-5,6-dihydro-5-methyl-4H-imidazo[1,5-a][1,4]benzodiazepin-6-on in 50 ml Dimethylformamid wurden 1.0 ml (12.7 mmol) Propylamin zugegeben, worauf 12 Stunden bei 55° gerührt wurde. Das Dimethylformamid wurde abgedampft, und der Rückstand wurde zwischen Methylenchlorid und 2N Natriumcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Methylenchlorid/Methanol/ wässeriges Ammoniak 140 : 10 :1) lieferte 1.40 g (65%) (S)-1-(5-Propylaminomethyl-1,3,4-oxadiazol-2-yl)-8-fluoro-5,6-dihydro-5-methyl-4H-imidazo[1,5-a]-[1,4]benzodiazepin-6-on als farblosen Schaum, Rf=0.49 (Kieselgel, Methylenchlorid/Methanol/wässeriges Ammoniak 110. : 10 : 1).

Beispiel 124

a) Eine Suspension von 3.06 g (9.22 mMol) (S)-8-Chlor-9-oxo-11,12,13,13a-tetrahydro-9H-imidazo[1,5-a]pyrrolo[2,1-c][1,4]benzodiazepin-1-carbonsäurehydrazid in 30 ml N,N-Dimethylformamid wurde mit 1.97 g (0.0115 Mol) Chloressigsäureanhydrid versetzt. Die erhaltene gelbe Lösung wurde 1½ Std. bei Raumtemperatur gerührt und dann vollständig von den Lösungsmitteln befreit. Der Rückstand wurde in Aether suspendiert und abgenutscht. Die erhaltenen beigen Kristalle (3.75 g, Smp. 262-264°C (Zers.)) wurden mit 27 ml Methansulfonsäure und 3 g Phosphorpentoxid versetzt, worauf 65 Std. bei Raumtemperatur nachgerührt wurde. Die orange-braune Lösung wurde auf 150 ml Eiswasser gegossen, mit wässriger Natriumhydroxidlösung basisch gestellt und mit Methylenchlorid extrahiert. Das kristalline Produkt wurde über Kieselgel mit Essigester als Laufmittel chromatographiert. Man erhielt 2.03 g (56%) (S)-8-Chlor-1-(5-chlormethyl-1,3,4-oxadiazol-2-yl)-11,12,13,13a-tetrahydro-9H-imidazo[1,5-a]pyrrolo[2,1-c]-[1,4]benzodiazepin-9-on als weisse Kristalle; Smp. 230-232° und $[\alpha]_D^{20}$ = +104.5° (DMF, c = 1%).

b) Eine Suspension von 1.30 g (3.33 mMol) (S)-8-Ch]or-1-(6-chlormethyl-1,3,4-oxadiazol-2-yl)-11,12,13,13a-tetrahydro-9H-imidazo[1,5-a]pyrrolo[2,1-c]-[1,4]benzodiazepin-9-on in 10 ml N,N-Dimethylformamid wurde mit 1.7 g (0.017 Mol) Dipropylamin versetzt. Nach 20 Std. Rühren bei Raumtempera-tur und 4 Std. bei 80°C wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether umkristallisiert. Man erhielt 1.27 g (85%) (S)-8-Chlor-1-(5-dipropylaminomethyl-1,3,4-oxadiazol-2-yl)-11,12,13,13a-tetrahydro-9H-imidazo[1,5-a]pyrrolo[2,1-c][1,4]benzodiazepin-9-on als weisse Kristalle; Smp. 158-160° und $[\alpha]_D^{20}$ = +48.8° ($CH_2Cl_2$, c = 1%).

c) 1.27 g (2.79 mMol) (S)-8-Chlor-1-(5-dipropylaminomethyl-1,3,4-oxadiazol-2-yl)-11,12,13,13a-tetrahydro-9H-imidazo[1,5-a]pyrrolo[2,1-c][1,4]-benzodiazepin-9-on in 30 ml Aethanol wurden mit 0.83 ml (3.07 mMol) 3.7 N aethanolischer Salzsäure versetzt. Nach 15 Minuten Rühren bei 0° wurde die Lösung tropfenweise mit 150 ml

Aether versetzt, und die erhaltene weisse Suspension wurde abgenutscht. Man erhielt 1.27 g (93%) (S)-8-Chlor-1-(5-dipropylaminomethyl-1,3,4-oxadiazol-2-yl)-11,12,13,13a-tetrahydro-9H-imidazo[1,5-a]pyrrolo[2,1-c][1,4]benzodiazepin-9-on hydrochlorid (1:1) als weisse Kristalle; Smp. 204-206° und $[\alpha]_D^{20}$ = -43.6° ($H_2O$, c = 1%).

Beispiel 125

a) Zu einer Suspension von 5.47 g (0.00162 Mol) 7-Chlor-8-fluor-5-methyl-6-oxo-5,6-dihydro-4H-imidazo[1,5-a][1,4]benzodiazepin-3-carbonsäureaethylester in 55 ml Aethanol wurden 9.15 ml Hydrazinhydrat zugegeben, und das Gemisch wurde 18 Stunden am Rückfluss erhitzt. Nach Abkühlen auf 0° filtrierte man die erhaltenen Kristalle ab und erhielt 3.28 g (63 %) 7-Chlor-8-fluor-5-methyl-6-oxo-5,6-dihydro-4H-imidazo[1,5-a][1,4]-benzodiazepin-3-carbonsäurehydrazid als weisse Kristalle; Smp. 308-310°.

b) Eine Suspension von 0.50 g (0.00154 Mol) 7-Chlor-8-fluor-5-methyl-6-oxo-5,6-dihydro-4H-imidazo[1,5-a][1,4]benzodiazepin-3-carbonsäurehydrazid in 5 ml N,N-Dimethylformamid wurde mit 0.33 g (0.00193 Mol) Chloressigsäureanhydrid versetzt. Die erhaltene Lösung wurde 1½ Std. bei Raumtempratur gerührt, wobei eine Suspension entstand. Man kühlte auf 0° ab, versetzte mit 40 ml Aether und nutschte ab. Die erhaltenen weissen Kristalle (0.643 g, Smp. 264-266° (Zers.)) wurden mit 4.5 ml Methansulfonsäure und 0.5 g Phosphorpentoxid versetzt, worauf das Gemisch erwärmt und die Lösung 1 Std. bei Raumtemperatur nachgerührt wurde. Die gelbe Lösung wurde auf 75 ml Eiswasser gegossen, worauf mit wässriger Natriumhydroxidlösung basisch gestellt und mit Methylenchlorid extrahiert wurde. Das kristalline Produkt wurde über Kieselgel mit Essigester als Laufinittel chromatographiert. Man erhielt 0.193 g (33%) 7-Chlor-3-(5-chlormethyl-1,3,4-oxadiazol-2-yl)-8-fluor-5-methyl-5,6-dihydro-4H-imidazo[1,5-a][1,4]benzodiazepin-6-on als weisse Kristalle; Smp. 234-236°.

c) Eine Suspension von 0.193 g (0.51 mMol) 7-Chlor-3-(5-chlormethyl-1,3,4-oxadiazol-2-yl)-8-fluor-5-methyl-5,6-dihydro-4H-imidazo [1,5-a][1,4]-benzodiazepin-6-on in 2 ml N,N-Dimethylformamid wurde mit 0.26 g (2.55 mMol) Dipropylamin versetzt. Nach 17 Std. Rühren bei Raumtemperatur wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether/n-Hexan bei 0°C umkristallisiert. Man erhielt 0.094 g (41%) 7-Chlor-3-(5-dipropylaminomethyl-1,3,4-oxadiazol-2-yl)-8-Fluor-5-methyl-5,6-dihydro-4H-imidazo[1,5-a]-[1,4]benzodiazepin-6-on als weisse Kristalle; Smp. 128-130°.

d) 0.094 g (0.21 mMol) 7-Chlor-3-(5-dipropylaminomethyl-1,3,4-oxadiazol-2-yl)-8-fluor-5-methyl-5,6-dihydro-4H-imidazo[1,5-a][1,4]benzodiazepin-6-on in 10 ml Aethanol wurden mit 0.062 ml (0.23 mMol) 3.7 N aethanolischer Salzsäure versetzt. Nach 30 Minuten Rühren bei 0° wurde die Lösung vollständig von den Lösungsmitteln befreit, worauf der Rückstand in 20 ml Aether suspendiert und abgenutscht wurde. Man erhielt 0.84 g (83%) 7-Chlor-3-(5-dipropylaminomethyl-1,3,4-oxadiazol-2-yl)-8-fluor-5-methyl-5,6-dihydro-4H-imidazo[1,5-a][1,4]benzodiazepin-6-on hydrochlorid (1:1) als weisse Kristalle; Smp. 152-154°.

Beispiel 126

a) 4.4 g (14.1 mMol) (12.3 mMol) (S)-8-Chlor-9-oxo-12,12a-dihydro-9H,11H-azeto[2,1-c]-imidazo[1,5-a][1,4]benzodiazepin-1-carbonsäurehydrazid wurden über Nacht in 30 ml N,N-Dimethylformamid mit 2.65 g (15.5 mMol) Chloressigsäureanhydrid bei Raumtemperatur gerührt. Nach Eindampfen des Lösungsmittels wurde der Rückstand während 48 Stunden mit 20 ml einer 10%iger Lösung von Phosphorpentoxid in Methansulfonsäure bei Raum-temperatur gerührt. Das Reaktionsgemisch wurde mit Eis versetzt, mit konz. Natronlauge alkalisch gestellt und viermal mit Methylenchlorid ausgeschüttelt. Durch Trocknen der organischen Phase über Magnesium-sulfat und Eindampfen des Lösungsmittels erhielt man 3.39 g (60%) (S)-8-Chlor-1-(5-chlormethyl-1,3,4-oxadiazol-2-yl)-12,12a-dihydro-9H,11H-azeto-[2,1-c]imidazo[1,5-a][1,4]benzodiazepin-9-on vom Smp. 185-189°.

b) 1.88 g (5 mMol) (S)-8-Chlor-1-(5-chlormethyl-1,3,4-oxadiazol-2-yl)-12,12a-dihydro-9H,11H-azeto[2,1-c]imidazo[1,5-a][1,4]benzodiazepin-9-on wurden über das Wochenende mit 1.12 g (11 mMol) Dipropylamin und 10 ml N,N-Dimethylformamid bei Raumtemperatur gerührt. Nach Eindampfen des Reaktionsgemisches wurde der Rückstand in Methylenchlorid gelöst, und die Lösung wurde zweimal mit Wasser gewaschen und über Magnesiumsulfat getrocknet. Nach Eindampfen des Lösungsmittels und Kristallisieren des Rückstandes aus Essigester und Hexan

erhielt man 2.15 g (98%) (S)-8-Chlor-1-(5-dipropylaminomethyl-1,3,4-oxadiazol-2-yl)-12,12a-dihydro-9H,11H-azeto[2,1-c]imidazo[1,5-a][1,4]benzodiazepin-9-on vom Smp. 134-136°, das in das Hydrochlorid vom Schmelzpunkt 235-237° überführt wurde.

Beispiel 127

a) 9.4 g (34.7 mMol) 5-Methyl-6-oxo-5,6-dihydro-4H-imidazo[1,5-a][1,4]-benzodiazepin-3-carbonsäurehydrazid wurden während 3 Stunden in 75 ml N,N-Dimethylformamid mit 27.1 g (41.5 mMol) Chloressigsäureanhydrid bei Raumtemperatur gerührt. Nach Eindampfen des Lösungsmittels wurde der Rückstand über Nacht mit 75 ml einer 10%iger Lösung von Phosphorpentoxid in Methansulfonsäure bei Raumtemperatur gerührt. Das Reaktionsgemisch wurde mit Eis versetzt, mit konz. Natronlauge alkalisch gestellt und viermal mit Methylenchlorid ausgeschüttelt. Durch Trocknen der organischen Phase über Magnesium-sulfat, Eindampfen des Lösungsmittels und Chromatographieren des Rückstandes an Kieselgel unter Eluieren mit Essigester erhielt man 6.9 g (61%) 3-(5-Chlormethyl-1,3,4-oxadiazol-2-yl)-5-methyl-5,6-dihydro-4H-imidazo[1,5-a][1,4]benzodiazepin-6-on vom Smp. 221-222°.

b) 0.33 g (1 mMol) 3-(5-Chlormethyl-1,3,4-oxadiazol-2-yl)-5-methyl-5,6-dihydro-4H-imidazo[1,5-a][1,4]benzodiazepin-6-on wurden über Nacht mit 2 ml (2 mMol) Diäthylamin und 5 ml N,N-Dimethylformamid bei Raumtemperarur gerührt. Nach Eindampfen des Reaktionsgemisches und Chromatographieren des Rückstandes an Kieselgel unter Eluieren mit Essigester/Aethanol 9/1 erhielt man 0.31 g (84%) 3-(5-Diäthylaminomethyl-1,3,4-oxadiazol-2-yl)-5-methyl-5,6-dihydro-4H-imidazo[1,5-a][1,4]benzodiazepin-6-on, das in das Hydrochlorid vom Smp. 235-238° überführt wurde.

Beispiel 128

a) 50 g (156.4 mMol) Aethyl 7-Chlor-5-methyl-6-oxo-5,6-dihydro-4H-imidazo[1,5-a][1,4]benzo-diazepin-3-carboxylat wurden während 6 Stunden mit 90 ml (1.85 Mol) Hydrazinhydrat und 500 ml Aethanol bei Siedetemperatur gerührt. Durch Abkühlen auf -10°, Abnutschen der Suspension und Trocknen des Kristallisats erhielt man 51.9 g (100%) 7-Chlor-5-methyl-6-oxo-5,6-dihydro-4H-imidazo[1,5-a][1,4]benzodiazepin-3-carbonsäurehydrazid vom Smp. 287°.

b) 15.3 g (50 mMol) 7-Chlor-5-methyl-6-oxo-5,6-dihydro-4H-imidazo-[1,5-a][1,4]-benzodiazepin-3-carbonsäurehydrazid wurden über Nacht in 160 ml N,N-Dimethylformamid mit 9.41 g (55 mMol) Chloressigsäureanhydrid bei Raumtemperatur gerührt. Nach Eindampfen des Lösungsmittels wurde der Rückstand über das Wochenende mit 100 ml einer 10%igen Lösung von Phosphorpentoxid in Methansulfonsäure bei Raumtemperatur gerührt. Das Reaktions-gemisch wurde mit Eis versetzt, mit konz. Natronlauge alkalisch gestellt und viermal mit Methylenchlorid ausgeschüttelt. Durch Trocknen der organischen Phase über Magnesium-sulfat, Eindampfen des Lösungsmittels und Chromatographieren des Rückstandes an Kieselgel unter Eluieren mit Essigester erhielt man 9.7 g (53%) 7-Chlor-3-(5-chlormethyl-1,3,4-oxadiazol-2-yl)-5-methyl-5,6-dihydro-4H-imidazo[1,5-a][1,4]benzodiazepin-6-on vom Smp. 201-203°.

c) 1.09 g (3 mMol) 7-Chlor-3-(5-chlormethyl-1,3,4-oxadiazol-2-yl)-5-methyl-5,6-dihydro-4H-imidazo[1,5-a][1,4] benzodiazepin-6-on wurden über Nacht mit 1.5 g (11.6 mMol) Dibutylamin und 15 ml N,N-Dimethylformamid bei Raumtemperatur gerührt. Nach Eindampfen des Reaktionsgemisches und Chromatographieren des Rückstandes an Kieselgel unter Eluieren mit Essigester erhielt man 1.09 g (79%) 7-Chlor-3-(5-dibutylaminomethyl-1,3,4-oxadiazol-2-yl)-5-methyl-5,6-dihydro-4H-imidazo[1,5-a][1,4]benzodiazepin-6-on, das in das Hydrochlorid vom Smp. 105-108° überführt wurde.

Beispiel 129

1.09 g (3 mMol) 7-Chlor-3-(5-chlormethyl-1,3,4-oxadiazol-2-yl)-5-methyl-5,6-dihydro-4H-imidazo[1,5-a][1,4] benzodiazepin-6-on wurden über Nacht mit 1 g (10 mMol) Dipropylamin und 15 ml N,N-Dimethylformamid bei Raumtemperarur gerührt. Nach Eindampfen des Reaktionsgemisches und Chromatographieren des Rückstandes an Kieselgel unter Eluieren mit Essigester erhielt man 1.17 g (91%) 7-Chlor-3-(5-dipropylaminomethyl-1,3,4-oxadiazol-2-yl)-5-methyl-5,6-dihydro-4H-imidazo[1,5-a][1,4]benzodiazepin-6-on, das in das Hydrochlorid vom Smp. 188-195° überführt wurde.

Beispiel 130

1.09 g (3 mMol) 7-Chlor-3-(5-chlormethyl-1,3,4-oxadiazol-2-yl)-5-methyl-5,6-dihydro-4H-imidazo[1,5-a][1,4] benzodiazepin-6-on wurden über Nacht mit 1 g (13.7 mMol) Diäthylamin und 15 ml N,N-Dimethylformamid bei Raumtemperatur gerührt. Nach Eindampfen des Reaktionsgemisches und Chromatographieren des Rückstandes an Kieselgel unter Eluieren mit Essigester/Aethanol 9/1 erhielt man 1.18 g (98%) 7-Chlor-3-(5-diäthylaminomethyl-1,3,4-oxadiazol-2-yl)-5-methyl-5,6-dihydro-4H-imidazo[1,5-a][1,4]-benzodiazepin-6-on, das in das Hydrochlorid vom Smp. 188-195° überführt wurde.

Beispiel 131

10 g (30.3 mMol) 3-(5-Chlormethyl-1,3,4-oxadiazol-2-yl)-5-methyl-5,6-dihydro-4H-imidazo[1,5-a][1,4]benzodiazepin-6-on wurden während 7 Stunden mit 10.1 g (100 mMol) Dipropylamin und 70 ml N,N-Dimethylformamid bei Raumtemperatur gerührt. Nach Eindampfen des Reaktionsgemisches und Chromatographieren des Rückstandes an Kieselgel unter Eluieren mit Methylenchlorid/Methanol 19/1 erhielt man nach Umkristallisieren aus Essigester 7.42 g (62%) 3-(5-Dipropylaminomethyl-1,3,4-oxadiazol-2-yl)-5-methyl-5,6-dihydro-4H-imidazo[1,5-a][1,4]-benzodiazepin-6-on vom Smp. 126-127°, das in das Hydrochlorid vom Smp. 208-210° überführt wurde.

Beispiel 132

0.66 g (2 mMol) 3-(5-Chlormethyl-1,3,4-oxadiazol-2-yl)-5-methyl-5,6-dihydro-4H-imidazo[1,5-a][1,4]benzodiazepin-6-on wurden über Nacht mit 1.2 g (9.3 mMol) Dibutylamin und 10 ml N,N-Dimethylformamid bei 75° gerührt. Nach Eindampfen des Reaktionsgemisches und Chromatographieren des Rückstandes an Kieselgel unter Eluieren mit Essigester erhielt man 0.72 g (85%) 3-(5-Dibutylaminomethyl-1,3,4-oxadiazol-2-yl)-5-methyl-5,6-dihydro-4H-imidazo[1,5-a][1,4]-benzodiazepin-6-on, das in das Hydrochlorid vom Smp. 211-213° überführt wurde.

Beispiel 133

1.64 g (5 mMol) 3-(5-Chlormethyl-1,3,4-oxadiazol-2-yl)-5-methyl-5,6-dihydro-4H-imidazo[1,5-a][1,4]benzodiazepin-6-on wurden über Nacht mit 1.62 g (12.5 mMol) Diisobutylamin und 20 ml N,N-Dimethylformamid bei Raumtemperarur gerührt. Nach Eindampfen des Reaktionsgemisches und Chromatographieren des Rückstandes an Kieselgel unter Eluieren mit Essigester erhielt man 1.63 g (77%) 3-(5-Diisobutylaminomethyl-1,3,4-oxadiazol-2-yl)-5-methyl-5,6-dihydro-4H-imidazo[1,5-a][1,4]-benzodiazepin-6-on vom Smp. 138-140°, das in das Hydrochlorid vom Smp. 177-180° überführt wurde.

Beispiel 134

1.09 g (3 mMol) 7-Chlor-3-(5-chlormethyl-1,3,4-oxadiazol-2-yl)-5-methyl-5,6-dihydro-4H-imidazo[1,5-a][1,4] benzodiazepin-6-on wurden über Nacht mit 0.97 g (7.5 mMol) Diisobutylamin und 15 ml N,N-Dimethylformamid bei Raumtemperatur gerührt. Nach Eindampfen des Reaktionsgemisches und Chromatographieren des Rückstandes an Kieselgel unter Eluieren mit Essigester erhielt man 1.19 g (86%) 7-Chlor-3-(5-diisobutylaminomethyl-1,3,4-oxadiazol-2-yl)-5-methyl-5,6-dihydro-4H-imidazo[1,5-a][1,4]benzodiazepin-6-on vom Smp. 181-182°, das in das Hydrochlorid vom Smp. 161-163° überführt wurde.

Beispiel 135

1.64 g (5 mMol) 3-(5-Chlormethyl-1,3,4-oxadiazol-2-yl)-5-methyl-5,6-dihydro-4H-imidazo[1,5-a][1,4]benzodiazepin-6-on wurden über Nacht mit 1.07 g (12.5 mMol) Piperidin und 40 ml N,N-Dimethylformamid bei Raumtemperatur gerührt. Nach Eindampfen des Reaktionsgemisches und Chromatographieren des Rückstandes an Kieselgel unter Eluieren mit Methylenchlorid/Methanol 19/1 erhielt man nach Umkristallisieren aus Essigester 1.3 g (68%) 5-Methyl-3-[5-(piperidin-1-yl)methyl-1,3,4-oxadiazol-2-yl]-5,6-dihydro-4H-imidazo[1,5-a][1,4]-benzodiazepin-6-on vom Smp. 160-162°, das in das Hydrochlorid vom Smp. 255-257° überführt wurde.

Beispiel 136

1.64 g (5 mMol) 3-(5-Chlormethyl-1,3,4-oxadiazol-2-yl)-5,6-dihydro-5-methyl-4H-imidazo[1,5-a][1,4]benzodiazepin-6-on wurden über Nacht mit 3 ml (21 mMol) Diisopropylamin und 30 ml N,N-Dimethylformamid bei 70° gerührt. Nach Eindampfen des Reaktionsgemisches und Chromatographieren des Rückstandes an Kieselgel unter Eluieren mit Methylenchlorid/Methanol 19/1 erhielt man nach Umkristallisieren aus Essigester 1.32 g (67%) 3-(5-Diisopropylaminomethyl-1,3,4-oxadiazol-2-yl)-5-methyl-5,6-dihydro-4H-imidazo[1,5-a][1,4]benzodiazepin-6-on vom Smp. 199-200°, das in das Hydrochlorid vom Smp. 232-234° überführt wurde.

Beispiel 137

1.64 g (5 mMol) 3-(5-Chlormethyl-1,3,4-oxadiazol-2-yl)-5-methyl-5,6-dihydro-4H-imidazo[1,5-a][1,4]benzodiazepin-6-on wurden über Nacht mit 2.26 g (17.5 mMol) Di-sek.-butylamin und 25 ml N,N-Dimethylformamid bei 75° gerührt. Nach Eindampfen des Reaktionsgemisches und Chromatographieren des Rückstandes an Kieselgel unter Eluieren mit Methylenchlorid/Methanol 19/1 erhielt man nach Umkristallisieren aus Essigester 1.35 g (64%) 3-(5-Di-sek.-butylaminomethyl-1,3,4-oxadiazol-2-yl)-5-methyl-5,6-dihydro-4H-imidazo[1,5-a][1,4]benzodiazepin-6-on vom Smp. 80-83°, das in das Hydrochlorid (amorph) überführt wurde.

Beispiel 138

Zu einer Lösung von 340 mg (1 mmol) (S)-1-(5-Aminomethyl-1,2,4-oxadiazol-3-yl)-10,11,12,12a-tetrahydro-8H-imidazo[5,1-c]pyrrolo[1,2-a]-thieno[3,2-e][1,4]diazepin-8-on in 15 ml Methylenchlorid wurden 0.87 ml (5 mmol) N-Aethyldiisopropylamin und 0.17 ml (2.1 mmol) Allylbromid zugegeben, und die Mischung wurde 12 Stunden bei Raumtemperatur gerührt. Die Reaktionslösung wurde anschliessend mit Methylenchlorid verdünnt und drei Mal mit Wasser gewaschen. Die organischen Phasen wurden über Magnesiumsulfat getrocknet, filtriert und eingedampft. Der erhaltene Rückstand wurde chromatographiert (Kieselgel, Methylenchlorid/Methanol/wässeriges Ammoniak 250:10:1). Man erhielt 280 mg (66%) (S)-1-(5-Diallylaminomethyl-1,2,4-oxadiazol-3-yl)-10,11,12,12a-tetrahydro-8H-imidazo[5,1-c]pyrrolo[1,2-a]thieno[3,2-e][1,4]diazepin-8-on, Rf=0.41 (Kieselgel, Essigsäureäthylester).

Beispiel 139

Zu einer Lösung von 756 mg (2 mmol) (S)-1-(3-Aminomethyl-1,2,4-oxadiazol-5-yl)-11,11a-dihydro-8H,10H-azeto[1,2-a]imidazo[5,1-c]thieno[3,2-e]-[1,4]diazepin-8-on in 20 ml Methylenchlorid wurden 2.4 ml (13.8 mmol) N-Aethyldiisopropylamin und 0.67 ml (8 mmol) Allylbromid zugegeben, und die Mischung wurde 20 Stunden bei Raumtemperatur gerührt. Die Reaktionslösung wurde anschliessend mit Methylenchlorid verdünnt und drei Mal mit Wasser gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 140:10:1). Man erhielt 645 mg (79 %) (S)-1-(5-Diallylaminomethyl-1,2,4-oxadiazol-5-yl)-11,11a-dihydro-8H,10H-azeto-[1,2-a]imidazo [5,1-c]thieno[3,2-e][1,4]diazepin-8-on als farblosen Schaum, Rf=0.52 (Kieselgel, Methylenchlorid/Methanol /wässeriges Ammoniak 140:10:1).

Beispiel 140

Zu einer Lösung von 453 mg (1.2 mmol) (S)-1-(3-Aminomethyl-1,2,4-oxadiazol-5-yl)-11,11a-dihydro-8H,10H-azeto[1,2-a]imidazo[5,1-c]thieno[3,2-e]-[1,4]diazepin-8-on in 20 ml Methylenchlorid wurden 1.5 ml (8.6 mmol) N-Aethyldiisopropylamin und 0.28 ml (2.4 mmol) Dimethylallylbromid zugegeben, und die Mischung wurde 20 Stunden bei Raumtemperatur gerührt. Die Reaktionslösung wurde anschliessend mit Methylenchlorid verdünnt und drei Mal mit Wasser gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 140:10: 1). Man erhielt 240 mg (43 %) (S)-1-[3-bis-[3-Methyl-but-2-enyl)-aminomethyl]-1,2,4-oxadiazol-5-yl]-11,11a-dihydro-8H,10H-azeto[1,2-a]imidazo[5,1-c]thieno[3,2-e][1,4]diazepin-8-on als farblosen Schaum, Rf=0.55 (Kieselgel, Methylenchlorid/Methanol/ wässeriges Ammoniak 140:10:1).

Beispiel 141

Zu einer Lösung von 600 mg (1.9 mmol) (S)-1-(3-Aminomethyl-1,2,4-oxadiazol-5-yl)-11,11a-dihydro-8H,10H-azeto[1,2-a]imidazo[5,1-c]thieno[3,2-e]-[1,4]diazepin-8-on in 20 ml Dimethylformamid wurden 4 ml (8.6 mmol) N-Aethyldiisopropylamin und 1.1 ml (12 mmol) 1-Brompropan zugegeben, und die Mischung wurde 12 Stunden bei 70°

gerührt. Die Reaktionslösung wurde anschliessend eingedampft, und der Rückstand wurde zwischen Methylenchlorid und 2N Natriumcarbonatlösung verteilt. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 140:10:1). Man erhielt 410 mg (54%) (S)-1-(Dipropylaminomethyl-1,2,4-oxadiazol-5-yl)-11,11a-dihydro-8H,10H-azeto-[1,2-a]imidazo[5,1-c]thieno[3,2-e][1,4]diazepin-8-on als farblosen Schaum, Rf=0.55 (Kieselgel, Methylenchlorid/Methanol /wässeriges Ammoniak 140:10:1).

Beispiel 142

Zu einer Lösung von 340 mg (1 mmol) (S)-1-(5-Aminomethyl-1,2,4-oxadiazol-3-yl)-10,11,12,12a-tetrahydro-8H-imidazo[5,1-c]pyrrolo[1,2-a]-thieno[3,2-e][1,4]diazepin-8-on in 10 ml Dimethylformamid wurden 1.74 ml (10 mmol) N-Aethyldiisopropylamin und 0.55 ml (6 mmol) 1-Brompropan zugegeben, und die Mischung wurde 12 Stunden bei 70° gerührt. Die Reaktionslösung wurde anschliessend eingedampft, und der Rückstand wurde zwischen Methylenchlorid und 2N Natriumcarbonatlösung verteilt. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 240:10:1). Man erhielt 162 mg (38 %) (S)-1-(5-Dipropylaminomethyl-1,2,4-oxadiazol-3-yl)-10,11,12,12a-tetrahydro-8H-imidazo[5,1-c]pyrrolo[1,2-a]thieno[3,2-e][1,4]-diazepin-8-on, Rf=0.26 (Kieselgel, Essigsäureäthylester/Methanol 40:1).

Beispiel 143

Zu einer Lösung von 1.5 g (4.74 mmol) 3-(5-Aminomethyl-1,2,4-oxadiazol-3-yl)-5-methyl-5,6-dihydro-4H-imidazo[1,5-a]thieno[2,3-f][1,4]-diazepin-6-on in 40 ml Dimethylformamid wurden 7 ml (23 mmol) N-Aethyldiisopropylamin und 0.9 ml (9.9 mmol) 1-Brompropan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/Methanol/wässeriges Ammoniak 250:10:1). Man erhielt 930 mg (49 %) 3-(5-Dipropylaminomethyl-1,2,4-oxadiazol-3-yl)-5-methyl-5,6-dihydro-4H-imidazo[1,5-a]thieno[2,3-f][1,4]diazepin-6-on vom Smp. 128.5-130.0°.

Beispiel 144

Zu einer Lösung von 0.6 g (1.9 mmol) 3-(3-Aminomethyl-1,2,4-oxadiazol-5-yl)-5-methyl-5,6-dihydro-4H-imidazo[1,5-a]thieno[2,3-f][1,4]-diazepin-6-on in 20 ml Dimethylformamid wurden 4 ml (23.4 mmol) N-Aethyldiisopropylamin und 1.1 ml (12 mmol) 1-Brompropan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Essigsäureäthylester/Methanol 25:1). Man erhielt 440 mg (57 %) 3-(3-Dipropylaminomethyl-1,2,4-oxadiazol-5-yl)-5-methyl-5,6-dihydro-4H-imidazo[1,5-a]thieno[2,3-f][1,4]-diazepin-6-on als farblosen Schaum, Rf=0.34 (Kieselgel, Essigsäureäthylester/Methanol 25:1).

Beispiel 145

Zu einer Lösung von 2.0 g (6.3 mmol) 7-(5-Aminomethyl-1,2,4-oxadiazol-3-yl)-5-methyl-5,6-dihydro-4H-imidazo[1,5-a]thieno[2,3-f][1,4]diazepin-4-on in 50 ml Dimethylformamid wurden 7.5 ml (43.6 mmol) N-Aethyldiisopropylamin und 2.3 ml (25.3 mmol) 1-Brompropan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 250 mg (10 %) 7-(5-Dipropylaminomethyl-1,2,4-oxadiazol-3-yl)-5-methyl-5,6-dihydro-4H-imidazo[1,5-a]thieno[3,2-f][1,4]diazepin-4-on als farblosen Schaum, Rf=0.46 (Kieselgel, Methylenchlorid/Methanol/wässeriges Ammoniak 110:10:1).

Beispiel 146

Zu einer Lösung von 2.0 g (6.3 mmol) in 50 ml 7-(5-Aminomethyl-1,2,4-oxadiazol-3-yl)-5-methyl-5,6-dihydro-4H-imidazo[1,5-a]thieno[3,2-f][1,4]-diazepin-4-on in 50 ml Dimethylformamid wurden 3.75 ml (21.8 mmol) N-Aethyldiisopropylamin und 1.15 ml (12.7mmol) 1-Brompropan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/Methanol/wässeriges Ammoniak 110:10:1). Man erhielt 460 mg (20 %) 7-(5-Propylaminomethyl-1,2,4-oxadiazol-3-yl)-5-methyl-5,6-dihydro-4H-imidazo[1,5-a]thieno[3,2-f][1,4]diazepin-4-on als farblosen Schaum, Rf=0.31 (Kieselgel, Methylenchlorid/Methanol/ wässeriges Ammoniak 110:10:1).

Beispiel 147

Zu einer Lösung von 1.0 g (3.16 mmol) 7-(5-Aminomethyl-1,2,4-oxadiazol-3-yl)-5-methyl-5,6-dihydro-4H-imidazo[1,5-a]thieno[3,2-f][1,4]-diazepin-4-on in 30 ml Dimethylformamid wurden 3.7 ml (21.8 mmol) N-Aethyldiisopropylamin und 1.4 ml (12.6 mmol) 1-Brombutan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 290 mg (22 %) 7-(5-Dibutylaminomethyl-1,2,4-oxadiazol-3-yl)-5-methyl-5,6-dihydro-4H-imidazo[1,5-a]thieno[3,2-f][1,4]diazepin-4-on als farblosen Schaum, Rf=0.37 (Kieselgel, Methylenchlorid/Methanol/wässeriges Ammoniak 110:10:1).

Beispiel 148

Zu einer Lösung von 1.0 g (3.16 mmol) 7-(5-Aminomethyl-1,2,4-oxadiazol-3-yl)-5-methyl-5,6-dihydro-4H-imidazo[1,5-a]thieno[3,2-f][1,4]-diazepin-4-on in 30 ml Dimethylformamid wurden 1.85 ml (10.9 mmol) N-Aethyldiisopropylamin und 0.7 ml (6.3 mmol) 1-Brombutan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 290 mg (25 %) 7-(5-Butylaminomethyl-1,2,4-oxadiazol-3-yl)-5-methyl-5,6-dihydro-4H-imidazo-[1,5-a]thieno[3,2-f][1,4]diazepin-4-on als farblosen Schaum, Rf=0.37 (Kieselgel, Methylenchlorid/Methanol/wässeriges Ammoniak 110:10:1).

Beispiel 149

Zu einer Lösung von 1.0 g (3.16 mmol) 7-(5-Aminomethyl-1,2,4-oxadiazol-3-yl)-5-methyl-5,6-dihydro-4H-imidazo[1,5-a]thieno[3,2-f][1,4]-diazepin-4-on in 30 ml Dimethylformamid wurden 3.75 ml (21.8 mmol) N-Aethyldiisopropylamin und 0.94 ml (12.6 mmol) 1-Bromäthan zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/Methanol/wässeriges Ammoniak 110:10:1). Man erhielt 250 mg (21 %) 7-(5-Diäthylaminomethyl-1,2,4-oxadiazol-3-yl)-5-methyl-5,6-dihydro-4H-imidazo[1,5-a]thieno[3,2-f][1,4]diazepin-4-on als farblosen Schaum, Rf=0.56 (Kieselgel, Methylenchlorid/Methanol/ wässeriges Ammoniak 110:10:1).

Beispiel 150

Zu einer Lösung von 0.54 g (1.7 mmol) 3-(5-Aminomethyl-1,2,4-oxadiazol-3-yl)-5-methyl-5,6-dihydro-4H-imidazo[1,5-a]thieno[2,3-f][1,4]-diazepin-6-on in 20 ml Dimethylformamid wurden 2.35 ml (13.6 mmol) N-Aethyldiisopropylamin und 1.1 ml (10.2 mmol) Butylbromid zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 265 mg (36 %) 3-(5-Dibutylaminomethyl-1,2,4-oxadiazol-3-yl)-5-methyl-5,6-dihydro-4H-imidazo-[1,5-a]thieno[2,3-f][1,4]diazepin-6-on als

farblosen Schaum, Rf=0.73 (Kieselgel, Methylenchlorid/Methanol/wässeriges Ammoniak 110:10:1).

Beispiel 151

Zu einer Lösung von 0.54 g (1.7 mmol) 3-(5-Aminomethyl-1,2,4-oxadiazol-3-yl)-5-methyl-5,6-dihydro-4H-imidazo[1,5-a]thieno[2,3-f][1,4]-diazepin-6-on in 20 ml Dimethylformamid wurden 2.35 ml (13.6 mmol) N-Aethyldiisopropylamin und 0.76 ml (10.2 mmol) Aethylbromid zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden über Magnesiumsulfat getrocknet, filtriert und eingedampft. Der erhaltene Rückstand wurde chromatographiert (Kieselgel, Methylenchlorid/Methanol/wässeriges Ammoniak 110:10:1). Man erhielt 301 mg (47 %) 3-(5-Diäthylaminomethyl-1,2,4-oxadiazol-3-yl)-5-methyl-5,6-dihydro-4H-imidazo[1,5-a]thieno[2,3-f][1,4]diazepin-6-on als farblosen Schaum, Rf=0.66 (Kieselgel, Methylenchlorid/Methanol/ wässeriges Ammoniak 110:10:1).

Beispiel 152

Zu einer Lösung von 0.500 g (1.52 mmol) (S)-1-(5-Aminomethyl-1,2,4-oxadiazol-3-yl)-11,11a-dihydro-8H,10H-azeto[1,2-a]imidazo[5,1-c]thieno[3,2-e]-[1,4]diazepin-8-on in 15 ml Dimethylformamid wurden 0.87 ml N-Aethyldiisopropylamin und 0.28 ml (3 mmol) 1-Brompropan zugegeben, worauf 12 Stunden bei 70° gerührt wurde. Das Dimethylformamid wurde eingedampft, und der Rückstand wurde zwischen Methylenchlorid und 2N Natriumcarbonatlösung verteilt. Die wässerige Phase wurde zwei Mal mit Methylenchlorid extrahiert, und die organischen Phasen wurden mit Natriumsulfat getrocknet, filtriert und eingedampft. Chromatographie (Kieselgel, Essigsäureäthylester/Methanol 20:1) lieferte 0.324 g (57 %) (S)-1-(5-Propylaminomethyl-1,2,4-oxadiazol-3-yl)-11,11a-dihydro-8H,10H-azeto-[1,2-a]imidazo[5,1-c]thieno[3,2-e][1,4]diazepin-8-on als farblosen Schaum, Rf=0.24 (Kieselgel, Essigsäureäthylester/Methanol 20:1).

Beispiel 153

Zu einer Lösung von 0.54 g (1.7 mmol) 3-(5-Aminomethyl-1,2,4-oxadiazol-3-yl)-5-methyl-5,6-dihydro-4H-imidazo [1,5-a]thieno [2,3-f][1,4]diazepin-6-on in 20 ml Dimethylformamid wurden 1.17 ml (6.8 mmol) N-Aethyldiisopropylamin und 0.55 ml (5.1 mmol) Butylbromid zugegeben, und die Mischung wurde 1 Stunde bei 70° gerührt. Die Reaktionslösung wurde anschliessend eingedampft, worauf der Rückstand zwischen Methylenchlorid und 2N Natriumcarbonatlösung verteilt wurde. Die wässerige Phase wurde drei Mal mit Methylenchlorid gewaschen. Die organischen Phasen wurden mit Magnesiumsulfat getrocknet, filtriert und eingedampft. Der erhaltene Rückstand wurde chromatographiert (Kieselgel, Methylenchlorid/ Methanol/wässeriges Ammoniak 110:10:1). Man erhielt 310 mg (47 %) 3-(5-Butylaminomethyl-1,2,4-oxadiazol-3-yl)-5-methyl-5,6-dihydro-4H-imidazo[1,5-a]thieno[2,3-f][1,4]diazepin-6-on als farblosen Schaum, Rf=0.73 (Kieselgel, Methylenchlorid/Methanol/wässeriges Ammoniak 110:10:1).

Beispiel 154

a) Eine frisch hergestellten Lösung von Natriummethanolat in Methanol (aus 53.46 g (2.32 Mol) Natrium in 1 1 Methanol) wurde innerhalb 10 Min zu einer Suspension von 141.4 g (2.32 Mol) Hydroxylaminhydrochlorid in 1.2 1 Methanol zugetropft, worauf 30 Min. bei Raumtemperatur gerührt wurde. Das ausgefallene NaCl wurde abfiltriert. Das erhaltene Filtrat versetzte man portionenweise mit 433 g (2.32 Mol) Phthtalimidoacetonitril (Ber. **55**, 2981 (1921)) so dass die Temperatur nicht über 40° stieg. Man rührte die Suspension über Nacht, filtrierte und trocknete die erhaltenen Kristalle bei 60°/10 Torr. Man erhielt 475 g (E und/oder Z)-N'-Hydroxy-1,3-dioxo-2-isoindolinacetamidin als farblose Kristalle vom Smp. 192-195°.

b) Zu einer Suspension von 50 g (228 mmol) (E und/oder Z)-N'-Hydroxy-1,3-dioxo-2-isoindolinacetamidin in 11 Dimethylformamid wurden portionenweise 40 g (228 mmol) Chloressigsäureanhydrid zugegeben, worauf zwei Stunden bei Raumtemperatur gerührt wurde. Anschliessend erhitzte man für 20 Stunden auf 100°. Die braune Lösung wurde eingedampft und zwischen Methylenchlorid und Wasser verteilt. Die wässerige Phase wurde ein Mal mit Methylenchlorid nachgewaschen. Die organischen Phasen wurden mit Magnesiumsulfat getrocknet, filtriert und eingedampft. Der Rückstand wurde chromatographiert (Kieselgel, Methylenchlorid/Essigsäureäthylester 20 : 1) und anschliessend aus Methylenchlorid/Diäthyläther kristallisiert. Man erhielt 36 g (57%) 2-(5-Chloromethyl-1,2,4-oxadiazol-3-ylmethyl)-2,3-dihydro-1H-isoindol-1,3-dion vom Smp. 96.5 - 98°.

c) Zu einer Lösung von 36 g (130 mmol) 2-(5-Chloromethyl-1,2,4-oxadiazol-3-ylmethyl)-2,3-dihydro-1H-isoindol-1,3-dion in 300 ml Methylenchlorid wurden bei Raumtemperatur 35.6 ml (260 mmol) Dipropylamin zugetropft, worauf 24 Stunden bei Raumtemperatur gerührt wurde. Die gelbe Lösung wurde mit ges. Natriumhydrogencarbonat-Lösung gewaschen, mit Natriumsulfat getrocknet, filtriert und eingedampft. Der erhaltene Rückstand wurde chromatographiert (Kieselgel, Methylenchlorid/ wässeriges Ammoniak 19 : 1) Man erhielt 38 g (85%) 2-(5-Dipropylaminomethyl-1,2,4-oxadiazol-3-ylmethyl)-2,3-dihydro-1H-isoindol-1,3-dion als beiges Oel, Rf= 0.12 (Kieselgel, Methylenchlorid/wässeriges Ammoniak 19:1).

d) Zu einer Lösung von 38 g (111 mMol) 2-(5-Dipropylaminomethyl-1,2,4-oxadiazol-3-ylmethyl)-2,3-dihydro-1H-isoindol-1,3-dion in 300 ml Aethanol wurden 400 ml Methylamin (33% in Aethanol) zugetropft. Man rührte 1.5 Stunden bei 70° und dampfte anschliessend ein. Der Rückstand wurde in Methylenchlorid verrührt und abfiltriert. Das Filtrat wurde eingeengt und chromatographiert (Kieselgel, Methylenchlorid/Methanol 19 : 1). Man erhielt 15.6 g (66%) (3-Aminomethyl-1,2,4-oxadiazol-5-ylmethyl)-dipropylamin als gelbes Oel, Rf=0.25 (Kieselgel, Methylenchlorid/Methanol 9:1).

e) Eine Lösung von 15.6 g (73.5 mMol) (3-Aminomethyl-1,2,4-oxadiazol-5-ylmethyl)-dipropyl-amin in 100 ml Ameisensäuremethylester wurde sieben Stunden am Rückfluss erhitzt. Anschliessend wurde die Lösung eingedampft, und der Rückstand wurde chromatographiert (Kieselgel, Methylenchlorid/Methanol 19 : 1). Man erhielt 14 g (79 %) N-(5-Dipropylaminomethyl-1,2,4-oxadiazol-3-ylmethyl)-formamid, Rf=0.28 (Kieselgel, Methylenchlorid/Methanol 19 : 1).

f) Zu einer Lösung von 2.4 g ( 10 mmol) N-(5-Dipropylaminomethyl-1,2,4-oxadiazol-3-ylmethyl)-formamid in 25 ml Methylenchlorid und 4.3 ml (30 mmol) Diisopropylamin tropfte man bei 0° 0.92 ml (10 mmol) Phosphoroxidchlorid in 5 ml Methylenchlorid dazu und rührte 45 Minuten bei 0°.

Man goss die Lösung auf Eiswasser und extrahierte zwei Mal mit Methylenchlorid. Die organischen Phasen wurden mit Magnesiumsulfat getrocknet, filtriert und eingedampft. Das erhaltene 5-(Dipropylaminomethyl-1,2,4-oxadiazol-3-ylmethyl)isocyanid wurde ohne weitere Reinigung in die nachstehend unter h) beschriebene Reaktion eingesetzt.

g) Eine Lösung von 7.23 g (42.75 mmol) 6H-Thieno-[2,3-d] [1,3]oxazin-4,6(7H)-dion (J. chem. Res. (M), 1986, 1468) und 1.99 g (24.75 mmol) L-Azetidin-2-carbonsäure in 30 ml Dimethylformamid und 6 ml Essigsäure wurde bei 120° während 16 Stunden gerührt. Die braune Lösung wurde eingedampft, und der erhaltene braune Rückstand wurde aus Aethanol kristallisiert. Man erhielt 3.74 g (42%) (S)-7,7a-Dihydroazeto[1,2-a]thieno-[2,3-e] [1,4] diazepin-4,8(6H,9H)-dion als farblose Nadeln vom Smp. 272-274°.

h) Zu einer Suspension von 0.45 g (10.3 mmol) NaH (55%, gewaschen mit Hexan) in 10 ml Dimethylformamid wurde bei -30° eine Lösung von 1.94 g (9.3 mmol) (S)-7,7a-Dihydroazeto[1,2-a]thieno[2,3-e] [1,4]diazepin-4,8(6H, 9H)-dion in 20 ml Dimethylformamid zugetropft, worauf während 40 Minuten bei -30° gerührt wurde. Nach Abkühlen auf -60° wurde eine Lösung von 2 ml (9.3 mmol) Phosphorsäure-diphenylesterchlorid in 5 ml Dimethylformamid so zugetropft, dass die Temperatur nicht über -45° stieg. Anschliessend rührte man noch 30 Minuten nach.

Unterdessen wurden 1.2 g (10.3 mmol) Kalium-tert.butylat in 20 ml Dimethylformamid gelöst und bei -60° mit 2.2 g (10mmol) 5-(Dipropylaminomethyl-1,2,4-oxadiazol-3-ylmethyl) isocyanid in 20 ml Dimethylformamid versetzt. Zu der so erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und nach Neutralisation mit 10 ml Essigsäure bei -40° auf Eiswasser gegossen, worauf drei Mal mit Methylenchlorid extrahiert wurde. Die vereinigten organischen Phasen wurden mit Natriumsulfat getrocknet, filtriert und eingedampft. Chromatographie (Kieselgel, Essigsäureäthylester/Methanol 20 :1) lieferte 0.370 g (10 %) (S)-1-(5-Dipropylaminomethyl-1,2,4-oxadiazol-3-yl)-11,11a-dihydro-8H,10H-azeto[1,2-a]imidazo[5,1-c]-thieno[3,2-e][1,4]diazepin-8-on als farblosen Schaum, Rf=0.21 (Kieselgel, Essigsäureäthylester/Methanol 20 : 1).

Beispiel 155

Zu einer Suspension von 0.45 g (10.3 mmol) NaH (55%, gewaschen mit Hexan) in 10 ml N,N-Dimethylformamid wurde bei -30° eine Lösung von 1.94 g (9.3 mmol) 6-Fluor-3,4-dihydro-4-methyl-2H-1,4-benzodiazepin-2,5-(1H)-dion (EPA 27 214) in 20 ml N,N-Dimethylformamid zugetropft, worauf während 40 Minuten bei -30° gerührt wurde. Nach Abkühlen auf -60° wurde eine Lösung von 2 ml (9.3 mmol) Phosphorsäure-diphenylesterchlorid in 5 ml N,N-Dimethylformamid so zugetropft, dass die Temperatur nicht über -45° stieg. Anschliessend rührte man noch 30 Minuten

nach.

Unterdessen wurden 1.2 g (10.3 mmol) Kalium-tert.butylat in 20 ml N,N-Dimethylformamid gelöst und bei -60° mit 2.2 g (10mmol) 5-(Dipropylaminomethyl-1,2,4-oxadiazol-3-ylmethyl) isocyanid in 20 ml N,N-Dimethylformamid versetzt. Zu der so erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und nach Neutralisation mit 10 ml Essigsäure bei -40° auf Eiswasser gegossen, worauf drei Mal mit Methylenchlorid extrahiert wurde. Die vereinigten organischen Phasen wurden mit Natriumsulfat getrocknet, filtriert und eingedampft. Chromatographie (Kieselgel, Essigsäureäthylester/Methanol 20 :1) lieferte 0.480 g (12 %) 7-Fluor-5-methyl-3-(5-dipropylaminomethyl-1,2,4-oxadiazol-3-yl)-5,6-dihydro-4H-imidazo[1,5-a]-[1,4]benzodiazepin-6-on als farblosen Schaum, Rf=0.26 (Kieselgel, Methylenchlorid/ Methanol/ wässeriges Ammoniak 140 :10 : 1).

Beispiel 156

a) Eine Lösung von 10 g (55.2 mmol) 5-Fluoro-2H-3,1-benzoxazin-2,4(1H)-dion und 5.66g ( 55.2 mmol) L-Azetidin-2-carbonsäure in 75 ml Dimethylformamid und 15 ml Essigsäure wurde bei 120° während 16 Stunden gerührt. Die braune Lösung wurde eingedampft, und der erhaltene braune Rückstand wurde aus Aethanol kristallisiert. Man erhielt 6 g (42%) (S)-5-Fluoro-1,2,4,9,10,10a-hexahydroazeto[2,1-c] [1,4]benzodiazepin-4,10-dion als beige Nadeln vom Smp. 232-233.5°.

b) Zu einer Suspension von 0.45 g (10.3 mmol) NaH (55%, gewaschen mit Hexan) in 10 ml Dimethylformamid wurde bei -30° eine Lösung von 2.05 g (9.3 mmol) (S)-5-Fluoro-1,2,4,9,10,10a-hexahydroazeto[2,1-c][1,4]benzodiazepin-4,10-dion in 20 ml Dimethylformamid zugetropft und während 40 Minuten bei -30° gerührt. Nach Abkühlen auf -60° wurde eine Lösung von 2 ml (9.3 mmol) Phosphorsäure-diphenylesterchlorid in 5 ml Dimethylformamid so zugetropft, dass die Temperatur nicht über -45° stieg. Anschliessend rührte man noch 30 Minuten nach.

Unterdessen wurden 1.2 g (10.3 mmol) Kalium-tert.butylat in 20 ml Dimethylformamid gelöst und bei -60° mit 2.2 g (10mmol) 5-(Dipropylaminomethyl-1,2,4-oxadiazol-3-ylmethyl) isocyanid in 20 ml Dimethylformamid versetzt. Zu der so erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und nach Neutralisation mit 10 ml Essigsäure bei -40° auf Eiswasser gegossen, worauf drei Mal mit Methylenchlorid extrahiert wurde. Die vereinigten organischen Phasen wurden über Natriumsulfat getrocknet, filtriert und eingedampft. Chromatographie (Kieselgel, Essigsäureäthylester/Methanol 20 :1) lieferte 0.360 g (9 %) (S)-8-Fluoro-1-(5-Dipropylaminomethyl-1,2,4-oxadiazol-3-yl)-12,12a-dihydro-9H,11H-azeto[2,1-c]imidazo-[1,5-a][1,4]benzodiazepin-9-on als farblosen Schaum, Rf=0.28 (Kieselgel, Methylenchlorid/ Methanol/ wässeriges Ammoniak 140 :10: 1).

Beispiel 157

Zu einer Suspension von 0.45 g (10.3 mmol) NaH (55%, gewaschen mit Hexan) in 10 ml Dimethylformamid wurde bei -30° eine Lösung von 2.2 g (9.3 mmol) (S)-5,6-Difluor-1,2,4,9,10,10a-hexahydroazeto[2,1-c][1,4]benzodiazepin-4,10-dion in 20 ml N,N-Dimethylformamid zugetropft und während 40 Minuten bei -30° gerührt. Nach Abkühlen auf -60° wurde eine Lösung von 2 ml (9.3 mmol) Phosphorsäure-diphenylesterchlorid in 5 ml N,N-Dimethylformamid so zugetropft, dass die Temperatur nicht über -45° stieg. Anschliessend rührte man noch 30 Minuten nach.

Unterdessen wurden 1.2 g (10.3 mmol) Kalium-tert.butylat in 20 ml N,N-Dimethylformamid gelöst und bei -60° mit 2.2 g (10 mmol) 5-Dipropylaminomethyl-1,2,4-oxadiazol-3-ylmethyl isocyanid in 20 ml N,N-Dimethylformamid versetzt. Zu der so erhaltenen Lösung wurde bei -70° das oben erhaltene Reaktionsgemisch via einen auf -40° gekühlten Tropftrichter zugetropft. Die erhaltene dunkelbraune zähe Lösung wurde 1 Stunde bei -60° nachgerührt und nach Neutralisation mit 10 ml Essigsäure bei -40° auf Eiswasser gegossen, worauf drei Mal mit Methylenchlorid extrahiert ' wurde. Die vereinigten organischen Phasen wurden über Natriumsulfat getrocknet, filtriert und eingedampft. Chromatographie (Kieselgel, Essigsäureäthylester/Methanol 20 :1) lieferte 0.380 g (9 %) (S)-7,8-Difluor-1-(5-Dipropylaminomethyl-1,2,4-oxadiazol-3-yl)-12,12a-dihydro-9H,11H-azeto-[2,1-c]imidazo[1,5-a][1,4]benzodiazepin-9-on als farblosen Schaum, Rf=0.25 (Kieselgel, Methylenchlorid/ Methanol/ wässeriges Ammoniak 140 :10 : 1).

Beispiel 158

a) Zu einer Lösung von 2.8 g (9.68 mmol) (E)-und/oder (Z)- (S)-1-(Aminohydroxyimino-methyl)-11,11a-dihydro-8H,10H-azeto[1,2-a]imidazo[5,1-c]-thieno[3,2-e][1,4]diazepin-8-on in 25 ml N,N-Dimethylformamid gab man 1.9 g (11.1 mmol) Chloressigsäureanhydrid und rührte 1.5 Stunden bei Raumtemperatur. Anschliessend erhitzte man 2 Stunden auf 105°. Die Lösung wurde eingedampft, und der Rückstand wurde zwischen 2N Natronlauge und Methylenchlorid verteilt. Die wässerige Phase wurde mit Methylenchlorid nachgewaschen, und die organischen Phasen wurden mit Magnesiumsulfat getrocknet, filtriert und eingedampft. Der erhaltene Rückstand wurde aus Methylenchlorid/Aethanol kristallisiert. Man erhielt 2.4 g (71 %) (S)-1-(5-Chlormethyl-1,2,4-oxadiazol-3-yl)-11,11a-dihydro-8H,10H-azeto[1,2-a]imidazo[5,1-c]thieno[3,2-e][1,4]diazepin-8-on als beiges Pulver vom Smp. 210-213°.

b) Zu einer Suspension von 400 mg (1.15 mmol) (S)-1-(5-Chlormethyl-1,2,4-oxadiazol-3-yl)-11,11a-dihydro-8H, 10H-azeto[1,2-a]imidazo[5,1-c]thieno-[3,2-e][1,4]diazepin-8-on in 10 ml N,N-Dimethylformamid gab man 0.36 ml (3.45 mmol) Diäthylamin zu und rührte 12 Stunden bei Raumtemperatur. Die Lösung wurde eingedampft, und der Rückstand wurde zwischen Methylenchlorid und 2N Natriumcarbonatlösung verteilt. Die wässerige Lösung wurde mit Methylenchlorid extrahiert, und die organischen Phasen wurden mit Magnesiumsulfat getrocknet, filtriert und eingedampft. Der Rückstand wurde chromatographiert (Kieselgel, Essigsäureäthylester/ Methanol 10 : 1). Man erhielt 410 mg (92 %) (S)-1-(5-Diäthylaminomethyl-1,2,4-oxadiazol-3-yl)-11,11a-dihydro-8H,10H-azeto[1,2-a]imidazo[5,1-c]thieno-[3,2-e][1,4]diazepin-8-on als farblosen Schaum, Rf=0.31 (Kieselgel, Essigsäureäthylester/Methanol 10 : 1).

Beispiel 159

Zu einer Suspension von 400 mg (1,15 mmol) (S)-1-(5-Chlormethyl-1,2,4-oxadiazol-3-yl)-11,11a-dihydro-8H, 10H-azeto[1,2-a]imidazo[5,1-c]thieno[3,2-e]-[1,4]diazepin-8-on in 10 ml N,N-Dimethylformamid gab man 0.6 ml (3.45 mmol) Dibutylamin zu und rührte 12 Stunden bei Raumtemperatur. Die Lösung wurde eingedampft, und der Rückstand wurde zwischen Methylenchlorid und 2N Natriumcarbonatlösung verteilt. Die wässerige Lösung wurde mit Methylenchlorid extrahiert, und die organischen Phasen wurden mit Magnesiumsulfat getrocknet, filtriert und eingedampft. Der Rückstand wurde chromatographiert (Kieselgel, Essigsäuremethylester/ Methanol 10 : 1). Man erhielt 425 mg (84%) (S)-1-(5-Dibutylaminomethyl-1,2,4-oxadiazol-3-yl)-11,11a-dihydro-8H,10H-azeto[1,2-a]imidazo(5,1-c]thieno-[3,2-e][1,4]diazepin-8-on als farblosen Schaum, Rf=0.31 (Kieselgel, Essigsäuremethylester/Methanol 10 : 1).

Beispiel 160

Zu einer Suspension von 400 mg (1,15 mmol) (S)-1-(5-Chlormethyl-1,2,4-oxadiazol-3-yl)-11,11a-dihydro-8H, 10H-azeto[1,2-a]imidazo[5,1-c]thieno[3,2-e]-[1,4]diazepin-8-on in 10 ml N,N-Dimethylformamid gab man 0.34 ml (3.45 mmol) Piperidin zu und rührte 12 Stunden bei Raumtemperatur. Die Lösung wurde eingedampft, und der Rückstand wurde zwischen Methylenchlorid und 2N Natriumcarbonatlösung verteilt. Die wässerige Lösung wurde mit Methylenchlorid extrahiert, und die organischen Phasen wurden getrocknet, filtriert und eingedampft. Der Rückstand wurde chromatographiert (Kieselgel, Essigsäuremethylester/Methanol 15 : 1). Man erhielt 375 mg (82%) (S)-1-(5-Piperidin-1-ylmethyl-1,2,4-oxadiazol-3-yl)-11,11a-dihydro-8H,10H-azeto[1,2-a]imidazo[5,1-c]thieno[3,2-e][1,4]diazepin-8-on als farblosen Schaum, Rf=0.22 (Kieselgel, Essigsäuremethylester/Methanol 15:1).

Beispiel 161

a) Zu einer Lösung von 18.92 g (68.48 mmol) (E)-und/oder (Z)-3-(Aminohydroxyimino-methyl)-5-methyl-5,6-dihydro-4H-imidazo[1,5-a]thieno[2,3-f]-[1,4]diazepin-6-on in 250 ml N,N-Dimethylformamid gab man 13.44 g (78.5 mmol) Chloressigsäureanhydrid und rührte 1.5 Stunden bei Raum-temperatur. Anschliessend erhitzte man 2 Stunden auf 105°. Die Lösung wurde eingedampft, und der Rückstand wurde zwischen 2N Natronlauge und Methylenchlorid verteilt.Die wässerige Phase wurde mit Methylenchlorid nachgewaschen, und die organischen Phasen wurden mit Magnesiumsulfat getrocknet, filtriert und eingedampft. Der erhaltene Rückstand wurde aus Methylenchlorid/Aethanol kristallisiert. Man erhielt 18 g (78 %) 3-(5-Chlormethyl-1,2,4-oxadiazol-3-yl)-5-methyl-5,6-dihydro-4H-imidazo[1,5-a]thieno[2,3-f][1,4]diazepin-6-on als beiges Pulver vom Smp. 221-223°.

b) Zu einer Suspension von 1 g (3 mmol ) 3-(5-Chlormethyl-1,2,4-oxadiazol-3-yl)-5-methyl-5,6-dihydro-4H-imidazo [1,5-a]thieno[2,3-f][1,4]-diazepin-6-on in 30 ml N,N-Dimethylformamid gab man 0.73 ml (9 mmol) Propylamin zu und rührte 12 Stunden bei Raumtemperatur. Die Lösung wurde eingedampft, und der Rückstand wurde zwischen Methylenchlorid und 2N Natriumcarbonatlösung verteilt. Die wässerige Lösung wurde mit Methylenchlorid extrahiert, und die organischen Phasen wurden getrocknet, filtriert und eingedampft. Der Rückstand wurde chromatographiert (Kieselgel, Methylenchlorid/Methanol / wässeriges Ammoniak 140 : 10 : 1). Man erhielt 0.86 g (80 %) (S)-1-(5-Propylaminomethyl-1,2,4-oxadiazol-3-yl)-11,11a-dihydro-8H,10H-azeto[1,2-a]imidazo[5,1-c]thieno [3,2-e]-[1,4]diazepin-8-on als farblosen Schaum, Rf=0.38 (Kieselgel, Methylenchlorid/Methanol/wässeriges Ammoniak 140 : 10 : 1).

Beispiel 162

0.93 g (3 mMol) 3-(3-Aminomethyl-1,2,4-oxadiazol-5-yl)-5-methyl-5,6-dihydro-4H-imidazo[1,5-a][1,4]benzodiazepin-6-on wurden über Nacht mit 0.51 g (3 mMol) Benzylbromid und 0.52 g (4 mMol) N-Aethyldiisopropylamin in 10 ml Methylenchlorid bei Raumtemperatur gerührt. Durch Eindampfen des Lösungsmittels und Chromatographieren des Rückstandes an Kieselgel unter Eluieren mit Essigester/Methanol 6/1 erhielt man 3-(3-Benzylaminomethyl-1,2,4-oxadiazol-5-yl)-5-methyl-5,6-dihydro-4H-imidazo[1,5-a][1,4]-benzodiazepin-6-on, das in das Hydrochlorid (0.25 g: 17%) vom Smp. 195-198° überführt wurde.

Beispiel 163

0.93 g (3 mMol) 3-(3-Aminomethyl-1,2,4-oxadiazol-5-yl)-5-methyl-5,6-dihydro-4H-imidazo[1,5-a][1,4]benzodiazepin-6-on wurden über Nacht mit 0.99 g (6.6 mMol) 5-Brom-1-penten und 1.04 g (8 mMol) N-Aethyldiisopropylamin in 20 ml N,N-Dimethylformamid bei 100° gerührt. Durch Eindampfen des Lösungsmittels und Chromatographieren des Rückstandes an Kieselgel unter Eluieren mit Essigester erhielt man 3-(3-[Bis-(pent-4-enyl)aminomethyl]-1,2,4-oxadiazol-5-yl) -5-methyl-5,6-dihydro-4H-imidazo[1,5-a][1,4]-benzodiazepin-6-on, das in das Hydrochlorid (0.55 g; 38%) vom Smp. 167-169° überführt wurde.

Beispiel 164

3.4 g (10 mMol) 3-(3-Aminomethyl-1,2,4-oxadiazol-5-yl)-7-chlor-5-methyl-5,6-dihydro-4H-imidazo[1,5-a][1,4] benzodiazepin-6-on wurden über das Wochenende mit 2.7 g (20 mMol) 4-Brom-1-buten und 2.7 g (23 mMol) N-Aethyldiisopropylamin in 30 ml N,N-Dimethylformamid bei 70° gerührt. Durch Eindampfen des Lösungsmittels und Chromatographieren des Rückstandes an Kieselgel unter Eluieren mit Methylenchlorid/Methanol 19/1 erhielt man nach Umkristallisieren aus Essigester 0.61 g (13.5%) 3-(3-[Bis-(but-3-enyl)aminomethyl]-1,2,4-oxadiazol-5-yl}-7-chlor-5-methyl-5,6-dihydro-4H-imidazo[1,5-a][1,4]benzodiazepin-6-on vom Schmelzpunkt 143-5°, das in das Hydrochlorid vom Smp. 145-150° überführt wurde.

Beispiel 165

a) Zu einer Suspension von 17 g (64.6 mmol) 5-Methyl-4-oxo-5,6-dihydro-4H-imidazo[1,5-a]thieno[3,2-f][1,4]diazepin-7-carbonsäure (EP 285 837 Al) in 100 ml N,N-Dimethylformamid wurden portionenweise 11.5 g (71 mmol) 1,1'-Carbonyldiimidazol zugegeben. Die entstandene hellbraune Lösung erwärmte man während 45 Minuten auf 50°. Anschliessend kühlte man die Lösung auf Raumtemperatur ab und tropfte 20 ml wässerige Ammoniaklösung dazu. Nach weiterem 30- minütigem Rühren wurde das Reaktions-gemisch auf 100 ml Eiswasser gegossen, und der entstandene Niederschlag wurde abfiltriert und mit Wasser, Aethanol und anschliessend mit Aether nachgewaschen. Man erhielt nach Trocknen bei 70°/10 Torr 15 g (89 %) 5-Methyl-4-oxo-5,6-dihydro-4H-imidazo[1,5-a] thieno[3,2-f][1,4]diazepin-7-carbonsäureamid als farblose Kristalle vom Smp. >250°.

b) Zu einer Suspension von 15 g (57.2 mmol) 5-Methyl-4-oxo-5,6-dihydro-4H-imidazo[1,5-a]thieno[3,2-f][1,4]diazepin-7-carbonsäureamid in 80 ml ml Dioxan und 20 ml Pyridin wurden bei 5-8° 8.7 ml (62.9 mmol) Trifluoressigsäureanhydrid zugetropft. Die erhaltene beige Lösung wurde während 2.5 Stunden bei 50° gerührt und anschliessend auf 220 ml Eiswasser gegossen. Der entstandene Niederschlag wurde abfiltriert. Man erhielt nach Trocknen bei 70°/10 Torr 12.70 g (91 %) 5-Methyl-4-oxo-5,6-dihydro-4H-imidazo[1,5-a]-thieno[3,2-f][1,4]diazepin-7-carbonsäurenitril als weisses Pulver vom Smp. 197-200°.

c) Zu einer frisch hergestellten Lösung von Natriummethylat in Methanol (aus 1.1 g (49.9 mmol) Natrium in 200 ml Methanol) wurden 8.7 g (35.6 mmol) 5-Methyl-4-oxo-5,6-dihydro-4H-imidazo[1,5-a]thieno[3,2-f][1,4]-diazepin-7-carbonsäurenitril und 3.7 g (53.4 mmol) Hydroxylamin-hydrochlorid zugegeben, worauf die Mischung 16 Stunden bei Raum-temperatur gerührt wurde. Anschliessend wurde die Suspension eingedampft, und der Rückstand wurde mit 100 ml Wasser versetzt. Der erhaltene Niederschlag wurde abfiltriert und am Hochvakuum getrocknet. Man erhielt 7.70 g (78%) (E)-und/oder (Z)-7-(Amino-hydroxyimino-methyl)-5-methyl-5,6-dihydro-4H-imidazo[1,5-a]thieno[3,2-f][1,4]diazepin-4-on als farbloses Pulver vom Smp. 216 - 217 °.

d) Zu einer Lösung von 5.40 g (30.5 mmol) BOC-Glycin in 150 ml N,N-Dimethylformamid wurden 5.40 g (33.3 mmol) 1,1'-Carbonyldiimidazol zugegeben, und die Mischung wurde während 30 Minuten bei 50° gerührt. Anschliessend gab man 7.70 g (7.58 mmol) (E)-und/oder (Z)-7-(Aminohydroxyimino-methyl)-5-methyl-5,6-dihydro-4H-imidazo[1,5-a]thieno[3,2-f]-[1,4]diazepin-4-on zu und rührte 15 Stunden bei 90°. Die erhaltene braune Lösung wurde am Hochvakuum eingedampft, und der erhaltene braune Rückstand wurde chromatographiert (Kieselgel, Methylenchlorid /Methanol 19:1). Man erhielt 8.40 g (73 %) 7-(5-BOC-Aminomethyl-1,2,4-oxadiazol-3-yl)-5-methyl-5,6-dihydro-4H-imidazo-[1,5-a]thieno[3,2-f][1,4]diazepin-4-on als farblosen Schaum, Rf=0.35 (Kieselgel, Methylenchlorid/Methanol 19:1).

e) Eine Lösung von 8.40 g (20.2 mmol) 7-(5-BOC-Aminomethyl-1,2,4-oxadiazol-3-yl)-5-methyl-6-oxo-5,6-dihydro-4H-imidazo-[1,5-a]thieno[3,2-f]-[1,4]diazepin-4-on in 50 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 10 ml wässeriger Ammoniaklösung basisch gestellt und sechs Mal mit Methylenchlorid extrahiert. Die organischen Phasen wurden mit Natriumsulfat getrocknet, filtriert und eingedampft. Der erhaltene Rückstand wurde aus Methylenchlorid /Aethanol kristallisiert. Man erhielt 5 g (78%) 7-(5-Aminomethyl-1,2,4-oxadiazol-3-yl)-5-methyl-5,6-dihydro-4H-imidazo[1,5-a]-thieno[3,2-f][1,4]diazepin-4-on als beiges Pulver vom Smp. 181 - 183°.

Beispiel 166

2.98 g (9.25 mMol) (S)-1-(3-Aminomethyl-1,2,4-oxadiazol-5-yl)-12,12a-dihydro-9H,11H-azeto[2,1-c]imidazo[1,5-a][1,4]benzodiazepin-9-on wurden während 60 Stunden mit 3.2 g (25 mMol) N-Aethyldiisopropylamin und 2.5 g (18.5 mMol) 4-Brom-1-buten in 20 ml N,N-Dimethyl-formamid bei 75° gerührt. Durch Eindampfen des Lösungsmittels und Chromatographieren des Rückstandes an Kieselgel unter Eluieren mit Essigester erhielt man 0.62 g (15%) (S)-1-{3-[Bis-(but-3-enyl)aminomethyl]-1,2,4-oxadiazol-5-yl}-12,12a-dihydro-9H,11H-azeto[2,1-c]-imidazo[1,5-a][1,4]benzodiazepin-9-on das in das Hydrochlorid vom Smp. 80-87° überführt wurde.

Beispiel 167

1.5 g (4.5 mMol) 3-(5-Chlormethyl-1,2,4-oxadiazol-3-yl)-5-methyl-5,6-dihydro-4H-imidazo[1,5-a][1,4]benzodiazepin-6-on wurden während 24 Stunden in 10 ml Benzylamin bei Raumtem-peratur gerührt. Durch Eindampfen des Reaktionsgemisches, Chromatographieren des Rückstandes an Kieselgel unter Eluieren mit Methylenchlorid/Methanol 19/1 und Kristallisieren aus Essigester und Hexan erhielt man 1.3 g (71%) 3-(5-Benzylaminomethyl-1,2,4-oxadiazol-3-yl)-5-methyl-5,6-dihydro-4H-imidazo[1,5-a][1,4]benzodiazepin-6-on vom Schmelzpunkt 88-92°, das in das Hydrochlorid vom Smp. 178-182° überführt wurde.

Beispiel 168

1.8 g (4 mMol) (S)-1-(3-Diallylaminomethyl-1,2,4-oxadiazol-5-yl)-11,12,13,13a-tetrahydro-9H-imidazo[1,5-a]pyrrolo[2,1-c][1,4]-benzodiazepin-9-on wurden in 50 ml Methanol gelöst und in Gegenwart von 60 mg 5%iger Palladiumkohle bei Normaldruck und Raumtemperatur hydriert. Der Katalysator wurde abgetrennt, und die Lösung wurde eingedampft. Durch Chromatographieren des Rückstandes an Kieselgel unter Eluieren mit Methylenchlorid/Methanol 19.5/0.5 erhielt man 1 g (60%) (S)-1-(3-Dipropylaminomethyl-1,2,4-oxadiazol-5-yl)-11,12,13,13a-tetrahydro-9H-imidazo[1,5-a]-pyrrolo[2,1-c][1,4]benzodiazepin-9-on, das in das Hydrochlorid vom Smp. 198-201° überführt wurde. Ro 48-6686.

Beispiel 169

1.5 g (4.5 mMol) 3-(5-Chlormethyl-1,2,4-oxadiazol-3-yl)-5-methyl-5,6-dihydro-4H-imidazo[1,5-a][1,4]benzodiazepin-6-on wurden während 2 Stunden in 1.1 ml (14 mMol) Pyrrolidin und 30 ml N,N-Dimethylformamid bei 80°

gerührt. Durch Eindampfen des Reaktionsgemisches und Chromatographieren des Rückstandes an Kieselgel unter Eluieren mit Methylenchlorid/Methanol 19/1 erhielt man 1.4 g (84%) 5-Methyl-3-[5-(pyrrolidin-1-yl)methyl-1,2,4-oxadiazol-3-yl]-5,6-dihydro-4H-imidazo[1,5-a]-[1,4]benzodiazepin-6-on, das in das Hydrochlorid vom Smp. 251-254° überführt wurde.

Beispiel 170

1.5 g (4.2 mMol) (S)-1-(3-Aminomethyl-1,2,4-oxadiazol-5-yl)-8-chlor-12,12a-dihydro-9H,11H-azeto[2,1-c]imidazo[1,5-a][1,4]benzodiazepin-9-on wurden während 2.5 Stunden mit 0.8 ml (9.7 mMol) Aethyljodid und 2.5 ml (14.7 mMol) N-Aethyldiisopropylamin in 30 ml N,N-Dimethyl-formamid bei 80° gerührt. Durch Eindampfen des Reaktionsgemisches und Chromatographieren des Rückstandes an Kieselgel unter Eluieren mit Methylenchlorid/Methanol 19.5/0.5 erhielt man 0.8 g (46%) (S)-8-Chlor-1-(3-diäthylaminomethyl-1,2,4-oxadiazol-5-yl)-12,12a-dihydro-9H,11H-azeto[2,1-c]-imidazo[1,5-a][1,4]benzodiazepin-9-on, das in das Hydrochlorid vom Smp. 169-171° überführt wurde.

Beispiel 171

1.5 g (4.5 mMol) 3-(5-Chlormethyl-1,2,4-oxadiazol-3-yl)-5-methyl-5,6-dihydro-4H-imidazo[1,5-a][1,4]benzodiazepin-6-on wurden während 20 Stunden in 1 ml (14 mMol) 3-Pyrrolin und 30 ml N,N-Dimethylformamid bei 80° gerührt. Durch Eindampfen des Reaktionsgemisches und Chromatographieren des Rückstandes an Kieselgel unter Eluieren mit Methylenchlorid/Methanol 19/1 erhielt man 0.2 g (12%) 5-Methyl-3-[5-(3-pyrrolin-1-yl)methyl-1,2,4-oxadiazol-3-yl]-5,6-dihydro-4H-imidazo[1,5-a][1,4]benzodiazepin-6-on, das in das Hydrochlorid vom Smp. 215-220° überführt wurde.

Beispiel 172

1.7 g (5 mMol) 3-(5-Chlormethyl-1,2,4-oxadiazol-3-yl)-8-fluor-5-methyl-5,6-dihydro-4H-imidazo[1,5-a][1,4] benzodiazepin-6-on wurden während 2 Stunden mit 5 ml (7.2 mMol) Cyclopropylamin und 15 ml N,N-Dimethylformamid bei Raumtemperatur gerührt. Nach Eindampfen des Reaktionsgemisches wurde der Rückstand in Methylenchlorid aufgenommen und mit wässrigem Ammoniak gewaschen. Nach Trocknung der organischen Phase über Magnesiumsulfat, Eindampfen des Lösungsmittels und Umkristallisieren des Rückstandes aus Essigester und Hexan erhielt man 1.17 g (65%) 3-(5-Cyclopropylaminomethyl-1,2,4-oxadiazol-3-yl)-8-fluor-5-methyl-5,6-dihydro-4H-imidazo[1,5-a][1,4] benzodiazepin-6-on vom Smp. 176-178°, das in das Hydrochlorid überführt wurde.

Beispiel 173

2.2 g (6.3 mMol) 3-(5-Chlormethyl-1,2,4-oxadiazol-3-yl)-8-fluor-5-methyl-5,6-dihydro-4H-imidazo[1,5-a][1,4] benzodiazepin-6-on wurden mit 20 ml (183 mMol) Benzylmethylamin über Nacht bei Raumtemperatur und während 1 Stunde bei 70° gerührt. Nach Eindampfen des Reaktionsgemisches wurde der Rückstand an Kieselgel chromatographiert unter Eluieren mit Essigester. Nach Kristallisieren aus Essigester erhielt man 1.80 g (66%) 3-[5-(N-Benzyl-N-methylamino)methyl-1,2,4-oxadiazol-3-yl]-8-fluor-5-methyl-5,6-dihydro-4H-imidazo[1,5-a]-[1,4]benzodiazepin-6-on vom Smp. 161-162°, das in das Hydrochlorid überführt wurde.

Beispiel 174

a) 4.1 g (10 mMol) 3-(5-BOC-Aminomethyl-1,2,4-oxadiazol-3-yl)-5-methyl-5,6-dihydro-4H-imidazo[1,5-a][1,4]benzodiazepin-6-on wurden in 15 ml N,N-Dimethylformamid gelöst und mit 0.44 g (10mMol) Natriumhydrid-Dispersion 55%ig in Oel deprotoniert. Nach Zugabe von 1.7 g (10 mMol) Propyljodid wurde über Nacht bei Raumtemperatur gerührt. Durch Eindampfen des Lösungsmittels und Chromatographieren des Rückstandes an Kieselgel unter Eluieren mit Essigester erhielt man nach Kristallisieren aus Essigester 2.46 g (54%) 3-(5-N-BOC-N-propylaminomethyl-1,2,4-oxadiazol-3-yl)-5-methyl-5,6-dihydro-4H-imidazo-[1,5-a][1,4]benzodiazepin-6-on vom Smp. 148-150°.

b) 2.53 g (6.6 mMol) 3-(5-N-BOC-N-propylaminomethyl-1,2,4-oxadiazol-3-yl)-5-methyl-5,6-dihydro-4H-imidazo-[1,5-a][1,4]benzodiazepin-6-on wurden über Nacht mit 30 ml 3N methanolischer Salzsäure bei Raumtemperatur gerührt. Die erhaltene Suspension wurde mit Aether verdünnt, auf 0° gekühlt und abgenutscht. Man erhielt 1.99 g (77%) 5-Methyl-3-(5-propylaminomethyl-1,2,4-oxadiazol-3-yl)-5,6-dihydro-4H-imidazo-[1,5-a][1,4]benzodiazepin-6-on als Hydrochlorid vom Smp. 248-250°.

Beispiel 175

a) 10.5 g (60 mMol) BOC-Glycin wurden in 50 ml N,N-Dimethyl-formamid gelöst, portionenweise mit 9.73 g (60 mMol) 1,1'-Carbonyldiimidazol versetzt und während 20 Minuten bei 55° gerührt. Nach Zugabe von 15.5 g (57.1 mMol) 5-Methyl-6-oxo-5,6-dihydro-4H-imidazo[1,5-a]-[1,4]benzodiazepin-3-carboxamidoxim wurde über Nacht bei 90° weitergerührt. Das Reaktionsgemisch wurde eingeengt, der Rückstand wurde in Methylenchlorid gelöst, und die Lösung wurde dreimal mit Wasser gewaschen. Nach Trocknung der Lösung, Eindampfen des Lösungsmittels und Kristallisieren des Rückstandes aus Aethanol erhielt man 20.4 g (87%) 3-(5-BOC-Amino-methyl-1,2,4-oxadiazol-3-yl)-5-methyl-5,6-dihydro-4H-imidazo[1,5-a][1,4]benzodiazepin-6-on vom Smp. 108-113°.

b) 15.9 g (38.7 mMol) 3-(5-BOC-Aminomethyl-1,2,4-oxadiazol-3-yl)-5-methyl-5,6-dihydro-4H-imidazo[1,5-a][1,4] benzodiazepin-6-on wurden während 3 Stunden in 40 ml Trifluoressigsäure bei Raumtemperatur gerührt. Die Lösung wurde eingeengt, der Rückstand wurde in Methylenchlorid aufgenommen, und die Lösung wurde mit gesättigter Natriumbicarbonatlösung gewaschen. Man extrahierte die wässrige Phase dreimal mit Methylenchlorid und sechsmal mit Essigester. Nach Trocknung und Eindampfen der vereinigten organischen Phasen und Chromatographieren des Rückstandes an Kieselgel unter Eluieren mit Methylenchlorid/Methanol 19/1 erhielt man 2.03 g (17%) 3-(5-Aminomethyl-1,2,4-oxadiazol-3-yl)-5-methyl-5,6-dihydro-4H-imidazo[1,5-a][1,4]benzodiazepin-6-on vom Smp. 192-195°.

Beispiel 176

a) 192.4 g (714.6 mMol) (S)-9-Oxo-12,12a-dihydro-9H,11H-azeto[2,1-c]imidazo[1,5-a][1,4]benzo-diazepin-1-carbonsäure wurden in 900 ml N,N-Dimethylformamid suspendiert, bei Raumtemperatur mit 116 g (715 mMol) 1,1'-Carbonyldiimidazol versetzt und während 30 Minuten bei 50° gerührt. 173 ml 25%igem Ammoniak wurden bei 25 -30° innert 30 Minuten zugetropft. Das Reaktionsgemisch wurde nach 30 minütigem Rühren eingeengt, und der Rückstand wurde in 500 ml Alkohol gelöst. Nach Zugabe von 250 ml Aether wurde auf 0° gekühlt und das Kristallisat genutscht und getrocknet. Man erhielt 133 6 g (69%) (S)-9-Oxo-12,12a-dihydro-9H,11H-azeto[2,1-c]-imidazo [1,5-a][1,4]benzodiazepin-1-carboxamid vom Smp. 228-230°.

b) 78 g (290 mMol) (S)-9-Oxo-12,12a-dihydro-9H,11H-azeto[2,1-c]imidazo-[1,5-a][1,4]benzodiazepin-1-carboxamid wurden in 380 ml Dioxan und 68 ml Pyridin suspendiert und bei 0° tropfenweise mit 59 ml (424 mMol) Trifluoressigsäureanhydrid versetzt. Nach zweistündigem Rühren bei 50° wurde das Reaktionsgemisch auf 2 l Eiswasser gegossen, und die Kristalle wurden abgenutscht, mit Wasser nachgewaschen und getrocknet. Man erhielt 60 g (82%) (S)-9-Oxo-12,12a-dihydro-9H,11H-azeto[2,1-c]imidazo[1,5-a][1,4]benzodiazepin-1-carbonitril vom Smp. 232-234°.

c) 64.7 g (258 mMol) (S)-9-Oxo-12,12a-dihydro-9H,11H-azeto[2,1-c]-imidazo[1,5-a][1,4]benzo-diazepin-1-carbonitril wurden während 2.5 Stunden mit 52.8 g (497 mMol) Natriumcarbonat und 42.36 g (608 mMol) Hydroxylaminhydrochlorid in 1.5 l Alkohol und 300 ml Wasser zum Rückfluss erhitzt. Der Alkohol wurde abgedampft, und die erhaltene Suspension wurde auf 0° gekühlt. Die Kristalle wurden abgenutscht, mit Wasser gewaschen und getrocknet. Man erhielt 68.5 g (93%) (S)-9-Oxo-12,12a-dihydro-9H,11H-azeto[2,1-c]imidazo[1,5-a][1,4]benzodiazepin-1-carboxamidoxim vom Zersetzungspunkt 216°.

d) 34.5g (122 mMol) (S)-9-Oxo-12,12a-dihydro-9H,11H-azeto[2,1-c]-imidazo[1,5-a][1,4]benzodiazepin-1-carboxamidoxim wurden mit 23 g (140 mMol) Chloressigsäureanhydrid in 200 ml N,N-Dimethylformamid über Nacht bei Raumtemperatur und 2 Stunden bei 110° gerührt. Nach Eindampfen des Reaktionsgemisches wurde der Rückstand in Methylenchlorid gelöst, und die Lösung wurde mit gesättigter Natriumbicarbonat-lösung gewaschen. Die organische Phase wurde über Magnesiumsulfatgetrocknet und eingeengt. Der Rückstand wurde an Kieselgel chromatographiert unter Eluieren mit Methylenchlorid/Methanol 19/1. Man erhielt 24 g (58%) (S)-1-(5-Chlor-methyl-1,2,4-oxadiazol-3-yl)-12,12a-dihydro-9H,11H-azeto[2,1-c]imidazo[1,5-a][1,4]benzodiazepin-9-on vom Smp. 205-207°.

e) 1.4 g (4 mMol) (S)-1-(5-Chlormethyl-1,2,4-oxadiazol-3-yl)-12,12a-dihydro-9H,11H-azeto[2,1-c]imidazo[1,5-a][1,4]benzodiazepin-9-on wurden während 1 Stunde mit 5.2 g (90 mMol) Propylamin und 15 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 19/1 erhielt man 1.07 g (73%) (S)-1-(5-Propylaminomethyl-1,2,4-oxadiazol-3-yl)-12,12a-dihydro-9H,11H-azeto[2,1-c]imidazo[1,5-a]-[1,4]benzodiazepin-9-on, das in das Hydrochlorid (amorph) überführt wurde.

Beispiel 177

a) 36.4 g (205 mMol) BOC-Glycin wurden in 300 ml N,N-Dimethylformamid gelöst und portionenweise mit 35.8 g (220 mMol) 1,1'-Carbonyldiimidazol versetzt. Man rührte die Lösung während 30 Minuten bei 50°, gab 54.7 g (194 mMol) (S)-9-Oxo-12,12a-dihydro-9H,11H-azeto[2,1-c]imidazo[1,5-a]-[1,4]benzodiazepin-1-carboxamidoxim zu und rührte über Nacht bei 90° weiter. Nach Eindampfen des Lösungsmittels wurde der Rückstand in Methylenchlorid gelöst, und die Lösung wurde mit Wasser gewaschen, über Magnesiumsulfat getrocknet und eingeengt. Durch Chromatographieren des Rückstandes an Kieselgel unter Eluieren mit Essigester erhielt man 73 g (89%) (S)-1-(5-BOC-Aminomethyl-1,2,4-oxadiazol-3-yl)-12,12a-dihydro-9H,11H-azeto[2,1-c]imidazo[1,5-a][1,4]benzodiazepin-9-on als weisser Schaum, der ohne weitere Reinigung in die nächste Stufe eingesetzt wurde.

b) 25 g (59.3 mMol) (S)-1-(5-BOC-Aminomethyl-1,2,4-oxadiazol-3-yl)-12,12a-dihydro-9H,11H-azeto[2,1-c]imidazo[1,5-a][1,4]benzodiazepin-9-on wurden über Nacht mit 60 ml 3 N methanolischer Salzsäure bei Raum-temperatur gerührt. Nach Eindampfen des Lösungsmittels wurde der Rückstand in Wasser gelöst, und die Lösung wurde dreimal mit Methylenchlorid extrahiert. Die wässrige Phase wurde mit konz. Ammoniak alkalisch gestellt und siebenmal mit Methylenchlorid ausgeschüttelt. Durch Trocknung der organischen Phase über Magnesiumsulfat und Eindampfen des Lösungsmittels erhielt man 16.9 g (88%) (S)-1-(5-Aminomethyl-1,2,4-oxadiazol-3-yl)-12,12a-dihydro-9H,11H-azeto[2,1-c]-imidazo[1,5-a][1,4]-benzodiazepin-9-on vom Smp. 211-214°.

Beispiel 178

6.9 g (21.4 mMol) (S)-1-(5-Aminomethyl-1,2,4-oxadiazol-3-yl)-12,12a-dihydro-9H,11H-azeto[2,1-c]imidazo[1,5-a][1,4]benzodiazepin-9-on wurden mit 7.65 g (45 mMol) Propyljodid in 80 ml 1,3-Dimethyl-3,4,5,6-tetrahydro-2(1H)-pyrimidinon und 6.5 g (50 mMol) N-Aethyldiisopropylamin über Nacht bei 80° und während 2 Stunden bei 100° gerührt. Durch Eindampfen des Reaktionsgemisches und Chromatographieren des Rückstandes an Kieselgel unter Eluieren mit Essigester erhielt man 3.32 g (38%) (S)-1-(5-Dipropylaminomethyl-1,2,4-oxadiazol-3-yl)-12,12a-dihydro-9H,11H-azeto-[2,1-c]imidazo[1,5-a][1,4]benzodiazepin-9-on, das in das Hydrochlorid vom Smp. 208-210° überführt wurde.

Beispiel 179

1.96 g (4.1 mMol) (S)-1-(5-Diallylaminomethyl-1,2.4-oxadiazol-3-yl)-7-fluor-11,12,13,13a-tetra-hydro-9H-imidazo[1,5-a]pyrrolo[2,1-c][1,4]-benzodiazepin-9-on wurden in 25 ml Essigester gelöst und in Gegenwart von 37 mg 5%iger Palladiumkohle bei Normaldruck und Raumtemperatur hydriert. Der Katalysator wurde abgetrennt, und die Lösung wurde eingedampft. Durch Chromatographieren des Rückstandes an Kieselgel unter Eluieren mit Essigester erhielt man 1.49 g (83%) (S)-1-(5-Dipropylaminomethyl-1,2,4-oxadiazol-3-yl)-7-fluor-11,12,13,13a-tetrahydro-9H-imidazo[1,5-a]pyrrolo[2,1-c][1,4]-benzodiazepin-9-on, das in das Hydrochlorid vom Smp. 222-225° überführt wurde.

Beispiel 180

a) Eine Suspension von 11.9 g (0.0394 Mol) (S)-8-Chloro-9-oxo-12,12a-dihydro-9H,11H-azeto[2,1-c]imidazo[1,5-a][1,4]benzodiazepin-1-carboxamid in einem Gemisch von 85 ml Dioxan und 6.8 ml Pyridin wurde bei 0° mit 7.1 ml (0.051 Mol) Trifluoressigsäureanhydrid versetzt. Die Suspension wurde 3 Std. bei 50° gerührt, abgekühlt und auf eiskaltes Wasser gegossen. Nach 1½ Std. Rühren wurde die Suspension abgenutscht. Man erhielt 11.2 g (100%) (S)-8-Chloro-9-oxo-12,12a-dihydro-9H,11H-azeto[2,1-c]imidazo[1,5-a][1,4]-benzodiazepin-1-carbonitril als weisse Kristalle; Smp. 130° (Zers.).

b) 178.7 g (628 mMol) (S)-8-Chloro-9-oxo-12,12a-dihydro-9H,11H-azeto[2,1-c]imidazo[1,5-a][1,4]-benzodiazepin-1-carbonitril und 65.5 g (941 mMol) Hydroxylaminhydrochlorid wurden zu einer Lösung, hergestellt aus 17.3 g (753 mMol) Natrium und 1.51 Methanol, gegeben. Das Reaktions-gemisch wurde über Nacht bei Raumtemperatur gerührt und anschliessend mit 1 1 Wasser verdünnt. Die erhaltene Suspension wurde genutscht, und die Kristalle wurden getrocknet. Man erhielt 169.7 g (85%) (S)-8-Chloro-9-oxo-12,12a-dihydro-9H,llH-azeto[2,1-c]imidazo [1,5-a][1,4]-benzodiazepin-1-carboxamidoxim vom Zersetzungspunkt 269°.

c) 31.7 g (100mMol) (S)-8-Chloro-9-oxo-12,12a-dihydro-9H,11H-azeto-[2,1-c]imidazo[1,5-a][1,4]-benzodiazepin-1-carboxamidoxim wurden in 200 ml N,N-Dimethylformamid suspendiert und mit 18.8 g (110 mMol) Chloressigsäureanhydrid versetzt. Das Reaktionsgemisch wurde während 2 Stunden auf 105° erhitzt und eingedampft. Durch Chromatographieren des Rückstandes an Kieselgel unter Eluieren mit Chloroform/Methanol 9/1 erhielt man 27.9 g (74%) (S)-8-Chlor-1-(5-chlormethyl-1,2,4-oxadiazol-3-yl)-12,12a-dihydro-9H,11H-azeto[2,1-c]-imidazo[1,5-a][1,4]benzodiazepin-9-on vom Smp. 211-212°.

d) 3.76 g (10 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 2 Stunden mit 10 g (82 mMol) Benzylmethylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 4.4 g (95%) (S)-1-(5-N-Benzyl-N-methyl-aminomethyl-1,2,4-oxadiazol-3-yl)-8-chlor-12,12a-dihydro-9H,11H-azeto[2,1-c]imidazo[1,5-a][1,4]benzodiazepin-9-on, das in das Hydrochlorid vom Smp. 213-215° überführt wurde.

Beispiel 181

3.76 g (10 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2 g (20 mMol) Isopropylmethylamin und 20 ml N,N-Dimethylformamid bei Raumtemperatur gerührt. Durch Eindampfen des Reaktionsgemisches und Chromatographieren des Rückstandes an Kieselgel unter Eluieren mit Essigester/Aethanol 9/1 erhielt man 3.4 g (90%) (S)-8-Chlor-1-(5-N-isopropyl-N-methylaminomethyl-1,2,4-oxadiazol-3-yl)-12,12a-dihydro-9H,11H-azeto[2,1-c]-imidazo[1,5-a][1,4]benzodiazepin-9-on, das in das Hydrochlorid vom Smp. 198-203° überführt wurde.

Beispiel 182

3.76 g (10 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2.19 g (30 mMol) Diäthylamin und 30 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 19/1 erhielt man 4.5 g (96%) (S)-8-Chlor-1-(5-diäthylamino-methyl-1,2,4-oxadiazol-3-yl)-12,12a-dihydro-9H,11H-azeto[2,1-c]-imidazo[1,5-a][1,4]benzodiazepin-9-on, das in das Hydrochlorid vom Smp. 218-220° überführt wurde.

Beispiel 183

a) 9.8 g (32 mMol) 7-Chlor-5-methyl-6-oxo-5,6-dihydro-4H-imidazo-[1,5-a][1,4]benzodiazepin-3-carboxamidoxim wurden in 60 ml N,N-Dimethylformamid mit 7 g (41mMol) Chloressigsäure-anhydrid während 1 Stunde bei Raumtemperatur und 4 Stunden bei 105° gerührt. Nach Eindampfen des Lösungsmittels wurde der Rückstand in Methylenchlorid gelöst, und die Lösung wurde mit gesättigter Natriumbicarbonat-Lösung gewaschen und über Magnesiumsulfat getrocknet. Durch Eindampfen des Lösungsmittels und Chromatographieren des Rückstandes an Kieselgel unter Eluieren mit Essigester erhielt man 6.63 g (65%) 7-Chlor-3-(5-chlormethyl-1,2,4-oxadiazol-3-yl)-5-methyl-5,6-dihydro-4H-imidazo[1,5-a][1,4]-benzodiazepin-6-on.

b) 1.09 g (3 mMol) 7-Chlor-3-(5-chlormethyl-1,2,4-oxadiazol-3-yl)-5-methyl-5,6-dihydro-4H-imidazo[1,5-a][1,4] benzodiazepin-6-on wurden während 4.5 Stunden mit 1.02 g (10 mMol) Dipropylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.16 g (90%) 7-Chlor-3-(5-dipropyl-aminomethyl-1,2,4-oxadiazol-3-yl)-5-methyl-5,6-dihydro-4H-imidazo[1,5-a][1,4]benzodiazepin-6-on, das in das Hydrochlorid vom Smp. 212-215° überführt wurde.

Beispiel 184

a) 4.4 g (10 mMol) 3-(5-BOC-Aminomethyl-1,2,4-oxadiazol-3-yl)-7-chlor-5-methyl-5,6-dihydro-4H-imidazo[1,5-a][1,4]benzodiazepin-6-on wurden in 15 ml N,N-Dimethylformamid bei 0° bis Raumtemperatur mit 0.44 g Natriumhydrid-Dispersion 55%ig in Oel deprotoniert. 1.7 g (10 mMol) Propyljodid wurden bei 10° zugegeben, und das Reaktionsgemisch wurde während 5 Stunden bei Rautemperatur weitergerührt. Durch Eindampfen des Reaktionsgemisches und Chromatographieren des Rückstandes an Kieselgel unter Eluieren mit Essigester erhielt man 2.50 g (51%) 7-Chlor-5-methyl-3-(5-N-BOC-N-propylamino-methyl-1,2,4-oxadiazol-3-yl)-5,6-dihydro-4H-imidazo[1,5-a][1,4]benzodiazepin-6-on als weissen Schaum, das ohne weitere Reinigung weiterverarbeitet wurde.

b) 2.5 g (5.1 mMol) 7-Chlor-5-methyl-3-(5-N-BOC-N-propylaminomethyl-1,2,4-oxadiazol-3-yl)-5,6-dihydro-4H-imidazo[1,5-a][1,4]benzodiazepin-6-on wurden während 3 Stunden mit 10 ml 3 N methanolischer Salzsäure bei Raumtemperatur gerührt. Die erhaltene Suspension wurde auf 0° gekühlt und abgenutscht. Nach Umkristallisieren aus Methanol erhielt man 0.94 g (41%) 7-Chlor-5-methyl-3-(5-propylaminomethyl-1,2,4-oxadiazol-3-yl)-5,6-dihydro-4H-imidazo[1,5-a][1,4]benzodiazepin-6-on hydrochlorid vom Zersetzungspunkt 235°.

Beispiel 185

1.5 g (4.1 mMol) 7-Chlor-3-(5-chlormethyl-1,2,4-oxadiazol-3-yl)-5-methyl-5,6-dihydro-4H-imidazo[1,5-a][1,4]benzodiazepin-6-on wurden während 2 Stunden mit 5 g (41.3 mMol) Benzylmethylamin und 10 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 19/1 erhielt man 1.29 g (72%) 3-(5-N-Benzyl-N-methylaminomethyl-1,2,4-oxadiazol-3-yl)-7-chlor-5-methyl-5,6-dihydro-4H-imidazo[1,5-a][1,4]benzodiazepin-6-on, das in das Hydrochlorid vom Smp. 214-216° überführt wurde.

Beispiel 186

3.76 g (10 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 5 g (39 mMol) Dibutylamin und 25 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 4.3 g (92%) (S)-8-Chlor-1-(5-dibutyl-aminomethyl-1,2,4-oxadiazol-3-yl)-12,12a-dihydro-9H,11H-azeto[2,1-c]imidazo[1,5-a][1,4]-benzodiazepin-9-on, das in das Hydrochlorid vom Smp. 120-125° überführt wurde.

Beispiel 187

3.76 g (10 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 5 g (58 mMol) Piperidin und 25 ml N,N-Dimethylformamid bei Raumtemperatur gerührt. Durch Eindampfen des Reaktionsgemisches und Chromatographieren des Rückstandes an Kieselgel unter Eluieren mit Essigester/Methanol 9/1 erhielt man 4.1 g (96%) (S)-8-Chlor-1-[5-(piperidin-1-yl)methyl-1,2,4-oxadiazol-3-yl]-12,12a-dihydro-9H,11H-azeto[2,1-c]imidazo[1,5-a][1,4]benzodiazepin-9-on vom Smp. 177-179°, das in das Hydrochlorid überführt wurde.

Beispiel 188

0.94 g (2.5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 4 Stunden mit 2 g (20 mMol) Hexamethylenimin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.01 g (92%) (S)-8-Chlor-1-[5-(hexamethylenimin-1-yl)methyl-1,2,4-oxadiazol-3-yl]-12,12a-dihydro-9H,11H-azeto[2,1-c]imidazo[1,5-a][1,4]benzodiazepin-9-on, das in das Hydrochlorid (amorph) überführt wurde.

Beispiel 189

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 1.5 g (13.3 mMol) Heptamethylenimin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.2 g (88%) (S)-8-Chlor-1-[5-(heptamethylenimin-1-yl)methyl-1,2,4-oxadiazol-3-yl]-12,12a-dihydro-9H,llH-azeto[2,1-c]imidazo[1,5-a][1,4]benzo-diazepin-9-on, das in das Hydrochlorid (amorph) überführt wurde.

Beispiel 190

0.94 g (2.5 mMol) (S)-8-Chlor-1-(5-chlormethyl-1,2,4-oxadiazol-3-yl)-12,12a-dihydro-9H,llH-azeto[2,1-c]imidazo[1,5-a][1,4]benzodiazepin-9-on wurden über Nacht mit 3 g (26.5 mMol) Methylcyclohexylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester/Methanol 19/1 erhielt man 0.79 g (69%) (S)-8-Chlor-1-(5-N-cyclohexyl-N-methylaminomethyl-1,2,4-oxadiazol-3-yl)-12,12a-dihydro-9H,11H-azeto[2,1-c]imidazo[1,5-a][1,4]benzodiazepin-9-on, das in das Hydrochlorid (amorph) überführt wurde.

Beispiel 191

0.94 g (2.5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2 g (23 mMol) tert.-Butylmethylamin und 15 ml N,N-Dimethyl-formamid bei Raumtemperatur gerührt. Durch Eindampfen des Reaktionsgemisches und Chromatographieren des Rückstandes an Kieselgel unter Eluieren mit Essigester/Methanol 9/1 erhielt man 0.81 g (75%) (S)-1-(5-N-tert.-Butyl-N-methylaminomethyl-1,2,4-oxadiazol-3-yl)-8-chlor-12,12a-dihydro-9H,11H-azeto[2,1-c]-imidazo[1,5-a][1,4]benzodiazepin-9-on, das in das Hydrochlorid (amorph) überführt wurde.

Beispiel 192

0.94 g (2.5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2 g (27 mMol) tert.-Butylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester/Methanol 9/1 erhielt man 0.91 g (88%) (S)-1-(5-tert.-Butylamino-methyl-1,2,4-oxadiazol-3-yl)-8-chlor-12,12a-dihydro-9H,11H-azeto[2,1-c]-imidazo[1,5-a][1,4]benzodiazepin-9-on, das in das Hydrochlorid (amorph) überführt wurde.

Beispiel 193

0.94 g (2.5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 5 Stunden mit 2 g (19.8 mMol) Diisopropyla-min und 15 ml N,N-Dimethylformamid bei 80° gerührt. Durch Eindampfen des Reaktionsgemisches und Chromatographieren des Rückstandes an Kieselgel unter Eluieren mit Essigester erhielt man 0.81 g (73%) (S)-8-Chlor-1-(5-diisopropylaminomethyl-1,2,4-oxadiazol-3-yl)-12,12a-dihydro-9H,11H-azeto[2,1-c]-imidazo[1,5-a][1,4]benzodiazepin-9-on, das in das Hydrochlorid (amorph) überführt wurde.

Beispiel 194

1.88 g (5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 2 Stunden mit 1.5 g (15 mMol) Diäthanolamin und 15 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 9/1 erhielt man 1.89 g (85%) (S)-8-Chlor-1-(5-diäthanolaminomethyl-1,2,4-oxadiazol-3-yl)-12,12a-dihydro-9H,11H-azeto[2,1-c]-imidazo[1,5-a][1,4]benzodiazepin-9-on vom Smp. 173-175°, das in das Hydrochlorid überführt wurde.

Beispiel 195

a) 10.1 g (231 mMol) Natriumhydrid-Dispersion 55%ig in Oel wurden mit Hexan gewaschen und in 180 ml N,N-Dimethylformamid suspendiert. Bei -40° bis -20° wurden 45.8 g (208 mMol) (S)-6-Fluor-1,2,4,9,10,10a-hexahydro-azeto[2,1-c][1,4]benzodiazepin-4,10-dion portionenweise zugegeben und während 1 Stunde bei -40° bis -20° deprotoniert. Man kühlte auf -60°, gab aufeinanderfolgend eine Lösung von 59 g (219 mMol) Diphenylchlorphosphat

in 10 ml N,N-Dimethylformamid und 24.9 g (220 mMol) Isocyanessigester zu und tropfte anschliessend eine Lösung von 24.5 g (218 mMol) Kalium-tert.-butylat in 50 ml N,N-Dimethylformamid bei -60° bis -55° zu. Man liess das Reaktionsgemisch auf Raumtemperatur erwärmen, neutralisierte es mit 7 ml Essigsäure und goss es auf 600 ml Eiswasser. Man extrahierte zehnmal mit Methylenchlorid, wusch die vereinigten organischen Phasen viermal mit Wasser und trocknete sie über Magnesiumsulfat. Durch Eindampfen des Lösungsmittels und Kristallisieren des Rückstandes aus Methylenchlorid erhielt man 13.6 g weisse Kristalle. Durch Chromatographieren der Mutterlauge an Kieselgel unter Eluieren mit Essigester erhielt man einen weitren Anteil von 10.9 g (Gesamtausbeute 62%) Aethyl (S)-7-fluor-12,12a-dihydro-9-oxo-9H,11H-azeto-[2,1-c]imidazo-[1,5-a][1,4]benzodiazepin-1-carboxylat vom Smp. 226-227°.

b) 40.58 g (128.7 mMol) Aethyl (S)-7-fluor-12,12a-dihydro-9-oxo-9H,11H-azeto-[2,1-c]imidazo-[1,5-a][1,4]benzodiazepin-1-carboxylat, 300 ml Aethanol und 32.5 ml (130 mMol) 4N-Natronlauge wurden während 1.5 Stunden auf dem Dampfbad zum Rückfluss erhitzt. Man dampfte den Alkohol am Rotationsverdampfer ab. Die wässrige Phase wurde zweimal mit Methylenchlorid gewaschen und mit 32.5 ml (130 mMol) 4N-Salzsäure auf pH 3-4 angesäuert. Die erhaltene Suspension wurde gekühlt und genutscht. Der Filterrückstand wurde mit wenig Eiswasser gewaschen und getrocknet. Man erhielt 36.67 g (99%) (S)-7-Fluor-9-oxo-12,12a-dihydro-9H,11H-azeto-[2,1-c]-imidazo[1,5-a][1,4]-benzodiazepin-1-carbonsäure vom Smp. 159-160°.

c) 100 g (348mMol) (S)-7-Fluor-9-oxo-12,12a-dihydro-9H,11H-azeto[2,1-c]-imidazo[1,5-a][1,4]-benzodiazepin-1-carbonsäure wurden in 600 ml N,N-Dimethylformamid suspendiert und mit 64.1 g (395 mMol) 1,1'-Carbonyldiimidazol versetzt. Nach 30 minütigem Rühren bei 50° wurden bei 13° bis 20° 80 ml 25% iges Ammoniak zugetropft, und das Gemisch wurde während 30 Minuten nachgerührt und auf 2 l Wasser gegossen. Durch Abnutschen der erhaltenen Suspension und Trocknen der Kristalle erhielt man 52.7 g (76%) (S)-7-Fluor-9-oxo-12,12a-dihydro-9H, 11H-azeto-[2,1-c]imidazo[1,5-a]-[1,4]benzodiazepin-1-carbonsäureamid vom Smp. 223-224°.

d) 75.6 g (264 mMol) (S)-7-Fluor-9-oxo-12,12a-dihydro-9H,11H-azeto-[2,1-c]imidazo[1,5-a][1,4]-benzodiazepin-1-carbonsäureamid wurden in 340 ml Dioxan und 45 ml Pyridin suspendiert und bei ca 7° tropfenweise mit 45 ml (323 mMol) Trifluoressigsäureanhydrid versetzt. Das Reaktionsgemisch wurde über Nacht bei Raumtemperatur gerührt und auf 2 1 Wasser gegossen. Durch Abnutschen der erhaltenen Suspension und Trocknen der Kristalle erhielt man 45.65 g (64%) (S)-7-Fluor-9-oxo-12,12a-dihydro-9H,11H-azeto[2,1-c]imidazo[1,5-a][1,4]-benzodiazepin-1-carbonitril das ohne weitere Reinigung in die nächste Stufe eingesetzt wurde.

e) 4.2 g (180 mMol) Natrium wurden in 225 ml Methanol gelöst. 13.55 g (195 mMol) Hydroxylaminhydrochlorid und 40 g (149 mMol) (S)-7-Fluor-9-oxo-12,12a-dihydro-9H,11H-azeto[2,1-c]imidazo[1,5-a][1,4]-benzodiazepin-1-carbonitril wurden zugegeben, und das Reaktionsgemisch wurde über Nacht bei Siedetemperatur gerührt. Die Suspension wurde auf 5° gekühlt und abgenutscht. Die Kristalle wurden mit Wasser nachgewaschen und getrocknet. Man erhielt 38.9 g (87%) (S)-7-Fluor-9-oxo-12,12a-dihydro-9H,11H-azeto[2,1-c]imidazo[1,5-a][1,4]-benzodiazepin-1-carboxamidoxim das ohne weitere Reinigung in die nächste Stufe eingesetzt wurde.

f) 15.9 g (52.8 mMol) (S)-7-Fluor-9-oxo-12,12a-dihydro 9H,11H-azeto-[2,1-c]imidazo[1,5-a][1,4]benzodiazepin-1-carboxamidoxim wurden mit 9.93 g (58 mMol) Chloressigsäure-anhydrid und 50 ml N,N-Dimethylformamid während 0.5 Stunde bei Raumtemperatur und 2 Stunden bei 105° gerührt. Nach Eindampfen des Reaktionsgemisches wurde der Rückstand in Methylenchlorid gelöst, und die Lösung wurde mit gesättigter Natriumbicarbonatlösung gewaschen. Durch Trocknung der organischen Phase über Magnesiumsulfat, Eindampfen des Lösungsmittels und Chromatographieren des Rückstandes an Kieselgel unter Eluieren mit Essigester erhielt man 11 g (58%) (S) 1-(5-Chlormethyl-1,2,4-oxadiazol-3-yl)-7-fluor-12,12a-dihydro-9H,11H-azeto[2,1-c]imidazo[1,5-a][1,4]benzodiazepin-9-on.

g) 1.8 g (5 mMol) (S) 1-(5-Chlormethyl-1,2,4-oxadiazol-3-yl)-7-fluor-12,12a-dihydro-9H,11H-azeto[2,1-c]imidazo [1,5-a][1,4]benzodiazepin-9-on wurden über Nacht mit 2g (20 mMol) Dipropylamin und 20 ml N,N-Dimethylformamid bei Raumtemperatur gerührt. Durch Eindampfen des Lösungsmittels und Chromatographieren des Rückstandes an Kieselgel unter Eluieren mit Essigester erhielt man 1.8 g (85%) (S)-1-(5-Dipropyl-aminomethyl-1,2,4-oxadiazol-3-yl)-7-fluor-12,12a-dihydro-9H,11H-azeto-[2,1-c]imidazo[1,5-a][1,4]benzodiazepin-9-on, das in das Hydrochlorid (amorph) überführt wurde.

Beispiel 196

1.8 g (5 mMol) (S)-1-(5-Chlormethyl-1,2,4-oxadiazol-3-yl)-7-fluor-12,12a-dihydro-9H,11H-azeto[2,1-c]imidazo[1,5-a][1,4]benzodiazepin-9-on wurden während 2 Stunden mit 2 g (34 mMol) Propylamin und 20 ml N,N-Dimethylformamid bei Raumtemperatur gerührt. Durch Abdampfen des Lösungsmittels und Chromatographieren des Rückstandes an Kieselgel unter Eluieren mit Essigester/Methanol 9/1 erhielt man 1.4 g (73%) (S)-7-Fluor-1-(5-propyl-aminomethyl-1,2,4-oxadiazol-3-yl)-12,12a-dihydro-9H,11H-azeto-[2,1-c]imidazo[1,5-a][1,4]-benzodiazepin-9-on, das in das Hydrochlorid (amorph) überführt wurde.

Beispiel 197

0.99 g (3 mMol) 3-(5-Chlormethyl-1,2,4-oxadiazol-3-yl)-5-methyl-5,6-dihydro-4H-imidazo[1,5-a]-[1,4]benzodiazepin-6-on wurden über Nacht in 1.2 g (16 mMol) Diäthylamin und 15 ml N,N-Dimethylformamid bei Raum-temperatur gerührt. Durch Eindampfen des Reaktionsgemisches und Chromatographieren des Rückstandes an Kieselgel unter Eluieren mit Essigester/Methanol 9/1 erhielt man 0.9 g (81%) 3-(5-Diäthylaminomethyl-1,2,4-oxadiazol-3-yl)-5-methyl-5,6-dihydro-4H-imidazo[1,5-a][1,4]benzodiazepin-6-on vom Smp. 153-154°, das in das Hydrochlorid überführt wurde.

Beispiel 198

0.99 g (3 mMol) 3-(5-Chlormethyl-1,2,4-oxadiazol-3-yl)-5-methyl-5,6-dihydro-4H-imidazo[1,5-a]-[1,4]benzodiazepin-6-on wurden über Nacht in 2 g (16 mMol) Di-butylamin und 15 ml N,N-Dimethylformamid bei Raum-temperatur gerührt. Durch Eindampfen des Reaktionsgemisches und Chromatographieren des Rückstandes an Kieselgel unter Eluieren mit Essigester erhielt man 0.82 g (65%) 3-(5-Dibutylaminomethyl-1,2,4-oxadiazol-3-yl)-5-methyl-5,6-dihydro-4H-imidazo[1,5-a][1,4]benzodiazepin-6-on, das in das Hydrochlorid vom Smp. 105-110° überführt wurde.

Beispiel 199

1.15 g (3.5 mMol) (S) 1-(5-Chlormethyl-1,2,4-oxadiazol-3-yl)-12,12a-dihydro-9H,11H-azeto[2,1-c]imidazo [1,5-a][1,4]benzodiazepin-9-on wurden über Nacht mit 0.73 g (10 mMol) Diäthylamin und 10 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/ Methanol 19/1 erhielt man 1.23 g (93%) (S) 1-(5-Diäthylaminomethyl-1,2,4-oxadiazol-3-yl)-12,12a-dihydro-9H,11H-azeto[2,1-c]imidazo[1,5-a][1,4]benzodiazepin-9-on, das in das Hydrochlorid vom Smp. 209-211° überführt wurde.

Beispiel 200

1.15 g (3.5 mMol) (S)-1-(5-Chlormethyl-1,2,4-oxadiazol-3-yl)-12,12a-dihydro-9H,11H-azeto[2,1-c]-imidazo [1,5-a][1,4]benzodiazepin-9-on wurden über Nacht mit 1.29 g (10 mMol) Dibutylamin und 10 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.04 g (68%) (S)-1-(5-Dibutylaminomethyl-1,2,4-oxadiazol-3-yl)-12,12a-dihydro-9H,11H-azeto[2,1-c]-imidazo[1,5-a][1,4]benzodiazepin-9-on, das in das Hydrochlorid (amorph) überführt wurde.

Beispiel 201

a) 30 g (106.7 mMol) (S)-5-Brom-1,10a-dihydro-2H-azeto[2,1-c][1,4]benzodiazepin-4,10(9H)-dion wurden in 50 ml N,N-Dimethylformamid gelöst, bei -20° mit 5.6 g (128 mMol) Natriumhydrid-Dispersion 55%ig in Oel (mit Hexan gewaschen) versetzt und während 30 Minuten bei -30° bis -20° deprotoniert. Bei -60° gab man eine Lösung von 43 g (160 mMol) Phosphorsäurediphenyl-esterchlorid in 25 ml N,N-Dimethylformamid zu und rührte während 35 Minuten bei max. -45°. Inzwischen wurden separat 12 g (107 mMol) Kalium-tert.-butylat in 20 ml N,N-Dimethylformamid gelöst und bei -60° mit 12.1 g (107 mMol) Isocyanessigsäure-aethylester versetzt. Man tropfte den deprotonierten Isocyanessigsäure-aethylester dem Reaktions-gemisch bei max. -65° innerhalb von 1 Stunden zu. Man rührte während 1 Stunde im Aceton/Trockeneisbad weiter, neutralisierte mit 10 ml Essigsäure und goss auf 500 ml Eiswasser. Man extrahierte fünfmal mit Methylenchlorid (insgesamt 1.2 1), trocknete über Magnesiumsulfat und dampfte zur Trockene ein. Durch Umkristallisieren des Rückstandes aus Aethanol und Aether erhielt man 23.7 g (49%) Aethyl (S)-8-Brom-9-oxo-12,12a-dihydro-9H,11H-azeto[2,1-c]imidazo[1,5-a]-[1,4]-benzodiazepin-1-carboxylat vom Smp. 184-185°.

b) 21.8 g (57.9 mMol) Aethyl (S)-8-Brom-9-oxo-12,12a-dihydro-9H,11H-azeto[2,1-c]imidazo[1,5-a][1,4]-benzodiazepin-1-carboxylat; wurden mit 17 ml 4N Natronlauge und 200 ml Aethanol während 15 Minuten zum Rückfluss erhitzt. Bei Raumtemperatur gab man 17 ml 4N Salzsäure und 250 ml Wasser zu. Der Alkohol wurde abgedampft, und die Suspension wurde auf 0° gekühlt. Durch Abnutschen und Trocknen der Kristalle erhielt man 19.8 g (98%) (S)-8-Brom-9-oxo-12,12a-dihydro-9H,11H-azeto[2,1-c]imidazo[1,5-a]-[1,4]-benzodiazepin-1-carbonsäure vom Smp. 178-180°.

c) 19.8 g (56.9 mMol) (S)-8-Brom-9-oxo-12,12a-dihydro-9H,11H-azeto-[2,1-c]imidazo[1,5-a][1,4]-benzodiazepin-1-carbonsäure wurden in 100 ml N,N-Dimethylformamid suspendiert, bei Raumtemperatur mit 10.1 g (62.6 mMol) 1,1'-Carbonyldiimidazol versetzt und während 30 Minuten bei 70° gerührt. 50 ml 25%iges Ammoniak wurden bei 25 -30° zugetropft. Das Reaktionsgemisch wurde mit 50 ml Wasser verdünnt und auf 0° gekühlt, und das Kristallisat wurde abgenutscht und getrocknet. Man erhielt 133 6 g (69%) (S)-8-Brom-9-oxo-12,12a-dihydro-9H,11H-azeto [2,1-c]imidazo[1,5-a]-[1,4]benzodiazepin-1-carboxamid vom Smp. 314-317°.

d) Eine Suspension von 19 g (54.7 mMol) (S)-8-Brom-9-oxo-12,12a-dihydro-9H,11H-azeto[2,1-c]imidazo[1,5-a][1,4]benzodiazepin-1-carboxamid in einem Gemisch von 100 ml Dioxan und 15 ml Pyridin wurde bei 7 bis 10° mit 15 ml Trifluoressigsäureanhydrid versetzt. Die Suspension wurde 1 Stunde bei Raumtemperatur gerührt und auf 600 ml Wasser gegossen. Nach 1 Stunde wurde die Suspension abgenutscht. Man erhielt 13.1 g (72%) (S)-8-Brom-9-oxo-12,12a-dihydro-9H,11H-azeto[2,1-c]imidazo[1,5-a][1,4]benzodiazepin-1-carbonitril vom Smp. 133-136°.

e) 13 g (39.5 mMol) (S)-8-Brom-9-oxo-12,12a-dihydro-9H,11H-azeto[2,1-c]-imidazo[1,5-a][1,4]benzodiazepin-1-carbonitril wurden mit 7.5 g (71 mMol) Natriumcarbonat und 5.7 g (82 mMol) Hydroxylaminhydrochlorid in 200 ml Aethanol und 40 ml Wasser während 1 Stunde bei 50° und 10 Minuten bei 75° gerührt. Nach Verdünnen mit 50 ml Wasser wurde die Suspension genutscht, worauf die Kristalle getrocknet wurden. Man erhielt 7.33 g (51%) (S)-8-Brom-9-oxo-12,12a-dihydro-9H,11H-azeto[2,1-c]imidazo[1,5-a][1,4]benzodiazepin-1-carboxamidoxim vom Smp. 265-266°.

f) 7 g (19.3 mMol) (S)-8-Brom-9-oxo-12,12a-dihydro-9H,11H-azeto[2,1-c]-imidazo[1,5-a][1,4]-benzodiazepin-1-carboxamidoxim wurden mit 3.8 g (22.2 mMol) Chloressigsäureanhydrid in 50 ml N,N-Dimethylformamid während 1 Stunde bei Raumtemperatur und 2 Stunden bei 105° gerührt. Nach Eindampfen des Reaktionsgemisches wurde der Rückstand in Methylenchlorid gelöst, und die Lösung wurde mit gesättigter Natriumbicarbonat-lösung gewaschen. Die organische Phase wurde über Magnesiumsulfat getrocknet und eingeengt. Nach Umkristallisieren des Rückstandes aus Methanol erhielt man 5.3 g (65%) (S)-8-Brom-1-(5-chlormethyl-1,2,4-oxadiazol-3-yl)-12,12a-dihydro-9H,11H-azeto[2,1-c]imidazo[1,5-a][1,4]benzodiazepin-9-on vom Smp. 210-211°.

g) 1.26 g (3 mMol) (S)-8-Brom-1-(5-chlormethyl-1,2,4-oxadiazol-3-yl)-12,12a-dihydro-9H,11H-azeto[2,1-c]imidazo [1,5-a][1,4]benzodiazepin-9-on wurden während 5 Stunden mit 0.44 g (6 mMol) Diäthylamin und 10 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 19/1 erhielt man 1.14 g (82%) (S)-8-Brom-1-(5-diäthylaminomethyl-1,2,4-oxadiazol-3-yl)-12,12a-dihydro-9H,11H-azeto[2,1-c]-imidazo[1,5-a][1,4] benzodiazepin-9-on, das in das Hydrochlorid vom Smp. 206-209° überführt wurde.

Beispiel 202

a) Eine Suspension von 12.8 g (35.4 mMol) (S)-8-Brom-9-oxo-11,12,13,13a-tetrahydro-9H-imidazo[1,5-a]pyrrolo [2,1-c]-[1,4]benzodiazepin-1-carboxamid in einem Gemisch von 50 ml Dioxan und 7 ml Pyridin wurde bei 7 bis 10° mit 6.5 ml Trifluoressigsäureanhydrid versetzt. Die Suspension wurde 2.5 Stunden bei Raumtemperatur gerührt und auf 200 ml Wasser gegossen. Nach 1 Stunde wurde die Suspension abgenutscht. Man erhielt 12.1 g (100%) (S)-8-Brom-9-oxo-11,12,13,13a-tetrahydro-9H-imidazo[1,5-a]-pyrrolo[2,1-c]-[1,4]benzodiazepin-1-carbonitril vom Smp. 253-254°.

b) 12 g (35 mMol) (S)-8-Brom-9-oxo-11,12,13,13a-tetrahydro-9H-imidazo[1,5-a]pyrrolo[2,1-c][1,4]benzodiazepin-1-carbonitril wurden während 2 Stunden mit 9.6 g (94 mMol) Natriumbicarbonat und 7.3 g (105 mMol) Hydroxylaminhydrochlorid in 170 ml Aethanol und 40 ml Wasser bei Siedetemperatur gerührt. Nach Abdampfen des Alkohols wurde die erhaltene Suspension gekühlt, und das Kristallisat wurde abgenutscht und getrocknet. Man erhielt 11.4 g (86%) (S)-8-Brom-9-oxo-11,12,13,13a-tetrahydro-9H-imidazo[1,5-a]pyrrolo[2,1-c][1,4]benzo-diazepin-1-carboxamidoxim vom Smp. 235-237°.

c) 11.5 g (30.6 mMol) (S)-8-Brom-9-oxo-11,12,13,13a-tetrahydro-9H-imidazo[1,5-a]pyrrolo[2,1-c][1,4]benzodiazepin-1-carboxamidoxim wurden mit 6.02 g (35.2 mMol) Chloressigsäure-anhydrid in 60 ml N,N-Dimethylformamid während 1 Stunde bei Raumtemperatur und 3 Stunden bei 105° gerührt. Nach Abdampfen des Lösungsmittels wurde der Rückstand in Methylenchlorid gelöst, und die Lösung wurde mit gesättigter Natriumbicarbonatlösung gewaschen. Durch Trocknen der organischen Phase über Magnesiumsulfat, Eindampfen des Lösungsmittels und Chromatographieren des Rückstandes an Kieselgel unter Eluieren mit Essigester erhielt man 10.2 g (76%) (S)-8-Brom-1-(5-chlormethyl-1,2,4-oxadiazol-3-yl)-11,12,13,13a-tetrahydro-9H-imidazo[1,5-a]pyrrolo[2,1-c][1,4]benzodiazepin-9on vom Smp. 242-244°.

d) 1.3 g (3 mMol) (S) 8-Brom-1-(5-chlormethyl-1,2,4-oxadiazol-3-yl)-11,12,13,13a-tetrahydro-9H-imidazo[1,5-a]pyrrolo[2,1-c][1,4]benzodiazepin-9-on wurden während 5 Stunden mit 1.02 g (10 mMol) Dipropylamin und 15 ml N,N-Dimethylformamid bei Raumtemperatur gerührt. Durch Abdampfen des Lösungsmittels und Chromatographieren des Rückstandes an Kieselgel unter Eluieren mit Methylenchlorid/Methanol 19/1 erhielt man 1.41 g (94%) (S) 8-Brom-1-(5-dipropylaminomethyl-1,2,4-oxadiazol-3-yl)-11,12,13,13a-tetrahydro-9H-imidazo[1,5-a]pyrrolo[2,1-c][1,4]benzodiazepin-9-on, das in das Hydrochlorid (amorph) überführt wurde.

Beispiel 203

a) 6.24 g (18.2 mMol) (S)-8-Chlor-7-fluor-9-oxo-12,12a-dihydro-9H,11H-azeto[2,1-c]imidazo[1,5-a][1,4]benzodiazepin-1-carboxamidoxim wurden mit 3.43 g (20 mMol) Chloressigsäure-anhydrid und 40 ml N,N-Dimethylformamid während 60 Stunden bei Raumtemperatur und 2.5 Stunden bei 105° gerührt. Durch Eindampfen des Reaktionsgemisches und Chromatographieren des Rückstandes an Kieselgel unter Eluieren mit Methylenchlorid/Methanol 19/1 erhielt man 1.88 g (26%) (S)-8-Chlor-1-(5-chlormethyl-1,2,4-oxadiazol-3-yl)-7-fluor-12,12a-dihydro-9H,11H-azeto[2,1-c]imidazo-[1,5-a][1,4]benzodiazepin-9-on vom Smp. 214-217°.

b) 1 g (2.5 mMol) (S)-8-Chlor-1-(5-chlormethyl-1,2,4-oxadiazol-3-yl)-7-fluor-12,12a-dihydro-9H,11H-azeto[2,1-c]imidazo[1,5-a][1,4]benzodiazepin-9-on wurden über Nacht mit 0.98 g (7.5 mMol) Dibutylamin und 10 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 0.44 g (36%) (S)-8-Chlor-1-(5-dibutylaminomethyl-1,2,4-oxadiazol-3-yl)-7-fluor-12,12a-dihydro-9H,11H-azeto[2,1-c]imidazo[1,5-a][1,4]benzodiazepin-9-on, das in das Hydrochlorid (amorph) überführt wurde.

Beispiel 204

0.88 g (2.5 mMol) (S)-8-Chlor-1-(5-chlormethyl-1,2,4-oxadiazol-3-yl)-7-fluor-12,12a-dihydro-9H,11H-azeto[2,1-c]imidazo[1,5-a][1.,4]benzodiazepin-9-on wurden über Nacht mit 0.5 g (6.8 mMol) Diäthylamin und 10 ml N,N-Dimethylformamid bei Raumtemperatur gerührt. Nach Eindampfen des Reaktionsgemisches wurde der Rückstand in Methylenchlorid gelöst, und die Lösung wurde mit Wasser gewaschen. Durch Trocknen der organischen Phase über Magnesiumsulfat und Abdampfen des Lösungsmittels erhielt man 0.9 g (95%) (S)-8-Chlor-1-(5-diäthylaminomethyl-1,2,4-oxadiazol-3-yl)-7-fluor-12,12a-dihydro-9H,11H-azeto[2,1-c]imidazo[1,5-a][1,4]benzo-diazepin-9-on, das in das Hydrochlorid (amorph) überführt wurde.

Beispiel 205

1.19 g (3 mMol) 3-(5-Chlormethyl-1,2,4-oxadiazol-3-yl)-5-methyl-7-trifluormethyl-5,6-dihydro-4H-imidazo[1,5-a][1,4]benzodiazepin-6-on wurden während 5 Stunden mit 1.02 g (10 mMol) Dipropylamin und 15 ml N,N-Dimethylformamid bei Raumtemperatur gerührt. Nach Eindampfen des Reaktionsgemisches wurde der Rückstand in Methylenchlorid gelöst, und die Lösung wurde mit Wasser gewaschen. Durch Trocknung der organischen Phase über Magnesiumsulfat und Abdampfen des Lösungsmittels erhielt man 1.33 g (96%) 3-(5-Dipropylaminomethyl-1,2,4-oxadiazol-3-yl)-5-methyl-7-trifluormethyl-5,6-dihydro-4H-imidazo[1,5-a][1,4]benzodiazepin-6-on, das in das Hydrochlorid vom Smp. 216-218° überführt wurde.

Beispiel 206

a) 7 g (20.6 mMol) 5-Methyl-6-oxo-7-trifluormethyl-5,6-dihydro-4H-imidazo[1,5-a][1,4]benzo-diazepin-3-carboxamidoxim wurden in 50 ml N,N-Dimethylformamid mit 4.05 g (23.7 mMol) Chloressigsäureanhydrid während 2 Stunden bei Raumtemperatur und 2 Stunden bei 105° gerührt. Durch Eindampfen des Reaktionsgemisches und Chromatographieren des Rückstandes an Kieselgel unter Eluieren mit Essigester erhielt man 7.12 g (87%) 3-(5-Chlormethyl-1,2,4-oxadiazol-3-yl)-5-methyl-7-trifluormethyl-5,6-dihydro-4H-imidazo[1,5-a][1,4]benzodiazepin-6-on vom Smp. 180-184°.

b) 1.19 g (3 mMol) 3-(5-Chlormethyl-1,2,4-oxadiazol-3-yl)-5-methyl-7-trifluormethyl-5,6-dihydro-4H-imidazo[1,5-a][1,4]benzodiazepin-6-on wurden über Nacht mit 5.3 g (90 mMol) Propylamin und 10 ml N,N-Dimethyl-formamid bei Raumtemperatur gerührt. Nach Eindampfen des Reaktionsgemisches wurde der Rückstand in Methylenchlorid gelöst, und die Lösung wurde mit Wasser gewaschen. Durch Trocknung der organischen Phase über Magnesiumsulfat und Abdampfen des Lösungsmittels erhielt man 0.84 g (67%) 5-Methyl-3-(5-propylaminomethyl-1,2,4-oxadiazol-3-yl)-7-trifluormethyl-5,6-dihydro-4H-imidazo[1,5-a][1,4]benzodiazepin-6-on, das in das Hydrochlorid vom Smp. 234-237° überführt wurde.

Beispiel 207

a) 24.9 g (74.1 mMol) 7-Brom-5-methyl-6-oxo-5,6-dihydro-4H-imidazo-[1,5-a][1,4]benzodiazepin-3-carbonsäure wurden in 100 ml N,N-Dimethylformamid suspendiert, bei Raumtemperatur mit 14.3 g (89 mMol) 1,1'-Carbonyldiimidazol versetzt und während 30 Minuten bei 70° gerührt. 50 ml 25%iges Ammoniak wurden bei 25 -30° zugetropft. Das Reaktionsgemisch wurde mit 200 ml Wasser verdünnt und auf 0° gekühlt, und das Kristallisat wurde abgenutscht und getrocknet. Man erhielt 21.6 g (86%) 7-Brom-5-methyl-6-oxo-5,6-dihydro-4H-imidazo[1,5-a][1,4]benzodiazepin-3-carboxamid vom Smp. 278-279°.

b) Eine Suspension von 21.5 g (64.1 mMol) 7-Brom-5-methyl-6-oxo-5,6-dihydro-4H-imidazo[1,5-a]-[1,4]benzodiazepin-3-carboxamid in einem Gemisch von 100 ml Dioxan und 15 ml Pyridin wurde bei 7 bis 10° mit 15 ml Trifluoressigsäureanhydrid versetzt. Die Suspension wurde 1 Stunde bei Raumtemperatur gerührt und auf 600 ml Wasser gegossen. Nach 1 Stunde wurde die Suspension abgenutscht. Man erhielt 17.8 g (88%) 7-Brom-5-methyl-6-oxo-5,6-dihydro-4H-imidazo[1,5-a][1,4]benzodiazepin-3-carbonitril vom Smp. 226-228°.

c) 17.85 g (56.3 mMol) 7-Brom-5-methyl-6-oxo-5,6-dihydro-4H-imidazo[1,5-a]-[1,4]benzodiazepin-3-carbonitril wurden mit 10.3 g (97.7 mMol) Natriumcarbonat und 8 g (115.1 mMol) Hydroxylaminhydrochlorid in 300 ml Aethanol und 60 ml Wasser während 1 Stunde bei 50° und 20 Minuten bei 75° gerührt. Nach Verdünnen mit 50 ml Wasser wurde die Suspension genutscht, und die Kristalle wurden getrocknet. Man erhielt 16.95 g (86%) 7-Brom-5-methyl-6-oxo-5,6-dihydro-4H-imidazo[1,5-a][1,4]benzodiazepin-3-carboxamidoxim vom Smp. 265-266°.

d) 16.9 g (48.3 mMol) 7-Brom-5-methyl-6-oxo-5,6-dihydro-4H-imidazo-[1,5-a][1,4]benzodiazepin-3-carboxamidoxim wurden mit 9.5 g (55.5 mMol) Chloressigsäure-anhydrid in 100 ml N,N-Dimethylformamid während 1 Stunde bei Raumtemperatur und 3 Stunden bei 105° gerührt. Nach Abdampfen des Lösungsmittels wurde der Rückstand in Methylenchlorid gelöst, und die Lösung wurde mit gesättigter Natriumbicarbonatlösung gewaschen. Durch Trocknen der organischen Phase über Magnesium-sulfat, Eindampfen des Lösungsmittels und Chromatographieren des Rückstandes an Kieselgel unter Eluieren mit Methylenchlorid/Methanol 9/1 erhielt man 16.3 g (84%) 7-Brom-3-(5-chlormethyl-1,2,4-oxadiazol-3-yl)-5-methyl-5,6-dihydro-4H-imidazo[1,5-a][1,4]benzodiazepin-6-on vom Smp. 242-244°.

e) 2.04 g (5 mMol) 7-Brom-3-(5-chlormethyl-1,2,4-oxadiazol-3-yl)-5-methyl-5,6-dihydro-4H-imidazo[1,5-a][1,4]benzodiazepin-6-on wurden während 4 Stunden mit 1.5 g (15 mMol) Dipropylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.61 g (68%) 7-Brom-3-(5-dipropyl-aminomethyl-1,2,4-oxadiazol-3-yl)-5-methyl-5,6-dihydro-4H-imidazo[1,5-a][1,4]benzodiazepin-6-on, das in das Hydrochlorid vom Smp. 135-145° überführt wurde.

Beispiel 208

2.1 g (5 mMol) (S)-8-Brom-1-(5-chlormethyl-1,2,4-oxadiazol-3-yl)-12,12a-dihydro-9H,11H-azeto[2,1-c]imidazo[1,5-a][1,4]benzodiazepin-9-an wurden während 2 Stunden mit 1.52 g (15 mMol) Dipropylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man nach Umkristallisieren aus Essigester 1.2 g (50%) (S)-8-Brom-1-(5-dipropylaminomethyl-1,2,4-oxadiazol-3-yl)-12,12a-dihydro-9H,11H-azeto[2,1-c]-imidazo [1,5-a][1,4]benzodiazepin-9-on vom Smp. 122-124°, das in das Hydrochlorid vom Smp. 196-198° überführt wurde.

Beispiel 209

1.88 g (5 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 4 Stunden mit 2 g (2.2 mMol) Bis(2-methoxyäthyl)-amin und 15 ml N,N-Dimethylformamid bei Raumtemperatur gerührt. Nach Abdampfen des Lösungsmittels wurde das Reaktionsgemisch in 4 N Natronlauge aufgenommen, worauf dreimal mit Methylenchlorid extrahiert wurde. Durch Trocknen der organischen Phase über Magnesiumsulfat und Eindampfen des Lösungsmittels erhielt man 2.2 g (93%) (S)-8-Chlor-1-[5-di-(2-methoxyäthyl)aminomethyl-1,2,4-oxadiazol-3-yl]-12,12a-dihydro-9H,11H-azeto[2,1-c]imidazo[1,5-a][1,4] benzodiazepin-9-on, das in das Hydrochlorid vom Smp. 188-190°überführt wurde.

Beispiel 210

0.94 g (2.5 mMol) (S)-8-Chlor-1-(5-chlormethyl-1,2,4-oxadiazol-3-yl)-12,12a-dihydro-9H,11H-azeto[2,1-c] imidazo[1,5-a][1,4]benzodiazepin-9-on wurden über Nacht mit 1 g (17 mMol) Propylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester/Methanol 9/1 erhielt man 0.92 g (92%) (S)-8-Chlor-1-(5-propylaminomethyl-1,2,4-oxadiazol-3-yl)-12,12a-dihydro-9H,11H-azeto[2,1-c]imidazo[1,5-a][1,4]benzodiazepin-9-on vom Smp. 237-239°, das in das Hydrochlorid überführt wurde.

Beispiel 211

1.5 g (4.5 mMol) 3-(5-Chlormethyl-1,2,4-oxadiazol-3-yl)-5-methyl-5,6-dihydro-4H-imidazo[1,5-a][1,4]benzodiazepin-6-on wurden über Nacht mit 0.5 g (4.4 mMol) Heptamethylenimin und 10 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 0.2 g (12%) 3-[5-(Heptamethylenimin-1-yl)methyl-1,2,4-oxadiazol-3-yl]-5-methyl-5,6-dihydro-4H-imidazo[1,5-a][1,4]benzodiazepin-6-on, das in das Hydrochlorid vom Smp. 246-248° überführt wurde.

Beispiel 212

0.7 g (2.1 mMol) 3-(5-Chlormethyl-1,2,4-oxadiazol-3-yl)-5-methyl-5,6-dihydro-4H-imidazo[1,5-a][1,4]benzodiazepin-6-on wurden über Nacht mit 0.5 g (5 mMol) Hexamethylenimin und 10 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 0.76 g (90%) 3-[5-(Hexamethylenimin-1-yl)methyl-1,2,4-oxadiazol-3-yl]-5-methyl-5,6-dihydro-4H-imidazo[1,5-a][1,4]benzodiazepin-6-on vom Smp. 158-159°, das in das Hydrochlorid überführt wurde.

Beispiel 213

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 5 ml (25 mMol) Dicyclohexylamin und 7 ml N,N-Dimethylformamid bei 65° gerührt. Durch Eindampfen des Reaktionsgemisches und Chromatographieren des Rückstandes an Kieselgel unter Eluieren mit Essigester erhielt man 0.8 g (51%) (S)-8-Chlor-1-(5-dicyclohexylaminomethyl-1,2,4-oxadiazol-3-yl)-12,12a-dihydro-9H,11H-azeto[2,1-c]imidazo[1,5-a][1,4]-benzodiazepin-9-on, das in das Hydrochlorid vom Smp. 155-185° überführt wurde.

Beispiel 214

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden während 4 Stunden mit 2 g (17 mMol) 2,6-Dimethylpiperidin und 15 ml N, N-Dimethylformamid bei 70° gerührt. Durch Eindampfen des Reaktionsgemisches und Chromatographieren des Rückstandes an Kieselgel unter Eluieren mit Essigester/Methanol 9/1 erhielt man 0.83 g (61%) (S)-8-Chlor-1-[5-(2,6-dimethylpiperidin-1-yl)methyl-1,2,4-oxadiazol-3-yl]-12,12a-dihydro-9H,11H-azeto[2,1-c]-imidazo[1,5-a][1,4]benzodiazepin-9-on, das in das Hydrochlorid vom Smp. 233-236° überführt wurde.

Beispiel 215

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2 g (12.7 mMol) Dipentylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.01 g (68%) (S)-8-Chlor-1-(5-dipentylaminomethyl-1,2,4-oxadiazol-3-yl)-12,12a-dihydro-9H,11H-azeto[2,1-c]-imidazo[1,5-a][1,4]-benzodiazepin-9-on, das in das Hydrochlorid (amorph) überführt wurde.

Beispiel 216

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 2 g (17.7 mMol) 3,3-Dimethylpiperidin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.34 g (98%) (S)-8-Chlor-1-[5-(3,3-dimethyl-piperidin-1-yl)methyl-1,2,4-oxadiazol-3-yl]-12,12a-dihydro-9H,11H-azeto[2,1-c]imidazo[1,5-a][1,4]benzodiazepin-9-on, das in das Hydrochlorid (amorph) überführt wurde.

Beispiel 217

a) 9.8 g (32.1 mMol) 7-Chlor-5-methyl-6-oxo-5,6-dihydro-4H-imidazo-[1,5-a][1,4]-benzodiazepin-3-carboxamidoxim wurden mit 7 g (41 mMol) Chloressigsäureanhydrid in 60 ml N,N-Dimethylformamid während 1 Stunde bei Raumtemperatur und 4 Stunden bei 105° gerührt. Nach Eindampfen des Reaktionsgemisches wurde der Rückstand in Methylenchlorid gelöst, und die Lösung wurde mit gesättigter Natriumbicarbonat-lösung gewaschen. Die organische Phase wurde über Magnesiumsulfat getrocknet und eingeengt, und der Rückstand wurde an Kieselgel chromatographiert unter Eluieren mit Essigester. Man erhielt 6.63 g (57%) 7-Chlor-3-(5-chlormethyl-1,2,4-oxadiazol-3-yl)-5-methyl-5,6-dihydro-4H-imidazo[1,5-a][1,4]benzodiazepin-6-on vom Smp. 208-210°.

b) 1.6 g (4.4 mMol) 7-Chlor-3-(5-chlormethyl-1,2,4-oxadiazol-3-yl)-5-methyl-5,6-dihydro-4H-imidazo[1,5-a][1,4]benzodiazepin-6-on wurden während 4.5 Stunden mit 1.1 g (15 mMol) Diäthylamin und 10 ml N,N-Dimethylformamid bei Raumtemperatur gerührt. Durch Eindampfen des Reaktionsgemisches und Chromatographieren des Rückstandes an Kieselgel unter Eluieren mit Methylenchlorid/Methanol 19/1 erhielt man 1.50 g (85%) 7-Chlor-3-(5-diäthylaminomethyl-1,2,4-oxadiazol-3-yl)-5-methyl-5,6-dihydro-4H-imidazo[1,5-a][1,4]-benzodiazepin-6-on, das in das Hydrochlorid vom Smp. 250-252° überführt wurde.

Beispiel 218

1.6 g (4.4 mMol) 7-Chlor-3-(5-chlormethyl-1,2,4-oxadiazol-3-yl)-5-methyl-5,6-dihydro-4H-imidazo[1,5-a][1,4]benzodiazepin-6-on wurden während 48 Stunden mit 3.6 ml (21 mMol) Dibutylamin und 10 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.23 g (61%) 7-Chlor-3-(5-dibutyl-aminomethyl-1,2,4-oxadiazol-3-yl)-5-methyl-5,6-dihydro-4H-imidazo[1,5-a][1,4]benzodiazepin-6-on vom Smp. 140-142°, das in das Hydrochlorid vom Smp. 184-187° überführt wurde.

Beispiel 219

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 1.7 g (9 mMol) Dihexylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 0.89 g (56%) (S)-8-Chlor-1-(5-dihexyl-aminomethyl-1,2,4-oxadiazol-3-yl)-12,12a-dihydro-9H,11H-azeto[2,1-c]imidazo[1,5-a][1,4]-benzodiazepin-9-on, das in das Hydrochlorid (amorph) überführt wurde.

Beispiel 220

1.15 g (3.5 mMol) 3-(5-Chlormethyl-1,2,4-oxadiazol-3-yl)-5-methyl-5,6-dihydro-4H-imidazo[1,5-a][1,4]benzodiazepin-6-on wurden während 1 Stunde mit 1 g (8.8 mMol) 3,3-Dimethylpiperidin und 10 ml N,N-Dimethylformamid bei Raumtemperatur gerührt. Durch Eindampfen des Reaktionsgemisches und Chromatographieren des Rückstandes an Kieselgel unter Eluieren mit Essigester/Aethanol 9/1 erhielt man 1.32 g (92%) 3-[5-(3.3-Dimethylpiperidin-1-yl)methyl-1,2,4-oxadiazol-3-yl]-5-methyl-5,6-dihydro-4H-imidazo[1,5-a][1,4]-benzodiazepin-6-on vom Smp. 167-168°, das in das Hydrochlorid überführt wurde.

Beispiel 221

1.15 g (3.5 mMol) 3-(5-Chlormethyl-1,2,4-oxadiazol-3-yl)-5-methyl-5,6-dihydro-4H-imidazo[1,5-a][1,4]benzodiazepin-6-on wurden während 1 Stunde mit 1 g (11.7 mMol) Piperidin und 10 ml N,N-Dimethylformamid bei Raumtemperatur gerührt. Durch Eindampfen des Reaktionsgemisches und Chromatographieren des Rückstandes an Kieselgel unter Eluieren mit Essigester/Aethanol 9/1 erhielt man 0.98 g (74%) 5-Methyl-3-[5-(piperidin-1-yl)methyl-1,2,4-oxadiazol-3-yl]-5,6-dihydro-4H-imidazo[1,5-a][1,4]benzodiazepin-6-on vom Smp. 167-168°, das in das Hydrochlorid überführt wurde.

Beispiel 222

1.3 g (3.6 mMol) 7-Chlor-3-(5-chlormethyl-1,2,4-oxadiazol-3-yl)-5-methyl-5,6-dihydro-4H-imidazo[1,5-a][1,4] benzodiazepin-6-on wurden über Nacht mit 1.6 ml (8.9 mMol) Diisobutylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 0.69 g (42%) 7-Chlor-3-(5-diisobutyl-aminomethyl-1,2,4-oxadiazol-3-yl)-5-methyl-5,6-dihydro-4H-imidazo[1,5-a][1,4]benzodiazepin-6-on vom Smp. 158-160°, das in das Hydrochlorid vom Smp. 125-128° überführt wurde.

Beispiel 223

1.13 g (3 mMol) (S)-8-Chlor-1-(5-chlormethyl-1,2,4-oxadiazol-3-yl)-12,12a-dihydro-9H,11H-azeto[2,1-c]imidazo[1,5-a][1,4]benzodiazepin-9-on wurden über Nacht mit 0.97 g (7.5 mMol) Diisobutylamin und 15 ml N,N-Dimethylformamid bei Raumtemperatur gerührt. Durch Eindampfen des Reaktionsgemisches und Chromatographieren des Rückstandes an Kieselgel unter Eluieren mit Essigester erhielt man 1.07 g (76%) (S)-8-Chlor-1-(5-diisobutyl-aminomethyl-1,2,4-oxadiazol-3-yl)-12,12a-dihydro-9H,11H-azeto[2,1-c]imidazo[1,5-a][1,4]benzodiazepin-9-on, das in das Hydrochlorid vom Smp. 125-140° überführt wurde.

Beispiel 224

72 g (212 mMol) 3-(5-Chlormethyl-1,2,4-oxadiazol-3-yl)-5-methyl-5,6-dihydro-4H-imidazo[1,5-a][1,4]benzodiazepin-6-on wurden während 16 Stunden in 150 ml (1.06 Mol) Diisopropylamin und 300 ml N,N-Dimethylformamid bei 80° gerührt. Nach Eindampfen des Lösungsmittels wurde der Rückstand in Methylenchlorid gelöst, und die Lösung wurde zweimal mit 100 ml 2N Salzsäure extrahiert. Die saure Phase wurde mit konz. Natronlauge alkalisch gestellt und mit Methylenchlorid extrahiert. Die organische Phase wurde über Magnesiumsulfat getrocknet und eingedampft. Durch Chromatographieren des Rückstandes an Kieselgel unter Eluieren mit Essigester/Methanol/Essigsäure 95/3/2 und Umkristallisieren aus Ethanol erhielt man 51.6 g (61%) 3-(5-Diisopropylaminomethyl-1,2,4-oxadiazol-3-yl)-5-methyl-5,6-dihydro-4H-imidazo[1,5-a][1,4]benzodiazepin-6-on vom Smp. 117-119°.

Beispiel 225

1 g (3 mMol) (5-Chlormethyl-1,2,4-oxadiazol-3-yl)-5-methyl-5,6-dihydro-4H-imidazo[1,5-a][1,4]benzodiazepin-6-on wurden über Nacht in 3 ml (17 mMol) Diisobutylamin und 15 ml N,N-Dimethylformamid bei Raum-temperatur gerührt. Durch Eindampfen des Reaktionsgemisches und Chromatographieren des Rückstandes an Kieselgel unter Eluieren mit Essigester erhielt man 1.2 g (94%) 3-(5-Diisobutylaminomethyl-1,2,4-oxadiazol-3-yl)-5-methyl-5,6-dihydro-4H-imidazo[1,5-a][1,4]benzodiazepin-6-on vom Smp. 170-172°, das in das Hydrochlorid vom Smp. 200-202° überführt wurde.

Beispiel 226

1.65 g (5 mMol) 3-(5-Chlormethyl-1,2,4-oxadiazol-3-yl)-5-methyl-5,6-dihydro-4H-imidazo[1,5-a][1,4]benzodiazepin-6-on wurden während 24 Stunden in 3 ml (17.5 mMol) Di-sek.-butylamin und 25 ml N,N-Dimethylformamid bei 75° gerührt. Durch Eindampfen des Reaktionsgemisches und Chromatographieren des Rückstandes an Kieselgel unter Eluieren mit Methylenchlorid/Methanol 19/1 und Kristallisieren aus Essigester erhielt man 1.23 g (58%) 3-(5-di-sek.-Butylaminomethyl-1,2,4-oxadiazol-3-yl)-5-methyl-5,6-dihydro-4H-imidazo[1,5-a][1,4]-benzodiazepin-6-on vom Smp. 155-157°, das in das Hydrochlorid vom Smp. 133-140° überführt wurde.

Beispiel 227

1.82 g (5 mMol) 7-Chlor-3-(5-chlormethyl-1,2,4-oxadiazol-3-yl)-5-methyl-5,6-dihydro-4H-imidazo[1,5-a][1,4] benzodiazepin-6-on wurden über Nacht mit 3 ml (21.3 mMol) Diisopropylamin und 20 ml N,N-Dimethylformamid bei 80° gerührt. Durch Eindampfen des Reaktionsgemisches und Chromatographieren des Rückstandes an Kieselgel unter Eluieren mit Cyclohexan/ Aether/Isopropanol/Ammoniak 15/15/5/0.5 erhielt man 1.41 g (66%) 7-Chlor-3-(5-diisopropylaminomethyl-1,2,4-oxadiazol-3-yl)-5-methyl-5,6-dihydro-4H-imidazo[1,5-a][1,4]benzodiazepin-6-on vom Smp. 58-63°, das in das Hydrochlorid vom Smp. 230-231° überführt wurde.

Beispiel 228

3.30 g (10 mMol) 3-(5-Chlormethyl-1,2,4-oxadiazol-3-yl)-5-methyl-5,6-dihydro-4H-imidazo[1,5-a][1,4]benzodiazepin-6-on wurden während 2.5 Stunden mit 3 g (50 mMol) Isopropylamin und 20 ml N,N-Dimethylformamid bei Raumtemperatur gerührt. Nach Abdampfen des Lösungsmittels wurde der Rückstand in Methylenchlorid gelöst, und die Lösung wurde zweimal mit Wasser gewaschen. Die organische Lösung wurde über Magnesiumsulfat getrocknet und eingeengt. Durch Umkristallisieren des Rückstandes aus Essigester erhielt man 2.37 g (67%) 3-(5-Isopropylaminomethyl-1,2,4-oxadiazol-3-yl)-5-methyl-5,6-dihydro-4H-imidazo[1,5-a][1,4]-benzodiazepin-6-on vom Smp. 142-144°, das in das Hydrochlorid vom Smp. 252-254° überführt wurde.

Beispiel 229

a) Eine Suspension von 7.07 g (21.3 mMol) (S)-8-Chlor-9-oxo-11,12,13,13a-tetrahydro-9H-imidazo[1,5-a]pyrrolo [2,1-c][1,4]benzodiazepin-1-carboxamidoxim in 50 ml N,N-Dimethylformamid wurde mit 4.0 g (23.4 mMol) Chloressigsäureanhydrid versetzt. Die erhaltene gelbe Lösung wurde 2 Std. bei 105° gerührt und dann vollständig von den Lösungsmitteln befreit. Das ölige Produkt wurde über Kieselgel mit Methylenchlorid/Methanol 9:1 als Laufmittel chromatographiert, und das erhaltene ölige Produkt wurde aus Essigester/Aether umkristallisiert. Die Mutterlauge wurde eingeengt, der Rückstand wurde nochmals über Kieselgel mit Acetonitril als Laufmittel chromatographiert, und die erhaltene zusätzliche Portion des Produkts wurde aus Essigester/Aether umkristallisiert. Man erhielt insgesamt 6.27 g (75%) (S)-8-Chlor-1-(5-chlormethyl-1,2,4-oxadiazol-3-yl)-11,12,13,13a-tetrahydro-9H-imidazo [1,5-a]pyrrolo[2,1-c][1,4]benzodiazepin-9-on als weisse Kristalle; Smp. 184-186° und $[\alpha]_D^{20}$ = +36.1° (MeOH, c = 1%).

b) Eine Suspension von 1.85 g (4.74 mMol) (S)-8-Chlor-1-(5-chlormethyl-1,2,4-oxadiazol-3-yl)-11,12,13,13a-tetrahydro-9H-imidazo[1,5-a]pyrrolo[2,1-c]-[1,4]benzodiazepin-9-on in 30 ml N,N-Dimethylformamid wurde mit 1.73 g (23.7 mMol) Diaethylamin versetzt. Nach 16 Std. Rühren bei Raum-temperatur wurde die erhaltene Lösung vollständig von den Lösungsmitteln befreit. Der Rückstand wurde über Kieselgel mit Methylenchlorid/Methanol 19:1 als Laufmittel chromatographiert. Man erhielt 1.54 g (76%) (S)-8-Chlor-1-(5-diaethylaminomethyl-1,2,4-oxadiazol-3-yl)-11,12,13,13a-tetrahydro-9H-imidazo[1,5-a]pyrrolo[2,1-c][1,4]benzodiazepin-9-on als hellgelbes Öl. Ein Muster wurde aus Aether umkristallisiert und ergab weisse Kristalle; Smp. 151-153° und $[\alpha]_D^{20}$ = +35.5° (MeOH, c = 1%).

c) 0.84 g (1.96 mMol) (S)-8-Chlor-1-(5-diaethylaminomethyl-1,2,4-oxadiazol-3-yl)-11,12,13,13a-tetrahydro-9H-imidazo[1,5-a]pyrrolo[2,1-c][1,4]-benzodiazepin-9-on in 10 ml Aethanol wurden mit 0.41 ml (1.96 mMol) 4.78 N aethanolischer Salzsäure versetzt. Nach 10 Minuten Rühren bei Raum-temperatur wurde die erhaltene Lösung vollständig von den Lösungsmitteln befreit. Der Rückstand wurde aus Aethanol/Aether umkristallisiert. Man erhielt 0.70 g (77%) (S)-8-Chlor-1-(5-diaethylaminomethyl-1,2,4-oxadiazol-3-yl)-11,12,13,13a-tetrahydro-9H-imidazo [1,5-a]pyrrolo[2,1-c][1,4]benzodiazepin-9-on hydrochlorid (1:1) als weisse Kristalle; Smp. 205-207° und $[\alpha]_D^{20}$ =

+25.2° (MeOH, c = 1%).

Beispiel 230

a) Eine Suspension von 1.85 g (4.74 mMol) (S)-8-Chlor-1-(5-chlormethyl-1,2,4-oxadiazol-3-yl)-11,12,13,13a-tetrahydro-9H-imidazo[1,5-a]pyrrolo[2,1-c]-[1,4]benzodiazepin-9-on in 30 ml N,N-Dimethylformamid wurde mit 2.40 g (23.7 mMol) Dipropylamin versetzt. Nach 16 Std. Rühren bei Raum-temperatur wurde die erhaltene Lösung vollständig von den Lösungsmitteln befreit. Der Rückstand wurde über Kieselgel mit Methylenchlorid/Methanol/ Methanol 19:1 als Laufmittel chromatographiert. Man erhielt 2.03 g (94%) (S)-8-Chlor-1-(5-dipropylaminomethyl-1,2,4-oxadiazol-3-yl)-11,12,13,13a-tetrahydro-9H-imidazo[1,5-a]pyrrolo[2,1-c][1,4]benzodiazepin-9-on als hellgelbes Öl; $[\alpha]_D^{20}$ = +29.3° (MeOH, c = 1%).

b) 1.93 g (4.24 mMol) (S)-8-Chlor-1-(5-dipropylaminomethyl-1,2,4-oxadiazol-3-yl)-11,12,13,13a-tetrahydro-9H-imidazo[1,5-a]pyrrolo[2,1-c][1,4]-benzodiazepin-9-on in 20 ml Aethanol wurden mit 0.88 ml (4.2 mMol) 4.78 N aethanolischer Salzsäure versetzt. Nach 10 Minuten Rühren bei Raum-temperatur wurde die erhaltene Lösung vollständig von den Lösungsmitteln befreit. Der Rückstand wurde aus Aethanol/Aether umkristallisiert. Man erhielt 1.42 g (68%) (S)-8-Chlor-1-(5-dipropylaminomethyl-1,2,4-oxadiazol-3-yl)-11,12,13,13a-tetrahydro-9H-imidazo [1,5-a]pyrrolo[2,1-c][1,4]benzodiazepin-9-on hydrochlorid (1:1) als weisse Kristalle; Smp. 183-185° und $[\alpha]_D^{20}$ = +24.4° (MeOH, c = 1%).

Beispiel 231

a) Eine Suspension von 1.85 g (4.74 mMol) (S)-8-Chlor-1-(5-chlormethyl-1,2,4-oxadiazol-3-yl)-11,12,13,13a-tetrahydro-9H-imidazo[1,5-a]pyrrolo[2,1-c]-[1,4]benzodiazepin-9-on in 30 ml N,N-Dimethylformamid wurde mit 3.06 g (23.7 mMol) Dibutylamin versetzt. Nach 16 Std. Rühren bei Raum-temperatur wurde die erhaltene Lösung vollständig von den Lösungsmitteln befreit. Der Rückstand wurde über Kieselgel mit Methylenchlorid/Methanol 19:1 als Laufmittel chromatographiert. Man erhielt 2.15 g (94%) (S)-8-Chlor-1-(5-dibutylaminomethyl-1,2,4-oxadiazol-3-yl)-11,12,13,13a-tetrahydro-9H-imidazo(1,5-a]pyrrolo[2,1-c][1,4]benzodiazepin-9-on als hellgelbes Öl; $[\alpha]_D^{20}$ = +28.7° (MeOH, c = 1%).

b) 2.01 g (4.16 mMol) (S)-8-Chlor-1-(5-dibutylaminomethyl-1,2,4-oxadiazol-3-yl)-11,12,13,13a-tetrahydro-9H-imidazo[1,5-a]pyrrolo[2,1-c][1,4]-benzodiazepin-9-on in 20 ml Aethanol wurden mit 1.12 ml (4.14 mMol) 3.70 N aethanolischer Salzsäure versetzt. Nach 10 Minuten Rühren bei Raum-temperatur wurde die erhaltene Lösung vollständig von den Lösungsmitteln befreit. Der Rückstand wurde aus Aethanol/Aether umkristallisiert. Man erhielt 1.99 g (92%) (S)-8-Chlor-1-(5-dibutylaminomethyl-1,2,4-oxadiazol-3-yl)-11,12,13,13a-tetrahydro-9H-imidazo [1,5-a]pyrrolo[2,1-c][1,4]benzodiazepin-9-on hydrochlorid (1:1) als weisse Kristalle; Smp. 163-165° und $[\alpha]_D^{20}$ = +23.2° (MeOH, c = 1%).

Beispiel 232

a) 0.66 g (1.9 mMol) 3-(5-Chlormethyl-1,2,4-oxadiazol-3-yl)-8-fluor-5-methyl-5,6-dihydro-4H-imidazo[1,5-a][1,4] benzodiazepin-6-on wurden in 20 ml N,N-Dimethylformamid gelöst. Während 1 Std. wurde bei Raum-temperatur ein schwacher Strom von Aethylamingas eingeleitet, wobei die Reaktiontemperatur bis 36° anstieg. Nach 16 Std. Rühren bei Raum-temperatur wurde die erhaltene Lösung vollständig von den Lösungsmitteln befreit. Der Rückstand wurde über Kieselgel mit Methylenchlorid/Methanol 19:1 als Laufmittel chromatographiert. Nach Einengen erhielt man einen beigen Schaum, der in Aether aufgenommen wurde. Man erhielt 0.40 g (59%) 3-(5-Aethylaminomethyl-1,2,4-oxadiazol-3-yl)-8-fluor-5-methyl-5,6-dihydro-4H-imidazo[1,5-a][1,4]benzodiazepin-6-on als beige Kristalle; Smp. 166-168°.

b) 0.37 g (1.04 mMol) 3-(5-Aethylaminomethyl-1,2,4-oxadiazol-3-yl)-8-fluor-5-methyl-5,6-dihydro-4H-imidazo [1,5-a][1,4]benzodiazepin-6-on in 6 ml Aethanol wurden mit 0.31 ml (1.14 mMol) 3.70 N aethanolischer Salzsäure versetzt. Nach Zugabe von 20 ml Essigester bei 0° fielen Kristalle aus. Man erhielt 0.30 g (73%) 3-(5-Aethylaminomethyl-1,2,4-oxadiazol-3-yl)-8-fluor-5-methyl-5,6-dihydro-4H-imidazo[1,5-a][1,4]benzodiazepin-6-on hydrochlorid (1:1) als weissliche Kristalle; Smp. 250-252° (Zers.).

Beispiel 233

a) 19.8 g (0.0639Mol) 7-Chlor-8-fluor-5-methyl-6-oxo-5,6-dihydro-4H-imidazo[1,5-a][1,4]benzodiazepin-3-carbonsäure wurden in 100 ml N,N-Dimethylformamid unter Argonbegasung suspendiert und bei Raum-temperatur portionenweise mit 10.9 g (0.0671 Mol) 1,1'-Carbonyldiimidazol versetzt. Nach Beendigung der $CO_2$-Abspaltung wurde die beige Suspension während 30 min bei 50° gerührt, gekühlt und bei einer Temperatur unter 25° innerhalb von ca 10 min tropfenweise mit 20 ml 25 prozentiger Ammoniak versetzt. Nach 15-minütigem Rühren wurde die erhaltene bräunliche Lösung auf 600 ml Eiswasser gegossen. Das Gemisch wurde 30 min bei Raumtemperatur gerührt und filtriert, worauf die Kristalle mit total 200 ml Wasser nachgewaschen wurden. Nach Trocknung erhielt man 14.4 g (73%) 7-Chlor-8-fluor-5 -methyl-6-oxo-5,6-dihydro-4H-imidazo[1,5-a][1,4]benzodiazepin-3-carboxamid als beige Kristalle; Smp. 292-294°.

b) 14.4 g (0.0466Mol) 7-Chlor-8-fluor-5-methyl-6-oxo-5,6-dihydro-4H-imidazo[1,5-a][1,4]benzodiazepin-3-carboxamid wurden in 60 ml Dioxan und 8 ml Pyridin suspendiert und bei einer Temperatur von ~8° innerhalb von 10 min tropfenweise mit 10.3 g (0.049 Mol) Trifluoressigsäureanhydrid versetzt. Man rührte während 3 Stunden bei 50° und goss auf 400 ml Wasser. Man extrahierte mit Essigester, trocknete über Magnesiumsulfat, filtrierte und engte das Filtrat ein. Der Rückstand wurde in 300 ml heissem Essigester gelöst, die Lösung wurde über 100 g Kieselgel abgenutscht, das Kieselgel wurde mit 200 ml Essigester nachgewaschen, und das Filtrat wurde eingedampft. Man erhielt 12 g (89%) 7-Chlor-8-fluor-5-methyl-6-oxo-5,6-dihydro-4H-imidazo[1,5-a][1,4]benzodiazepin-3-carbonitril als leicht gelbliche Kristalle; Smp. 221-223°.

c) 1.0 g (0.0432 Mol) Natrium wurden in 60 ml Methanol gelöst. Bei Raumtemperatur gab man aufeinanderfolgend 8.85 g (0.0304 Mol) 7-Chlor-8-fluor-5-methyl-6-oxo-5,6-dihydro-4H-imidazo[1,5-a][1,4]benzodiazepin-3-carbonitril und 3.25 g (0.0469 Mol) Hydroxylaminhydrochlorid zu. Man rührte die weisse Suspension 67 Std. bei Raumtemperatur und dampfte ein, worauf man den Rückstand in 100 ml Wasser aufschlämmte und die Kristalle abfiltrierte. Durch Trocknung des Kristallisats erhielt man 8.2 g (83%) 7-Chlor-8-fluor-5-methyl-6-oxo-5,6-dihydro-4H-imidazo [1,5-a][1,4]-benzodiazepin-3-carboxamidoxim als weisse Kristalle; Smp. 228-231°.

Die wässrige Phase wurde mit Essigester extrahiert, über Magnesium-sulfat getrocknet, abfiltriert und eingeengt, wobei 0.95 g (10%) zusätzliches Material (Smp. 225-228°) erhalten wurde.

d) Eine Suspension von 7.0 g (0.0216 Mol) 7-Chlor-8-fluor-5-methyl-6-oxo-5,6-dihydro-4H-imidazo[1,5-a][1,4]benzodiazepin-3-carboxamidoxim in 70 ml N,N-Dimethylformamid wurde mit 4.38 g (0.0256 Mol) Chloressigsäureanhydrid versetzt. Die erhaltene gelbe Lösung wurde 2 Std. bei 100° gerührt und dann vollständig von den Lösungsmitteln befreit. Das ölige Produkt wurde in 300 ml Acetonitril aufgenommen, worauf die Lösung mit Aktivkohle versetzt, am Rückfluss gekocht und heiss über 100 g Kieselgel filtriert wurde; man wusch das Kieselgel mit 200 ml Acetonitril nach und dampfte das Filtrat ein. Der kristalline Schaum wurde in 100 ml Aether suspendiert und abgenutscht. Aus der Mutterlauge konnte noch zusätzliches Material gewonnen werden. Man erhielt insgesamt 5.65 g (68%) 7-Chlor-3-(5-chlormethyl-1,2,4-oxadiazol-3-yl)-8-fluor-5-methyl-5,6-dihydro-4H-imidazo[1,5-a][1,4] benzodiazepin-6-on als weissgelbliche Kristalle; Smp. 215-218°.

e) Eine Suspension von 1.30 g (3.4 mMol) 7-Chlor-3-(5-chlormethyl-1,2,4-oxadiazol-3-yl)-8-fluor-5-methyl-5,6-dihydro-4H-imidazo[1,5-a][1,4]benzodiazepin-6-on in 13 ml N,N-Dimethylformamid wurde mit 1.24 g (0.017 Mol) Diaethylamin versetzt. Nach 16 Std. Rühren bei Raumtemperatur wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether/n-Hexan umkristallisiert. Man erhielt 1.3 g (91%) 7-Chlor-3-(5-diaethylaminomethyl-1,2,4-oxadiazol-3-yl)-8-fluor-5-methyl-5,6-dihydro-4H-imidazo[1,5-a][1,4]-benzodiazepin-6-on als weisse Kristalle; Smp. 140-144° (Zers.).

f) 1.28 g (3.06 mMol) 7-Chlor-3-(5-diaethylaminomethyl-1,2,4-oxadiazol-3-yl)-8-fluor-5-methyl-5,6-dihydro-4H-imidazo[1,5-a][1,4]benzodiazepin-6-on in 20 ml Aethanol wurden mit 0.91 ml (3.36 mMol) 3.7 N aethanolischer Salzsäure versetzt. Nach 10 Minuten Rühren bei Raumtemperatur wurde die erhaltene weisse Suspension mit 100 ml Aether versetzt und abgenutscht. Man erhielt 1.31 g (94%) 7-Chlor-3-(5-diaethylaminomethyl-1,2,4-oxadiazol-3-yl)-8-fluor-5-methyl-5,6-dihydro-4H-imidazo[1,5-a][1,4]-benzodiazepin-6-on hydrochlorid (1:1) als weisse Kristalle; Smp. 206-209° (Zers.).

Beispiel 234

a) Eine Suspension von 1.30 g (3.4 mMol) 7-Chlor-3-(5-chlormethyl-1,2,4-oxadiazol-3-yl)-8-fluor-5-methyl-5,6-dihydro-4H-imidazo[1,5-a][1,4]benzodiazepin-6-on in 13 ml N,N-Dimethylformamid wurde mit 1.72 g (0.017 Mol) Dipropylamin versetzt. Nach 16 Std. Rühren bei Raumtemperatur wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether/n-Hexan umkristallisiert. Man erhielt 1.32 g (87%) 7-Chlor-3-(5-dipropylaminomethyl-1,2,4-oxadiazol-3-yl)-8-fluor-5-methyl-5,6-dihydro-4H-imidazo[1,5-a][1,4]-benzodiazepin-6-on als weisse Kristalle; Smp. 143-146° (Zers.).

b) 1.25 g (2.8 mMol) 7-Chlor-3-(5-dipropylaminomethyl-1,2,4-oxadiazol-3-yl)-8-fluor-5-methyl-5,6-dihydro-4H-imidazo[1,5-a][1,4]benzodiazepin-6-on in 20 ml Aethanol wurden mit 0.84 ml (3.07 mMol) 3.7 N aethanolischer Salzsäure versetzt. Nach 30 Minuten Rühren bei 0° wurde die Lösung mit 100 ml Aether versetzt, und die erhaltene weisse Suspension wurde abgenutscht. Man erhielt 1.13 g (84%) 7-Chlor-3-(5-dipropylaminomethyl-1,2,4-oxadiazol-3-yl)-8-fluor-5-methyl-5,6-dihydro-4H-imidazo[1,5-a][1,4]-benzodiazepin-6-on hydrochlorid (1:1) als weisse Kristalle; Smp. 209-211° (Zers.).

Beispiel 235

a) Eine Suspension von 1.30 g (3.4 mMol) 7-Chlor-3-(5-chlormethyl-1,2,4-oxadiazol-3-yl)-8-fluor-5-methyl-5,6-dihydro-4H-imidazo[1,5-a][1,4]benzodiazepin-6-on in 13 ml N,N-Dimethylformamid wurde mit 1.44 g (0.017 Mol) Dipropylamin versetzt. Nach 16 Std. Rühren bei Raumtemperatur wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether/n-Hexan umkristallisiert. Man erhielt 1.39 g (95%) 7-Chlor-8-fluor-5-methyl-3-(5-piperidin-1-ylmethyl-1,2,4-oxadiazol-3-yl)-5,6-dihydro-4H-imidazo[1,5-a]-[1,4]benzodiazepin-6-on als weisse Kristalle; Smp. 210-212° (Zers.).

b) 1.37 g (3.18 mMol) 7-Chlor-8-fluor-5-methyl-3-(5-piperidin-1-ylmethyl-1,2,4-oxadiazol-3-yl)-5,6-dihydro-4H-imidazo[1,5-a][1,4]benzodiazepin-6-on wurden in 50 ml Aethanol heiss gelöst und bei 40° mit 0.95 ml (3.5 Mol) 3.7 N aethanolischer Salzsäure versetzt. Nach 30 Minuten Rühren bei 0° wurde die Lösung auf ein Volumen von ~20 ml eingeengt und mit 100 ml Aether versetzt. Die erhaltene weisse Suspension wurde abgenutscht. Man erhielt 1.30 g (88%) 7-Chlor-8-fluor-5-methyl-3-(5-piperidin-1-ylmethyl-1,2,4-oxadiazol-3-yl)-5,6-dihydro-4H-imidazo[1,5-a][1,4]benzodiazepin-6-on hydrochlorid (1:1) als weisse Kristalle; Smp. 240-243° (Zers.).

Beispiel 236

a) Eine Suspension von 1.20 g (3.14 mMol) 7-Chlor-3-(5-chlormethyl-1,2,4-oxadiazol-3-yl)-8-fluor-5-methyl-5,6-dihydro-4H-imidazo[1,5-a][1,4]-benzodiazepin-6-on in 20 ml N,N-Dimethylformamid wurde mit 1.59 g (0.0166 Mol) Diisopropylamin versetzt. Nach 20 Std. Rühren bei 80° wurde die erhaltene Lösung vollständig von den Lösungsmitteln befreit. Der Rückstand wurde über Kieselgel mit Methylenchlorid/Methanol 39:1 als Laufmittel chromatographiert. Das Produkt wurde aus Aether/n-Hexan umkristallisiert. Man erhielt 0.84 g (60%) 7-Chlor-3-(5-diisopropylaminomethyl-1,2,4-oxadiazol-3-yl)-8-fluor-5-methyl-5,6-dihydro-4H-imidazo[1,5-a]-[1,4]benzodiazepin-6-on als weissliche Kristalle; Smp. 153-156°.

b) 0.82 g (1.84 mMol) 7-Chlor-3-(5-diisopropylaminomethyl-1,2,4-oxadiazol-3-yl)-8-fluor-5-methyl-5,6-dihydro-4H-imidazo[1,5-a][1,4]benzodiazepin-6-on wurden in 20 ml Aethanol heiss gelöst und bei 0° mit 0.55 ml (2.02 mMol) 3.7 N aethanolischer Salzsäure versetzt. Nach 30 Minuten Rühren bei 0° wurde die Lösung total eingeengt. Das Produkt wurde aus heissem Acetonitril/Essigester umkristallisiert. Man erhielt 0.74 g (83%) 7-Chlor-3-(5-diisopropylaminomethyl-1,2,4-oxadiazol-3-yl)-8-fluor-5-methyl-5,6-dihydro-4H-imidazo[1,5-a][1,4]benzodiazepin-6-on hydrochlorid (1:1) als weisse Kristalle; Smp. 206-210° (Zers.).

Beispiel 237

a) Zu einer Suspension von 13.8 g (50.3 mmol) 7-Fluor-5-methyl-6-oxo-5,6-dihydro-4H-imidazo-[1,5-a][1,4]benzodiazepin-3-carbo:nsäureamid (EPA 150 040) in 80 ml Dioxan und 20 ml Pyridin wurden bei 5-8° 7.7 ml (55.3 mmol) Trifluoressigsäureanhydrid zugetropft. Die erhaltene beige Lösung wurde während 2.5 Stunden bei 50° gerührt und anschliessend auf 220 ml Eiswasser gegossen. Der entstandene Niederschlag wurde abfiltriert. Man erhielt nach Trocknen bei 70°/10 Torr 11 g (85%) 7-Fluor-5-methyl-6-oxo-5,6-dihydro-4H-imidazo-[1,5-a][1,4]benzodiazepin-3-carbonsäurenitril als weisses Pulver vom Smp. >250°.

b) Zu einer frisch hergestellten Lösung von Natriummethylat in Methanol (aus 0.27 g (11.7 mmol) Natrium in 50 ml Methanol) wurden 2 g (7.8 mmol) 7-Fluor-5-methyl-6-oxo-5,6-dihydro-4H-imidazo-[1,5-a][1,4]benzodiazepin-3-carbonsäurenitril und 1.1 g (15.6 mmol) Hydroxylamin-hydrochlorid zugegeben, worauf die Mischung 16 Stunden bei Raum-temperatur gerührt wurde. Anschliessend wurde die Suspension eingedampft und mit 100 ml Wasser versetzt. Der erhaltene Niederschlag wurde abfiltriert und am Hochvakuum getrocknet. Man erhielt 2 g (89 %) (E)- und/oder (Z)- 3-(Amino-hydroxyimino-methyl)-7-fluor-5-methyl-5,6-dihydro-4H-imidazo-[1,5-a][1,4]benzodiazepin-6-on als farblosen Schaum, Rf= 0.25 (Kieselgel, Methylenchlorid/Methanol 9 : 1).

c) Zu einer Lösung von 1.8 g (6.2 mmol) (E)-und/oder (Z)- 3-(Aminohydroxyimino-methyl)-7-fluor-5-methyl-5,6-dihydro-4H-imidazo-[1,5-a][1,4]-benzodiazepin-6-on in 50 ml N,N-Dimethylformamid gab man 1.3 g (7.5 mmol) Chloressigsäureanhydrid und rührte 1.5 Stunden bei Raum-temperatur. Anschliessend erhitzte man 2 Stunden auf 105°. Die Lösung wurde eingedampft, und der Rückstand wurde zwischen 2N Natronlauge und Methylenchlorid verteilt. Die wässerige Phase wurde mit Methylenchlorid nachgewaschen, und die organischen Phasen wurden mit Magnesiumsulfat getrocknet, filtriert und eingedampft. Der erhaltene Rückstand wurde aus Methylenchlorid/Aethanol kristallisiert. Man erhielt 1.75 g (81 %) 3-(5-Chlormethyl-1,2,4-oxadiazol-3-yl)-7-fluor-5-methyl-5,6-dihydro-4H-imidazo[1,5-a][1,4]benzodiazepin-6-on als beiges Pulver 205.5-206.5°.

d) Zu einer Suspension von 1.7 g (4.9 mmol ) 3-(5-Chlormethyl-1,2,4-oxadiazol-3-yl)-7-fluor-5-methyl-5,6-dihydro-4H-imidazo-[1,5-a][1,4]benzodiazepin-6-on in 40 ml N,N-Dimethylformamid gab man 1 ml (12.2 mmol) Propylamin zu und rührte 12 Stunden bei Raumtemperatur. Die Lösung wurde eingedampft, und der Rückstand wurde zwischen Methylenchlorid und 2N Natriumcarbonatlösung verteilt. Die wässerige Lösung wurde mit Methylenchlorid extrahiert, und die organischen Phasen wurden mit Magnesiumsulfat getrocknet, filtriert und eingedampft. Der Rückstand wurde chromatographiert (Kieselgel, Essigsäureäthylester/Methanol 9 : 1). Man erhielt 1.21 g (67 %) 3-(5-Propylaminomethyl-1,2,4-oxadiazol-3-yl)-7-fluor-5-methyl-5,6-dihydro-4H-imidazo[1,5-a][1,4]benzodiazepin-6-on, Rf=0.22 (Kieselgel, Essigsäureäthylester/Methanol 4 : 1).

Beispiel 238

490 mg (1.2 mMol) 3-(3-Diallylaminomethyl-1,2,4-oxadiazol-5-yl)-8-fluor-5-methyl-5,6-dihydro-4H-imidazo [1,5-a][1,4]benzodiazepin-6-on wurden in 10 ml Essigester in Gegenwart von 24 mg 5%iger Palladiumkohle bei Raumtemperatur und Normaldruck hydriert. Nach Abtrennen des Katalysators wurde die Lösung eingeengt. Das noch mit wenig Edukt verunreinigte 3-(3-Dipropylaminomethyl-1,2,4-oxadiazol-5-yl)-8-fluor-5-methyl-5,6-dihydro-4H-imidazo [1,5-a][1,4]benzodiazepin-6-on wurde an 40 g Kieselgel mit Essigester chromatographiert, das Eluat wurde eingedampft, der Rückstand wurde in 4 ml Isopropanol gelöst, und die Lösung wurde mit ätherischer Salzsäure angesäuert. Die ausgefallenen Kristalle wurden abfiltriert und getrocknet. Man erhielt 0.37 g (69%) 3-(3-Dipropylaminomethyl-1,2,4-oxadiazol-5-yl)-8-fluor-5-methyl-5,6-dihydro-4H-imidazo[1,5-a][1,4]benzodiazepin-6-on hydrochlorid (1:1) als weisse Kristalle vom Smp.167-169°.

Beispiel 239

Unter Argon wurde eine Suspension von 0.49 g (1.5 mMol) 3-(5-Aminomethyl-1,2,4-oxadiazol-3-yl)-8-fluor-5-methyl-5,6-dihydro-4H-imidazo[1,5-a][1,4]benzodiazepin-6-on in 8 ml N,N,-Dimethylformanid mit 0.41 ml (4.5 mMol) N-Propylbromid und 1.04 g (7.5 mMol) Kaliumcarbonat versetzt und 73 Std. unter Argon bei Raumtemperatur gerührt. Die Lösung wurde filtriert, und das Filtrat wurde eingedampft, wobei ein gelbliches Oel erhalten wurde. Das Rohprodukt wurde durch Chromatographie an 50 g Kieselgel (Methylenchlorid/Aceton 9:1, 4:1, 2:1, schliesslich Methylenchlorid/Methanol 19:1) gereinigt. Das Eluat wurde eingedampft, der Rückstand wurde in 5 ml Methanol aufgenommen, und die Lösung wurde mit ätherischer Salzsäure angesäuert, wobei das Produkt auskristallisierte. Die Lösungsmittel wurden erneut eingedampft, und der Rückstand wurde in 30 ml Essigester erhitzt. Man kühlte im Eisbad und saugte die ausgefallenen Kristalle ab. Man erhielt nach Trocknung im Vakuum 315 mg (51%) 3-[5-Propylaminomethyl)-1,2,4-oxadiazol-3-yl]-8-fluor-5-methyl-5,6-dihydro-4H-imidazo[1,5-a][1,4]benzodiazepin-6-on hydrochlorid (1:1) vom Smp. 144-148° (Zers.).

Beispiel 240

a) Zu einer frisch hergestellten Lösung von Natriummethylat in Methanol (aus 230 mg (10.0 mMol) Natrium in 10 ml Methanol) wurden unter Argon 0.79 g (11.4 mMol) Hydroxylamin-hydrochlorid und 2.0 g (7.6 mMol) (S)-9-Oxo-11,12,13,13a-tetrahydro-9H-imidazo[1,5-a]pyrrolo[2,1-c][1,4]-benzodiazepin-1-carbonitril zugegeben und 90 Std. bei Raumtemperatur gerührt. Anschliessend wurde die Suspension eingedampft, und der Rückstand wurde in 30 ml Wasser aufgenommen. Der unlösliche Anteil wurde abgesaugt. Die Wasserphase wurde mit Kochsalz gesättigt, wobei weiteres Produkt auskristallisierte. Dieses wurde abgesaugt und zusammen mit mit dem vorher erhaltenen Material im Vakuum getrocknet. Man erhielt 2.15 g (95%) (E)- und/oder (Z)-(S)-1-(Amino-hydroximino-methyl)-11,12,13,13a-tetrahydro-9H-imidazo[1,5-a]pyrrolo[2,1-c][1,4]benzodiazepin-9-on vom Smp. 253-254° (Zers., Methanol/Diethylether).

b) Zu einer Suspension von 1.68 g (5.65 mMol) (E)- und/oder (Z)-(S)-1-(Amino-hydroximino-methyl)-11,12,13,13a-tetrahydro-9H-imidazo[1,5-a]-pyrrolo[2,1-c][1,4]benzodiazepin-9-on in 11 ml Dimethylformamid wurden unter Argon 1.1 g (6.5 mMol) Chloressigsäureanhydrid zugegeben und während 30 Min. bei ca. 25° gerührt und darauf 1 Std. auf 110° erhitzt. Die Lösung wurde am Hochvakuum eingedampft, und der braune Rückstand wurde in 20 ml Methylenchlorid aufgenommen. Die Lösung wurde dreimal mit 10 ml gesättigter Natriumhydrogencarbonat-Lösung gewaschen, und das Lösungsmittel wurde im Vakuum entfernt. Das Rohmaterial wurde an 100 g Kieselgel (Methylenchlorid/Aceton 9:1, dann 4:1) chromatographiert. Man erhielt 1.45 g (72%) (S)-1-(5-Chloromethyl-1,2,4-oxadiazol-3-yl)-11,12,13,13a-tetrahydro-9H-imidazo[1,5-a]pyrrolo[2,1-c][1,4]benzodiazepin-9-on als farblose Kristalle vom Smp. 205-206°(Acetonitril).

c) Eine Lösung von 711 mg (2.0 mMol) (S)-1-(5-Chloromethyl-1,2,4-oxadiazol-3-yl)-11,12,13,13a-tetrahydro-9H-imidazo[1,5-a]pyrrolo[2,1-c][1,4]-benzodiazepin-9-on in 10 ml N,N-Dimethylformamid wurde mit 0.82 ml (6.0 mMol) Dipropylamin versetzt und unter Argon 2 Std. bei Raumtemperatur gerührt. Die Lösung wurde eingedampft, der Rückstand wurde in 10 ml Wasser verrührt, und die Kristalle wurden abfiltriert. Das Rohprodukt wurde an 30 g Kieselgel chromatographiert (Methylenchlorid/ Aceton 9:1, dann 4:1). Man erhielt 840 mg (S)-1-(5-Dipropylaminomethyl-1,2,4-oxadiazol-3-yl)-11,12,13,13a-tetrahydro-9H-imidazo[1,5-a]pyrrolo[2,1-c][1,4]benzodiazepin-9-on , welches mit aetherischer Salzsäure in das Hydrochlorid überführt wurde. Durch Umkristallisation aus Acetonitril erhielt man 685 mg (73%) (S)-1-(5-Dipropylaminomethyl-1,2,4-oxadiazol-3-yl)-11,12,13,13a-tetrahydro-9H-imidazo[1,5-a]pyrrolo[2,1-c][1,4]benzodiazepin-9-on hydrochlorid (1:1.3) vom Smp. 137-140° (Zers.).

Beispiel 241

Eine Lösung von 710 mg (2.0 mMol) (S)-1-(5-Chlormethyl-1,2,4-oxadiazol-3-yl)-11,12,13,13a-tetrahydro-9H-imidazo[1,5-a]pyrrolo[2,1-c][1,4]-benzodiazepin-9-on in 10 ml N,N-Dimethylformamid wurde mit 0.5 ml (6.0 mMol) Propylamin versetzt und unter Argon 2 Std. bei Raumtemperatur gerührt. Die Lösung wurde eingedampft, der Rückstand wurde in 5 ml Wasser verrührt, und die Kristalle wurden abfiltriert. Weiteres Rohprodukt wurde durch Extraktion der Wasserphase mit Methylenchlorid gewonnen. Die vereinigten Rohprodukte (0.65 g) wurden an 25 g Kieselgel chromatographiert (Methylenchlorid/ Methanol 2%, 5%, 10%). Man erhielt 540 mg (S)-1-(5-Propylaminomethyl-1,2,4-oxadiazol-3-yl)-11,12,13,13a-tetrahydro-9H-imidazo[1,5-a]pyrrolo[2,1-c][1,4]benzodiazepin-9-on, welches in 5 ml Acetonitril gelöst und darauf mit aetherischer Salzsäure in das Hydrochlorid überführt wurde. Durch Umkristallisation aus Acetonitril erhielt man 560 mg (62%) (S)-1-(5-Propylaminomethyl-1,2,4-oxadiazol-3-yl)-11,12,13,13a-tetrahydro-9H-imidazo[1,5-a]pyrrolo[2,1-c][1,4]benzodiazepin-9-on hydrochlorid (1:2) vom Smp. 169-173° (Zers.).

Beispiel 242

Eine Lösung von 710 mg (2.0 mMol) (S)-1-(5-Chlormethyl-1,2,4-oxadiazol-3-yl)-11,12,13,13a-tetrahydro-9H-imidazo[1,5-a]pyrrolo[2,1-c][1,4]-benzodiazepin-9-on in 10 ml N,N-Dimethylformamid wurde mit 0.78 ml (6.0 mMol) N,N,N'-Trimethylaethylendiamin versetzt und unter Argon 2 Std. bei Raumtemperatur gerührt. Die Lösung wurde eingedampft, und der Rückstand wurde in 10 ml Wasser gelöst. Die Lösung wurde mit Kochsalz gesättigt und mehrmals mit

Methylenchlorid extrahiert. Die vereinigten Extrakte wurden mit Natriumsulfat getrocknet, filtriert und eingedampft, wobei 0.85 eines gelblichen Schaums erhalten wurde. Das Rohprodukt wurde an 30 g Kieselgel chromatographiert (Methylenchlorid/Aceton 9:1, Methylenchlorid/ Methanol 9:1, 4:1 und schliesslich Methylenchlorid/ Methanol/Triaethylamin 80:19:1). Man erhielt 0.64 g (76%) (S)-1-[5-[Methyl-(2-dimethylamino-aethyl)-aminomethyl]-1,2,4-oxadiazol-3-yl]-11,12,13,13a-tetrahydro-9H-imidazo[1,5-a]pyrrolo[2,1-c][1,4]benzodiazepin-9-on. Das Rohprodukt wurde in 10 ml Acetonitril gelöst und über Celit filtriert. Darauf wurde mit aetherischer Salzsäure angesäuert und eingedampft. Der Rückstand wurde aus 15 ml heissem Acetonitril kristallisiert. Man erhielt 690 mg (65%) (S)-1-[5-[Methyl-(2-dimethylamino-aethyl)-aminomethyl]-1,2,4-oxadiazol-3-yl]-11,12,13,13a-tetrahydro-9H-imidazo[1,5-a]pyrrolo[2,1-c][1,4]-benzodiazepin-9-on hydrochlorid (1:3) vom Smp. 198-200° (Zers.).

Beispiel 243

Eine Lösung von 711 mg (2.0 mMol) (S)-1-(5-Chlormethyl-1,2,4-oxadiazol-3-yl)-11,12,13,13a-tetrahydro-9H-imidazo[1,5-a]pyrrolo[2,1-c][1,4]-benzodiazepin-9-on in 10 ml N,N-Dimethylformamid wurde mit 1.0 ml (6.0 mMol) Dibutylamin versetzt und unter Argon 3 Std. bei Raumtemperatur gerührt. Die Lösung wurde eingedampft, der Rückstand wurde in 10 ml Wasser verrührt, und die Kristalle wurden abgesaugt. Das Rohprodukt wurde in Methylenchlorid gelöst, die Lösung wurde mit Natriumsulfat getrocknet, worauf an 30 g Kieselgel chromatographiert wurde (Methylenchlorid/ Aceton 9:1, dann 4:1). Man erhielt 0.73 g (S)-1-(5-Dibutylaminomethyl-1,2,4-oxadiazol-3-yl)-11,12,13,13a-tetrahydro-9H-imidazo[1,5-a]-pyrrolo[2,1-c][1,4]benzodiazepin-9-on, welches in 10 ml Acetonitril mit aetherischer Salzsäure in das Hydrochlorid überführt wurde. Durch Kristallisation aus heissem Essigester erhielt man 0.75 g (77%) (S)-1-(5-Dibutylaminomethyl-1,2,4-oxadiazol-3-yl)-11,12,13,13a-tetrahydro-9H-imidazo[1,5-a]pyrrolo[2,1-c][1,4]benzodiazepin-9-on hydrochlorid (1:1) vom Smp. 115-123° (Zers.).

Beispiel 244

a) Zu einer Suspension von 6.9 g (21.6 mMol) 8-Chlor-5-methyl-6-oxo-5,6-dihydro-4H-imidazo[1,5-a][1,4]benzodiazepin-3-carbonsäure aethylester in 5 ml Aethanol und 7 ml Wasser wurden 5.4 ml 5N Natronlauge zugetropft und 30 Minuten zum Rückfluss erhitzt. Die Lösung wurde heiss filtriert, auf ca. 6 ml eingeengt und mit 5.5 ml 5N Salzsäure angesäuert. Das Produkt wurde abgesaugt. Man erhielt 4.7 g (75%) 8-Chlor-5-methyl-6-oxo-5,6-dihydro-4H-imidazo[1,5-a][1,4]benzodiazepin-3-carbonsäure als farblose Kristalle vom Smp. 263-267° (Zers.).

b) Zu einer Suspension von 4.7 g (16.0 mMol) 8-Chlor-5-methyl-6-oxo-5,6-dihydro-4H-imidazo[1,5-a][1,4]benzodiazepin-3-carbonsäure in 25 ml N,N-Dimethylformamid wurden 2.7 g (16.8 mMol) 1,1'-Carbonyldiimidazol portionenweise zugegeben. Nach beendeter $CO_2$-Entwicklung erwärmte man während 45 Min. auf 60°. Anschliessend kühlte man die Lösung auf Raumtemperatur ab und tropfte 3.9 ml konz. wässerige Ammoniaklösung dazu. Nach weiterem 90 minütigem Rühren wurde das Reaktionsgemisch auf 90 ml Eiswasser gegossen und 1 Std. gerührt. Die weissen Kristalle wurden abgesaugt und mit wenig Wasser gewaschen. Nach Trocknen im Hochvakuum erhielt man 3.84 g (83%) 8-Chlor-5-methyl-6-oxo-5,6-dihydro-4H-imidazo[1,5-a][1,4]benzodiazepin-3-carbonsäureamid vom Smp. 275-277°.

c) Zu einer Suspension von 3.72 g (12.8 mMol) 8-Chlor-5-methyl-6-oxo-5,6-dihydro-4H-imidazo[1,5-a][1,4]benzodiazepin-3-carbonsäureamid in 17 ml Dioxan und 2 ml Pyridin wurden bei <8° 1.85 ml (13.4 mMol) Trifluoressigsäure-anhydrid zugetropft. Die erhaltene beige Lösung wurde während 4 Std. bei 50 - 60° gerührt, abgekühlt und mit 80 ml Wasser versetzt. Die weissen Kristalle wurden abgesaugt und im Hochvakuum getrocknet. Man erhielt 3.08 g (88%) 8-Chlor-5-methyl-6-oxo-5,6-dihydro-4H-imidazo[1,5-a]-[1,4]benzodiazepin-3-carbonitril als farblose Kristalle vom Smp. 272-275°.

d) Zu einer Natriummethylat-Lösung (hergestellt aus 342 mg (14.8 mMol) Natrium und 14 ml Methanol) wurden portionenweise 1.1 g (15.8 mMol) Hydroxylamin-hydrochlorid und 2.90 g (10.6 mMol) 8-Chlor-5-methyl-6-oxo-5,6-dihydro-4H-imidazo[1,5-a][1,4]benzodiazepin-3-carbonitril zugegeben und während 18 Std. bei Raumtemperatur gerührt. Die erhaltene gelbe Suspension wurde im Eisbad abgekühlt und abgesaugt. Anschliessend wurde das Rohmaterial in 10 ml Wasser suspendiert, erneut abgesaugt und im Hochvakuum getrocknet. Man erhielt 2.9 g (89%) (E)- und/oder (Z)-3-(Amino-hydroximino-methyl)-8-chlor-5-methyl-5,6-dihydro-4H-imidazo-[1,5-a][1,4] benzodiazepin-6-on vom Smp. 266-270° (Acetonitril/DMF).

e) Zu einer Suspension von 0.90 g (2.9 mMol) (E)- und/oder (Z)-3-(Aminohydroximino-methyl)-8-chlor-5-methyl-5,6-dihydro-4H-imidazo[1,5-a][1,4]-benzodiazepin-6-on in 9 ml Dimethylformamid wurden unter Argon 0.55 g (3.2 mMol) Chloressigsäureanhydrid zugegeben und während 1 Std. bei ca. 25° gerührt und darauf 1 Std. auf 110° erhitzt. Die Lösung wurde am Hochvakuum eingedampft, und der braune Rückstand wurde in 50 ml Methylenchlorid aufgenommen. Die Lösung wurde dreimal mit 10 ml gesättigter Natriumhydrogencarbonat Lösung gewaschen, und das Lösungsmittel wurde im Vakuum entfernt. Das Rohmaterial wurde an 30 g Kieselgel (Methylenchlorid/Aceton 9:1) chromatographiert. Man erhielt 0.76 g (71%) 8-Chlor-3-(5-chlormethyl-1,2,4-oxadiazol-3-yl)-5-methyl-5,6-dihydro-4H-imidazo[1,5-a][1,4]benzodiazepin-6-on als fast farblose Kristalle vom Smp. 229°(Acetonitril).

f) Eine Lösung von 370 mg (1.01 mMol) 8-Chlor-3-(5-chlormethyl-1,2,4-oxadiazol-3-yl)-5-methyl-5,6-dihydro-4H-imidazo[1,5-a][1,4]benzodiazepin-6-on in 5 ml N,N-Dimethylformamid wurde mit 0.42 ml (3.03 mMol) Dipropylamin versetzt und unter Argon 2 Std. bei Raumtemperatur gerührt. Die Lösung wurde eingedampft, und der Rückstand wurde in 10 ml Wasser verrührt. Die weissen Kristalle wurden abfiltriert. Das Produkt wurde an 20 g Kieselgel chromatographiert (Essigester/Hexan 1:1, dann Essigester). Man erhielt 259 mg 8-Chlor-3-(5-dipropylaminomethyl-1,2,4-oxadiazol-3-yl)-5-methyl-5,6-dihydro-4H-imidazo[1,5-a][1,4]benzodiazepin-6-on, welches mit aetherischer Salzsäure in das Hydrochlorid überführt wurde. Durch Umkristallisation aus Acetonitril erhielt man 268 mg (54%) 8-Chlor-1-(5-dipropylaminomethyl-1,2,4-oxadiazol-3-yl)-5-methyl-5,6-dihydro-4H-imidazo[1,5-a][1,4]benzodiazepin-6-on hydrochlorid (1:1.6) vom Smp. 180-192° (Zers.).

Beispiel 245

Eine Lösung von 278 mg (0.76 mMol) 8-Chlor-3-(5-chlormethyl-1,2,4-oxadiazol-3-yl)-5-methyl-5,6-dihydro-4H-imidazo[1,5-a][1,4]benzodiazepin-6-on in 5 ml N,N-Dimethylformamid wurde mit 0.39 ml (2.3 mMol) Dibutylamin versetzt und unter Argon 1.5 Std. bei Raumtemperatur gerührt. Die Lösung wurde eingedampft, der Rückstand wurde in 20 ml Methylenchlorid gelöst, und die Lösung wurde dreimal mit Wasser gewaschen. Die organische Phase wurde mit Natriumsulfat getrocknet, filtriert und eingedampft. Das Rohprodukt wurde an 20 g Kieselgel chromatographiert (Methylenchlorid/Aceton 4:1). Man erhielt 250 mg 8-Chlor-3-(5-dibutylaminomethyl-1,2,4-oxadiazol-3-yl)-5-methyl-5,6-dihydro-4H-imidazo[1,5-a][1,4]benzodiazepin-6-on, welches mit ätherischer Salzsäure in das Hydrochlorid überführt wurde. Durch Kristallisation aus Acetonitril erhielt man 151 mg (39%) 8-Chlor-1-(5-Dibutylaminomethyl-1,2,4-oxadiazol-3-yl)-5-methyl-5,6-dihydro-4H-imidazo[1,5-a][1,4]benzodiazepin-6-on hydrochlorid (1:1.3) vom Smp. 165-175° (Zers.).

Beispiel 246

Eine Lösung von 200 mg (0.54 mMol) 8-Chlor-3-(5-chlormethyl-1,2,4-oxadiazol-3-yl)-5-methyl-5,6-dihydro-4H-imidazo[1,5-a][1,4]benzodiazepin-6-on in 4 ml N,N-Dimethylformamid wurde mit 0.17 ml (1.65 mMol) Diäthylamin versetzt und unter Argon 1.5 Std. bei Raumtemperatur gerührt. Die Lösung wurde eingedampft, und der Rückstand wurde in 10 ml Wasser verrührt. Die Kristalle wurden abgesaugt, das Filtrat wurde einmal mit Essigester extrahiert, und der Extrakt wurde eingedampft. Die vereinigten Rohprodukte wurden an 20 g Kieselgel chromatographiert (Methylenchlorid/ Aceton 4:1, 2:1). Man erhielt 124 mg 8-Chlor-3-(5-diäthylaminomethyl-1,2,4-oxadiazol-3-yl)-5-methyl-5,6-dihydro-4H-imidazo[1,5-a][1,4]benzodiazepin-6-on, welches in Acetonitril mit ätherischer Salzsäure in das Hydrochlorid überführt wurde. Durch Kristallisation aus Acetonitril erhielt man 108 mg (42%) 8-Chlor-1-(5-diäthylaminomethyl-1,2,4-oxadiazol-3-yl)-5-methyl-5,6-dihydro-4H-imidazo[1,5-a][1,4]benzodiazepin-6-on hydrochlorid (1:1.9) vom Smp. 191-205° (Zers.).

Beispiel 247

Eine Lösung von 200 mg (0.54 mMol) 8-Chlor-3-(5-chlormethyl-1,2,4-oxadiazol-3-yl)-5-methyl-5,6-dihydro-4H-imidazo[1,5-a][1,4]benzodiazepin-6-on in 4 ml N,N-Dimethylformamid wurde mit 0.136 ml (1.65 mMol) Propylamin versetzt und unter Argon 1.5 Std. bei Raumtemperatur gerührt. Die Lösung wurde eingedampft, und der Rückstand wurde in 10 ml Wasser verrührt. Die Wasserphase wurde mit Methylenchlorid extrahiert, und die vereinigten Extrakte wurden zweimal mit Wasser gewaschen, mit Natriumsulfat getrocknet, filtriert und eingedampft. Die vereinigten Rohprodukte wurden an 30 g Kieselgel chromatographiert (Essigester, Methylenchlorid/Methanol 9:1). Man erhielt 126 mg 8-Chlor-3-(5-propylaminomethyl-1,2,4-oxadiazol-3-yl)-5-methyl-5,6-dihydro-4H-imidazo[1,5-a]-[1,4]benzodiazepin-6-on als Schaum, welcher in Acetonitril mit ätherischer Salzsäure in das Hydrochlorid überführt wurde. Durch Kristallisation aus heissem Acetonitril erhielt man 102 mg (42%) 8-Chlor-1-(5-propylaminomethyl-1,2,4-oxadiazol-3-yl)-5-methyl-5,6-dihydro-4H-imidazo[1,5-a][1,4]-benzodiazepin-6-on hydrochlorid (1:1.8) vom Smp. 230-240° (Zers.).

Beispiel 248

Eine Suspension von 695 mg (2.0 mMol) 3-(5-Chlormethyl-1,2,4-oxadiazol-3-yl)-8-fluoro-5-methyl-5,6-dihydro-4H-imidazo[1,5-a][1,4]benzodiazepin-6-on in 7 ml N,N-Dimethylformamid wurde mit 0.78 ml (6.0 mMol) N,N,N'-Trimethyläthylendiamin versetzt. Nach 2 Std. Rühren bei Raumtemperatur wurde die Lösung eingeengt, und der Rückstand wurde in 70 ml Wasser aufgenommen. Die Kristalle wurden abfiltriert und mit 20 ml gesättigter Kochsalzlösung aufgenommen, worauf viermal mit Essigester und dreimal mit Methylenchlorid extrahiert wurde. Die Extrakte wurden mit Natriumsulfat getrocknet, filtriert und eingedampft. Der Rückstand wurde aus Acetonitril umkristallisiert. Man erhielt 460 mg (56%) 3-[5-[Methyl-(2-dimethylamino-äthyl)-aminomethyl]-1,2,4-oxadiazol-3-yl]-8-fluor-5-methyl-5,6-dihydro-4H-imidazo[1,5a][1,4]benzodiazepin-6-on als weisse Kristalle. Diese wurden in Acetonitril gelöst, und die Lösung wurde mit ätherischer Salzsäure angesäuert. Nach Kristallisation aus heissem Acetonitril erhielt man 408 mg (40%) 3-[5-[Methyl-(2-dimethylamino-äthyl)-aminomethyl]-1,2,4-oxadiazol-3-yl]-8-fluor-5-methyl-5,6-dihydro-4H-imidazo[1,5a][1,4]benzodiazepin-6-on hydrochlorid (1:2.5) als weisse Kristalle vom Smp. 217-222° (Zers.).

Beispiel 249

a) 59.3 g (0.3 Mol) 5-Chlor-isatosäureanhydrid und 30.3 g (0.3 Mol) (S)-Azetidin-2-carbonsäure wurden in 400ml N,N-Dimethylformamid/Essigsäure 5:1 suspendiert und im Oelbad unter Argon 64 Std. auf 87 - 90° erhitzt. Das Lösungsmittel wurde im Vakuum entfernt, und der Rückstand wurde in 500 ml Methanol aufgenommen, worauf 30 Min. bei Raumtemperatur gerührt wurde. Die weissen, reinen Kristalle wurden abgesaugt. Das Lösungsmittel wurde im Vakuum entfernt, und der halbkristalline Rückstand wurde aus 60 ml Methanol umkristallisiert (Heissfiltration). Die Produkte wurden vereinigt und im Vakuum getrocknet. Man erhielt 61.3 g (86%) (S)-6-Chlor-1,2,4,9,10,10a-hexahydro-azeto[2,1-c][1,4]benzodiazepin-4,10-dion vom Smp. 229-231° (Zers.).

b) 23.6 g (0.1 Mol) (S)-6-Chlor-1,2,4,9,10,10a-hexahydro-azeto[2,1-c][1,4]-benzodiazepin-4,10-dion in 300 ml THF/DMPU 5:1 wurden unter Argon bei ca.-75° zu einer LDA-Lösung (hergestellt auf übliche Weise aus 16 ml (0.11 Mol) Diisopropylamin in 200 ml THF und 69 ml 1.6 M n-Butyllithium-Lösung in Hexan) getropft. Man rührte noch 40 Min. Bei ca. -75° gab man tropfenweise 23 ml (0.11 Mol) Phosphorsäurediphenylesterchlorid zu und rührte während 35 Min. bei -75°. Inzwischen wurde separat eine weitere LDA-Lösung wie oben beschrieben hergestellt (100 ml THF). Dazu wurde nun bei ca. -75° eine Lösung von 12.0 ml (0.11 Mol) Isocyanessigsäure-aethylester in 20 ml THF/DMPU 1:1 tropfenweise zugegeben. Man tropfte den deprotonierten Isocyanessigsäure-äthylester via einen mit Trockeneis gekühlten Tropftrichter bei ca.- 70° innerhalb von 30 Min. zum anfänglich beschriebenen Reaktionsgemisch zu. Man rührte während 2 Std. im Aceton/Trockeneisbad weiter, setzte bei <-60° 100 ml 20% Ammoniumchlorid-Lösung zu und goss auf 800 ml Eiswasser. Man extrahierte dreimal mit 200 ml Methylenchlorid, trocknete mit Natriumsulfat und dampfte ein. Das braune Oel wurde in 300 ml Essigester gelöst, worauf langsam 300 ml n-Hexan zugesetzt wurde. Die Kristalle wurden abfiltriert. Weiteres Produkt wurde durch Chromatographie der Mutterlauge an 300 g Kieselgel (Methylenchlorid/Aceton 9:1) gewonnen. Nach Trocknung im Vakuum erhielt man 17.8 g (54%) (S)-7-Chlor-12,12a-dihydro-9-oxo-9H,11H-azeto-[2,1-c]imidazo[1,5-a][1,4]benzodiazepin-1-carbonsäureäthylester vom Smp. 190-194° (Essigester).

c) 20.8 g (62.8 mMol) (S)-7-Chlor-12,12a-dihydro-9-oxo-9H,11H-azeto-[2,1-c]imidazo[1,5-a][1,4]benzodiazepin-1-carbonsäureäthylester in 250 ml Aethanol und 75 ml (75 mMol) IN Natronlauge wurden während 30 Min. zum Rückfluss erhitzt. Man dampfte den Alkohol am Rotationsverdampfer ab. Der Rückstand wurde in 250 ml Wasser gelöst und mit IN Salzsäure angesäuert. Die Kristalle wurden abgesaugt, mit Wasser gewaschen und im Vakuum getrocknet. Man erhielt 18.2 g (96%) (S)-7-Chlor-12,12a-dihydro-9-oxo-9H,llH-azeto[2,1-c]imidazo[1,5-a][1,4]benzodiazepin-1-carbonsäure vom Smp. 249-250° (Zers.).

d) 3.04 g (10.0 mMol) (S)-7-Chlor-12,12a-dihydro-9-oxo-9H,11H-azeto-[2,1-c]imidazo[1,5-a][1,4]benzodiazepin-1-carbonsäure wurden in 15 ml N,N-Dimethylformamid suspendiert und bei Raumtemperatur portionenweise mit 1.78 g (11.0 mMol) 1,1'-Carbonyldiimidazol versetzt. Nach Beendigung der $CO_2$-Abspaltung wurde die klare braune Lösung während 30 Min. bei 50° gerührt, gekühlt und bei einer Temperatur unter 25° innerhalb von ca. 10 Min. tropfenweise mit 2.5 ml konz. Ammoniak versetzt. Nach 30-minütigem Rühren wurde die erhaltene Suspension eingedampft, und der Rückstand wurde mit 30 ml Wasser verrührt . Die weissen Kristalle wurden abgesaugt und im Vakuum getrocknet. Man erhielt 2.8 g (92%) (S)-7-Chlor-12,12a-dihydro-9-oxo-9H,11H-azeto[2,1-c]imidazo[1,5-a][1,4]benzodiazepin-1-carboxamid vom Smp. 251-253°.

e) 2.38 g (7.85 mMol) (S)-7-Chlor-12,12a-dihydro-9-oxo-9H,11H-azeto-[2,1-c]imidazo[1,5-a][1,4]benzodiazepin-1-carboxamid wurden in 15 ml Dioxan und 1.4 ml Pyridin suspendiert und bei einer Temperatur von ca. 10° tropfenweise mit 1.2 ml (8.25 mMol) Trifluoressigsäureanhydrid versetzt. Man rührte während 45 Min. bei Raumtemperatur und setzte nochmals 0.26 ml Pyridin und 0.11 ml Trifluoressigsäureanhydrid zu. Man rührte das Reaktionsgemisch 30 Min. Darauf wurden die Lösungsmittel im Vakuum entfernt, und der Rückstand wurde mit 30 ml Wasser verrührt. Das Rohmaterial wurde an 50 g Kieselgel (Methylenchlorid/Aceton 9:1) chromatographiert. Das Produkt wurde aus Acetonitril unkristallisiert. Man erhielt 1.75 g (80%) (S)-7-Chlor-12,12a-dihydro-9-oxo-9H,11H-azeto-[2,1-c]imidazo[1,5-a][1,4]benzodiazepin-1-carbonitril vom Smp. 258-261°.

f) Zu einer Natriummethylat-Lösung (hergestellt aus 1.49 g (64.7 mMol) Natrium und 67 ml Methanol) wurden portionenweise 4.5 g (65.1 mMol) Hydroxylamin-hydrochlorid und 12.35 g (43.3 mMol) (S)-7-Chlor-12,12a-dihydro-9-oxo-9H,11H-azeto[2,1-c]imidazo[1,5-a][1,4]benzodiazepin-1-carbonitril zugegeben und während 108 Std. bei Raumtemperatur gerührt. Die Kristalle wurden abgesaugt, in 40 ml Wasser 30 Min. gerührt, erneut abgesaugt und im Hochvakuum getrocknet. Man erhielt 13.3 g (96%) (E)-und/oder (Z)-1-(Amino-hydroximino-methyl)-7-chlor-12,12a-dihydro-9H,11H-azeto[2,1-c]imidazo[1,5-a][1,4]benzodiazepin-9-on vom Smp. 282-283° (Zers.).

g) Zu einer Suspension von 1.59 g (5.0 mMol) (E)- und/oder (Z)-1-(Amino-hydroximino-methyl)-7-chlor-12,12a-dihydro-9H,11H-azeto[2,1-c]-imidazo[1,5-a][1,4]benzodiazepin-9-on in 10 ml N,N-Dimethylformamid wurden unter Argon 1.0 ml (5.75 mMol) Chloressigsäureanhydrid zugegeben und während 1 Std. bei ca. 25° gerührt und darauf Std. auf 110° erhitzt. Die Lösung wurde am Hochvakuum eingedampft, und der erhaltene braune Rückstand wurde in 120 ml Methylenchlorid gelöst. Die Lösung wurde dreimal mit 20 ml gesättigter Natriumhydrogencarbonat Lösung gewaschen, und das Lösungsmittel wurde im Vakuum entfernt. Das Rohmaterial wurde an 80 g Kieselgel (Methylenchlorid/Aceton 9:1, dann 4:1) chromatographiert. Man erhielt nach Trocknung im Hochvakuum 1.47 g (78%) (S)-7-Chlor-1-(5-chlormethyl-1,2,4-oxadiazol-3-yl)-12,12a-dihydro-9H,11H-azeto[2,1-c]imidazo[1,5-a][1,4]benzodiazepin-9-on als farblose Kristalle vom Smp. 238-241°(Acetonitril).

h) Eine Suspension von 1.13 g (3.0 mMol) (S)-7-Chlor-1-(5-chlormethyl-1,2,4-oxadiazol-3-yl)-12,12a-dihydro-9H,11H-azeto[2,1-c]imidazo[1,5-a][1,4]-benzodiazepin-9-on in 15 ml N,N-Dimethylformamid wurde mit 0.74 ml (9.0 mMol) Propylamin versetzt und unter Argon 3 Std. bei Raumtemperatur gerührt. Die Lösung wurde eingedampft, der Rückstand wurde in 20 ml Wasser aufgenommen, und die wässerige Phase wurde mit Methylenchlorid extrahiert. Die vereinigten Extrakte wurden zweimal mit Wasser gewaschen, mit Natriumsulfat getrocknet, filtriert und eingedampft. Das Rohprodukt wurde an 30 g Kieselgel chromatographiert (Methylenchlorid/ Aceton 4:1, dann 2:1 und schliesslich Methylenchlorid/Methanol 9:1). Man erhielt 840 mg (S)-7-Chlor-1-(5-propylaminomethyl-1,2,4-oxadiazol-3-yl)-12,12a-dihydro-9H,11H-azeto[2,1-c]imidazo[1,5-a][1,4]benzodiazepin-9-on, welches in Acetonitril in Gegenwart von Fumarsäure in das Fumarat überführt wurde. Durch Umkristallisation aus Acetonitril erhielt man 588 mg (35%) (S)-7-Chlor-1-(5-propylaminomethyl-1,2,4-oxadiazol-3-yl)-12,12a-dihydro-9H,11H-azeto[2,1-c]imidazo[1,5-a][1,4]benzodiazepin-9-on fumarat (1:1.4) vom Smp. 135 -140° (Zers.).

Beispiel 250

Eine Suspension von 1.04 g (3.0 mMol) 3-(5-Chlormethyl-1,2,4-oxadiazol-3-yl)-8-fluor-5-methyl-5,6-dihydro-4H-imidazo[1,5-a][1,4]benzodiazepin-6-on in 10 ml N,N-Dimethylformamid wurde mit 1.27 ml (9.0 mMol) Diisopropylamin versetzt. Nach 18 Std. Rühren bei Raumtemperatur wurde die erhaltene Lösung eingeengt, und der Rückstand wurde in 10 ml Wasser aufgenommen und 2 Std. im Ultraschallbad gerührt. Die Kristalle wurden abgesaugt und in Methylenchlorid gelöst, und die Lösung wurde mit Natriumsulfat getrocknet, filtriert und eingedampft. Das Rohmaterial wurde an Kieselgel (Essigester) chromatographiert, in Acetonitril gelöst und mit ätherischer Salzsäure angesäuert. Die Lösung wurde erneut eingedampft, und der Rückstand wurde aus Acetonitril umkristallisiert. Man erhielt nach Trocknung im Vakuum 542 mg (38%) 3-(5-Diisopropylaminomethyl-1,2,4-oxadiazol-3-yl)-8-fluor-5-methyl-5,6-dihydro-4H-imidazo[1,5-a][1,4]benzodiazepin-6-on als weisse Kristalle vom Smp. 173-178° (Zers.).

Beispiel 251

a) Zu einer Suspension von 22 g (76 mmol) (S)-10,11,12,12a-Tetrahydro-8-oxo-8H-imidazo[5,1-c]pyrrolo[1,2-a]thieno[3,2-e][1,4]diazepin-1-carbonsäure (EP 59 390 A1) in 100 ml Dimethylformamid wurden portionenweise 12.33 g (76 mmol) 1,1'-Carbonyldiimidazol zugegeben. Die entstandene hellbraune Lösung erwärmte man während 45 Minuten auf 50°. Anschliessend kühlte man die Lösung auf Raumtemperatur ab und tropfte 14 ml wässerige Ammoniaklösung dazu. Nach weiteren 30 Minuten wurde das Reaktions-gemisch auf 100 ml Eiswasser gegossen, und der entstandene Niederschlag wurde abfiltriert und mit Wasser, Aethanol und anschliessend mit Aether nachgewaschen. Man erhielt nach Trocknen bei 70°/10 Torr 20 g (91 %) (S)-10,11,12,12a-Tetrahydro-8-oxo-8H-imidazo[5,1-c]pyrrolo[1,2-a]thieno[3,2-e]-[1,4]diazepin-1-carbonsäureamid als farblose Kristalle vom Smp. 222-223°.

b) Zu einer Suspension von 8.98 g (31.1 mmol) (S)-10,11,12,12a-Tetrahydro-8-oxo-8H-imidazo[5,1-c]pyrrolo [1,2-a]thieno[3,2-e][1,4]diazepin-1-carbonsäureamid in 50 ml Dioxan und 5.4 ml Pyridin wurden bei 5-8° 4.44 ml (31.9 mmol) Trifluoressigsäureanhydrid zugetropft. Die erhaltene beige Lösung wurde während 2.5 Stunden bei 50° gerührt und anschliessend auf 220 ml Eiswasser gegossen. Der entstandene Niederschlag wurde abfiltriert. Man erhielt nach Trocknen bei 70°/10 Torr 6.78 g (80 %) (S)-10,11,12,12a-Tetrahydro-8-oxo-8H-imidazo[5,1-c] pyrrolo[1,2-a]thieno[3,2-e][1,4]diazepin-1-carbonsäurenitril als weisses Pulver vom Smp. 249-251°.

c) Zu einer frisch hergestellten Lösung von Natriummethylat in Methanol (aus 1.55 g (67 mmol) Natrium in 70 ml Methanol) wurden 12.7 g (47 mmol) (S)-10,11,12,12a-Tetrahydro-8-oxo-8H-imidazo[5,1-c]pyrrolo[1,2-a]-thieno [3,2-e)[1,4]diazepin-1-carbonsäurenitril und 5 g (72.2 mmol) Hydroxylamin-hydrochlorid zugegeben, worauf die Mischung 48 Stunden bei Raumtemperatur gerührt wurde. Anschliessend wurde die Suspension eingedampft und mit 100 ml Wasser versetzt. Der erhaltene Niederschlag wurde abfiltriert und am Hochvakuum getrocknet. Man erhielt 9.46 g (66 %) (E)-und/oder (Z)- (S)-1-(Amino-hydroxyimino-methyl)-10,11,12,12a-tetrahydro-8H-imidazo [5,1-c]pyrrolo[1,2-a]thieno[3,2-e][1,4]diazepin-8-on als farbloses Pulver vom Smp. 106 - 108 °.

d) Zu einer Lösung von 1.42 g (8.07 mmol) BOC-Glycin in 13 ml Dimethylformamid wurden 1.40 g (8.04 mmol) 1,1'-Carbonyldiimidazol zugegeben, und die Mischung wurde während 30 Minuten bei 50° gerührt. Anschliessend gab man 2.30 g (7.58 mmol) (E)-und/oder (Z)- (S)-1-(Aminohydroxyimino-methyl)-10,11,12,12a-tetrahydro-8H-imidazo[5,1-c]pyrrolo-[1,2-a]thieno[3,2-e][1,4]diazepin-8-on zu und rührte 15 Stunden bei 90°. Die erhaltene braune Lösung wurde am Hochvakuum eingedampft, und der erhaltene braune Rückstand wurde chromatographiert (Kieselgel, Methylenchlorid /Methanol 10:1). Man erhielt 1.50 g (44 %) (S)-1-(5-BOC-Aminomethyl-1,2,4-oxadiazol-3-yl)-10,11,12,12a-tetrahydro-8H-imidazo-[5,1-c]pyrrolo[1,2-a]thieno[3,2-e][1,4]diazepin-8-on als farblosen Schaum, Rf=0.62 (Kieselgel, Methylenchlorid/Methanol 10:1).

e) Eine Lösung von 1.50 g (3.4 mmol) (S)-1-(5-BOC-Aminomethyl-1,2,4-oxadiazol-3-yl)-10,11,12,12a-tetrahydro-8H-imidazo[5,1-c]pyrrolo[1,2-a]-thieno[3,2-e][1,4]diazepin-8-on in 6 ml Trifluoressigsäure wurde 2 Stunden bei Raumtemperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 10 ml wässeriger Ammoniaklösung basisch gestellt und sechs Mal mit Methylenchlorid extrahiert. Die organischen Phasen wurden mit Natriumsulfat getrocknet, filtriert und eingedampft. Der erhaltene Rückstand wurde chromatographiert (Kieselgel, Methylenchlorid/Methanol 9:1). Man erhielt 850 mg (73 %) (S)-1-(5-Aminomethyl-1,2,4-oxadiazol-3-yl)-10,11,12,12a-tetrahydro-8H-imidazo[5,1-c]pyrrolo[1,2-a]thieno[3,2-e][1,4]-diazepin-8-on als farblosen Schaum, Rf=0.25 (Kieselgel, Methylenchlorid/ Methanol 9:1).

Beispiel 252

a) Zu einer Lösung von 5.13 g (18.6 mmol) (S)-11,lla-Dihydro-9-oxo-9H,10H-azeto[1,2-a]imidazo[5,1-c]thieno [3,2-e][1,4]diazepin-1-carbonsäure in 30 ml Dimethylformamid wurden bei Raumtemperatur 3.15 g (19.4 mmol) 1,1'-Carbonyldiimidazol auf einmal zugegeben, und die Mischung wurde 30 Minuten bei 50° gerührt. Anschliessend wurden 4.16 g (18.9 mmol) Phthaloylglycinamidoxim auf einmal zugegeben, und die Mischung wurde 15 Stunden bei 110° gerührt. Das Dimethylformamid wurde am Hochvakuum eingedampft, und der erhaltene Rückstand wurde mit 150 ml Wasser versetzt. Extraktion mit Methylenchlorid (zwei Mal), Trocknen mit Natriumsulfat, Filtrieren und Eindampfen lieferten einen rötlichen Rückstand, welcher anschliessend chromatographiert wurde (Kieselgel, Methylenchlorid/Methanol 20:1). Man erhielt 3.92 g (46 %) (S)-2-[5-(8-Oxo-11,11a-dihydro-8H,10H-azeto[1,2-a]imidazo[5,1-c]thieno[3,2-e][1,4]diazepin-1-yl)-1,2,4-oxadiazol-3-ylmethyl]-2,3-dihydro-1H-isoindol-1,3-dion als hellbraune Kristalle vom Smp. 155-157°.

b) Zu einer Lösung von 2.4 g (5.23 mmol) (S)-2-[5-(8-Oxo-11,11a-dihydro-8H,10H-azeto[1,2-a]imidazo[5,1-c]thieno[3,2-e][1,4]diazepin-1-yl)- 1,2,4-oxadiazol-3-yl-methyl]-2,3-dihydro-1H-isoindol-1,3-dion in 40 ml Aethanol tropfte man 30 ml Methylamin (33% in Aethanol) bei 70° zu und rührte noch 2 Stunden bei 70°. Das Reaktionsgemisch

wurde eingedampft, und der Rückstand wurde chromatographiert (Kieselgel, Methylenchlorid/ Methanol 20:1). Man erhielt 1.60 g (93 %) (S)-1-(3-Aminomethyl-1,2,4-oxadiazol-5-yl)-11,11a-dihydro-8H,10H-azeto[1,2-a]imidazo [5,1-c]thieno[3,2-e][1,4]diazepin-8-on als farblose Kristalle vom Smp. 227° (Zers.).

Beispiel 253

a) Zu einer Suspension von 8.8 g (33.4 mmol) 5-Methyl-6-oxo-5,6-dihydro-4H-imidazo[1,5-a]thieno[2,3-f][1,4]diazepin-3-carbonsäure (EP 150 040 A2) in 80 ml Dimethylformamid wurden portionenweise 5.66 g (34.9 mmol) 1,1'-Carbonyldiimidazol zugegeben. Die entstandene hellbraune Lösung erwärmte man während 45 Minuten auf 50°. Anschliessend kühlte man die Lösung auf Raumtemperatur ab und tropfte 8.1 ml wässerige Ammoniaklösung dazu. Nach weiteren 30 Minuten wurde das Reaktionsgemisch auf 100 ml Eiswasser gegossen, und der entstandene Niederschlag wurde abfiltriert und mit Wasser, Aethanol und anschliessend mit Aether nachgewaschen. Man erhielt nach Trocknen bei 70°/10 Torr 7.6 g (86 %) 5-Methyl-6-oxo-5,6-dihydro-4H-imidazo[1,5-a]-thieno[2,3-f][1,4] diazepin-3-carbonsäureamid als farblose Kristalle vom Smp. 294-296°.

b) Zu einer Suspension von 7.43 g (28.3 mmol) 5-Methyl-6-oxo-5,6-dihydro-4H-imidazo[1,5-a]thieno[2,3-f[1,4]diazepin-3-carbonsäureamid in 40 ml Dioxan und 5 ml Pyridin wurden bei 5-8° 4.05 ml (29 mmol) Trifluoressigsäureanhydrid zugetropft. Die erhaltene beige Lösung wurde während 2.5 Stunden bei 50° gerührt und anschliessend auf 220 ml Eiswasser gegossen. Der entstandene Niederschlag wurde abfiltriert. Man erhielt nach Trocknen bei 70°/10 Torr 5.6 g (81 %) 5-Methyl-6-oxo-5,6-dihydro-4H-imidazo[1,5-a]thieno[2,3-f][1,4]diazepin-3-carbonsäurenitril als weisses Pulver vom Smp. 206-208°.

c) Zu einer frisch hergestellten Lösung von Natriummethylat in Methanol (aus 0.74 g (32.3 mmol) Natrium in 40 ml Methanol) wurden 5.54 g (22.7 mmol) 5-Methyl-6-oxo-5,6-dihydro-4H-imidazo[1,5-a]thieno[2,3-f][1,4]-diazepin-3-carbonsäurenitril und 2.5 g (36.1 mmol) Hydroxylamin-hydrochlorid zugegeben, worauf die Mischung 48 Stunden bei Raum-temperatur gerührt wurde. Anschliessend wurde die Suspension eingedampft und mit 100 ml Wasser versetzt. Der erhaltene Niederschlag wurde abfiltriert und am Hochvakuum getrocknet. Man erhielt 5.7 g (90 %) (E)-und/oder (Z)-3-(Amino-hydroxyimino-methyl)-5-methyl-5,6-dihydro-4H-imidazo[1,5-a]thieno[2,3-f][1,4]diazepin-6-on als farbloses Pulver vom Smp. 248-250°.

d) Zu einer Lösung von 3.85 g (21.9 mmol) BOC-Glycin in 45 ml Dimethylformamid wurden 3.82 g (23.5 mmol) 1,1'-Carbonyldiimidazol zugegeben, und die Mischung wurde während 30 Minuten bei 50° gerührt. Anschliessend gab man 5.7 g (20.56 mmol) (E)-und/oder (Z)-3-(Aminohydroxyimino-methyl)-5-methyl-5,6-dihydro-4H-imidazo [1,5-a]thieno[2,3-f]-[1,4]diazepin-6-on zu und rührte 15 Stunden bei 90°. Die erhaltene braune Lösung wurde am Hochvakuum eingedampft, und der erhaltene braune Rückstand wurde chromatographiert (Kieselgel, Methylenchlorid /Methanol 10:1). Man erhielt 7.2 g (84 %) nicht ganz reines 3-(5-BOC-Aminomethyl-1,2,4-oxadiazol-3-yl)-5-methyl-5,6-dihydro-4H-imidazo[1,5-a]thieno[2,3-f][1,4]-diazepin-6-on als farblosen Schaum, Rf=0.28 (Kieselgel, Methylenchlorid/ Methanol 10:1).

e) Eine Lösung von 7.2 g (17.2 mmol) 3-(5-BOC-Aminomethyl-1,2,4-oxadiazol-3-yl)-5-methyl-5,6-dihydro-4H-imidazo[1,5-a]thieno[2,3-f][1,4]-diazepin-6-on in 15 ml Trifluoressigsäure wurde 2 Stunden bei Raum-temperatur gerührt. Die gelbe Lösung wurde eingedampft, der Rückstand wurde in Wasser gelöst, und die Wasserphase wurde drei Mal mit Methylenchlorid gewaschen. Anschliessend wurde die Wasserphase mit 10 ml wässeriger Ammoniaklösung basisch gestellt und sechs Mal mit Methylenchlorid extrahiert. Die organischen Phasen wurden mit Natriumsulfat getrocknet, filtriert und eingedampft. Der erhaltene Rückstand wurde chromatographiert (Kieselgel, Methylenchlorid/Methanol 10:1). Man erhielt 3.2 g (58 %) 3-(5-Aminomethyl-1,2,4-oxadiazol-3-yl)-5-methyl-5,6-dihydro-4H-imidazo[1,5-a]thieno[2,3-f][1,4]diazepin-6-on als farblose Kristalle vom Smp.202-204°

Beispiel 254

a) Zu einer Lösung von 5.27 g (20 mmol) 5-Methyl-6-oxo-5,6-dihydro-4H-imidazo[1,5-a]thieno[2,3-f][1,4]diazepin-3-carbonsäure in 50 ml Dimethylformamid wurden bei Raumtemperatur 3.39 g (20 mmol) 1,1'-Carbonyldiimidazol auf einmal zugegeben, und die Mischung wurde 30 Minuten bei 50° gerührt. Anschliessend wurden 4.47 g (20 mmol) Phthaoylglycinamidoxim auf einmal zugegeben, und die Mischung wurde 15 Stunden bei 110° gerührt. Das

Dimethylformamid wurde am Hochvakuum eingedampft, und der erhaltene Rückstand wurde mit 150 ml Wasser versetzt. Extraktion mit Methylenchlorid (zwei Mal), Trocknen mit Natriumsulfat, Filtrieren und Eindampfen lieferte einen rötlichen Rückstand, welcher anschliessend chromatographiert wurde (Kieselgel, Methylenchlorid/Methanol 20:1). Man erhielt nach Umkristallisieren aus Acetonitril 4.21 g (47 %) 5-(5-Methyl-6-oxo-5,6-dihydro-4H-imidazo [1,5-a]thieno[2,3-f][1,4]diazepin-3-ylmethyl)-2,3-dihydro-lH-isoindol-1,3-dion als hellbraune Kristalle vom Smp. 245 -246 °.

b) Zu einer Lösung von 4.2 g (9.41 mmol) 5-(5-Methyl-6-oxo-5,6-dihydro-4H-imidazo[1,5-a]thieno[2,3-f][1,4]diazepin-3-ylmethyl)-2,3-dihydro-1H-isoindol-1,3-dion in 50 ml Aethanol tropfte man 50 ml Methylamin (33% in Aethanol) bei 70° zu und rührte noch zwei Stunden bei 70°. Das Reaktions-gemisch wurde abgekühlt, und die erhaltenen Kristalle wurden abfiltriert. Man erhielt 1.2 g (40 %) 3-(3-Aminomethyl-1,2,4-oxadiazol-5-yl)-5-methyl-5,6-dihydro-4H-imidazo[1,5-a]thieno[2,3-f][1,4]diazepin-6-on als farblose Kristalle vom Smp. 203° (Zers.).

Beispiel 255

a) Zu einer Suspension von 4.0 g (12.6 mmol) (S)-10,11,12,12a-Tetrahydro-8-oxo-8H-imidazo[5,1-c]pyrrolo[1,2-a] thieno[3,2-e][1,4]diazepin-1-carbonsäureäthylester in 40 ml Aethanol wurden 8 ml Hydrazinhydrat zugegeben, und das Gemisch wurde 3 Stunden am Rückfluss erhitzt. Nach Abkühlen auf 0° filtrierte man die erhaltenen Kristalle ab und erhielt 3.6 g (94 %) (S)-10,11,12,12a-Tetrahydro-8-oxo-8H-imidazo[5,1-c]pyrrolo[1,2-a]-thieno[3,2-e][1,4] diazepin-1-carbonsäurehydrazid als farblose Nadeln vom Smp. > 260°.

b) Eine Lösung von 1.0 g ( 4.9 mmol) N-Phthaloylglycin in 8 ml Dimethylformamid versetzte man bei Raumtemperatur mit 0.83 g (5.11 mmol) 1,1'-Carbonyldiimidazol und erhitzte anschliessend auf 50°. Nach 30 Minuten kühlte man auf Raumtemperatur ab, gab 1.5 g (5 mmol) (S)-10,11,12,12a-Tetrahydro-8-oxo-8H-imidazo[5,1-c]pyrrolo [1,2-a]thieno[3,2-e]-[1,4]diazepin-1-carbonsäurehydrazid dazu und rührte 12 Stunden bei Raumtemperatur. Die erhaltene Suspension wurde filtriert, und das erhaltene farblose Pulver wurde mit Aethanol und Diäthyläther gewaschen. Man erhielt 2.2 g ( 100 %) (S)-N'-(1,3-Dioxo-2,3-dihydro-1H-isoindol-2-ylacetyl)-8-oxo-10,11,12,12a-tetrahydro-8H-imidazo[5,1-c]pyrrolo[1,2-a]-thieno[3,2-e][1,4]diazepine-1-carbonsäurehydrazid vom Smp. > 260°.

c) Eine Lösung von 10 g (20.4 mmol) (S)-N'-(1,3-Dioxo-2,3-dihydro-1H-isoindol-2-ylacetyl)-8-oxo-10,11,12,12a-tetrahydro-8H-imidazo[5,1-c]pyrrolo-[1,2-a]thieno[3,2-e][1,4]diazepine-1-carbonsäurehydrazid in 90 g Polyphosphosphorsäure wurde während 1.5 Stunden bei 100° gerührt. Nach Abkühlen auf Raumtemperatur wurde das Gemisch unter gutem Rühren auf 300 ml Eiswasser gegossen, worauf bis zu pH=8 festes Natriumcarbonat zugegeben wurde. Extraktion mit Methylenchlorid und Chromatographie (Kieselgel, Methylenchlorid/Methanol 20 : 1) lieferte 7.5 g (78 %) (S)-2-[5-(8-Oxo-10,11,12,12a-tetrahydro-8H-imidazo[5,1-c]pyrrolo[1,2-a]thieno[3,2-e]-[1,4] diazepin-1-yl)-1,3,4-oxadiazol-2-ylmethyl]-2.3-dihydro-1H-isoindol-1,3-dion als farbloses Pulver vom Smp. >250.

d) Zu einer Suspension von 2.5 g (5.3 mmol) (S)-2-[5-(8-Oxo-10,11,12,12a-tetrahydro-8H-imidazo[5,1-c]pyrrolo [1,2-a]thieno[3,2-e][1,4]diazepin-1-yl)-1,3,4-oxadiazol-2-ylmethyl]-2,3-dihydro-lH-isoindol-1,3-dion in 30 ml Aethanol tropfte man bei 70° 30 ml Methylamin (33 % in Aethanol) zu und rührte während einer Stunde bei 70°. Der erhaltene Niederschlag wurde heiss abfiltriert, und das erhaltene gelbliche Pulver wurde mit Aethanol farblos gewaschen. Man erhielt 1.23 g (68 %) (S)-1-(5-Aminomethyl-1,3,4-oxadiazol-2-yl)-10,11,12,12a-tetrahydro-8H-imidazo[5,1-c]pyrrolo[1,2-a]-thieno[3,2-e][1,4]diazepin-8-on als farbloses Pulver vom Smp. 214-216°.

Beispiel 256

a) Zu einer Suspension von 6.0 g (19.8 mmol) (S)-8-Oxo-11,11a-dihydro-8H,10H-azeto[1,2-a]imidazo[5,1-c]thieno [3,2-e][1,4]diazepin-1-carbonsäureäthylester in 50 ml Aethanol wurden 10 ml Hydrazinhydrat zugegeben, und das Gemisch wurde 3 Stunden am Rückfluss erhitzt. Nach Abkühlen auf 0° filtrierte man die erhaltenen Kristalle ab und erhielt 5.6 g (98 %) (S)-8-Oxo-11,11a-dihydro-8H,10H-azeto[1,2-a]imidazo[5,1-c]thieno[3,2-e][1,4]diazepin-1-carbonsäurehydrazid als farblose Nadeln vom Smp. 260 - 263°.

b) Eine Lösung von 5.8 g ( 19 mmol) N-Phthaloylglycin in 30 ml Dimethylformamid versetzte man bei Raumtemperatur mit 3.2 g (19.75 mmol) 1,1'-Carbonyldiimidazol und erhitzte anschliessend auf 50°. Nach 30 Minuten kühlte

man auf Raumtemperatur ab, gab 5.6 g (19.63 mmol) (S)-8-Oxo-11,11a-dihydro-8H,10H-azeto[1,2-a]imidazo [5,1-c]thieno[3,2-e][1,4]-diazepin-1-carbonsäurehydrazid dazu und rührte 12 Stunden bei Raum-temperatur. Die erhaltene Suspension wurde filtriert, und das erhaltene farblose Pulver wurde mit Aethanol und Diäthyläther gewaschen. Man erhielt 8.7 g ( 96 %) (S)-N'-(1,3-Dioxo-2,3-dihydro-1H-isoindol-2-ylacetyl)-8-oxo-11,11a-dihydro-8H,10H-azeto[1,2-a]imidazo[5,1-c]thieno[3,2-e][1,4]diazepin-1-carbonsäurehydrazid vom Smp. > 260°.

c) Eine Lösung von 8.5 g (16.8 mmol) (S)-N'-(1,3-Dioxo-2,3-dihydro-1H-isoindol-2-ylacetyl)-8-oxo-11,11a-dihydro-8H,10H-azeto[1,2-a]imidazo[5,1-c]-thieno[3,2-e][1,4]diazepin-1-carbonsäurehydrazid in 70 g Polyphosphosphorsäure wurde während 1.5 Stunden bei 100° gerührt. Nach Abkühlen auf Raumtemperatur wurde das Gemisch unter gutem Rühren auf 300 ml Eiswasser gegossen, worauf bis zu pH=8 festes Natriumcarbonat zugegeben wurde. Extraktion mit Methylenchlorid und Chromatographie (Kieselgel, Methylenchlorid/Methanol 20 : 1) lieferte 6.8 g (88 %) (S)-2-[5-(8-Oxo-11,11a-dihydro-8H,10H-azetc[1,2-a]imidazo[5,1-c]thieno[3,2-e][1,4]diazepin-1-yl)-1,3,4-oxadiazol-2-ylmethyl]-2,3-dihydro-1H-isoindol-1,3-dion als farbloses Pulver vom Smp. >250.

d) Zu einer Suspension von 2.5 g (5.4 mmol) (S)-2-[5-(8-Oxo-11,11a-dihydro-8H,10H-azeto[1,2-a]imidazo[5,1-c] thieno[3,2-e][1,4]diazepin-1-yl)-1,3,4-oxadiazol-2-ylmethyl]-2,3-dihydro-1H-isoindol-1,3-dion in 30 ml Aethanol tropfte man bei 70° 30 ml Methylamin (33 % in Aethanol) zu und rührte während einer Stunde bei 70°. Der erhaltene Niederschlag wurde heiss abfiltriert, und das erhaltene gelbliche Pulver wurde mit Aethanol farblos gewaschen. Man erhielt 1.18 g (66 %) (S)-1-(5-Aminomethyl-1,3,4-oxadiazol-2-yl)-11,11a-dihydro-8H,10H-azeto[1,2-a]imidazo [5,1-c]thieno-[3,2-e][1,4]diazepin-8-on als farbloses Pulver vom Smp. 233 -235°.

Beispiel 257

a) Zu einer Suspension von 7.0 g (24 mmol) 5-Methyl-6-oxo-5,6-dihydro-4H-imidazo[1,5-a]thieno[2,3-f][1,4]diazepin-3-carbonsäureäthylester in 70 ml Aethanol wurden 13 ml Hydrazinhydrat zugegeben, und das Gemisch wurde 3 Stunden am Rückfluss erhitzt. Nach Abkühlen auf 0° filtrierte man die erhaltenen Kristalle ab und erhielt 6.5 g (97 %) 5-Methyl-6-oxo-5,6-dihydro-4H-imidazo[1,5-a]thieno[2,3-f][1,4]diazepin-3-carbonsäurehydrazid als farblose Nadeln vom Smp. > 260°.

b) Eine Lösung von 4.74 g ( 23.12 mmol) N-Phthaloylglycin in 35 ml Dimethylformamid versetzte man bei Raumtemperatur mit 3,9 g (24.07 mmol) 1,1'-Carbonyldiimidazol und erhitzte anschliessend auf 50°. Nach 30 Minuten kühlte man auf Raumtemperatur ab, gab 6.53 g 5-Methyl-6-oxo-5,6-dihydro-4H-imidazo[1,5-a]thieno[2,3-f][1,4] diazepin-3-carbonsäurehydrazid dazu und rührte 12 Stunden bei Raumtemperatur. Die erhaltene Suspension wurde filtriert, und das erhaltene farblose Pulver wurde mit Aethanol und Diäthyläther gewaschen. Man erhielt 10.4 g ( 97 %) (S)-N'-(1,3-Dioxo-2,3-dihydro-1H-isoindol-2-ylacetyl)-5-methyl-6-oxo-5,6-dihydro-4H-imidazo[1,5-a]-thieno[2,3-f][1,4]diazepin-3-carbonsäurehydrazid vom Smp. > 260°.

c) Eine Lösung von 5 g (10.8 mmol) (S)-N'-(1,3-Dioxo-2,3-dihydro-1H-isoindol-2-ylacetyl)-5-methyl-6-oxo-5,6-dihydro-4H-imidazo[1,5-a]thieno[2,3-f]-[1,4]diazepin-3-carbonsäurehydrazid in 35 g Polyphosphorsäure wurde während 1.5 Stunden bei 100° gerührt. Nach Abkühlen auf Raum-temperatur wurde das Gemisch unter gutem Rühren auf 300 ml Eiswasser gegossen, worauf bis zu pH=8 festes Natriumcarbonat zugegeben wurde. Extraktion mit Methylenchlorid und Chromatographie (Kieselgel, Methylenchlorid/Methanol 20 : 1) lieferte 4.3 g (89 %) (S)-2-[5-(5-Methyl-6-oxo-5,6-dihydro-4H-imidazo[1,5-a]thieno[2,3-f][1,4]diazepin-3-yl)-1,3,4-oxadiazol-2-ylmethyl]-2,3-dihydro-lH-isoindol-1,3-dion als farbloses Pulver vom Smp. 262-264°.

d) Zu einer Suspension von 4.3 g (5.3 mmol) (S)-2-[5-(5-Methyl-6-oxo-5,6-dihydro-4H-imidazo[1,5-a]thieno[2,3-f] [1,4]diazepin-3-yl)-1,3,4-oxadiazol-2-ylmethyl]-2,3-dihydro-1H-isoindol-1,3-dion in 100 ml Aethanol tropfte man bei 70° 60 ml Methylamin (33 % in Aethanol) zu und rührte während einer Stunde bei 70°. Der erhaltene Niederschlag wurde heiss abfiltriert, und das erhaltene gelbliche Pulver wurde mit Aethanol farblos gewaschen. Man erhielt 2.7 g (88 %) 3-(5-Aminomethyl-1,3,4-oxadiazol-2-yl)-5-methyl-5,6-dihydro-4H-imidazo[1,5-a]thieno[2,3-f] [1,4]diazepin-6-on als farbloses Pulver vom Smp. 217-219°.

Beispiel 258

Zu einer Lösung von 0.632 g (2 mmol) 3-(5-Aminometh,yl-1,3,4-oxadiazol-2-yl)-5-methyl-5,6-dihydro-4H-imidazo[1,5-a]thieno[2,3-f][1,4]-diazepin-6-on in 20 ml Dimethylformamid wurden 3.48 ml (20 mmol) N-Aethyldiisopropylamin und 1.1 ml (12 mmol) Propylbromid zugegeben, worauf 12 Stunden bei 70° gerührt wurde. Das Dimethylformamid wurde abgedampft, und der Rückstand wurde zwischen Methylenchlorid und 2N Natriumcarbonatlösung verteilt. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 20:1) lieferte 0.395 g (49 %) 3-(5-Dipropylaminomethyl-1,3,4-oxadiazol-2-yl)-5-methyl-5,6-dihydro-4H-imidazo[1,5-a]thieno[2,3-f][1,4]diazepin-6-on als farblosen Schaum, Rf=0.33 (Kieselgel, Essigsäureäthylester/Methanol 20:1).

Beispiel 259

Zu einer Lösung von 0.500 g (1.58 mmol) 3-(5-Aminomethyl-1,3,4-oxadiazol-2-yl)-5-methyl-5,6-dihydro-4H-imidazo[1,5-a]thieno[2,3-f][1,4]-diazepin-6-on in 40 ml Methylenchlorid wurden 2 ml (11.5 mmol) N-Aethyldiisopropylamin und 0.97 ml (8 mmol) Allylbromid zugegeben, worauf 12 Stunden bei 70° gerührt wurde. Die Reaktionslösung wurde mit Methylenchlorid verdünnt und mit 2N Natriumcarbonatlösung gewaschen. Die wässerige Phase wurde zwei Mal mit Methylenchlorid gewaschen, und die organischen Phasen wurden mit Natriumsulfat getrocknet, filtriert und eingedampft. Chromatographie (Kieselgel, Essigsäureäthylester/Methanol 10:1) lieferte 0.510 g (81 %) 3-(5-Diallylaminomethyl-1,3,4-oxadiazol-2-yl)-5-methyl-5,6-dihydro-4H-imidazo[1,5-a]thieno[2,3-f][1,4]diazepin-6-on als farblosen Schaum, Rf=0.54 (Kieselgel, Essigsäureäthylester/ Methanol 10:1).

Beispiel A

(S)-8-Chlor-1-(5-dipropylaminomethyl-1,2,4-oxadiazol-3-yl)-12,12a-dihydro-9H,11H-azeto[2,1-c]imidazo[1,5-a][1,4]benzodiazepin-9-on, 3-(5-Dipropylaminomethyl-1,2,4-oxadiazol-3-yl)-8-fluoro-5-methyl-5,6-dihydro-4H-imidazo[1,5-a][1,4]-benzodiazepin-6-on oder eine andere der eingangs als besonders bevorzugt hervorgehobenen Verbindungen kann als Wirkstoff zur Herstellung einer Injektionslösung folgender Zusammensetzung verwendet werden:

| | |
|---|---|
| Wirkstoff | 1 mg |
| 1 n HCl | 20 µl |
| Essigsäure | 0,5 mg |
| NaCl | 8 mg |
| Benzolalkohol | 10 mg |
| 1 n NaOH | q.s. ad pH 5 |
| $H_2O$ | q.s. ad 1 ml |

**Patentansprüche**

**1.** Imidazodiazepine der allgemeinen Formel

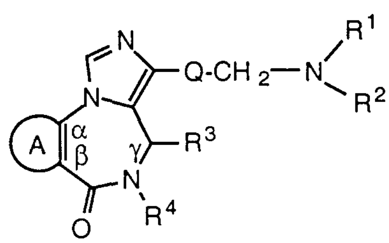

I

worin A zusammen mit den beiden mit α und β bezeichneten Kohlenstoffatomen einen der Reste

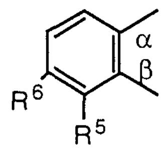 , 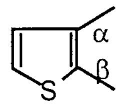 und 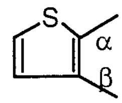

($A^1$) ($A^2$) ($A^3$)

Q einen der Reste

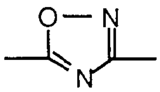 , 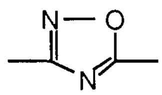 und 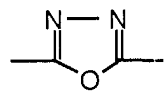

($Q^1$) ($Q^2$) ($Q^3$)

$R^1$ und $R^2$ je Wasserstoff, ($C_1$-$C_7$)-Alkyl, ($C_1$-$C_7$)-Alkenyl, ($C_1$-$C_7$)-Alkinyl, ($C_1$-$C_7$)-Hydroxyalkyl, ($C_1$-$C_7$)-Alkoxy-($C_1$-$C_7$)-Alkyl, ($C_3$-$C_6$)-Cycloalkyl, ($C_3$-$C_6$)-Cycloalkyl-($C_1$-$C_7$)-Alkyl, Amino-($C_1$-$C_7$)-Alkyl, ($C_1$-$C_7$)-Alkylamino-($C_1$-$C_7$)-Alkyl, Di-($C_1$-$C_7$)-Alkylamino-($C_1$-$C_7$)-Alkyl oder Phenyl-($C_1$-$C_7$)-Alkyl, wobei der Phenylrest gegebenenfalls durch Halogen, Trifluormethyl, ($C_1$-$C_7$)-Alkyl oder ($C_1$-$C_7$)-Alkoxy substituiert ist, oder zusammen mit dem Stickstoffatom einen 5- bis 8-gliedrigen, gegebenenfalls ein weiteres Heteroatom oder einen ankondensierten Benzolring enthaltenden Heterocyclus,

$R^3$ Wasserstoff und $R^4$ ($C_1$-$C_7$)-Alkyl oder $R^3$ und $R^4$ zusammen eine Di-oder Trimethylengruppe, und

$R^5$ und $R^6$ je Wasserstoff, Halogen, Trifluormethyl, ($C_1$-$C_7$)-Alkoxy oder Nitro bedeuten,

wobei das mit $\gamma$ bezeichnete Kohlenstoffatom die S-Konfiguration aufweist, wenn $R^3$ von Wasserstoff verschieden ist,
und pharmazeutisch annehmbare Säureadditionssalze davon.

**2.** Verbindungen nach Anspruch 1, worin Q den Rest $Q^2$ bedeutet.

**3.** Verbindungen nach Anspruch 1, worin Q den Rest $Q^3$ bedeutet.

**4.** Verbindungen nach einem der Ansprüche 1 bis 3, worin $R^1$ und $R^2$ je ($C_1$-$C_7$)-Alkyl, ($C_1$-$C_7$)-Hydroxyalkyl oder ($C_3$-$C_6$)-Cycloalkyl-($C_1$-$C_7$)-Alkyl oder zusammen mit dem Stickstoffatom Piperidino oder Isoindolin-2-yl bedeuten.

**5.** Verbindungen nach einem der Ansprüche 1-4, worin A den Rest $A^1$ oder $A^2$, $R^5$ Wasserstoff, Chlor, Fluor, Trifluormethyl oder ($C_1$-$C_7$)-Alkoxy und $R^6$ Wasserstoff oder Fluor bedeuten.

**6.** Verbindungen nach einem der Ansprüche 1-5, worin $R^3$ Wasserstoff und $R^4$ Methyl oder $R^3$ und $R^4$ zusammen Dimethylen bedeuten.

**7.** Verbindung nach Anspruch 1, 3-(5-Dipropylaminomethyl-1,2,4-oxadiazol-3-yl)-8-fluoro-5-methyl-5,6-dihydro-4H-imidazo [1,5-a] [1,4]benzodiazepin-6-on.

**8.** Verbindung nach Anspruch 1, (S)-8-Chlor-1-(5-dipropylaminomethyl-1,2,4-oxadiazol-3-yl)-12, 12a-dihydro-9H, 11H-azeto[2,1-c]imidazo[1,5-a] [1,4]benzodiazepin-9-on.

**9.** Verbindung nach Anspruch 1, (S)-1-(5-Dipropylaminomethyl-1,2,4-oxadiazol-3-yl)-11,11a-dihydro-8H,10H-azeto [1,2-a]imidazo[5,1-c]thieno [3,2-e] [1,4]diazepin-8-on.

**10.** Verbindung nach Anspruch 1, 8-Fluor-5-methyl-3-[5-(piperidin-1-ylmethyl)-1,2,4-oxadiazol-3-yl]-5,6-dihydro-4H-imidazo[1,5-a] [1,4]benzodiazepin-6-on.

**11.** Verbindung nach Anspruch 1, (S)-1-(5-Diallylaminomethyl-1,2,4-oxadiazol-3-yl)-8-chlor-7-fluor-12,12a-dihydro-9H,11H-azeto[2,1-c]imidazo[1,5-a] [1,4]benzodiazepin-9-on.

**12.** Verbindung nach Anspruch 1, 3-(5-Dipropylaminomethyl-1,2,4-oxadiazol-3-yl)-5-methyl-5,6-dihydro-4H-imidazo [1,5-a] [1,4]benzodiazepin-6-on.

**13.** Verbindung nach Anspruch 1, 3-(5-Dipropylaminomethyl-1,3,4-oxadiazol-2-yl)-8-fluoro-5-methyl-5,6-dihydro-4H-imidazo [1,5-c] [1,4]benzodiazepin-6-on.

**14.** Verbindung nach Anspruch 1, 3-(5-Diethylamiomethyl-1,2,4-oxadiazol-3-yl)-8-fluoro-5-methyl-5,6-dihydro-4H-imidazo[1,5-a] [1,4]benzodiazepin-6-on.

**15.** Verbindung nach Anspruch 1, (S)-8-Chlor-1-[5-(piperidin-1-yl)methyl-1,2,4-oxadiazol-3-yl]-12,12a-dihydro-9H, 11H-azeto[2,1-c]imidazo[1,5-a] [1,4]benzodiazepin-9-on.

**16.** Verbindung nach Anspruch 1, 7-Fluor-5-methyl-3-(5-dipropylaminomethyl-1,2,4-oxadiazol-3-yl)-5,6-dihydro-4H-imidazo [1,5-a] [1,4]benzodiazepin-6-on.

**17.** Verbindung nach Anspruch 1, 7-Chlor-3-(5-diäthylaminomethyl-1,2,4-oxadiazol-3-yl)-5-methyl-5,6-dihydro-4H-imidazo[1,5-a][1,4] benzodiazepin-6-on.

**18.** Verbindung nach Anspruch 1, 7-Chlor-3-(5-dipropylaminomethyl-1,3,4-oxadiazo-2-yl)-5-methyl-5,6-dihydro-4H-imidazo[1,5-a] [1,4]benzodiazepin-6-on.

**19.** Verbindung nach Anspruch 1, (S)-1-(5-Diäthylaminomethyl-1,2,4-oxadiazol-3-yl)-11,11a-dihydro-8H,10H-azeto [1,2-a]imidazo[5,1-c]thieno[3,2-e] [1,4]diazepin-8-on.

**20.** Verbindung nach Anspruch 1, (S)-1-(5-Dibutylaminomethyl-1,3,4-oxadiazol-2-yl)-11,11a-dihydro-8H,10H-azeto [1,2-a] imidazo [5,1-c] thieno [3,2-e] [1,4]diazepin-8-on.

**21.** Verbindung nach Anspruch 1, 3-(5-Diisopropylaminomethyl-1,2,4-oxadiazol-3-yl)-5-methyl-5,6-dihydro-4H-imidazo [1,5-a] [1,4]benzodiazepin-6-on.

**22.** Verbindung nach Anspruch 1, 3-(5-Diisopropylaminomethyl-1,2,4-oxadiazol-3-yl)-8-fluor-5-methyl-5,6-dihydro-4H-imidazo [1,5-a] [1,4]benzodiazepin-6-on.

**23.** Verbindungen nach Anspruch 1, (S)-1-(5-Diallylaminomethyl-1,2,4-oxadiazol-3-yl)-8-chlor-12,12a-dihydro-9H, 11H-azeto [2,1-c] imidazo [1,5-a] [1,4]benzodiazepin-9-on;

(S)-1-(5-Diallylaminomethyl-1,2,4-oxadiazol-3-yl)-8-trifluormethyl-12,12a-dihydro-9H,11H-azeto [2,1-c]imidazo[1,5-a] [1,4]benzodiazepin-9-on;

3-(5-Diallylaminomethyl-1,2,4-oxadiazol-3-yl)-8-fluor-5-methyl-5,6-dihydro-4H-imidazo [1,5-a] [1,4]benzodiazepin-6-on;

8-Fluor-3-(5-isoindolin-2-ylmethyl-1,2,4-oxadiazol-3-yl)-5-methyl-5,6-dihydro-4H-imidazo[1,5-a] [1,4]benzodiazepin-6-on; und

(S)-1-(5-Diallylaminomethyl-1,2,4-oxazdiazol-3-yl)-11,11a-dihydro-8H,10H-azeto [1,2-a]imidazo [5,1-c]thieno [3,2-e] [1,4]diazepin-8-on.

**24.** Verbindungen nach Anspruch 1,3-(5-Dipropylaminomethyl-1,2,4-oxadiazol-3-yl)-5-methyl-5,6-dihydro-4H-imidazo [1,5-a]thieno[2,3-fl [1,4]diazepin-6-on;

3-(5-Dipropylaminomethyl-1,2,4-oxadiazol-3-yl)-5-methyl-7-trifluormethyl-5,6-dihydro-4H-imidazo[1,5-a]

[1,4]benzodiazepin-6-on; und

(S)-8-Chlor-1-(5-dipropylaminomethyl-1,3,4-oxadiazol-2-yl)-12,12a-dihydro-9H,11H-azeto[2,1-c]imidazo [1,5-a][1,4]benzodiazepin-9-on.

**25.** Arzneimittel, insbesondere anxiolytisches und/oder antikonvulsives und/oder muskelrelaxierendes und/oder sedativ-hypnotisches Mittel, enthaltend eine Verbindung nach einem der Ansprüche 1-24 und einen therapeutisch inerten Träger.

**26.** Verfahren zur Herstellung von Verbindungen nach einem der Ansprüche 1-24, dadurch gekennzeichnet, dass man

a) ein reaktionsfähiges funktionelles Derivat einer Carbonsäure der allgemeinen Formel

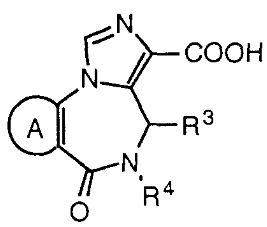 II oder 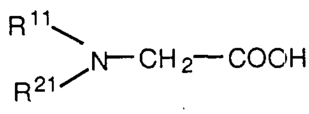 III

mit einem Amidoxim der allgemeinen Formel

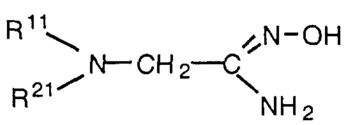 IV bzw. 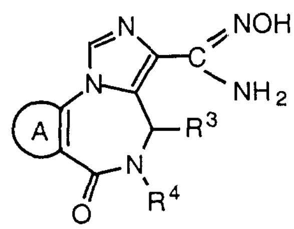 V

oder mit einem Hydrazid der allgemeinen Formel

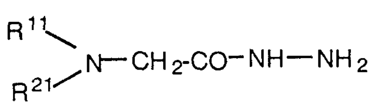 VI bzw. 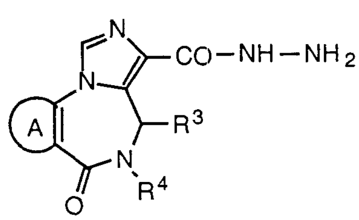 VII

wobei A, $R^3$ und $R^4$ die in Anspruch 1 angegebene Bedeutung besitzen und $R^{11}$ und $R^{21}$ je $(C_1\text{-}C_7)$-Alkyl, $(C_1\text{-}C_7)$-Alkenyl, $(C_1\text{-}C_7)$-Alkinyl, $(C_1\text{-}C_7)$-Alkoxy-$(C_1\text{-}C_7)$-Alkyl, $(C_3\text{-}C_6)$-Cycloalkyl, $(C_3\text{-}C_6)$-Cycloalkyl-$(C_1\text{-}C_7)$-Alkyl, Di-$(C_1\text{-}C_7)$-Alkylamino-$(C_1\text{-}C_7)$-Alkyl oder Aryl-$(C_1\text{-}C_7)$-Alkyl oder zusammen mit dem Stickstoffatom einen 5-bis 8-gliedrigen, gegebenenfalls ein weiteres Heteroatom oder einen ankondensierten Benzolring enthaltenden Heterocyclus bedeuten,
umsetzt, oder

b) eine Verbindung der allgemeinen Formel

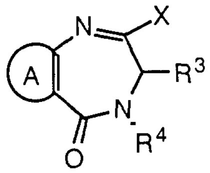 VIII

worin A, $R^3$ und $R^4$ die in Anspruch 1 angegebene Bedeutung besitzen und X eine Abgangsgruppe bedeutet,
in Gegenwart einer Base mit einem Isonitril der allgemeinen Formel

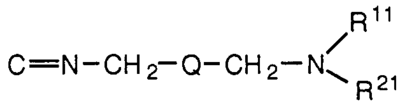 IX

worin Q die in Anspruch 1 angegebene und $R^{11}$ und $R^{21}$ obige Bedeutung besitzen,
umsetzt, oder

c) eine Verbindung der allgemeinen Formel

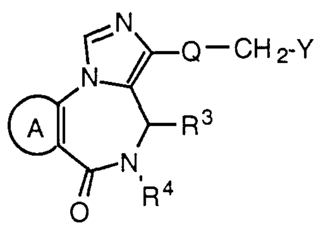 X

worin A, Q, $R^3$ und $R^4$ die in Anspruch 1 angegebene Bedeutung besitzen und Y eine Abgangsgruppe bedeutet,
mit einem Amin der allgemeinen Formel

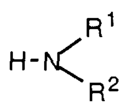 XI

worin $R^1$ und $R^2$ die in Anspruch 1 angegebene Bedeutung besitzen, umsetzt, oder

d) aus einer Verbindung der allgemeinen Formel

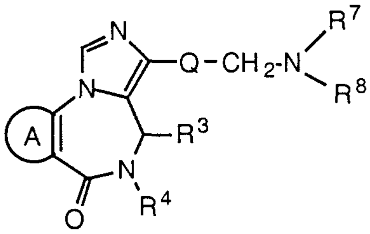 XII

worin A, Q, $R^3$ und $R^4$ die in Anspruch 1 angegebene Bedeutung, besitzen und $R^7$ eine Schutzgruppe, geschütztes ($C_1$-$C_7$)-Hydroxyalkyl, geschütztes Amino-($C_1$-$C_7$)-Alkyl oder geschütztes ($C_1$-$C_7$)-Alkylamino-($C_1$-$C_7$)-Alkyl und $R^8$ Wasserstoff, ($C_1$-$C_7$)-Alkyl, ($C_1$-$C_7$)-Alkenyl, ($C_1$-$C_7$)-Alkinyl, geschütztes ($C_1$-$C_7$)-Hydro-

xyalkyl, $(C_1\text{-}C_7)$-Alkoxy-$(C_1\text{-}C_7)$-Alkyl, $(C_3\text{-}C_6)$-Cycloalkyl, $(C_3\text{-}C_6)$-Cycloalkyl-$(C_1\text{-}C_7)$-Alkyl, geschütztes Amino-$(C_1\text{-}C_7)$-Alkyl, geschütztes $(C_1\text{-}C_7)$-Alkylamino-$(C_1\text{-}C_7)$-Alkyl, Di-$(C_1\text{-}C_7)$-Alkylamino-$(C_1\text{-}C_7)$-Alkyl oder Aryl-$(C_1\text{-}C_7)$-Alkyl oder $R^7$ und $R^8$ zusammen eine Schutzgruppe bedeuten,
die Schutzgruppe (n) abspaltet, oder

e) eine Verbindung der allgemeinen Formel

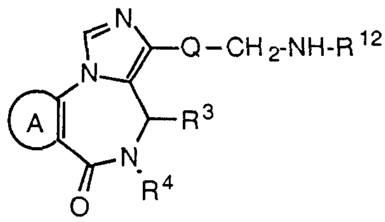

Ia

worin A, Q, $R^3$ und $R^4$ die in Anspruch 1 angegebene Bedeutung besitzen und $R^{12}$ Wasserstoff, $(C_1\text{-}C_7)$-Alkyl, $(C_1\text{-}C_7)$-Alkenyl-$(C_1\text{-}C_7)$-Alkinyl, $(C_1\text{-}C_7)$-Hydroxyalkyl, $(C_1\text{-}C_7)$-Alkoxy-$(C_1\text{-}C_7)$-Alkyl, $(C_3\text{-}C_6)$-Cycloalkyl, $(C_3\text{-}C_6)$-Cycloalkyl-$(C_1\text{-}C_7)$-Alkyl, Amino-$(C_1\text{-}C_7)$-Alkyl, $(C_1\text{-}C_7)$-Alkylamino-$(C_1\text{-}C_7)$-Alkyl, Di-$(C_1\text{-}C_7)$-Alkylamino-$(C_1\text{-}C_7)$-Alkyl oder Aryl-$(C_1\text{-}C_7)$-Alkyl bedeutet,
entsprechend N-alkyliert, oder

f) eine Verbindung der allgemeinen Formel

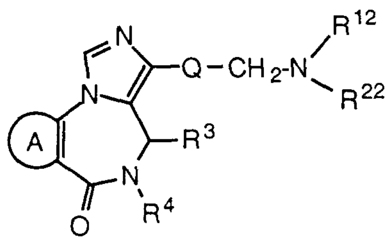

Ib

worin A, Q, $R^3$ und $R^4$ die in Anspruch 1 angegebene und $R^{12}$ obige Bedeutung besitzen und $R^{22}$ $(C_1\text{-}C_7)$-Alkenyl oder $(C_1\text{-}C_7)$-Alkinyl bedeutet,
reduziert und erwünschtenfalls

g) eine Verbindung der allgemeinen Formel I in ein pharmazeutisch anwendbares Säureadditionssalz überführt.

**27.** Verbindungen gemäss einem der Ansprüche 1-24 zur Anwendung als therapeutische Wirkstoffe, insbesondere als anxiolytische und/oder antikonvulsive und/oder muskelrelaxierende und/oder sedativ-hypnotische Wirkstoffe.

**28.** Verwendung von Verbindungen nach einem der Ansprüche 1-24 zur Herstellung von anxiolytischen und/oder antikonvulsiven und/oder muskelrelaxierenden und/oder sedativ-hypnotischen Mitteln.

**Claims**

**1.** Imidazodiazepines of the general formula

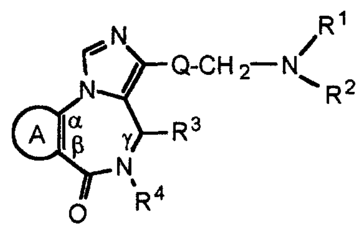 I

wherein A and the two carbon atoms denoted by α and β together signify one of the residues

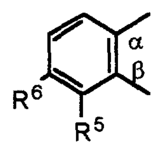 , 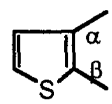 and 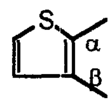

($A^1$) ($A^2$) ($A^3$)

Q signifies one of the residues

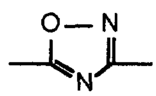 , 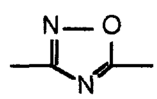 and 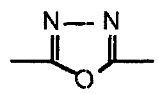

($Q^1$) ($Q^2$) ($Q^3$)

$R^1$ and $R^2$ each signify hydrogen, ($C_1$-$C_7$)-alkyl, ($C_1$-$C_7$)-alkenyl, ($C_1$-$C_7$)-alkynyl, ($C_1$-$C_7$)-hydroxyalkyl, ($C_1$-$C_7$)-alkoxy-($C_1$-$C_7$)-alkyl, ($C_3$-$C_6$)-cycloalkyl, ($C_3$-$C_6$)-cycloalkyl-($C_1$-$C_7$)-alkyl, amino-($C_1$-$C_7$)-alkyl, ($C_1$-$C_7$)-alkylamino-($C_1$-$C_7$)-alkyl, di-($C_1$-$C_7$)-alkylamino-($C_1$-$C_7$)-alkyl or phenyl-($C_1$-$C_7$)-alkyl, whereby the phenyl residue is optionally substituted by halogen, trifluoromethyl, ($C_1$-$C_7$)-alkyl or ($C_1$-$C_7$)-alkoxy, or together with the nitrogen atom signify a 5- to 8-membered heterocycle optionally containing a further hetero atom or a fused benzene ring,

$R^3$ signifies hydrogen and $R^4$ signifies ($C_1$-$C_7$)-alkyl or $R^3$ and $R^4$ together signify a di- or trimethylene group and

$R^5$ and $R^6$ each signify hydrogen, halogen, trifluoromethyl, ($C_1$-$C_7$)-alkoxy or nitro, whereby the carbon atom denoted by γ has the S-configuration when $R^3$ is different from hydrogen,

and pharmaceutically acceptable acid addition salts thereof.

**2.** Compounds according to claim 1, wherein Q signifies the residue $Q^2$.

**3.** Compounds according to claim 1, wherein Q signifies the residue $Q^3$.

**4.** Compounds according to any one of claims 1 to 3, wherein $R^1$ and $R^2$ each signify ($C_1$-$C_7$)-alkyl, ($C_1$-$C_7$)-hydroxyalkyl or ($C_3$-$C_6$)-cycloalkyl-($C_1$-$C_7$)-alkyl or together with the nitrogen atom signify piperidino or isoindolin-2-yl.

**5.** Compounds according to any one of claims 1-4, wherein A signifies the residue $A^1$ or $A^2$, $R^5$ signifies hydrogen, chlorine, fluorine, trifluoromethyl or ($C_1$-$C_7$)-alkoxy and $R^6$ signifies hydrogen or fluorine.

**6.** Compounds according to any one of claims 1-5, wherein $R^3$ signifies hydrogen and $R^4$ signifies methyl or $R^3$ and

$R^4$ together signify dimethylene.

7. The compound according to claim 1, 3-(5-dipropylaminomethyl-1,2,4-oxadiazol-3-yl)-8-fluoro-5-methyl-5,6-dihydro-4H-imidazo [1,5-a][1,4] benzodiazepin-6-one.

8. The compound according to claim 1, (S)-8-chloro-1-(5-dipropylaminomethyl-1,2,4-oxadiazol-3-yl)-12,12a-dihydro-9H,11H-azeto[2,1-c]imidazo[1,5-a][1,4]benzodiazepin-9-one.

9. The compound according to claim 1, (S)-1-(5-dipropylaminomethyl-1,2,4-oxadiazol-3-yl)- 11,11a-dihydro-8H,10H-azeto[1,2-a] imidazo[5,1-c]thieno[3,2-e] [1,4]diazepin-8-one.

10. The compound according to claim 1, 8-fluoro-5-methyl-3-[5-(piperidin-1-ylmethyl)-1,2,4-oxadiazol-3-yl]-5,6-dihydro-4H-imidazo[1,5-a][1,4]benzodiazepin-6-one.

11. The compound according to claim 1, (S)-1-(5-diallylaminomethyl-1,2,4-oxadiazol-3-yl)-8-chloro-7-fluoro-12,12a-dihydro-9H,11H-azeto [2,1-c]imidazo[1,5-a]-[1,4]benzodiazepin-9-one.

12. The compound according to claim 1, 3-(5-dipropylaminomethyl-1,2,4-oxadiazol-3-yl)-5-methyl-5,6-dihydro-4H-imidazo[1,5-a][1,4]benzodiazepin-6-one.

13. The compound according to claim 1, 3-(5-dipropylaminomethyl-1,3,4-oxadiazol-2-yl)-8-fluoro-5-methyl-5,6-dihydro-4H-imidazo[1,5-c] [1,4]benzodiazepin-6-one.

14. The compound according to claim 1, 3-(5-diethylaminomethyl-1,2,4-oxadiazol-3-yl)-8-fluoro-5-methyl-5,6-dihydro-4H-imidazo[1,5-a][1,4]benzodiazepin-6-one.

15. The compound according to claim 1, (S)-8-chloro-1-[5-(piperidin-1-yl)methyl-1,2,4-oxadiazol-3-yl]-12,12a-dihydro-9H,11H-azeto[2,1-c]imidazo[1,5-a][1,4]benzodiazepin-9-one.

16. The compound according to claim 1, 7-fluoro-5-methyl-3-(5-dipropylaminomethyl-1,2,4-oxadiazol-3-yl)-5,6-dihydro-4H-imidazo[1,5-a] [1,4]benzodiazepin-6-one.

17. The compound according to claim 1, 7-chloro-3-(5-diethylaminomethyl-1,2,4-oxadiazol-3-yl)-5-methyl-5,6-dihydro-4H-imidazo[1,5-a] [1,4]benzodiazepin-6-one.

18. The compound according to claim 1, 7-chloro-3-(5-dipropylaminomethyl-1,3,4-oxadiazo-2-yl)-5-methyl-5,6-dihydro-4H-imidazo[1,5-a] [1,4]benzodiazepin-6-one.

19. The compound according to claim 1, (S)-1-(5-diethylaminomethyl-1,2,4-oxadiazol-3-yl)-11,11a-dihydro-8H,10H-azeto[1,2-a]imidazo[5,1-c]thieno[3,2-e] [1,4] diazepin-8-one.

20. The compound according to claim 1, (S)-1-(5-dibutylaminomethyl-1,3,4-oxadiazol-2-yl)-11,11a-dihydro-8H,10H-azeto[1,2-a]imidazo[5,1-c]thieno[3,2-e][1,4]diazepin-8-one.

21. The compound according to claim 1, 3-(5-diisopropylarninomethyl-1,2,4-oxadiazol-3-yl)-5-methyl-5,6-dihydro-4H-imidazo[1,5-a][1,4]benzodiazepin-6-one.

22. The compound according to claim 1, 3-(5-diisopropylaminomethyl-1,2,4-oxadiazol-3-yl) -8-fluoro-5-methyl-5,6-dihydro-4H-imidazo[1,5-a] [1,4]benzodiazepin-6-one.

23. The compounds according to claim 1, (S)-l-(5-diallylaminomethyl-1,2,4-oxadiazol-3-yl) -8-chloro-12,12a-dihydro-9H,11H-azeto[2,1-c]imidazo[1,5-a][1,4]benzodiazepin-9-one;

(S)-1-(5-diallylaminomethyl-1,2,4-oxadiazol-3-yl) -8-trifluoromethyl-12,12a-dihydro-9H,11H-azeto[2,1-c]imidazo[1,5-a][1,4]benzodiazepin-9-one;
3-(5-diallylaminomethyl-1,2,4-oxadiazol-3-yl)-8-fluoro-5-methyl-5,6-dihydro-4H-imidazo[1,5-a][1,4]benzodiazepin-6-one;
8-fluoro-3-(5-isoindolin-2-ylmethyl-1,2,4-oxadiazol-3-yl)-5-methyl-5,6-dihydro-4H-imidazo [1,5-a] [1,4]benzo-

diazepin-6-one; and
(S)-1-(5-diallylaminomethyl-1,2,4-oxazdiazol-3-yl)-11,11 a-dihydro-8H,10H-azeto [1,2-a]imidazo [5,1-c]thieno [3,2-e] [1,4] diazepin-8-one.

**24.** The compounds according to claim 1, 3-(5-dipropylaminomethyl-1,2,4-oxadiazol-3-yl)-5-methyl-5,6-dihydro-4H-imidazo[1,5-a]thieno[2,3-f][1,4] diazepin-6-one;

3-(5-dipropylaminomethyl-1,2,4-oxadiazol-3-yl)-5-methyl-7-trifluoromethyl-5,6-dihydro-4H-imidazo[1,5-a][1,4]benzodiazepin-6-one; and
(S)-8-chloro-1-(5-dipropylaminomethyl-1,3,4-oxadiazol-2-yl)-12,12a-dihydro-9H,11H-azeto[2,1-c] imidazo [1,5-a][1,4]benzodiazepin-9-one.

**25.** A medicament, especially an anxiolytic and/or anticonvulsant and/or muscle relaxant and/sedative-hypnotic medicament, containing a compound according to any one of claims 1-24 and a therapeutically inert carrier.

**26.** A process for the manufacture of compounds according to any one of claims 1-24, characterized by

a) reacting a reactive functional derivative of a carboxylic acid of the general formula

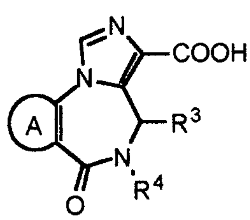 II or 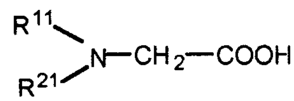 III

with an amidoxime of the general formula

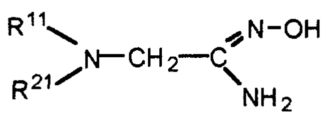 or, respectively, 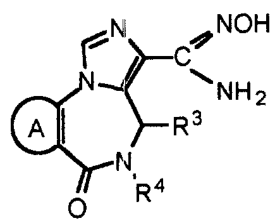

IV V

or with a hydrazide of the general formula

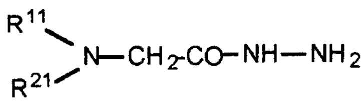 or, respectively, 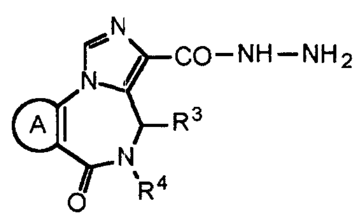

VI VII

wherein A, $R^3$ and $R^4$ have the significance given in claim 1 and $R^{11}$ and $R^{21}$ each signify ($C_1$-$C_7$)-alkyl, ($C_1$-$C_7$)-alkenyl, ($C_1$-$C_7$)-alkynyl, ($C_1$-$C_7$)-alkoxy-($C_1$-$C_7$)-alkyl, ($C_3$-$C_6$)-cycloalkyl, ($C_3$-$C_6$)-cycloalkyl-($C_1$-$C_7$)-

alkyl, di-($C_1$-$C_7$)-alkylamino-($C_1$-$C_7$)-alkyl or aryl-($C_1$-$C_7$)-alkyl or together with the nitrogen atom signify a 5- to 8-membered heterocycle optionally containing a further hetero atom or a fused benzene ring, or

b) reacting a compound of the general formula

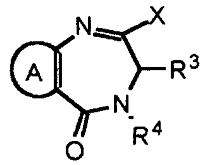

VIII

wherein A, $R^3$ and $R^4$ have the significance given in claim 1 and X signifies a leaving group, in the presence of a base with an isonitrile of the general formula

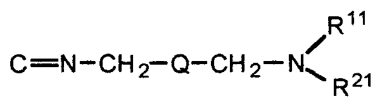

IX

wherein Q has the significance given in claim 1 and $R^{11}$ and $R^{21}$ have the above significance, or

c) reacting a compound of the general formula

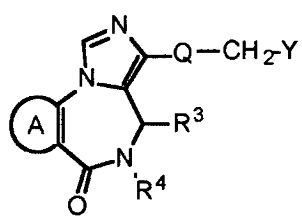

X

wherein A, Q, $R^3$ and $R^4$ have the significance given in claim 1 and Y signifies a leaving group, with an amine of the general formula

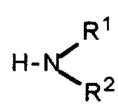

XI

wherein $R^1$ and $R^2$ have the significance given in claim 1, or

d) cleaving off the protecting group(s) from a compound of the general formula

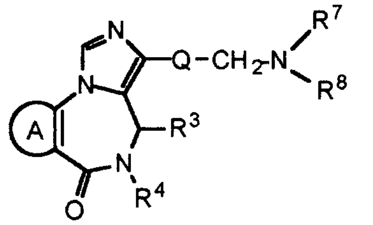

XII

wherein A, Q, $R^3$ and $R^4$ have the significance given in claim 1 and $R^7$ signifies a protecting group, protected ($C_1$-$C_7$)-hydroxyalkyl, protected amino-($C_1$-$C_7$)-alkyl or protected ($C_1$-$C_7$)-alkylamino-($C_1$-$C_7$)-alkyl and $R^8$ signifies hydrogen, ($C_1$-$C_7$)-alkyl, ($C_1$-$C_7$)-alkenyl, ($C_1$-$C_7$)-alkynyl, protected ($C_1$-$C_7$)-hydroxyalkyl, ($C_1$-$C_7$)-alkoxy-($C_1$-$C_7$)-alkyl, ($C_3$-$C_6$)-cycloalkyl, ($C_3$-$C_6$)-cycloalkyl-($C_1$-$C_7$)-alkyl, protected amino-($C_1$-$C_7$)-alkyl, protected ($C_1$-$C_7$)-alkylamino-($C_1$-$C_7$)-alkyl, di-($C_1$-$C_7$)-alkylamino-($C_1$-$C_7$)-alkyl or aryl-($C_1$-$C_7$)-alkyl or R7 and R8 together signify a protecting group,
or

e) appropriately N-alkylating a compound of the general formula

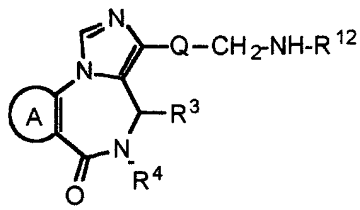

Ia

wherein A, Q, $R^3$ and $R^4$ have the significance given in claim 1 and $R^{12}$ signifies hydrogen, ($C_1$-$C_7$)-alkyl, ($C_1$-$C_7$)-alkenyl-($C_1$-$C_7$)-alkynyl, ($C_1$-$C_7$)-hydroxyalkyl, ($C_1$-$C_7$)-alkoxy-($C_1$-$C_7$)-alkyl, ($C_3$-$C_6$)-cycloalkyl, ($C_3$-$C_6$)-cycloallcyl-($C_1$-$C_7$)-alkyl, amino-($C_1$-$C_7$)-alkyl, ($C_1$-$C_7$)-alkylamino-($C_1$-$C_7$)-alkyl, di-($C_1$-$C_7$)-alkylamino-($C_1$-$C_7$)-alkyl or aryl-($C_1$-$C_7$)-alkyl,
or

f) reducing a compound of the general formula

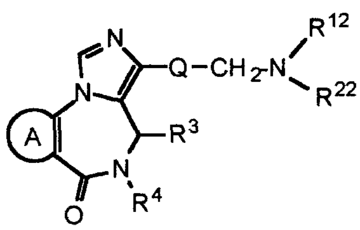

Ib

wherein A, Q, $R^3$ and $R^4$ have the significance given in claim 1 and $R^{12}$ has the above significance and $R^{22}$ signifies ($C_1$-$C_7$)-alkenyl or ($C_1$-$C_7$)-alkynyl,
and, if desired,

g) converting a compound of general formula I into a pharmaceutically usable acid addition salt.

**27.** Compounds according to any one of claims 1-24 for use as therapeutically active substances, especially as anxiolytic and/or anticonvulsant and/or muscle relaxant and/or sedative-hypnotic active substances.

**28.** The use of compounds according to any one of claims 1-24 for the production of anxiolytic and/or anticonvulsant and/or muscle relaxant and/or sedative-hypnotic medicaments.

**Revendications**

**1.** Imidazodiazépines de formule générale

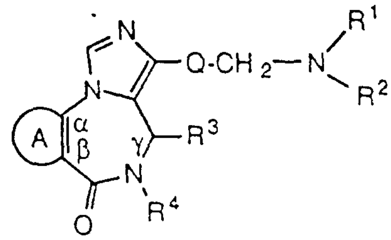

I

dans laquelle R représente avec les deux atomes de carbone désignés par α et β l'un des radicaux

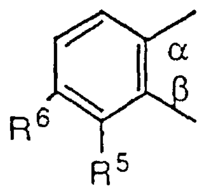

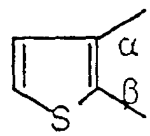

et

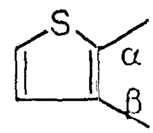

$(A^1)$ $(A^2)$ $(A^3)$

Q représente l'un des radicaux

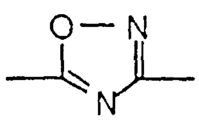

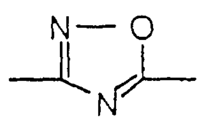

et

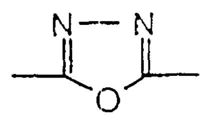

$(Q^1)$ $(Q^2)$ $(Q^3)$

$R^1$ et $R^2$ représentent chacun un hydrogène, un alkyle en $C_1$ à $C_7$, un alcényle en $C_1$ à $C_7$, un alcynyle en $C_1$ à $C_7$, un hydroxyalkyle en $C_1$ à $C_7$, un alcoxy en $C_1$ à $C_7$-alkyle en $C_1$ à $C_7$, un cycloalkyle en $C_3$ à $C_6$, un cycloalkyle en $C_3$ à $C_6$-alkyle en $C_1$ à $C_7$, un aminoalkyle en $C_1$ à $C_7$, un alkylamino en $C_1$ à $C_7$-alkyle en $C_1$ à $C_7$, un dialkylamino en $C_1$ à $C_7$-alkyle en $C_1$ à $C_7$ ou un phénylalkyle en $C_1$ à $C_7$, où le radical phényle est éventuellement substitué par un halogène, un trifluorométhyle, un alkyle en $C_1$ à $C_7$ ou un alcoxy en $C_1$ à $C_7$, ou, forment avec l'atome d'azote, un hétérocycle à 5 à 8 chaînons, contenant le cas échéant un autre hétéroatome ou un noyau benzénique condensé,

$R^3$ représente un hydrogène et $R^4$ représente un alkyle en $C_1$ à $C_7$, ou $R^3$ et $R^4$ représentent ensemble un groupe di- ou triméthylène, et

$R^5$ et $R^6$ représentent chacun un hydrogène, un halogène, un trifluorométhyle, un alcoxy en $C_1$ à $C_7$ ou un nitro,

où l'atome de carbone désigné par γ présente la configuration S lorsque $R^3$ est différent d'un hydrogène,

et leurs sels d'addition d'acide pharmaceutiquement acceptables.

**2.** Composés selon la revendication 1, dans lesquels Q représente le radical $Q^2$.

**3.** Composés selon la revendication 1, dans lesquels Q représente un radical $Q^3$.

**4.** Composés selon les revendications 1 à 3, dans lesquels $R^1$ et $R^2$ représentent chacun un alkyle en $C_1$ à $C_7$, un

hydroxyalkyle en $C_1$ à $C_7$ ou un cycloalkyle en $C_3$ à $C_6$-alkyle en $C_1$ à $C_7$, ou représentent ensemble avec l'atome d'azote un pipéridino ou un isoindolin-2-yle;

5. Composés selon l'une des revendications 1 à 4, dans lesquels A représente un radical $A^1$ ou $A^2$, $R^5$ représente un hydrogène, un chlore, un fluor, un tri-fluorométhyle ou un alcoxy en $C_7$ et $R^6$ représente un hydrogène ou un fluor.

6. Composés selon l'une des revendications 1 à 5, dans lesquels $R^3$ représente un hydrogène et $R^4$ un méthyle, ou $R^3$ et $R^4$ représentent ensemble un diméthylène.

7. Composé selon la revendication 1, la 3-(5-dipropylaminométhyl-1,2,4-oxadiazol-3-yl)-8-fluoro-5-méthyl-5,6-dihydro-4H-imidazo[1,5-a] [1,4]benzodiazépine-6-one.

8. Composé selon la revendication 1, la (S)-8-chloro-1-(5-dipropylaminométhyl-1,2,4-oxadiazol-3-yl)-12,12a-dihydro-9H,11H-azéto[2,1-c]imidazo-[1,5-a][1,4]benzodiazépine-9-one.

9. Composé selon la revendication 1, la (S)-1-(5-dipropylaminométhyl-1,2,4-oxadiazol-3-yl)- 11,11a-dihydro-8H,10H-azéto[1,2-a]imidazo[5,1-c]thiéno[3, 2-e] [1,4]diazépine-8-one.

10. Composé selon la revendication 1, la 1,8-fluoro-5-méthyl-3-[5-(pipéridin-1-ylméthyl)-1,2,4-oxadiazol-3-yl]-5,6-dihydro-4H-imidazo[1,5-a][1,4]benzodiazépine-6-one.

11. Composé selon la revendication 1, la (S)-1-5-diallylaminométhyl-1,2,4-oxadiazol-3-yl)-8-chloro-7-fluoro-12,12a-dihydro-9H,11H-azéto[2,1-c]-imidazo[1,5-a] [1,4]benzodiazépine-9-one.

12. Composé selon la revendication 1, la 3-(5-dipropylaminométhyl-1,2,4-oxadiazol-3-yl)-5-méthyl-5,6-dihydro-4H-imidazo[1,5-a] [1,4]benzodiazépine-6-one.

13. Composé selon la revendication 1, la 3-(5-dipropyl-amlnométhyl-1,3,4-oxadiazol-2-yl)-8-fluoro-5-méthyl-5,6-dihydro-4H-imidazo[1,5-c][1,4]benzodiazépine-6-one.

14. Composé selon la revendication 1, la 3-(5-diéthylaminométhyl-1,2,4-oxadiazol-3-yl)-8-fluoro-5-méthyl-5,6-dihydro-4H-imidazo[1,5-a] [1,4]benzodiazépine-6-one.

15. Composé selon la revendication 1, la (S)-8-chloro-11-[5-(pipéridin-1-yl)méthyl-1,2,4-oxadiazol-3-yl]-12,12a-dihydro-9H,11H-azéto[2,1-c]-imidazo[1,5-a][1,4]benzodiazépine-9-one.

16. Composé selon la revendication 1, la 7-fluoro-5-méthyl-3-(5-dipropylaminométhyl-1,2,4-oxadiazol-3-yl)-5,6-dihydro-4H-imidazo[1,5-a]benzodiazépine-6-one.

17. Composé selon la revendication 1, la 7-chloro-3-(5-diéthylaminométhyl-1,2,4-oxadiazol-3-yl)-5-méthyl-5,6-dihydro-4H-imidazo[1,5-a][1,4]benzodiazépine-6-one.

18. Composé selon la revendication 1, 7-chloro-3-(5-dipropylaminométhyl-1,3,4-oxadiazo-2-yl)-5-méthyl-5,6-dihydro-4H-imidazo[1,5-a][1,4]benzodiazépine-6-one.

19. Composé selon la revendication 1, la (S)-1-(5-diéthylaminométhyl-1,2,4-oxadiazol-3-yl)-11,11a-dihydro-8H,10H-azéto[1,2-a]imidazo[5,1-c]thiéno-[3,2-e]diazépine-8-one.

20. Composé selon la revendication 1, la (S)-1-(5-dibutylaminométhyl-1,3,4-oxadiazol-2-yl)-11,11a-dihydro-8H,10H-azéto[1,2a]-imidazo[5,1c]-thiéno[3,2-e]diazépine-8-one.

21. Composé selon la revendication 1, la 3-(5-diisopropylaminométhyl-1,2,4-oxadiazol-3-yl)-5-méthyl-5,6-dihydro-4H-imidazo[1,5-a][1,4]benzodiazépine-6-one.

22. Composé selon la revendication 1, la 3-(5-diiso-propylaminométhyl-1,2, 4-oxadiazol-3-yl)-8-fluoro-5-méthyl-5,6-dihydro-4H-imidazo[1,5-a][1,4]-benzodiazépine-6-one.

23. Composés selon la revendication 1, la (S)-1-(5-diallylaminométhyl-1,2,4-oxadiazol-3-yl)-8-chloro-12,12a-dihydro-9H,11H-azéto[2,1-c]imidazo[1,5-a][1,4]benzodiazépine-9-one;

(S)-1-(5-diallylaminométhyl-1,2,4-oxadiazol-3-yl)-8-trifluorométhyl-12,12a-dihydro-9H-11H, azéto[2,1-c]imidazo[1,5-a][1,4]benzodiazépine-9-one ;

3-(5-diallylaminométhyl-1,2,4-oxadiazol-3-yl)-8-fluoro-5-méthyl-5,6-dihydro-4H-imidazo[1,5-a][1,4][benzodiazépine-6-one

8-fluoro-3-(5-isoindolin-2-ylméthyl-1,2,4-oxadiazol-3-yl)-5-méthyl-5,6-dihydro-4H-imidazo[1,5-a][1,4]benzodiazépine-6-one; et

(S)-1-(5-diallylaminométhyl-1,2,4-oxadiazol-3-yl)-11,11a-dihydro-8H,10H-azéto[1,2-a]imidazo[5,1-c]thiéno[3,2-e][1,4]diazépine-8-one.

**24.** Composés selon la revendication 1, 3-(5-dipropylaminométhyl-1,2,4-oxa-diazol-3-yl)-5-méthyl-5,6-dihydro-4H-imidazo[1,5-a]thiéno[2,3-f][1,4]di-azépine-6-one ;

3-(5-dipropylaminométhyl-1,2,4-oxadiazol-3-yl)-5-méthyl-7-trifluorométhyl-5,6-dihydro-4H-imidazo[1,5-a][1,4]benzodiazépine-6-one ; et

(S)-8-chloro-1-(5-dipropylaminométhyl-1,3,4-oxadiazol-2-yl)-12,12a-dihydro-9H,11H-azéto[2,1-c]imidazo[1,5-a][1,4]benzodiazépine-9-one.

**25.** Médicament, en particulier agent à action anxiolytique et/ou anti-convulsive et/ou de relaxation musculaire et/ou sédative-hypnotique, contenant un composé selon l'une des revendications 1 à 24 et un support thérapeutiquement inerte.

**26.** Procédé de préparation de composés selon l'une des revendications 1 à 24, caractérisé en ce que

a) on fait réagir un dérivé fonctionnel réactif d'un acide carboxylique de formule générale

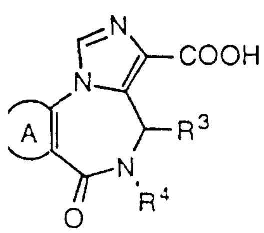 II ou 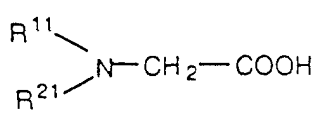 II

avec une amidoxime de formule générale

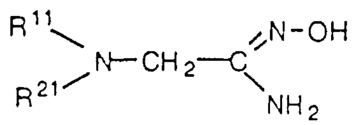 IV

ou selon les cas

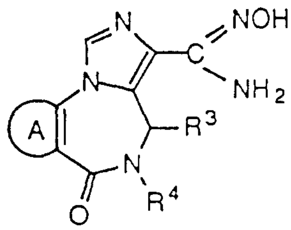

V

ou avec un hydrazide de formule générale

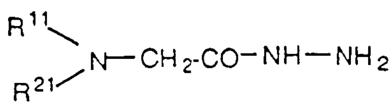

VI

ou selon les cas

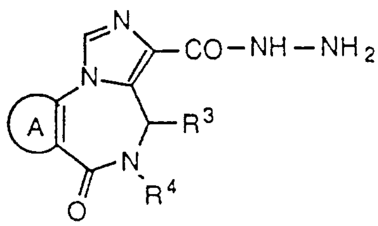

VII

dans lesquelles A, $R^3$ et $R^4$ ont la signification indiquée dans la revendication 1 et $R^{11}$ et $R^{21}$ représentent chacun un alkyle en $C_1$ à $C_7$, un alcényle en $C_1$ à $C_7$, un alcynyle en $C_1$ à $C_7$, un alcoxy en $C_1$ à $C_7$-alkyle en $C_1$ à $C_7$, un cycloalkyle en $C_3$ à $C_6$, un cycloalkyle en $C_3$ à $C_6$-alkyle en $C_1$ à $C_7$, un dialkylamino en $C_1$ à $C_7$-alkyle en $C_1$ à $C_7$, ou un arylalkyle-alkyle en $C_1$ à $C_7$ ou représentent ensemble avec l'atome d'azote un hétérocycle à 5 à 8 chaînons, contenant le cas échéant un autre hétéroatome ou un noyau benzénique condensé, ou,

b) on fait réagir un composé de formule générale

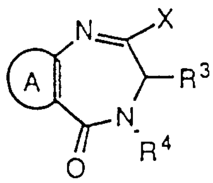

VIII

dans laquelle A, $R^3$ et $R^4$ ont la signification indiquée dans la revendication 1 et X représente un groupe partant, en présence d'une base avec un isonitrile de formule générale

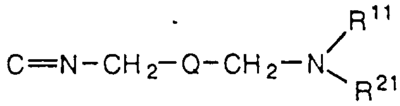

IX

dans laquelle Q a la signification indiquée dans la revendication 1 et $R^{11}$ et $R^{21}$ ont la signification indiquée ci-dessus, ou

c) on fait réagir un composé de formule générale

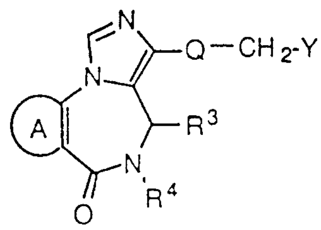

X

dans laquelle A, Q, $R^3$ et $R^4$ ont la signification indiquée dans la revendication 1, et Y représente un groupe partant,
avec une amine de formule générale

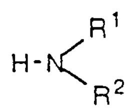

XI

dans laquelle $R^1$ et $R^2$ ont la signification indiquée dans la revendication 1 ou
d) à partir d'un composé de formule générale

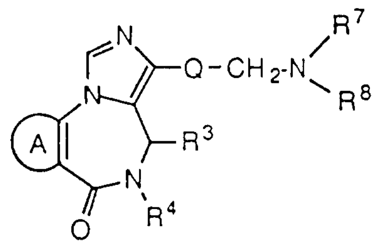

XII

dans laquelle A, Q, $R^3$ et $R^4$ ont la signification indiquée dans la revendication 1 et $R^7$ représente un groupe protecteur, un hydroxyalkyle en $C_1$ à $C_7$ protégé, un amino-alkyle en $C_1$ à $C_7$ protégé ou un alkylamino en $C_1$ à $C_7$-alkyle en $C_1$ à $C_7$ protégé et $R^8$ représente un hydrogène, un alkyle en $C_1$ à $C_7$, un alcényle en $C_1$ à $C_7$, un alcynyle en $C_1$ à $C_7$, un hydroxyalkyle en $C_1$ à $C_7$ protégé, un alcoxy en $C_1$ à $C_7$-alkyle en $C_1$ à $C_7$, un cycloalkyle en $C_3$ à $C_6$, un cycloalkyle en $C_3$ à $C_6$-alkyle en $C_1$ à $C_7$, ou un amino-alkyle en $C_1$ à $C_7$ protégé, un alkylamino en $C_1$ à $C_7$-alkyle en $C_1$ à $C_7$ protégé un dialkylamino en $C_1$ à $C_7$-alkyle en $C_1$ à $C_7$ ou un arylalkyle en $C_1$ à $C_7$ protégé, ou $R^7$ et $R^8$ représentent ensemble un groupe protecteur,
on sépare le ou les groupes protecteurs, ou
e) on N-alkyle un composé de formule générale

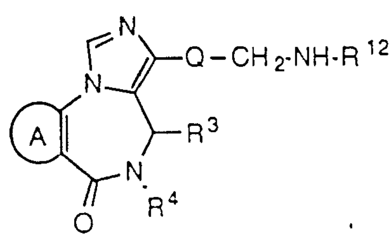

Ia

dans laquelle A, Q, $R^3$ et $R^4$ ont la signification indiquée dans la revendication 1 et $R^{12}$ représente un hydrogène, un alkyle en $C_1$ à $C_7$, un alcényle en $C_1$ à $C_7$-alcynyle en $C_1$ à $C_7$, un hydroxyalkyle en $C_1$ à $C_7$, un alcoxy en $C_1$ à $C_7$-alkyle en $C_1$ à $C_7$, un cycloalkyle en $C_3$ à $C_6$, un cycloalkyle en $C_3$ à $C_6$-alkyle en $C_1$ à $C_7$, un amino-alkyle en $C_1$ à $C_7$, un alkylamino en $C_1$ à $C_7$-alkyle en $C_1$ à $C_7$, un dialkylamino en $C_1$ à $C_7$-alkyle en $C_1$ à $C_7$ ou un arylalkyle en $C_1$ à $C_7$, ou

f) on réduit un composé de formule générale

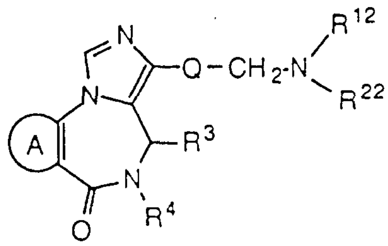

Ib

dans laquelle A, Q, $R^3$ et $R^4$ ont la signification indiquée dans la revendication 1 et $R^{12}$ a la signification indiquée ci-dessus et $R_{22}$ représente un alcényle en $C_1$ à $C_7$ ou un alcynyle en $C_1$ à $C_7$,
et si on le désire,
g) on transforme le composé de formule générale (I) en un sel d'addition d'acide pharmaceutiquement acceptable.

**27.** Composés selon l'une des revendications 1 à 24 aux fins d'application comme substances actives thérapeutiques, en particulier comme substances actives anxiolytiques et/ou anticonvulsives et/ou de relaxation musculaire et/ou sédative-hypnotique.

**28.** Utilisation de composés selon l'une des revendications 1 à 24, pour fabriquer des agents anxiolytiques et/ou anticonvulsifs et/ou de relaxation musculaire et/ou sédatifs-hypnotiques.